# FASCICULE DE BREVET EUROPEEN

(11) **EP 2 829 539 B1**
(45) Date de publication et mention de la délivrance du brevet: **21.12.2016**
(21) Numéro de dépôt: 14178016.3
(22) Date de dépôt: 22.07.2014
(51) Int. Cl.: C07D 401/14, C07D 405/14, A61K 31/4709, C07D 401/10, C07D 471/04, A61P 35/00, A61P 37/00, C07D 491/10, C07D 498/04

(54) **Nouveaux derives de pyrrole, leur procede de preparation et les compositions pharmaceutiques qui les contiennent**
Neue Pyrrolderivate, ihr Herstellungsverfahren und pharmazeutische Zusammensetzungen, die sie enthalten
New pyrrole derivatives, method of preparing same and pharmaceutical compositions containing them

(30) Priorité: 23.07.2013 FR 1357258
(43) Date de publication de la demande: 28.01.2015
(73) Titulaire: Les Laboratoires Servier, 92284 Suresnes Cedex (FR); Vernalis (R&D) Ltd., Berkshire RG41 5RD (GB)
(72) Inventeur: Le Tiran, Arnaud, 78290 CROISSY SUR SEINE (FR); Le Diguarher, Thierry, 45550 Saint Denis de l'Hôtel (FR); Starck, Jérôme-Benoît, 92500 RUEIL-MALMAISON (FR); Henlin, Jean-Michel, 92150 SURESNES (FR); Guillouzic, Anne-Françoise, 92000 NANTERRE (FR); De Nanteuil, Guillaume, 92150 SURESNES (FR); Geneste, Olivier, 92500 RUEIL MALMAISON (FR); Fejes, Imre, 1012 BUDAPEST (HU); Tatai, Janos, 1041 BUDAPEST (HU); Nyerges, Miklos, 2016 LEANYFALU (HU); Davidson, James Edward Paul, CAMBRIDGE, CB22 5DJ (GB); Murray, James Brooke, LINTON, Cambridgeshire CB21 4YL (GB); Chen, I-Jen, CAMBRIDGE, CB1 3NY (GB); Durand, Didier, 78240 Chambourcy (FR)

(56) Documents cités:
- WO-A1-2012/162365
- WO-A1-2013/110890
- WO-A2-2006/023778

## Description

La présente invention concerne de nouveaux dérivés de pyrrole, leur procédé de préparation et les compositions pharmaceutiques qui les contiennent.

Les composés de la présente invention sont nouveaux et présentent des caractéristiques pharmacologiques très intéressantes dans le domaine de l'apoptose et de la cancérologie.

L'apoptose, ou mort cellulaire programmée, est un processus physiologique crucial pour le développement embryonnaire et le maintien de l'homéostasie tissulaire.
La mort cellulaire de type apoptotique fait intervenir des changements morphologiques, tels que la condensation du noyau, la fragmentation de l'ADN, ainsi que des phénomènes biochimiques, tels que l'activation des caspases qui vont dégrader des composants structuraux clés de la cellule pour induire son désassemblage et sa mort. La régulation du processus d'apoptose est complexe et implique l'activation ou la répression de plusieurs voies de signalisation intracellulaire (Cory S. et al., Nature Review Cancer, 2002, 2, 647-656).
Des dérégulations de l'apoptose sont impliquées dans certaines pathologies. Une apoptose accrue est liée aux maladies neurodégénératives telles que la maladie de Parkinson, la maladie d'Alzheimer et l'ischémie. Inversement, des déficiences dans l'exécution de l'apoptose jouent un rôle important dans le développement des cancers et leur chimiorésistance, des maladies auto-immunes, des maladies inflammatoires et des infections virales. Ainsi, l'échappement à l'apoptose fait partie des signatures phénotypiques du cancer (Hanahan D. et al., Cell 2000, 100, 57-70).

Les protéines anti-apoptotiques de la famille Bcl-2 sont associées à de nombreuses pathologies. L'implication des protéines de la famille Bcl-2 est décrite dans de nombreux types de cancer, tel que le cancer colorectal, le cancer du sein, le cancer du poumon à petites cellules, le cancer du poumon non à petites cellules, le cancer de la vessie, le cancer de l'ovaire, le cancer de la prostate, la leucémie lymphoïde chronique, le lymphome folliculaire, le myélome... La surexpression des protéines anti-apoptotiques de la famille Bcl-2 est impliquée dans la tumorogenèse, dans la résistance à la chimiothérapie et dans le pronostique clinique des patients atteints de cancer. Il existe donc un besoin thérapeutique de composés inhibant l'activité anti-apoptotique des protéines de la famille Bcl-2.

Parmi les inhibiteurs de Bcl-2 déjà connus dans la littérature , on distingue les dérivés de 2-(1 ,2,3 ,4-tétrahydroisoquinoline-2-carbonyl)-4-(sulfonylcarbamoyl)phényle décrits clans WO2012/162365, les dérivés de 1-(1,2,3,4-tétrahydroisoquinoline-2-carbonyl)-2,3,4- trihydroxyphényle décrits dans WO2006/023778 et les dérivés de 3-[2-(1,2,3,4- tétrahydroisoquinoline-2-carbonyl)phényl]indolizine décrits dans WO2013/110890. Tous présentent un intérêt potentiel dans le traitement du cancer.

Les composés de la présente invention outre leur nouveauté, présentent des propriétés pro-apoptotiques permettant de les utiliser dans les pathologies impliquant un défaut d'apoptose, comme par exemple dans le traitement du cancer, des maladies auto-immunes et du système immunitaire.

La présente invention concerne plus particulièrement les composés de formule (I) : 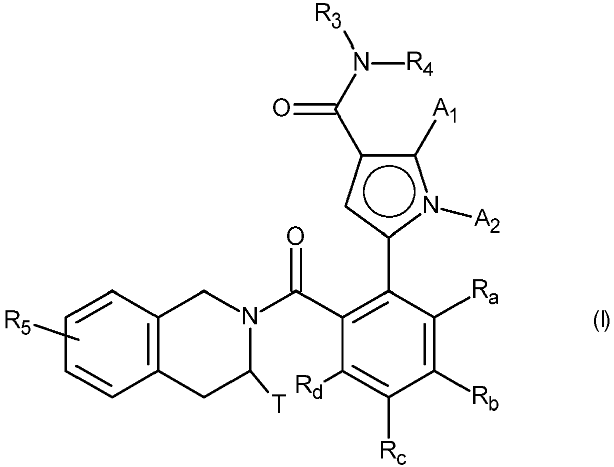 dans laquelle :
◆ A₁ représente un atome d'hydrogène ou d'halogène, un polyhalogénoalkyle (C₁-C₆) linéaire ou ramifié, un groupement alkyle (C₁-C₆) linéaire ou ramifié ou un groupement cycloalkyle,
◆ A₂ représente un groupement alkyle (C₁-C₆) linéaire ou ramifié éventuellement substitué par un groupement choisi parmi halogène, hydroxy, alkoxy (C₁-C₆) linéaire ou ramifié, NR'R" et morpholine, ou bien A₂ représente un polyhalogénoalkyle (C₁-C₆) linéaire ou ramifié ou un groupement cyclopropyle, étant entendu que R' et R" représentent, indépendamment l'un de l'autre, un atome d'hydrogène ou un groupement alkyle (C₁-C₆) linéaire ou ramifié,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
   ou bien R₁ et R₂ forment avec l'atome d'azote qui les porte un hétérocycloalkyle,
◆ R₃ représente un groupement aryle ou hétéroaryle, étant entendu qu'un ou plusieurs atomes de carbone des groupements précédents, ou de leurs éventuels substituants, peut(vent) être deutéré(s),
◆ R₄ représente un groupement phényle, 4-hydroxyphényle, 3-fluoro-4-hydroxyphényle, 2-hydroxypyrimidine ou 3-hydroxypyridine, étant entendu qu'un ou plusieurs atomes de carbone des groupements précédents, ou de leurs éventuels substituants, peut(vent) être deutéré(s),
◆ R₅ représente un atome d'hydrogène ou d'halogène, un groupement alkyle (C₁-C₆) linéaire ou ramifié, ou un groupement alkoxy (C₁-C₆) linéaire ou ramifié,
◆ R₆ représente un atome d'hydrogène ou un groupement alkyle (C₁-C₆) linéaire ou ramifié,
◆ Rₐ et R_{d} représentent chacun un atome d'hydrogène et (R_{b},R_{c}) forment ensemble avec les atomes de carbone qui les portent un groupe 1,3-dioxolane, un groupe 1,4-dioxane, ou bien Rₐ, R_{c} et R_{d} représentent chacun un atome d'hydrogène et R_{b} représente un atome d'hydrogène ou d'halogène ou un groupement méthoxy, ou bien Rₐ et R_{d} représentent chacun un atome d'hydrogène, R_{b} représente un atome d'hydrogène ou d'halogène et R_{c} un groupement hydroxy ou méthoxy, ou bien : Rₐ et R_{d} représentent chacun un atome d'hydrogène, R_{b} représente un groupement hydroxy ou méthoxy et R_{c} un atome d'halogène,
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié, spiro (C₃-C₆), alkoxy (C₁-C₆) linéaire ou ramifié, (C₁-C₆)alkyl-S-, hydroxy, oxo (ou N-oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle, hétéroaryle, aryloxy, arylthio, cycloalkyle, hétérocycloalkyle éventuellement substitué par un ou plusieurs atomes d'halogène ou groupements alkyles, ,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la *tert*butylamine, etc...

De manière avantageuse, A₁ représente un atome d'hydrogène ou un groupement méthyle.

Plus préférentiellement encore, A₁ et A₂ représentent tous les deux un groupement méthyle.

Dans un mode de réalisation préféré de l'invention, T représente un alkyle (C₁-C₆) linéaire ou ramifié. Dans un autre mode de réalisation préféré, T représente un groupement alkyl(C₁-C₄)-NR₁R₂, et plus particulièrement un groupement alkyl(C₁-C₄)-NR₁R₂ dans lequel R₁ et R₂ forment avec l'atome d'azote qui les porte un hétérocycloalkyle.
Dans les composés préférés de l'invention, T représente un méthyle, un groupement aminométhyle, (morpholin-4-yl)méthyle, (4-méthylpipérazin-1-yl)méthyle, 2-(morpholin-4-yl)éthyle, [2-(morpholin-4-yl)éthoxy]méthyle, hydroxyméthyle, [2-(diméthylamino) éthoxy]méthyle, hexahydropyrazino[2,1-*c*][1,4]oxazin-8(1*H*)-ylméthyle, 1-oxa-6-azaspiro[3.3]hept-6-ylméthyle, 3-(morpholin-4-yl)propyle, ou trifluorométhyle. Plus préférentiellement encore, T représente un groupement (morpholin-4-yl)méthyle, méthyle ou 3-(morpholin-4-yl)propyle.

Plus préférentiellement encore, Rₐ et R_{d} représentent chacun un atome d'hydrogène et (R_{b},R_{c}) forment ensemble avec les atomes de carbone qui les portent un groupe 1,3-dioxolane, ou bien Rₐ, R_{c} et R_{d} représentent chacun un atome d'hydrogène et R_{b} représente un halogène, préférentiellement un atome de chlore ou de fluor.

Plus préférentiellement encore, R₄ représente un groupement 4-hydroxyphényle.

Dans les composés préférés de l'invention, R₃ représente préférentiellement un groupement choisi parmi phényle, 1*H-*yrazole, 1*H*-indole, 1*H*-indazole, pyridine, pyrimidine, 1*H*-pyrrolo[2,3-*b*]pyridine, 2,3-dihydro-1*H*-pyrrolo[2,3-*b*]pyridine, 1*H-*benzimidazole, 1*H*-pyrrole, 1*H*-pyrrolo[2,3-*c*]pyridine, 1*H*-pyrrolo[3,2-*b*]pyridine, 5*H-*pyrrolo[3,2-*d*]pyrimidine, thiophène, pyrazine, 1*H*-pyrazolo[3,4-*b*]pyridine, 1,2-oxazole, 1*H*-pyrazolo[1,5-*a*]pyrimidine, ces groupements comportant éventuellement un ou plusieurs substituants choisis parmi halogène, alkyle (C₁-C₆) linéaire ou ramifié, alkoxy (C₁-C₆) linéaire ou ramifié, cyano, cyclopropyle, oxétane, tétrahydrofurane, -CO-O-CH₃, trideutériométhyle, 2-(morpholin-4-yl)éthyle, ou 2-(morpholin-4-yl)éthoxy. Plus préférentiellement, R₃ représente un groupement 1-méthyl-1*H*-pyrazol-4-yl, pyridin-4-yl, 1-méthyl-1*H*-pyrrolo[2,3-*b*]pyridin-5-yl, 5-cyano-1-méthyl-1*H*-pyrrol-3-yl, 5-cyano-1,2-diméthyl-1*H*-pyrrol-3-yl, 1-méthyl-2,3-dihydro-1*H-*pyrrolo[2,3-*b*]pyridin-5-yl, or 5-cyano-2-méthyl-1-(trideutériométhyl)-1*H*-pyrrol-3-yl.

Les composés préférés selon l'invention sont compris dans le groupe suivant :
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide,
- 5-(5-chloro-2-{[(3*R*)-3-méthyl-3,4-dihydroisoquinolin-2(1*H*)-yl]carbonyl}phényl)-*N*-(4-hydroxyphényl)-1,2-diméthyl-*N*-(pyridin-4-yl)-1*H*-pyrrole-3-carboxamide,
- *N*-(4-hydroxyphényl)-1,2-diméthyl-*N*-(1-méthyl-1*H*-pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide,
- *N*-(4-hydroxyphényl)-1,2-diméthyl-5-(6-{[(3*R*)-3-méthyl-3,4-dihydroisoquinolin-2(1*H*)-yl]carbonyl}-1,3-benzodioxol-5-yl)-*N*-(pyridin-4-yl)-1*H*-pyrrole-3-carboxamide,
- 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H-*pyrrolo[2,3-*b*]pyridin-5-yl)-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(pyridin-4-yl)-1*H-*pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(5-cyano-1-méthyl-1*H*-pyrrol-3-yl)-*N*-(4-hydroxyphényl)-1,2-diméthyl-1*H*-pyrrole-3-carboxamide,
- *N*-(5-cyano-1-méthyl-1*H*-pyrrol-3-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide,
- *N*-(5-cyano-1,2-diméthyl-1*H*-pyrrol-3-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-[5-cyano-2-méthyl-1-(trideutériométhyl)-1*H*-pyrrol-3-yl]-*N*-(4-hydroxyphényl)-1,2-diméthyl-1*H*-pyrrole-3-carboxamide,
leurs énantiomères et diastéréoisomères, ainsi que leurs sels d'addition à un acide ou à une base pharmaceutiquement acceptable.

L'invention s'étend également au procédé de préparation des composés de formule (I) caractérisé en ce que l'on utilise comme produit de départ le composé de formule (II) : 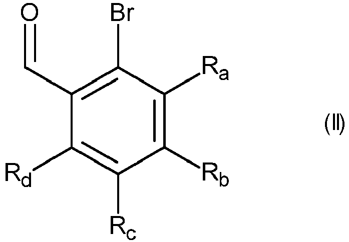 dans laquelle Rₐ, R_{b}, R_{c} et R_{d} sont tels que définis dans la formule (I),
composé de formule (II) qui est soumis à une réaction de Heck, en milieu aqueux ou organique, en présence d'un catalyseur au palladium, d'une base, d'une phosphine et du composé de formule (III) : 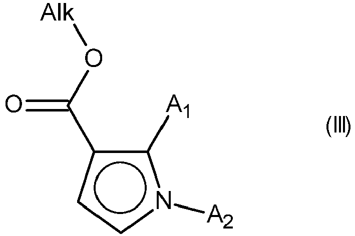 dans laquelle les groupements A₁ et A₂ sont tels que définis dans la formule (I) et Alk représente un alkyle (C₁-C₆) linéaire ou ramifié,
pour obtenir le composé de formule (IV) : 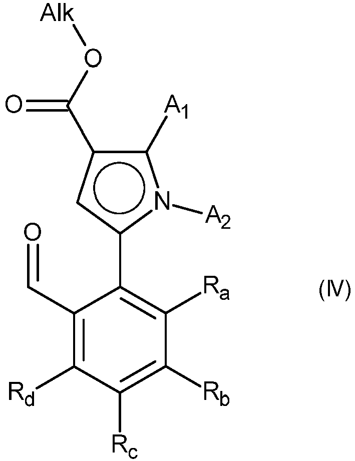 dans laquelle A₁, A₂, Rₐ, R_{b}, R_{c} et R_{d} sont tels que définis dans la formule (I) et Alk est tel que défini précédemment,
composé de formule (IV) dont la fonction aldéhyde est oxydée en acide carboxylique pour former le composé de formule (V) : 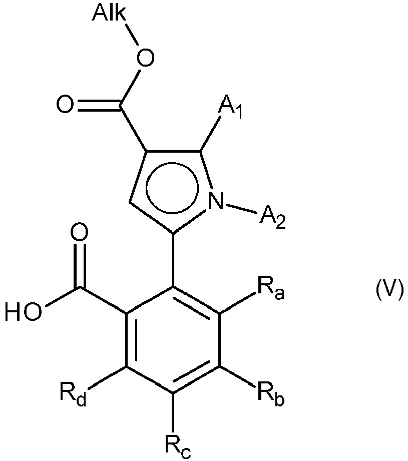 dans laquelle A₁, A₂, Rₐ, R_{b}, R_{c} et R_{d} sont tels que définis dans la formule (I) et Alk est tel que défini précédemment,
composé de formule (V) qui subit ensuite un couplage peptidique avec un composé de formule (VI) : 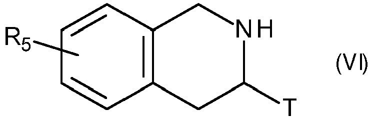 dans laquelle T et R₅ sont tels que définis dans la formule (I),
pour conduire au composé de formule (VII) : 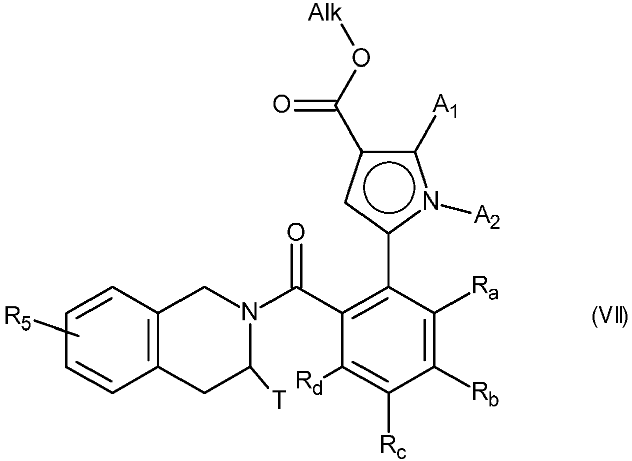 dans laquelle A₁, A₂, Rₐ, R_{b}, R_{c}, R_{d}, T et R₅ sont tels que définis dans la formule (I) et Alk est tel que défini précédemment,
composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

Plus particulièrement, lorsque l'un des groupements R₃ ou R₄ de l'amine NHR₃R₄ est substitué par une fonction hydroxy, cette dernière peut être préalablement soumise à une réaction de protection avant tout couplage avec l'acide carboxylique du composé de formule (VII), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

Les composés de formules (II), (III), (II'), (IV'), (VI), ainsi que l'amine NHR₃R₄, sont soit commerciaux, soit accessibles à l'homme du métier par des réactions chimiques classiques et décrites dans la littérature.

L'étude pharmacologique des dérivés de l'invention a montré qu'ils possédaient des propriétés pro-apoptotiques. La capacité à réactiver le processus apoptotique dans les cellules cancéreuses représente un intérêt thérapeutique majeur dans le traitement des cancers, des maladies auto-immunes et du système immunitaire.

Plus particulièrement, les composés selon l'invention seront utiles dans le traitement des cancers chimio ou radiorésistants, ainsi que dans les hémopathies malignes et le cancer du poumon à petites cellules.
Parmi les traitements des cancers envisagés on peut citer, sans s'y limiter, le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules. Parmi les lymphomes non hodgkiniens, on peut citer plus préférentiellement les lymphomes folliculaires, les lymphomes des cellules du manteau, les lymphomes diffus à grandes cellules B, les petits lymphomes lymphocytiques et les lymphomes à cellules B de la zone marginale.

La présente invention a également pour objet les compositions pharmaceutiques contenant au moins un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Par ailleurs, la présente invention concerne également l'association d'un composé de formule (I) avec un agent anticancéreux choisi parmi les agents génotoxiques, les poisons mitotiques, les anti-métabolites, les inhibiteurs du protéasome, les inhibiteurs de kinase, ou les anticorps, ainsi que les compositions pharmaceutiques contenant ce type d'association et leur utilisation pour la fabrication de médicaments utiles dans le traitement du cancer.

Les composés de l'invention peuvent également être utilisés en association avec une radiothérapie dans le traitement du cancer.

Enfin, les composés de l'invention peuvent être liés à des anticorps monoclonaux ou à des fragments de ceux-ci ou peuvent être liés à des protéines de charpente qui peuvent être apparentées, ou non, à des anticorps monoclonaux.
Par fragments d'anticorps, on doit entendre des fragments du type Fv, scFv, Fab, F(ab')2, F(ab'), scFv-Fc, ou des diabodies, qui en général ont la même spécificité de liaison que l'anticorps duquel ils sont issus. Selon la présente invention, les fragments d'anticorps de l'invention peuvent être obtenus à partir d'anticorps par des méthodes telles que digestion par enzymes telles que la pepsine ou la papaïne, et/ou par clivage des ponts disulfures par réduction chimique. D'autre manière, les fragments d'anticorps compris dans la présente invention peuvent être obtenus par des techniques de recombinaison génétique également bien connues à l'homme du métier ou bien par synthèse de peptides au moyen de synthétiseurs automatiques de peptides par exemple, tels que ceux fournis par la société Applied Biosystems etc...
Par protéines de charpente qui peuvent être apparentées, ou non, à des anticorps monoclonaux, on entend une protéine qui comprend, ou non, un repliement d'immunoglobuline et qui livre une capacité de liaison similaire à celle d'un anticorps monoclonal. L'homme du métier sait comment sélectionner la protéine de charpente. Plus particulièrement, il est connu que, pour être sélectionné, une telle charpente devrait présenter plusieurs caractéristiques comme suivant (Skerra A., J. Mol. Recogn. 13, 2000, 167-187): une bonne conservation phylogénétique, une architecture robuste avec une organisation moléculaire en trois dimensions bien connue (telle que la cristallographie ou la RMN, par exemple), une petite taille, aucune ou peu de modification(s) post-traductionnelle(s), la facilité de production, d'expression et de purification. Une telle protéine de charpente peut être, sans s'y limiter, une structure choisie parmi le groupe consistant en la fibronectine et de préférence le dixième domaine type III de la fibronectine (FNfn10), la lipocaline, l'anticaline (Skerra A., J. Biotechnol., 2001, 74(4):257-75), la protéine Z dérivée du domaine B de la protéine A des staphylocoques, la thiorédoxine A ou une protéine quelconque avec un domaine répété tel qu'une "répétition ankyrine" (Kohl et al., PNAS, 2003, vol.100, no.4, 1700-1705), une "répétition armadillo", une "répétition riche en leucine" ou une "répétition tétratricopeptide". On pourrait aussi mentionner une charpente dérivée de toxines (telles que des toxines de scorpions, d'insectes, de plantes ou des mollusques, par exemple) ou des protéines inhibitrices de l'oxyde nitrique synthase (PIN).

Les Préparations et Exemples suivants illustrent l'invention et ne la limitent en aucune façon.

### Préparation 1: Acide 4-chloro-2-[4-(éthoxycarbonyl)-1,5-diméthyl-1H-pyrrol-2-yl]benzoïque

### Stade A : 1,2-Diméthyl-1H-pyrrole-3-carboxylate d'éthyle

A une solution de 10 g de 2-méthyl-1*H*-pyrrole-3-carboxylate d'éthyle (65,3 mmol) et 8,95 mL (130,6 mmol) d'iodure de méthyle dans 70 mL de diméthylformamide placée à 0°C, on ajoute en trois portions 2,61 g (65,3 mmol) d'hydure de sodium (NaH) à 60% . L'ensemble est ensuite agité à 0°C pendant 1 heure. Puis, le milieu réactionnel est hydrolysé par l'addition de 420 mL d'eau glacée. Le milieu réactionnel est ensuite dilué avec de l'acétate d'éthyle, lavé successivement avec une solution aqueuse d'acide chlorhydrique (HCl) 0,1 M, une solution aqueuse saturée en LiCl, puis de la saumure. La phase organique est alors séchée sur MgSO₄, filtrée, concentrée à sec, et purifiée par chromatographie sur gel de silice (gradient éther de pétrole/ AcOEt).
**RMN ¹H** : δ (400 MHz ; dmso-d6 ; 300K) : 6.65 (d, 1H pyrrole) ; 6.3 (1d, 1H pyrrole) ; 4.1 (1q, 2H, O**CH₂**CH₃); 3.5 (s, 3H N-pyrrole); 2.4 (s, 3H pyrrole) ; 1.5 (1t, 3H OCH₂**CH**₃) **IR :** v : >C=O: 1688 cm⁻¹ ; v : C-O-C: 1172 cm⁻¹

### Stade B : 5-(5-Chloro-2-formylphényl)-1,2-diméthyl-1H-pyrrole-3-carboxylate d'éthyle

A une solution de 10,5 g du composé obtenu au Stade A (62,8 mmol) dans 65 mL de *N,N-*diméthylacétamide, on ajoute successivement 15,2 g de 2-bromo-4-chlorobenzaldéhyde (69 mmol), 12,3 g d'acétate de potassium (125,6 mmol), puis l'ensemble est agité sous argon pendant 20 minutes. On ajoute alors 2,2 g de catalyseur au palladium PdCl₂(PPh₃)₂ (3,14 mmol). Le milieu réactionnel est ensuite chauffé à 130°C pendant la nuit. On laisse le milieu revenir à température ambiante, puis on le dilue avec du dichlorométhane. On ajoute du noir animal (2 g par g de produit) et l'ensemble est agité à température ambiante pendant 1 heure, puis filtré. La phase organique est alors lavée avec de l'eau, séchée sur MgSO₄ et concentrée à sec. Le produit brut ainsi obtenu est purifié par chromatographie sur gel de silice (gradient éther de pétrole/ AcOEt). Le produit du titre est obtenu sous la forme d'un solide.
**RMN ¹H** : δ (400 MHz ; dmso-d6 ; 300K) : 9.8 (s, 1H, formyle) ; 7,91-7,69-7,61 (d, 3H aromatiques); 6,5 (s, 1H pyrrole); 4,2 (q, 2H, O**CH**₂CH₃ ); 3,4 (s, 3H, **CH**₃-N-pyrrole) ; 2,55 (s, 3H pyrrole) ; 1,28 (t, 3H, OCH₂**CH**₃)

### Stade C : Acide 4-chloro-2-[4-(éthoxycarbonyl)-1,5-diméthyl-1H-pyrrol-2-yl]benzoïque

On prépare une solution contenant 12,85 g du composé obtenu au Stade B (42 mmol) et 35,7 mL (336 mmol) de 2-méthyl-2-butène dans un mélange constitué de 20 mL d'acétone et 20 mL de tétrahydrofurane. On ajoute, goutte à goutte, 200 mL d'une solution aqueuse contenant un mélange de 13,3 g de chlorite de sodium (NaClO₂) (147 mmol) et de 14,5 g d'hydrogénophosphate de sodium (NaHPO₄)(105 mmol). L'ensemble est ensuite agité énergiquement à température ambiante durant 7 heures. Puis, le milieu réactionnel est concentré pour éliminer l'acétone. On ajoute de l'acétate d'éthyle et on lave à l'eau la phase organique, la sèche sur MgSO₄, puis on concentre à sec. Le résidu est ensuite repris dans un minimum d'éther éthylique. Le solide alors obtenu est filtré, lavé à l'éther puis séché sous vide à 40°C pendant une nuit. Le produit du titre est obtenu sous la forme d'un solide, qui est utilisé pour la suite sans autre purification.
**RMN¹H:** δ (400 MHz ; dmso-d6 ; 300K) : 13 (m, 1H COO**H**), ; 7.85-7.6-7.41(d,dd,df, 3H, H aromatiques) ; 6.3 (s, 1H, H pyrrole) ; 4.15 (q, 2H, O**CH₂**CH₃) ; 3.25 (s, 3H, **CH₃**-N-pyrrole); 2.5 (s, 3H, **CH₃**-pyrrole) ; 1.25 (t, 3H, OCH₂**CH₃**)
**IR :** v : -OH : 3100-2500 cm⁻¹ acide ; v : >C=O : 1681 cm⁻¹ ester + acide

### Préparation 2: Acide 2-[4-(éthoxycarbonyl)-1-méthyl-1H-pyrrol-2-yl]benzoïque

On procède selon le procédé de la Préparation 1 en remplaçant d'une part le 2-méthyl-1*H-*pyrrole-3-carboxylate d'éthyle utilisé au Stade A par le 1*H*-pyrrole-3-carboxylate d'éthyle, et d'autre part le 2-bromo-4-chlorobenzaldéhyde utilisé au Stade B par le 2-bromo-benzaldéhyde.

### Préparation 3 : Acide 4-chloro-2-[4-(éthoxycarbonyl)-1-méthyl-1H-pyrrol-2-yl]benzoïque

On procède selon le procédé de la Préparation 1 en remplaçant le 2-méthyl-1*H*-pyrrole-3-carboxylate d'éthyle utilisé au Stade A par le 1*H*-pyrrole-3-carboxylate d'éthyle.

### Préparation 4 : Acide 6-[4-(éthoxycarbonyl)-1-méthyl-1H-pyrrol-2-yl]-1,3-benzodioxole-5-carboxylique

On procède selon le procédé de la Préparation 1 en remplaçant d'une part le 2-méthyl-1*H-*pyrrole-3-carboxylate d'éthyle utilisé au Stade A par le 1*H*-pyrrole-3-carboxylate d'éthyle, et d'autre part le 2-bromo-4-chlorobenzaldéhyde utilisé au Stade B par le 6-bromo-1,3-benzodioxole-5-carbaldéhyde.
**IR :** v : -OH : 3500-2300 cm⁻¹ acide ; v : >C=O : 1688-1670 cm⁻¹ ester + acide

### Préparation 5 : Acide 4-chloro-2-[4-(éthoxycarbonyl)-1-éthyl-1H-pyrrol-2-yl]benzoïque

On procède selon le procédé de la Préparation 1 en remplaçant au Stade A d'une part le 2-méthyl-1*H*-pyrrole-3-carboxylate d'éthyle par le 1*H*-pyrrole-3-carboxylate d'éthyle, d'autre part l'iodure de méthyle par l'iodure d'éthyle (voir le protocole décrit dans US 6,258,805 B1).

### Préparation 6 : Acide 4-chloro-2-[1-cyclopropyl-4-(éthoxycarbouyl)-1H-pyrrol-2-yl]benzoïque

On procède selon le procédé de la Préparation 1 en remplaçant au Stade A d'une part le 2-méthyl-1*H*-pyrrole-3-carboxylate d'éthyle par le 1*H*-pyrrole-3-carboxylate d'éthyle, d'autre part l'iodure de méthyle par l'acide cyclopropylboronique (voir le protocole décrit dans Bénard S. et al, Journal of Organic Chemistry 73(16), 6441-6444, 2008).

### Préparation 7 : Acide 4-chloro-2-[4-(éthoxycarbonyl)-1-(propan-2-yl)-1H-pyrrol-2-yl]benzoïque

On procède selon le procédé de la Préparation 1 en remplaçant au Stade A d'une part le 2-méthyl-1*H*-pyrrole-3-carboxylate d'éthyle par le 1*H*-pyrrole-3-carboxylate d'éthyle, d'autre part l'iodure de méthyle par l'iodure d'isopropyle (voir le protocole décrit dans Okada E. et al, Heterocycles 34(7), 1435-1441, 1992).

### Préparation 8 : Acide 4-fluoro-2-[4-(méthoxycarbonyl)-1,5-diméthyl-1H-pyrrol-2-yl]benzoïque

On procède selon le procédé de la Préparation 1 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 2-bromo-4-fluorobenzaldéhyde.
**IR :** v : -OH : 2727-2379 cm⁻¹ acide ; v : >C=O_{:} 1687 cm⁻¹

### Préparation 9 : Acide 6-{1-[2-(benzyloxy)éthyl]-4-(éthoxycarbonyl)-5-méthyl-1H-pyrrol-2-yl}-1,3-benzodioxole-5-carboxylique

### Stade A : 1-[2-(benzyloxy)éthyl]-2-méthyl-1H-pyrrole-3-carboxylate d'éthyle

On procède selon le procédé du Stade A de la Préparation 1 en remplaçant l'iodure de méthyle utilisé comme agent alkylant par le 2-bromoéthyl éther de benzyle.
**RMN ¹H** : δ (400 MHz ; dmso-d6 ; 300K) : 7.32 (t, 2H, H aromatiques, H *méta* benzyle éther) ; 7.3 (t, 1H, H aromatiques, H *para* benzyle éther) ; 7.23 (d, 2H, H aromatiques, H *ortho* benzyle éther) ; 6.72 (d, 1H, H- pyrrole) ; 6.35 (d, 1H, H- pyrrole) ; 4.48 (s, 2H, H aliphatiques, O-**CH₂**-Ph) ; 4.15 (q, 2H, H aliphatiques, O-**CH₂**-CH₃) ; 4.1 (t, 2H, H aliphatiques, **CH₂**-O-CH₂-Ph) ; 3.7 (t, 2H, H aliphatiques, **CH₂**-CH₂-O-CH₂-Ph) ; ; 2.45 (s, 3H, **CH₃**- pyrrole) ; 1.25 (t, 3H, H aliphatiques, O-CH₂-**CH₃**)
**IR** : v : >C=O : 1689 cm⁻¹

### Stade B : Acide 6-{1-[2-(benzyloxy)éthyl]-4-(éthoxycarbonyl)-5-méthyl-1H-pyrrol-2-yl}-1,3-beuzodioxole-5-carboxylique

On procède selon les procédés des Stades B et C de la Préparation 1 en remplaçant le 2-bromo-4-chlorobenzaldéhyde par le 6-bromo-1,3-benzodioxole-5-carbaldéhyde.
**RMN ¹H** : δ (400 MHz ; dmso-d6 ; 300K) : 7.35 (s, 1H, H aromatique, H benzodioxole) ; 7.30 (m, 3H, H aromatiques, H benzyle éther) ; 7.25 (s, 1H, H aromatique, H benzodioxole) ; 7.10 (d, 2H, H aromatiques, H *ortho* benzyle éther) ; 12.55 (s large , 1H, COO**H**) ; 6.75 (s, 1H, H- pyrrole) ; 6.15 (s large, 2H, H aliphatiques, O-**CH₂**-O) ; 4.30 (s, 2H, H aliphatiques, O-**CH₂**-Ph) ; 4.15 (q, 2H, H aliphatiques, O-**CH₂**-CH₃) ; 3.9 (m, 2H, H aliphatiques, CH₂-**CH₂**-O-CH₂-Ph) ; 3.40 (t, 2H, H aliphatiques, **CH₂**-CH₂-O-CH₂-Ph) ; 2.50 (s, 3H, **CH₃**- pyrrole) ; 1.25 (t, 3H, H aliphatiques, O-CH₂-**CH₃**)
**IR :** v : -OH : 3200-2300 cm⁻¹ acide ; v : >C=O : 1687 cm⁻¹ acide

### Préparation 10 : Acide 2-{1-[3-(benzyloxy)propyl]-4-(éthoxycarbonyl)-5-méthyl-1H-pyrrol-2-yl}-4-fluorobenzoïque

On procède selon le procédé de la Préparation 9 en remplaçant le 2-bromoéthyl éther de benzyle utilisé au Stade A par le 3-bromopropyl éther de benzyle, ainsi que le 6-bromo-1,3-benzodioxole-5-carbaldéhyde utilisé au Stade B par le 2-bromo-4-fluorobenzaldéhyde.
**IR :** v : -OH : 3200-2305 cm⁻¹ acide ; v : >C=O : 1690 cm⁻¹ acide

### Préparation 11 : Acide 6-[4-(éthoxycarbonyl)-1,5-diméthyl-1H-pyrrol-2-yl]-1,3-benzodioxole-5-carboxylique

On procède selon le procédé de la Préparation 1 en remplaçant le 2-bromo-4-chlorobenzaldéhyde utilisé au Stade B par le 6-bromo-1,3-benzodioxole-5-carbaldéhyde.

### Préparation 12 : Acide 4-méthoxy-2-[4-(méthoxycarbonyl)-1,5-diméthyl-1H-pyrrol-2-yl]benzoïque

On procède selon le procédé de la Préparation 1 en remplaçant le 2-méthyl-1*H*-pyrrole-3-carboxylate d'éthyle utilisé au Stade A par le 2-méthyl-1*H*-pyrrole-3-carboxylate de méthyle, ainsi que le 2-bromo-4-chlorobenzaldéhyde utilisé au Stade B par le 2-bromo-4-méthoxybenzaldéhyde.
**IR :** v : -OH : 3000-2500 cm⁻¹ acide ; v : >C=O: 1693 + 1670 cm⁻¹ acide + ester

### Préparation 13 : Acide 7-[4-(méthoxycarbonyl)-1,5-diméthyl-1H-pyrrol-2-yl]-2,3-dihydro-1,4-benzodioxine-6-carboxylique

On procède selon le procédé de la Préparation 1 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 7-bromo-2,3-dihydro-1,4-benzodioxine-6-carbaldéhyde.
**IR :** v : -OH : 3100-2500 cm⁻¹ acide ; v : >C=O : 1690 + 1674 cm⁻¹ acide + ester

### Préparation 14 : Acide 4-fluoro-5-méthoxy-2-[4-(méthoxycarbonyl)-1,5-diméthyl.1H-pyrrol-2-yl]benzoïque

On procède selon le procédé de la Préparation 1 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 2-bromo-4-fluoro-5-méthoxybenzaldéhyde.

### Préparation 15: Acide 4-fluoro-5-bydroxy-2-[4-(méthoxycarbonyl)-1,5-diméthyl-1H-pyrrol-2-yl]benzoïque

On procède selon le procédé de la Préparation 1 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 2-bromo-4-fluoro-5-hydroxy-benzaldéhyde.

### Préparation 16: Acide 6-[4-(éthoxycarbonyl)-1-méthyl-5-trifluorométhyl-1H-pyrrol-2-yl]-1,3-benzodioxole-5-carboxylique

On procède selon le procédé de la Préparation 1 en remplaçant au Stade A le 2-méthyl-1*H-*pyrrole-3-carboxylate d'éthyle par le 2-(trifluorométhyl)-1*H*-pyrrole-3-carboxylate d'éthyle, ainsi que le 2-bromo-4-chlorobenzaldéhyde utilisé au Stade B par le 6-bromo-1,3-benzodioxole-5-carbaldéhyde.

### Préparation 17: Acide 6-[4-(éthoxycarbonyl)-1-éthyl-5-méthyl-1H-pyrrol-2-yl]-1,3-benzodioxole-5-carboxylique

On procède selon le procédé de la Préparation 11 en remplaçant l'iodure de méthyle par l'iodure d'éthyle (voir le protocole décrit dans US 6,258,805 B1).

### Préparation 18: Acide 6-[4-(éthoxycarbonyl)-1-méthyl-5-(trifluorométhyl)-1H-pyrrol-2-yl]-1,3-benzodioxole-5-carboxylique

### Stade A : 1-Méthyl-2-(trifluorométhyl)pyrrole-3-carboxylate d'éthyle

Du 4,4,4-trifluoro-3-oxo-butanoate d'éthyle (29 ml, 0,219 mmol) est ajouté dans de la méthylamine (solution à 40% dans l'eau) (50 ml, 0,580 mmol) refroidie à 10°C ; un précipité blanc se forme. De l'acétate de 1,2-dibromoéthyle (préparé selon Molecules, 16, 9368-9385; 2011) est additionné au goutte-à-goutte. Le réacteur est alors scellé et chauffé à 70°C pendant 45 minutes. Le milieu réactionnel est refroidi, puis extrait avec de l'acétate d'éthyle, séché sur sulfate de sodium (Na₂SO₄) et évaporé à sec. Le brut réactionnel obtenu est purifié par chromatographie sur gel de silice en utilisant l'heptane et l'acétate d'éthyle comme éluants. Le composé attendu est obtenu sous la forme de cristaux.
**RMN ¹H** (400 MHz, dmso-d6) δ ppm : 7.11 (d, 1 H), 6.52 (d, 1 H), 4.21 (quad, 2 H), 3.8 (s, 3 H), 1.27 (t, 3 H)
**RMN ¹⁹F** (400 MHz, dmso-d6) δ ppm :-53.9
**IR (ATR)** cm⁻¹: 3145 + 3128 v -CH, 1711 v >C=O, 1183 + 1117 + 1078 v -CF3

### Stade B : Acide 6-[4-(éthoxycarbonyl)-1-méthyl-5-(trifluorométhyl)-1H-pyrrol-2-yl]-1,3-benzodioxole-5-carboxylique

On procède selon le protocole décrit aux Stades B et C de la Préparation 1 en remplaçant le 2-bromo-4-chlorobenzaldéhyde utilisé au Stade B par le 6-bromo-1,3-benzodioxole-5-carbaldéhyde.

### Préparation 19 : Acide 6-[1-(2-benzyloxyéthyl)-4-éthoxycarbonyl-5-méthyl-1H-pyrrol-2-yl]-1,3-benzodioxole-5-carboxylique

### Stade A : 1-(2-Benzyloxyéthyl)-2-méthyl-pyrrole-3-carboxylate d'éthyle

Le 2-méthyl-1*H*-pyrrole-3-carboxylate d'éthyle (10 g, 65,3 mmol) est mis en solution dans 100 mL de diméthylformamide sous argon refroidi à 0°C, puis le 2-bromoéthoxyméthylbenzène (28,1 g, 130,6 mmol) est ajouté en une seule fois. Le mélange réactionnel est placé sous agitation. On y additionne ensuite à 0°C, en trois portions, le NaH (1,72g, 71,83 mmol) sur une durée de 15 minutes. Le mélange réactionnel est agité pendant 15 minutes à 0°C, puis 15h à température ambiante. Il est ensuite versé sur un bain de glace, puis extrait 3 fois avec de l'acétate d'éthyle. La phase organique est lavée 3 fois avec une solution aqueuse saturée en chlorure de lithium, séchée sur MgSO₄, filtrée puis évaporée à sec. Le résidu ainsi obtenu est purifié par chromatographie sur gel de silice en utilisant l'éther de pétrole et l'acétate d'éthyle comme éluants. On obtient le composé attendu sous la forme d'une huile.
**RMN ¹H** (400 MHz, dmso-d6) δ ppm : 7.32 (t, 2 H), 7.3 (t, 1 H), 7.23 (d, 2 H), 6.72 (d, 1 H), 6.35 (d, 1 H), 4.48 (s, 2 H), 4.15 (quad., 2 H), 4.1 (t, 2 H), 3.7 (t, 2 H), 2.45 (s, 3 H), 1.25 (t, 3 H)
**IR (ATR)** cm⁻¹ : 1689 v -C=O

### Stade B : 1-(2-Benzyloxyéthyl)-5-(6-formyl-1,3-benzodioxol-5-yl)-2-méthyl-pyrrole-3-carboxylate d'éthyle

On procède selon le procédé du Stade B de la Préparation 1 en remplaçant le 2-bromo-4-chlorobenzaldéhyde par le 6-bromo-1,3-benzodioxole-5-carbaldéhyde
**RMN 1H (400 MHz, dmso-d6) δ ppm:** 9.5 (s, 1 H), 7.3 (s, 1 H), 7.25 (m, 3 H), 7.05 (m, 2 H), 7 (s, 1 H), 6.4 (s, 1 H), 6.2 (sl, 2 H), 4.25 (s, 2 H), 4.2 (quad., 2 H), 4.05 (m, 2 H), 3.4 (m, 2 H), 2.55 (s, 3 H), 1.25 (t, 3 H)

### Stade C : Acide 6-[1-(2-benzyloxyéthyl)-4-éthoxycarbonyl-5-méthyl-1H-pyrrol-2-yl]-1,3-benzodioxole-5-carboxylique

On procède selon le procédé du Stade C de la Préparation 1.
**RMN ¹H** (400 MHz, dmso-d6) δ ppm: 12.55 (sl, 1 H), 7.35 (s, 1 H), 7.3 (m, 3 H), 7.2 (m, 3 H), 6.75 (s, 1 H), 6.15 (s, 2 H), 4.3 (s, 2 H), 4.15 (quad., 2 H), 3.9 (m, 2 H), 3.4 (t, 2 H), 2.5 (s, 3 H), 1.25 (t, 3 H)
**IR (ATR)** cm⁻¹ :3200-2300 v -OH, 1687 (+ épaulement) v-C=O acide carboxylique + ester conjugués

### Préparation 20: Acide 6-[4-(éthoxycarbonyl)-1-éthyl-5-méthy-1H-pyrrol-2-yl]-1,3-benzodioxole-5-carboxylique

On procède selon le protocole décrit à la Préparation 11 en remplaçant l'iodure de méthyle par l'iodure d'éthyle.
**RMN ¹H** (400 MHz, dmso-d6) δ ppm: 12.49 (sl, 1 H), 7.33 (s, 1 H), 6.89 (s, 1 H), 6.17 (s, 2 H), 6.13 (s, 1 H), 4.15 (quad, 2 H), 3.69 (quad, 2 H), 2.51 (s, 3 H), 1.24 (t, 3 H), 1.01 (t, 3 H)

### Préparation 21: Acide 6-[4-(éthoxycarbonyl,)-1-(2-fluoroéthyl)-5-méthyl-1H-pyrrol-2-yl]-1,3-benzodioxole-5-carboxylique

On procède selon le procédé de la Préparation 11 en remplaçant l'iodure de méthyle par le 1-bromo-2-fluoroéthane.
**RMN ¹H** (400 MHz, dmso-d6, 300K) δ ppm: 12.53 (sl, 1 H), 7.34 (s, 1 H), 6.9 (s, 1 H), 6.16 (s, 1 H), 6.16 (s, 2 H), 4.4 (dt, 2 H), 4.15 (quad, 2 H), 4.01 (m, 2 H), 2.51 (s, 3 H), 1.24 (t, 3 H)
**RMN ¹⁹F** (400 MHz, dmso-d6, 300 K) δ ppm: -222
**IR :** v : -OH : 3700-2400 cm⁻¹ acide ; v : >C=O_{:} 1689 cm⁻¹ acide ; v : >CF: 1213 cm⁻¹

### Préparation 22: Acide 6-[4-(éthoxycarbonyl)-1-méthyl-5-[2-(morpholin-4-yl)éthyl]-1H-pyrrol-2yl}-1,3-benzodioxole-5-carboxylique

### Stade A : Acide 3,3-diéthoxypropanoique

A une solution de 25 g de 3,3-diéthoxypropionate d'éthyle (131 mmol) dans 79 mL de méthanol sont additionnés 13,1 mL d'une solution aqueuse de soude à 35% (452 mmol). Le mélange réactionnel est agité à température ambiante durant 3 heures. Puis, le milieu réactionnel est concentré pour éliminer le méthanol. Après solubilisation de l'insoluble par addition d'eau, une solution aqueuse HCl 5N est ajoutée jusqu'à atteindre pH 5. On ajoute du dichlorométhane, puis la phase organique est lavée avec de la saumure. Après séchage sur MgSO₄ et concentration à sec, le produit du titre est obtenu sous forme d'une huile qui est utilisée au stade suivant sans autre purification.
**RMN ¹H** (400 MHz, dmso-d6) δ ppm : 12.2 (s, 1 H), 4.8 (t, 1 H), 3.58/3.47 (2m, 4 H), 2.5 (d, 2 H), 1.09 (t, 6 H)

### Stade B : 5,5-Diéthoxy-3-oxo-pentanoate d'éthyle

A une solution de 16 mL d'acide 3-éthoxy-3-oxomalonique (135 mmol) dans 40 mL de tétrahydrofurane sont ajoutés, sous argon, 21,9 g de magnésium en poudre (90,4 mmol). Le mélange obtenu est ensuite chauffé à 80°C pendant 7 heures. Après retour à la température ambiante, ce mélange est canulé sur une solution de 10 g du composé obtenu au Stade A (61,7 mmol) dans 64 mL de tétrahydrofurane à laquelle a été additionnée au préalable, par portions, 11 g de carbonyldiimidazole (66 mmol). Le milieu réactionnel est agité pendant 3 jours à température ambiante. Après concentration, le résidu est repris dans un mélange d'acétate d'éthyle et d'une solution aqueuse d'hydrogénosulfatede sodium (NaHSO₄)_{.} Le mélange est agité vigoureusement jusqu'à la fin du dégagement gazeux. Après décantation, la phase organique est lavée successivement avec de l'eau, une solution aqueuse saturée en NaHCO₃ et enfin de la saumure. Après séchage sur MgSO₄ et concentration à sec, le produit du titre est obtenu sous forme d'une huile qui est utilisée au stade suivant sans autre purification.
**RMN ¹H** (400 MHz, dmso-d6) δ ppm : 4.84 (t, 1 H), 4.08 (q, 2 H), 3.59 (s, 2 H), 3.56/3.46 (2m, 4 H), 2.8 (d, 2 H), 1.18 (t, 3 H), 1.09 (t, 6 H)

### Stade C : 2-(2,2-Diéthoxyéthyl)-1-méthyl-pyrrole-3-carboxylate d'éthyle

A une solution de 11,8 g du composé obtenu au Stade B (50,8 mmol) dans 76 mL d'eau sont additionnés, goutte à goutte à 0°C, 6,6 ml d'une solution aqueuse de méthylamine à 40% (76,2 mmol). Le milieu réactionnel est agité et réchauffé doucement jusqu'à la température ambiante pendant 5 heures. Après retour à 0°C, 8,8 mL d'une solution aqueuse de méthylamine à 40% (102 mmol), puis 16,6 mL d'une solution aqueuse de chloroacétaldéhyde à 50% (127 mmol) sont respectivement additionnés, goutte à goutte, à une température inférieure à 10°C. Le milieu réactionnel est agité à température ambiante pendant 16 heures, puis dilué dans l'acétate d'éthyle. La phase organique est lavée de la saumure, séchée sur MgSO₄ et concentrée à sec. Le produit brut ainsi obtenu est purifié par chromatographie sur gel de silice en utilisant l'éther de pétrole et l'acétate d'éthyle comme éluants. Le produit du titre est obtenu sous forme d'une huile.
**RMN ¹H** (400 MHz, dmso-d6) δ ppm : 6.7 (d, 1 H), 6.33 (d, 1 H), 4.55 (t, 1 H), 4.15 (q, 2 H), 3.6 (m, 2 H), 3.6 (s, 3 H), 3.3 (m, 2 H), 3.15 (d, 2 H), 1.25 (t, 3 H), 1.05 (t, 6 H)

### Stade D : 1-Méthyl-2-(2-morpholinoéthyl)pyrrole-3-carboxylate d'éthyle

A une solution de 3,8 g du composé obtenu au Stade C (14,05 mmol) dans 28 mL de tétrahydrofurane, sont additionnés 58 mL d'une solution aqueuse d'acide sulfurique 10%. Le milieu réactionnel est agité à température ambiante pendant 2 heures puis dilué dans un mélangé d'acétate d'éthyle et d'eau. Après séparation, la phase organique est lavée avec de la saumure, séchée sur MgSO₄ et concentrée à sec. A une solution du résidu ainsi obtenu dans 70 mL de dichloroéthane sont additionnés une solution de 13,5 mL de morpholine (14,5 mmol) dans un mélange constitué de 30 mL de dichloroéthane et de 3,6 mL d'une solution aqueuse de HCl 4N dans le dioxane (14,5 mmol), puis 7,4 g de triacétoxyborohydrure de sodium (NaBH(OAc)₃) (35,13 mmol). Le mélange réactionnel est agité à température ambiante pendant 3 heures puis dilué dans un mélange de dichlorométhane et d'une solution aqueuse saturée en NaHCO₃. Après séparation des phases et extraction de la phase aqueuse au dichlorométhane, les phases organiques sont séchées sur MgSO₄ et concentrées à sec. Le produit brut ainsi obtenu est purifié par chromatographie sur gel de silice en utilisnat le dichlorométhane et le méthanol comme éluants. Le produit du titre est isolé sous la forme d'une huile.
**RMN ¹H** (400 MHz, dmso-d6) δ ppm : 6.66 (d, 1 H), 6.31 (d, 1 H), 4.14 (q, 2 H), 3.58 (s, 3 H), 3.57 (m, 4 H), 3.05 (m, 2 H), 2.45-2.38 (m, 6 H), 1.24 (t, 3 H)

### Stade E : Acide 6-{4-(éthoxycarbonyl)-1-méthyl-5-[2-(morpholin-4-yl)éthyl]-1H-pyrrol-2-yl}-1,3-benzodioxole-5-carboxylique

On procède selon les Stades B et C de la Préparation 1 en remplaçant le 2-bromo-4-chlorobenzaldéhyde utilisé au Stade B par le 6-bromo-1,3-benzodioxole-5-carbaldéhyde.

### Préparation 23 : Acide 6-[4-(éthoxycarbonyl)-1-méthyl-5-(morpholin-4-ylméthyl)-1H-pyrrol-2-yl]-1,3-benzodioxole-5-carboxylique

### Stade A : 4,4-Diéthoxy-3-oxo-butanoate d'éthyle

A une solution de 40 g de diéthoxyacétate d'éthyle (227 mmol) dans 67 mL d'acétate d'éthyle, sont ajoutés sous argon, par portions, 6 g de sodium (261 mmol). Le milieu réactionnel est ensuite agité à température ambiante pendant 48 heures. On ajoute 10 mL de méthanol, puis le mélange est hydrolysé par 65 mL d'eau. Le pH du milieu réactionnel est ajusté à pH 6 par addition d'une solution aqueuse d'HCl 1N. Le mélange est décanté puis extrait avec de l'acétate d'éthyle. Les phases organiques sont réunies, lavées avec de la saumure, séchées sur MgSO₄, filtrées et concentrées à sec. Le produit du titre est obtenu sous forme d'une huile qui est utilisée au stade suivant sans autre purification.
**RMN ¹H** (400 MHz, CDCl₃) δ ppm: 3.6 (s, 2 H), 1.28 (t, 9 H), 3.7-3.6 (2m, 4 H), 4.7 (s, 1 H), 4.2 (quad, 2 H)

### Stade B : 2-(2,2-Diéthoxyméthyl)-1-méthyl-pyrrole-3-carboxylate d'éthyle

Le composé du titre est obtenu selon le procédé décrit au Stade C de la Préparation 22 à partir du 4,4-diéthoxy-3-oxo-butanoate d'éthyle obtenu au stade précédent.
**RMN ¹H** (400 MHz, dmso-d6) δ ppm: 6.73 (d, 1 H), 6.32 (d, 1 H), 6.23 (s, 1 H), 4.17 (q, 2 H), 3.7 (s, 3 H), 3.68/3.43 (2m, 4 H), 1.26 (t, 3 H), 1.13 (t, 6 H)

### Stade C : 1-Méthyl-2-(2-morpholinométhyl)pyrrole-3-carboxylate d'éthyle

Le composé du titre est obtenu selon le procédé décrit au Stade D de la Préparation 22 à partir du 2-(2,2-diéthoxyméthyl)-1-méthyl-pyrrole-3-carboxylate d'éthyle obtenu au stade précédent.
**RMN ¹H** (400 MHz, dmso-d6) δ ppm: 6.74 (d, 1 H), 6.32 (d, 1 H), 4.15 (q, 2 H), 3.8 (s, 2 H), 3.65 (s, 3 H), 3.5 (m, 4 H), 2.32 (m, 4 H), 1.22 (t, 3 H)

### Stade D : Acide 6-[4-(éthoxycarbonyl)-1-méthyl-5-(morpholin-4-ylméthyl)-1H-pyrrol-2-yl]-1,3-beuzodioxole-5-carboxylique

On procède selon le protocole décrit aux Stades B et C de la Préparation 1 en remplaçant le 2-bromo-4-chlorobenzaldéhyde utilisé au Stade B par le 6-bromo-1,3-benzodioxole-5-carbaldéhyde.

### Préparation 24: Acide 6-[4-(méthoxycarbonyl)-5-(2-méthoxyéthyl)-1-méthyl-1H-pyrrol-2-yl]-1,3-benzodioxole-5-carboxylique

### Stade A : 2-(2-Méthoxyéthyl)-1-méthyl pyrrole-3-carboxylate de méthyle

Le composé du titre est obtenu selon le protocole du Stade C de la Préparation 22 en utilisant le 5-méthoxy-3-oxovalérate de méthyle.
**RMN ¹H** (400 MHz, dmso-d6) δ ppm: 6.69 (df, 1 H), 6.31 (df, 1 H), 3.69 (s, 3 H), 3.59 (s, 3 H), 3.49 (t, 2 H), 3.2 (s, 3 H), 3.11 (t, 2 H)

### Stade B : Acide 6-[4-(méthoxycarbonyl)-5-(2-méthoxyéthyl)-1-méthyl-1H-pyrrol-2-yl]-1,3-benzodioxole-5-carboxylique

On procède selon le protocole décrit aux Stades B et C de la Préparation 1 en remplaçant le 2-bromo-4-chlorobenzaldéhyde utilisé au Stade B par le 6-bromo-1,3-benzodioxole-5-carbaldéhyde.

### Préparation 25 : Acide 2-[4-(éthoxycarbonyl)-1,5-diméthy-1H-pyrrol-2-yl]benzoïque

On procède selon le procédé de la Préparation 1 en remplaçant le 2-bromo-4-chlorobenzaldéhyde utilisé au Stade B par le 2-bromobenzaldéhyde.
**RMN ¹H** (400 MHz, dmso-d6) δ ppm: 12.81 (m, 1 H), 7.84 (m, 1 H), 7.59 (m, 1 H), 7.51 (m, 1 H), 7.35 (m, 1 H), 6.23 (s, 1 H), 4.16 (q, 2 H), 3.24 (s, 3 H), 2.5 (s, 3 H), 1.25 (t, 3 H)

### Préparation 26 : Acide 5-fluoro-2-[4-(méthoxycarbonyl)-1,5-diméthyl-1H-pyrrol-2-yl]benzoïque

On procède selon le procédé de la Préparation 1 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 2-bromo-5-fluorobenzaldéhyde.
**RMN ¹H** (400 MHz, dmso-d6) δ ppm: 7.6 (dd, 1 H), 7.42 (m, 2 H), 6.22 (s, 1 H), 4.15 (q, 2 H), 3.21 (s, 3 H), 2.5 (s, 3 H), 1.23 (t, 3 H)
**RMN ¹⁹F** (400 MHz, dmso-d6) δ ppm: -113

### Préparation 27 : Acide 2-[4-(éthoxycarbonyt)-1,5-diméthyl-1H-pyrrol-2-yl]-5-fluoro-4-méthoxybenzoïque

On procède selon le procédé de la Préparation 1 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 2-bromo-4-méthoxy-5-fluorobenzaldéhyde.
**RMN ¹H** (400 MHz, dmso-d6) δ ppm: 9.65 (d, 1 H), 7.67 (d, 1 H), 7.24 (d, 1 H), 6.48 (s, 1 H), 4.19 (q, 2 H), 3.97 (s, 3 H), 3.38 (s, 3 H), 2.56 (s, 3 H), 1.26 (t, 3 H).

### Préparation 28 : Acide 2-(4-éthoxycarbonyl-1,5-diméthyl-1H-pyrrol-2-yl)-4-fluoro-5-méthoxy-benzoïque

On procède selon la Préparation 1 en remplaçant le 2-bromo-4-chlorobenzaldéhyde utilisé au Stade B par le 4-fluoro-5-méthoxybenzaldéhyde.
**RMN ¹H** (400 MHz, dmso-d6) δ ppm: 12.9 (m, 1 H), 7.6 (d, 1 H), 7.22 (d, 1 H), 6.23 (s, 1 H), 4.2 (quad, 2 H), 3.95 (s, 3 H), 3.25 (s, 3 H), 2.5 (s, 3 H), 1.25 (t, 3 H)

### Préparation 29 : Acide 5-chloro-2-[4-(éthoxycarbonyl)-1,5-diméthyl-1H-pyrrol-2-yl]benzoïque

On procède selon le procédé de la Préparation 1 en remplaçant le 2-bromo-4-chlorobenzaldéhyde utilisé au Stade B par le 2-bromo-5-chlorobenzaldéhyde.

### Préparation 30: Acide 2-{1-[2-(benzyloxy)éthyl]-4-(éthoxycarbonyl)-5-méthyl-1H-pyrrol-2-yl}-4-chlorobenzoïque

On procède selon le procédé de la Préparation 19 en remplaçant le 6-bromo-1,3-benzodioxole-5-carbaldéhyde par le 2-bromo-4-chlorobenzaldéhyde.

### Préparation 31 : Acide 5-méthoxy-2-[4-(méthoxycarbonyl)-1,5-diméthyl-1H,pyrrol-2-yl]benzoïque

On procède selon le procédé de la Préparation 1 en remplaçant au Stade A le 2-méthyl-1*H-*pyrrole-3-carboxylate d'éthyle par le 2-méthyl-1*H*-pyrrole-3-carboxylate de méthyle, ainsi que le 2-bromo-4-chlorobenzaldéhyde utilisé au Stade B par le 2-bromo-5-methoxybenzaldéhyde.
**RMN ¹H** (400 MHz, dmso-d6) δ ppm: 12.8 (sl, 1 H), 7.34 (df, 1 H), 7.26 (d, 1 H), 7.15 (dd, 1 H), 6.19 (s, 1 H), 3.84 (s, 3 H), 3.69 (s, 3 H), 3.22 (s, 3 H), 2.5 (s, 3 H)

### Préparation 32 : Acide 6-[1-(2,2-difluoroéthyl)-4-(éthoxycarbonyl)-5-méthy-1H-pyrrol-2-yl]-1,3-benzodioxole-5-carboxylique

On procède selon le protocole de la Préparation 19 en remplaçant le 2-bromoéthoxyméthylbenzène utilisé au Stade A par du 2-bromo-1,1-difluoro-éthane.

### Préparation 1' : Chlorhydrate de (3R)-3-méthyl-1,2,3,4-tétrahydroisoqulinoline

### Stade A : {(3S)-2-[(4-Méthylphényl)sulfonyl]-1,2,3,4-tétrahydroisoquinolin-3-yl}méthyl 4-méthylbenzènesulfonate

A une solution de 30,2 g de [(3*S*)-1,2,3,4-tétrahydroisoquinolin-3-yl]méthanol (185 mmol) dans 750 mL de dichlorométhane sont ajoutés successivement 91,71 g de chlorure de tosyle (481 mmol), puis, au goutte à goutte, 122,3 mL de *N,N,N*-triéthylamine (740 mmol). Le milieu réactionnel est ensuite agité à température ambiante durant 20 h. Il est ensuite dilué avec du dichlorométhane, lavé successivement avec une solution HCl 1M, une solution aqueuse saturée en NaHCO₃, puis de la saumure jusqu'à neutralité. La phase organique est alors séchée sur MgSO₄, filtrée, concentrée à sec. Le solide obtenu est alors solubilisé dans un volume minimum de dichlorométhane, puis est ajouté du cyclohexane jusqu'à formation d'un précipité. Ce précipité est alors filtré et lavé avec du cyclohexane. Après séchage, le produit du titre est obtenu sous forme de cristaux.
**RMN ¹H** : δ (400 MHz ; dmso-d6 ; 300K) : 7.75 (d, 2H , H aromatiques, *ortho* O-tosyle); 7.6 (d, 2H , H aromatiques, *ortho N*-tosyle); 7.5 (d, 2H , H aromatiques, *méta* O-tosyle); 7.3 (d, 2H , H aromatiques, *méta N*-tosyle); 7.15-6.9 (m, 4H, H aromatiques, tétrahydro isoquinoline); 4.4-4.15 (dd, 2H, H aliphatiques, tétrahydroisoquinoline); 4.25 (m, 1H, H aliphatique, tétrahydroisoquinoline); 4.0-3.8 (2dd, 2H, H aliphatiques, **CH**₂-O-tosyle) ; 2.7 (2dd, 2H, H aliphatiques, tétrahydroisoquinoline); 2.45 (s, 3H, O-SO₂-Ph- **CH₃**); 2.35 (s, 3H, *N*-SO₂-Ph-**CH₃**)
**IR** : v : -SO₂ : 1339-1165 cm⁻¹

### Stade B : (3R)-3-Méthyl-2-[(4-méthylphényl)sulfonyl]-1,2,3,4-tétrahydroisoquinoline

A une suspension de 8,15 g (214,8 mmol) d'hydrure de lithium et d'aluminium (LiAlH₄) dans 800 mL de méthyl *tert*-butyl éther (MTBE), on ajoute 101,2 g du dérivé ditosylé obtenu au Stade A (214,8 mmol) en solution dans 200 mL de MTBE. L'ensemble est ensuite chauffé à 50°C pendant 2h. On laisse refroidir et on se place à 0°C, puis on ajoute, goutte à goutte, 12 mL d'une solution de NaOH 5N. L'ensemble est agité à température ambiante pendant 45 minutes. Le solide ainsi obtenu est alors filtré, lavé avec du MTBE puis avec du dichlorométhane. Le filtrat est ensuite concentré à sec. On obtient alors le produit du titre sous la forme d'un solide.
**RMN ¹H** : δ (400 MHz ; dmso-d6 ; 300K) : 7.70 (d, 2H , H aromatiques, *ortho N*-tosyle); 7.38 (d, 2H , H aromatiques, *méta N*-tosyle); 7.2-7.0 (m, 4H, H aromatiques, tétrahydroisoquinoline); 4.4 (m, 2H, H aliphatiques, tétrahydroisoquinoline); 4.3 (m, 1H, H aliphatique, tétrahydroisoquinoline); 2.85-2.51 (2dd, 2H, H aliphatiques, tétrahydro isoquinoline); 2.35 (s, 3H, *N*-SO₂-Ph- **CH₃**) ; 0.90 (d, 3H, , tétrahydroisoquinoline-**CH₃**)
**IR** : ν : -SO₂ : 1332-1154 cm⁻¹

### Stade C : (3R)-3-Méthyl-1,2,3,4-tétrahydroisoquinoline

A une solution de 31,15 g (103,15 mmol) du dérivé monotosylé obtenu au Stade B dans 500 mL de méthanol anhydre, on ajoute 3,92g (161 mmol) de tournure de magnésium par pelletés. L'ensemble est agité en présence d'ultra-sons pendant 96 h. Le milieu réactionnel est ensuite filtré et le solide est lavé plusieurs fois avec du méthanol. Le filtrat est ensuite concentré à sec. Après purification par chromatographie sur gel de silice en utilisant le dichlorométhane et l'éthanol contenant de l'ammoniac comme éluants, on obtient le produit du titre sous la forme d'une huile.
**RMN ¹H** : δ (400 MHz ; dmso-d6 ; 300K) : 7.05 (m, 4H, H aromatiques, tétrahydroisoquinoline); 3.90 (m, 2H, H aliphatiques, tétrahydroisoquinoline); 2.85 (m, 1H, H aliphatique, tétrahydroisoquinoline); 2.68-2.4 (2dd, 2H, H aliphatiques, tétrahydro isoquinoline); 1.12 (d, 3H, tétrahydroisoquinoline-**CH₃**) ; 2.9-2.3 (m, large, 1H, **HN** (tétrahydroisoquinoline))
**IR :** v : -NH : 3248 cm⁻¹

### Stade D : Chlorhydrate de (3R)-3-méthyl-1,2,3,4-tétrahydroisoquinoline

A une solution de 14,3 g (97.20 mmol) du composé obtenu au Stade C dans 20 mL d' éthanol anhydre, on ajoute ,goutte à goutte, 100 mL d'une solution d'éther chlorhydrique 1M. L'ensemble est agité à température ambiante pendant 1h, puis filtré. Les cristaux ainsi obtenus sont lavés avec de l'éther éthylique. Après séchage, le produit du titre sous la forme de cristaux.
**RMN ¹H** : δ (400 MHz ; dmso-d6 ; 300K) : 9.57 (m, large, 2H, **NH₂**⁺(tétrahydro isoquinoline).7.22 (m, 4H, H aromatiques, tétrahydroisoquinoline); 4.27 (s, 2H, H aliphatiques, tétrahydroisoquinoline); 3.52 (m, 1H, H aliphatique, tétrahydroisoquinoline); 3.03--2.85 (2dd, 2H, H aliphatiques, tétrahydroisoquinoline); 1.39 (d, 3H, tétrahydro isoquinoline-**CH₃**)
**IR :** v : -NH₂⁺ : 3000-2300 cm⁻¹ v : -CH aromatique : 766 cm⁻¹

### Préparation 2' :[(3S)-1,2,3,4-Tétrahydroisoquinolin-3-ylméthyl]carbamate de tert-butyle

### Stade A : (3S)-3-(Hydroxyméthyl)-3,4-dihydro-1H-isoquinoline-2-carboxylate de benzyle

Ce dérivé est obtenu en utilisant un protocole de la littérature (R. B. Kawthekar et al South Africa Journal of Chemistry 63, 195, 2009) à partir de 15 g de (3S)-1,2,3,4-tétrahydroisoquinolin-3-ylméthanol (91,9 mmol) en présence de chloroformate de benzyle et de triéthylamine en solution dans le dichlorométhane. Après purification par chromatographie sur gel de silice en utilisant l'éther de pétrole et l'acétate d'éthyle comme éluants, le produit du titre est obtenu sous la forme d'une huile.

**RMN¹H** : δ (300 MHz ; DMSO-d6 ; 300K) : 7.33 (m, 5H, H aromatiques, O-Benzyl); 7.15 (s, 4H, H aromatiques, H tétrahydroisoquinoline) ; 5.13 (s, 2H, **CH₂**-Ph) ; 4.73 (d, 1H, H tétrahydroisoquinoline) ; 4.47 (m, H, **CH₂OH**) ; 4.36 (m, 1H, H tétrahydroisoquinoline) ; 4.28 (d, 1H, H tétrahydroisoquinoline) ; 3.39 (dd, 1H, **CH₂OH**) ; 3.23 (dd, 1H, , **CH₂**OH**)** ; 2.93 (dd, 1H, H tétrahydroisoquinoline) ; 2.86 (dd, 1H, H tétrahydroisoquinoline)
**IR :** v : OH : 3416 cm⁻¹ ; v : <C=O 1694 cm⁻¹ ; v : >C-H aromatique: 754 cm⁻¹

### Stade B : (3S)-3-(Azidométhyl)-3,4-dihydroisoquinoline-2(1H)-carboxylate de benzyle

Ce dérivé est obtenu utilisant un protocole de la littérature (D. Pagé et al J. Med. Chem, 44, 2387, 2001) à partir de 23 g du composé obtenu au Stade A (77,3 mmol) en présence de diphénylphosphorylazide et de triphénylphosphine en solution dans le THF. Après purification par chromatographie sur gel de silice en utilisant l'éther de pétrole et l'acétate d'éthyle comme éluants, le produit du titre est obtenu sous la forme d'une huile.
**RMN¹H** : δ (400 MHz ; DMSO-d6 ; 300K) : 7.36 (m, 5H, H aromatiques, O-Benzyl); 7.19 (m, 4H, H aromatiques, H tétrahydroisoquinoline) ; 5.16 (s, 2H, **CH₂**-Ph) ; 4.76 (d, 1H, H tétrahydroisoquinoline) ; 4.53 (m, 1H, H tétrahydroisoquinoline) ; 4.30 (m, 1H, H tétrahydroisoquinoline) ; 3.28 (m, 2H, , **CH₂**N₃) ; ; 3.06 (dd, 1H, H tétrahydroisoquinoline) ; 2.78 (dd, 1H, H tétrahydroisoquinoline)
**IR :** v : N₃ : 2095 cm⁻¹ v : <C=O :1694 cm⁻¹ ; v : >C-H aromatique : 754 cm⁻¹

### Stade C : (3S)-3-(Aminométhyl)-3,4-dihydroisoquinoline-2(1H)-carboxylate de benzyle

A une solution de 20, 9 g (64,5 mmol) du dérivé azido obtenu au Stade B dans 650 mL de THF, on ajoute successivement 25,5g (97,2 mmol) de triphénylphosphine et 157 mL d'eau. L'ensemble est porté à reflux pendant 2h30. Le milieu réactionnel est ensuite concentré à sec, puis l'huile résiduelle est repris avec de l'éther isopropylique. Un précipité blanc apparait ; il est filtré et lavé avec de l'éther isopropylique. Le filtrat est alors concentré à sec puis purifié par chromatographie sur gel de silice en utilisant le dichlorométhane et le méthanol comme éluants. On obtient le produit du titre sous la forme d'une huile.
**RMN¹H** : δ (400 MHz ; DMSO-d6 ; 300K) : 7.40 (m, 5H, H aromatiques, O-Benzyl); 7.20 (m, 4H, H aromatiques, H tétrahydroisoquinoline) ; 5.15 (s, 2H, **CH₂**-Ph) ; 4.75-4.3 (m, 2H, H tétrahydroisoquinoline) ; 4.30 (d, 1H, H tétrahydroisoquinoline) ; 2.90 (m, 2H, , **CH**₂NH₂) ; 2.45 (m, 2H, H tétrahydroisoquinoline) ; 1.40 (m, 2H, NH₂)
**IR :** ν : NH₂ : 3400-3300 cm⁻¹ ; v : <C=O : 1688 cm⁻¹

### Stade D : (3S)-3-{[(tert-Butoxycarbonyl)amino]méthyl}-3,4-dihydroisoquinoline-2(1H)-carboxylate de benzyle

A une solution de 18,4 g (62,1 mmol) du composé obtenu au Stade C dans 630 mL de dichlorométhane, on ajoute successivement 17,5 mL (124 mmol) de triéthylamine et, par pelletés, 14,9 g (68,3 mmol) de di-*tert*-butyl dicarbonate. L'ensemble est agité à température ambiante pendant 2 h. On concentre ensuite le milieu réactionnel, puis on ajoute de l'actétate d'éthyle. La phase organique est lavée successivement avec une solution HCl 1M, de la saumure, une solution aqueuse saturée en NaHCO₃, puis de la saumure. Après séchage, concentration à sec et purification par chromatographie sur gel de silice en utilisant l'éther de pétrole et l'acétate d'éthyle comme éluants, le produit du titre est obtenu sous la forme d'une huile.
**RMN¹H** : δ (400 MHz ; DMSO-d6 ; 300K) : 7.35 (m, 5H, H aromatiques, O-Benzyl); 7.15 (m, 4H, H aromatiques, H tétrahydroisoquinoline) ; 6.51 (m, 1H, **NH**Boc) ; 5.12 (s, 2H, **CH₂**-Ph) ; 4.76 (d, 1H, H tétrahydroisoquinoline) ; 4.51 (m, 1H, H tétrahydroisoquinoline) ; 4.36 (d, 1H, H tétrahydroisoquinoline) ; 2.95 (m, 3H, H tétrahydroisoquinoline + **CH₂**NHBoc) ; 2.71 (d, 1H, H tétrahydroisoquinoline) ; 1.34 (s, 9H, NH**Boc**)
**IR:** v : NH : 3351 cm⁻¹ v : <C=O : 1686 cm⁻¹

### Stade E : [(3S)-1,2,3,4-Tétrahydroisoquinolin-3-ylméthyl]carbamate de tert-butyle

A une solution de 21 g (53 mmol) du composé obtenu au Stade D dans 600 mL d'acétate d'éthyle, on ajoute 2,1 g de palladium sur charbon 10%. L'ensemble est agité à température ambiante sous 1,3 bar de pression de dihydrogène pendant 5 h. Le milieu réactionnel est ensuite filtré, puis concentré à sec. Le produit du titre est obtenu sous la forme d'un solide.
**RMN¹H** : δ (400 MHz ; DMSO-d6 ; 300K) : 7.15 (m, 4H, H aromatiques, H tétrahydro isoquinoline) ; 6.85 (t, 1H, **NH**Boc) ; 3.90 (m, 2H, H tétrahydroisoquinoline) ; 3.00 (m, 2H, **CH₂**NHBoc) ; 2.80 (m, 1H, H tétrahydroisoquinoline) ; 2.65 (dd, 1H, H tétrahydro isoquinoline) ; 2.40 (dd, 1H, H tétrahydro isoquinoline) ; 1.40 (s, 9H, NH**Boc**)
**IR :** v : NH : 3386-3205 cm⁻¹ (NH amide); v : <C=O _{:} 1688 cm⁻¹ ; v : NH : 1526 cm⁻¹ (NH amine)

### Préparation 3' : (3S)-3-(4-Morpholinylméthyl)-1,2,3,4-tétrahydroisoquinoline

### Stade A : (3S)-3-(4-Morpholinylcarbonyl)-3,4-dihydro-2(1H)-isoquinoline carboxylate de benzyle

A une solution de 5 g d'acide (3*S*)-2-[(benzyloxy)carbonyl]-1,2,3,4-tétrahydro-3-isoquinolinecarboxylique (16 mmol) dans 160 mL de dichlorométhane sont ajoutés 1,5 mL de morpholine (17,6 mmol), puis 9 mL de *N,N,N*-triéthylamine (64 mmol), 3,3 g de 1-éthyl-3-(3'-diméthylaminopropyl)-carbodiimide (EDC) (19,2 mmol), et 2,6 g d'hydroxybenzotriazole (HOBt) (19,2 mmol). Le milieu réactionnel est agité à température ambiante pendant une nuit, puis il est versé sur une solution de chlorure d'ammonium et extrait avec de l'acétate d'éthyle. La phase organique est ensuite séchée sur MgSO₄, puis filtrée et évaporée à sec. Le produit brut ainsi obtenu est alors purifié par chromatographie sur gel de silice en utilisant le dichlorométhane et le méthanol comme éluants. Le produit est obtenu sous la forme d'une mousse.
**RMN¹H:** δ (400 MHz; dmso-d6; 353K): 7.30 (m, 5H benzyl); 7.15 (m, 4H aromatiques) ; 5.2-5.0 (m, 3H, 2H benzyl, 1H dihydroisoquinoline); 4.75-4.5 (2d, 2H dihydroisoquinoline); 3.55-3.3 (m, 8H morpholine); 3.15-2.9 (2dd, 2H dihydroisoquinoline)
**IR :** v : >C=O : 1694 ;1650 cm⁻¹

### Stade B : (3S)-3-(4-Morpholinylméthyl)-3,4-dihydro-2(1H)-isoquinoline carboxylate de benzyle

A une solution de 5,3 g du produit obtenu au Stade A (13,9 mmol) dans 278 mL de tétrahydrofurane sont ajoutés 14 mL de complexe borane-diméthylsulfure (BH₃Me₂S) (27,8 mmol) à température ambiante. Le tout est chauffé pendant 4 heures à 80°C. On laisse revenir à température ambiante, puis on ajoute 7 mL (14 mmol) de BH₃Me₂S. Le milieu réactionnel est de nouveau chauffé à 80°C pendant 2 heures. On évapore ensuite le tétrahydrofurane, puis on ajoute lentement du méthanol, puis 5,6 mL dune solution aqueuse de HCl 5N (27,8 mmol). Le mélange est agité à température ambiante pendant une nuit, puis à 80°C pendant 1h. On ajoute ensuite une solution aqueuse saturée en NaHCO₃ sur le milieu réactionnel placé à 0°C jusqu'à atteindre un pH=8, puis on extrait avec de l'acétate d'éthyle. La phase organique est ensuite séchée sur MgSO₄, puis filtrée et évaporée à sec. Le produit du titre est obtenu sous la forme d'une huile.
**RMN¹H** : δ (400 MHz ; dmso-d6 ; 353K) : 7.43-7.30 (massif, 5H benzyl) ; 7.19 (m, 4H aromatiques) ; 5.16 (m, 2H, 2H benzyl) ; 4.79-4.29 (d, 2H dihydroisoquinoline) ; 4.58 (m, 1H dihydroisoquinoline) ; 3.50 (m, 4H morpholine) ; 3.02-2.80 (dd, 2H dihydroisoquinoline) ; 2.42-2.28 (massif, 5H, 4H morpholine, 1H morpholine) ; 2.15 (dd, 1H morpholine)
**IR** : v : >CH : 2810 cm⁻¹ ; ν : >C=O : 1694 cm⁻¹ ; v : >C-O-C< : 1114 cm⁻¹ ; v : >CH-Ar : 751 ; 697 cm⁻¹

### Stade C : (3S)-3-(4-Morpholinylméthyl)-1,2,3,4-tétrahydroisoquinoline

A une solution de 4,9 g du composé du Stade B (13,4 mmol) dans 67 mL d'éthanol est ajouté 0,980 g de dihydroxyde de palladium (20% massique) à température ambiante. Le milieu réactionnel est placé sous 1,2 bar de d'hydrogène à température ambiante pendant 4 heures. Il est ensuite passé sur une filtre Wathman, puis le palladium est rincé plusieurs fois avec de l'éthanol. Le filtrat est évaporé à sec. Le produit du titre est obtenu sous la forme d'une huile.
**RMN¹H** : δ (400 MHz ; dmso-d6 ; 300K) : 7.12-7.0 (massif, 4H aromatiques) ; 3.92 (s, 2H tétrahydroisoquinoline) ; 3.60 (t, 4H morpholine) ; 2.98 (m, 1H tétrahydroisoquinoline) ; 2.68 (dd, 1H tétrahydroisoquinoline) ; 2.5-2.3 (massif, 8H, 1H tétrahydroisoquinoline, 6H morpholine, 1H NH)
**IR :** v : >NH : 3322 cm⁻¹ ; v : >C-O-C< : 1115 cm⁻¹ ; ν : >CH-Ar : 742 cm⁻¹

### Préparation 4' : (3S)-3-[(4-Méthyl-1-pipérazinyl)méthyl]-1,2,3,4-tétrahydroisoquinoline

On procède selon le procédé de la Préparation 3' en remplaçant la morpholine utilisée au Stade A par la 1-méthyl-pipérazine.

### Préparation 5' : Chlorhydrate de (3S)-3-[2-(morpholin-4-yl)éthyl]-1,2,3,4-tétrahydroisoquinoline

### Stade A : (3S)-3-(2-Morpholino-2-oxo-éthyl)-3,4-dihydro-1H-isoquinoline-2-carboxylate de tert-butyle

A une solution de 3 g (10,30 mmol) d'acide [(3*S*)-2-(*tert*-butoxycarbonyl)-1,2,3,4-tétrahydroisoquinolin-3-yl]acétique dans 100mL de dichlorométhane, on ajoute goutte à goutte 1,10 mL (11,32 mmol) de morpholine, toujours goutte à goutte 4,3 mL (30,9 mmol) de triéthylamine, 2,20 g (12,40 mmol) d'EDC et 1,70g (1,68 mmol) de HOBt'hydroxybenzotriazole. L'ensemble est agité à température ambiante pendant 15 h. Le milieu réactionnel est ensuite dilué avec du dichlorométhane, lavé successivement avec une solution HCl 1M, une solution aqueuse saturée en NaHCO₃, puis de la saumure jusqu'à neutralité. La phase organique est alors séchée sur MgSO₄, filtrée et concentrée à sec. Après purification par chromatographie sur gel de silice en utilisant le dichlorométhane et le méthanol comme éluants, on obtient le produit du titre sous la forme d'une huile.
**RMN ¹H** : δ (400 MHz; dmso-d6; 300K): 7.20-7.10 (m, 4H, H aromatiques, tétrahydroisoquinoline); 4.70 (m, 1H, H aliphatiques, CH tétrahydroisoquinoline); 4.75-4.20 (2m, 2H, H aliphatiques, CH₂ en alpha de N tétrahydroisoquinoline); 3.60 (m, 8H, H aliphatiques, morpholine); 3.00 et 2.70 (2dd, 2H, H aliphatique, tétrahydroisoquinoline); 2.50-2.20 (2d, 2H, H aliphatiques, CH₂CO); 1.40 (s, 9H, ^{t}Bu)
**IR :** v : C=O : 1687; 1625 cm⁻¹

### Stade B : Chlorhydrate de 1-(morpholin-4-yl)-2-[(3S)-1,2,3,4-tétrahydroisoquinolin-3-yl]éthanone

A une solution de 2,88 g (7,18 mmol) du composé obtenu au Stade A dans 16 mL de dichlorométhane, on ajoute goutte à goutte 80 mL (80 mmol) d'une solution d'éther chlorhydrique 1M. L'ensemble est agité à température ambiante pendant 15 h, puis la suspension est filtrée et le précipité lavé à l'éther. Après séchage, on obtient le produit du titre sous la forme d'un solide.
**RMN ¹H** : δ (400 MHz ; dmso-d6 ; 300K) : 9.80-9.50 (m, 2H, NH₂⁺); 7.30-7.10 (m, 4H, H aromatiques, tétrahydroisoquinoline); 4.30 (m, 2H, H aliphatiques, CH₂ en alpha de N tétrahydroisoquinoline); 3.80 (m, 1H, H aliphatiques, CH tétrahydroisoquinoline); 3.70-3.40 (2m, 8H, H aliphatiques, morpholine); 3.15 et 2.8 (m, 4H, H aliphatique, CH₂ tétrahydroisoquinoline et CH₂CO)
**IR :** v : -NH₂⁺ : 2800-1900 cm⁻¹ ; v : C=O : 1620 cm⁻¹

### Stade C : Chlorhydrate de (3S)-3-[2-(morpholin-4-yl)éthyl]-1,2,3,4-tétrahydroisoquinoline

On prépare une solution de 2,2 g (7,44 mmol) du composé obtenu au Stade B dans 22 mL de MTBE et 5 mL de dichlorométhane. Après refroidissement dans un bain de glace à 0°C, on y ajoute goutte à goutte 15 mL (15 mmol) d'une solution de LiAlH₄ 1M dans le tétrahydrofurane. L'ensemble est ensuite agité à température ambiante pendant 6 h. On se place à 0°C, puis on ajoute goutte à goutte 1 mL d'une solution de NaOH 5M. L'ensemble est agité à température ambiante pendant 45 minutes. Le solide est alors filtré et lavé avec du MTBE, puis avec du dichlorométhane et le filtrat est concentré à sec. L'huile ainsi obtenue est diluée au dichlorométhane et on ajoute goutte à goutte 6,3 mL d'une solution d'éther chlorhydrique 1M. L'ensemble est agité à température ambiante pendant 1h, puis filtré. Les cristaux ainsi obtenus sont lavés avec de l'éther éthylique. Après séchage, on obtient le produit du titre sous la forme d'un solide.
**RMN ¹H** : δ (400 MHz ; dmso-d6 ; 300K) : 11.35 + 9.80 (2m, 2H, NH₂⁺); 10.00 (m, H, NH⁺); 7.20 (m, 4H, H aromatiques, tétrahydroisoquinoline); 4.30 (s, 2H, H aliphatiques, CH₂ en alpha de N tétrahydroisoquinoline); 4.00 + 3.85 (2m, 4H, H aliphatiques, CH₂ en alpha de N morpholine); 3.70 (m, 1H, H aliphatiques, CH tétrahydroisoquinoline); 3.55-3.30 (m, 4H, H aliphatiques, CH en alpha de O morpholine et CH₂-Morpholine ); 3.15 (dd, 1H, H aliphatique, CH₂ tétrahydroisoquinoline); 3.10 (m, 2H, H aliphatique, CH en alpha de O morpholine) ; 2.90 (dd, 1H, H aliphatique, CH₂ tétrahydroisoquinoline); 2.30 + 2.15 (2m, 2H, H aliphatique, CH₂-tétrahydroisoquinoline)
**IR :** v : NH⁺ / -NH₂⁺ : entre 3500 et 2250 cm⁻¹ ; v : C=C : faible 1593 cm⁻¹ ; ν : C-H aromatique :765 cm⁻¹

### Préparation 6' : (3R)-3-[3-(Morpholin-4-yl)propyl]-1,2,3,4-tétrahydroisoquinoline

### Stade A : {(3S)-2-[(4-Méthylphényl)sulfonyl]-1,2,3,4-tétrahydroisoquinolin-3-yl}méthyl 4-méthylbenzènesulfonate

Le procédé est identique à celui du Stade A de la Préparation 1'.

### Stade B : 2-({(3R)-2-[(4-Méthylphényl)sulfonyl]-1,2,3,4-tétrahydroisoquinolin-3-yl}méthyl)-3-(morpholin-4-yl)-3-oxopropanoate de tert-butyle

A une suspension de 1 g de NaH (60%) (25,08 mmol) dans 30 mL de MTBE, on ajoute goutte à goutte une solution de 5 g de 3-morpholino-3-oxopropanoate de *tert*-butyle (21,81 mmol) dans 20 mL de MTBE anhydre. Cette suspension est agitée à température ambiante pendant 1h, puis on ajoute le composé obtenu au Stade A sous forme d'une poudre. L'ensemble est agité à 60°C pendant 30h. Une solution de 100 mL d'une solution aqueuse saturée en chlorure d'ammonium est ajoutée. Cette solution est extraite au dichlorométhane. La phase organique est alors séchée sur MgSO₄, filtrée et concentrée à sec. Après purification par chromatographie sur gel de silice en utilisant le dichlorométhane et le MeOH comme éluants, on obtient le produit attendu sous la forme d'une huile.
**RMN ¹H** (500 MHz, dmso-d6) δ ppm : 7.63/7.59 (2d, 2 H), 7.3/7.26 (2d, 2 H), 7.13 (m, 2 H), 7.09/6.97 (2t, 2 H), 4.64/4.55/4.36/4.28 (2AB, 2 H), 4.25/4.11 (2m, 1 H), 3.81 (m, 1 H), 3.73-3.48 (m, 4 H), 3.57-3.32 (m, 4 H), 2.51 (m, 2 H), 2.32/2.31 (2s, 3 H), 1.88/1.79 (2m, 2 H), 1.39/1.38 (2s, 9 H)
**IR (ATR)** cm⁻¹: v : >C=O : 1731 (ester) ; v : >C=O : 1644 (amide) ; v : -SO2 : 1334-1156 ; ν : >C-O-C< : 1115; γ : >CH-Ar : 815-746-709

### Stade C : Acide 2-({(3R)-2-[(4-méthylphényl)sulfonyl]-1,2,3,4-tétrahydroisoquinolin-3-yl}méthyl)-3-(morpholin-4-yl)-3-oxopropanoique

A une solution de 9,5 g (17,97 mmol) du composé obtenu au Stade B dans 40 mL de dioxane, on ajoute goutte à goutte 20 mL d'une solution de HCl 4M dans le dioxane. L'ensemble est agité à température ambiante pendant 48h, puis la solution est concentrée à sec. Après séchage, on obtient le produit attendu sous la forme d'une huile.
**RMN ¹H** (400 MHz, dmso-d6) δ ppm : 12.75 (m, 1 H), 7.6 (2*d, 2 H), 7.3 (2*d, 2 H), 7.1/6.95 (2*m, 4 H), 4.7-4.2 (d, 2 H), 4.25/4.12 (2*m, 1 H), 3.9-3.3 (m, 9 H), 2.55 (d, 2 H), 2.3 (2*s, 3 H), 1.8 (t, 2 H)
**IR (ATR)** cm⁻¹ : v : -OH : 3500 à 2000 ; v : >C=O _{:} 1727 (acide) ; v : >C=O : 1634 (amide) ; v : -SO2 : 1330-1155

### Stade D : 3-{(3R)-2-[(4-Méthylphényl)sulfonyl]-1,2,3,4-tétrahydroisoquinolin-3-yl}-1-(morpholin-4-yl)propan-1-one

A une solution de 7,80 g (16,51 mmol) du composé obtenu au Stade C dans 100mL de DMSO, on ajoute 1,16 g (19,83 mmol) de chlorure de sodium (NaCl) solide, puis goutte à goutte 5 mL d'eau. L'ensemble est agité à 130°C pendant 1h, puis la solution est concentrée au ¾. Le milieu réactionnel est ensuite dilué avec du dichlorométhane, lavé successivement avec une solution aqueuse saturée en chlorure de lithium, puis de la saumure. La phase organique est alors séchée sur MgSO₄, filtrée, concentrée à sec. Après purification par chromatographie sur gel de silice en utilisant le cyclohexane et l'acétate d'éthyle comme éluants, on obtient le produit attendu sous la forme d'une huile.
**RMN ¹H** (400 MHz, dmso-d6) δ ppm : 7.65 (d, 2 H), 7.3 (d, 2 H), 7.15/7 (2 m, 4 H), 4.6 (d, 1 H), 4.25 (d, 1 H), 4.2 (m, 1 H), 3.5 (m, 4 H), 3.4 (2 m, 4 H), 2.6 (2 dd, 2 H), 2.35 (s, 3 H), 2.3 (m, 2 H), 1.5 (quad., 2 H)
**IR (ATR)** cm⁻¹ : ν : >C=O : 1639 ; ν : -SO2 : 1331-1156 ; γ : >CH-Ar : 815-675

### Stade E : (3R)-2-((4-Méthylphéuyl)sulfouyl)-3-(3-(morpholin-4yl)propyl)-1,2,3,4-tétrahydroisoquinoline

A une solution de 6,0 g (14,0 mmol) du composé obtenu au Stade D dans 60mL de MTBE et 14 mL de dichlorométhane, on ajoute 1,06g (28 mmol) de LAH par portion sur 5 minutes. L'ensemble est agité à température ambiante pendant 15 h. On ajoute goutte à goutte 1,5 mL d'eau et on agite pendant 15min. Ensuite, on ajoute goutte à goutte 1,5mL de soude 5 M et on agite 15min. Le milieu réactionnel est ensuite dilué avec du MTBE et du dichlorométhane. Puis, la suspension est filtrée et le précipité est lavé avec du MTBE et du dichlorométhane. La phase organique est alors séchée sur MgSO₄, filtrée, et concentrée à sec. Après purification par chromatographie sur gel de silice en utilisant le dichlorométhane et l'éthanol contenant de l'ammoniac comme éluants, on obtient le produit attendu sous la forme d'une huile.
**RMN ¹H** (400 MHz, dmso-d6) δ ppm : 7.68 (d, 2 H), 7.32 (d, 2 H), 7.1 (massif, 4 H), 4.65/4.23 (AB, 2 H), 4.2 (m, 1 H), 3.55 (t, 4 H), 2.7/2.6 (ABx, 2 H), 2.35 (s, 3 H), 2.25 (t, 4 H), 2.2 (t, 2 H), 1.4/1.3 (2m, 4 H).
**IR (ATR)** cm⁻¹ : ν: -SO2 : 1333-1158

### Stade F : (3R)-3-[3-(Morpholin-4yl]propyl)-1,2,3,4-tétrahydroisoquiuoline

A une solution de 1,50 g (3,62 mmol) du dérivé obtenu au Stade E dans 20 mL de méthanol anhydre, on ajoute 2,0 g (82,3 mmol) de tournure de magnésium par pelletés. L'ensemble est agité en présence d'ultrasons pendant 96 h. Le milieu réactionnel est ensuite filtré, le solide est lavé plusieurs fois avec du méthanol, et le filtrat est concentré à sec. Après purification par chromatographie sur gel de silice en utilisant le dichlorométhane et l'éthanol contenant de l'ammoniac comme éluants, on obtient le produit attendu sous la forme d'une huile.
**RMN ¹H** (400 MHz, dmso-d6) δ ppm : 7.3 (d, 2 H), 7.1 (t, 2 H), 7.1 (d+t, 3 H), 7 (d, 2 H), 3.9 (s, 2 H), 3.55 (t, 4 H), 2.75 (m, 1 H), 2.72/2.45 (dd, 2 H), 2.35 (t, 4 H), 2.25 (t, 2 H), 1.6 (m, 2 H), 1.45 (m, 2 H)
**IR (ATR)** cm⁻¹ : ν : >NH2+/NH+ : 3500-2300 ; ν : >C-O-C< : 1115

### Masse haute résolution (ESI+-/FIA/HR) :

Formule brute : C₁₆H₂₄N₂O
[M+H]⁺calculé : 261.1961
[M+H]⁺ mesuré : 261.1959

### Préparation 7' : Trichlorhydrate de (3S)-3-[(9aS)-octahydropipérazino[2,1-c]morpholin-8-ylméthyl]-1,2,3,4-tétrahydroisoquinoline

### Stade A: (9aS)-4-Oxo-octahydropipéraziuo[2,1-c]morpholine-8-carboxylate de tert-butyle

La synthèse de ce composé est connue dans la littérature (J. Med. Chem. 2012, 55, 5887 pour l'énantiomère opposé).

### Stade B: Chlorhydrate de (9aS)-octahydropipérazino[2,1-c]morpholin-4-one

Une solution de HCl 4 M dans le dioxane (60 mL, 240 mmol) est ajoutée au composé (9*aS*)-4-oxo-octahydropipérazino[2,1-c]morpholine-8-carboxylate de *tert*-butyle (11,8 g, 46,0 mmol) refroidi avec un bain de glace. La solution est ensuite agitée à la température ambiante durant 2 h, puis à 50-60°C durant 1.5 h. Puis, la solution est évaporée à sec. Le résidu est co-évaporé avec du dioxane (3 x 20 mL), puis séché sous vide pour obtenir le composé attendu sous la forme d'un solide.
**RMN ¹H** (400 MHz, dmso-d6) δ ppm: 2.80 - 2.94 (m, 2 H), 2.94 - 3.05 (m, 1 H), 3.23 - 3.37 (m, 2 H), 3.59 - 3.69 (m, 1 H), 3.83 - 3.93 (m, 1 H), 3.95 - 4.04 (m, 1 H), 4.02 - 4.13 (m, 2 H), 4.45 - 4.55 (m, 1 H), 9.58 (br s, 2 H)

### Stade C : (3S)-3-[(9aS)-4-Oxo-octahydropipérazino[2,1-c]morpholine-8-carbonyl]-1,2,3,4-tétrahydroisoquinoline-2-carboxylate de tert-butyle

De l'EDC (3,90 g, 20,3 mmol) est ajouté à une solution du composé du Stade B (3,02 g, 15,7 mmol), d'acide (3*S*)-2-[(*tert*-butoxy)carbonyl]-1,2,3,4-tétrahydroisoquinoline-3-carboxylique (4,6 g, 16,6 mmol), de triéthylamine (8,0 mL, 57,4 mmol) et de HOBt (2,72 g, 20,1 mmol) dans le dichlorométhane (150 mL). Le mélange est agité à la température ambiante pendant 21 h. Une solution aqueuse de HCl 1 N (105 mL) est ajoutée et le précipité formé est filtré sur Büchner. Les phases du filtrat sont séparées. La phase aqueuse est extraite avec du dichlorométhane (2 x 10 mL). Les phases organiques combinées sont lavées avec une solution aqueuse de HCl 3 N (35 mL), puis avec une solution aqueuse 5% de bicarbonate de potassium (2 x 35 mL) et enfin avec de la saumure (35 mL). La phase organique est séchée sur Na₂SO₄, filtrée puis concentrée sous pression réduite. Le produit est purifié par chromatographie sur gel de silice en utilisant l'acétate d'éthyle et l'heptane comme éluants pour obtenir le composé attendu sous la forme d'un solide.
**RMN ¹H** (400 MHz, CDCl₃) δ ppm: 1.38 - 1.57 (m, 9 H), 2.39 - 2.89 (m, 2 H), 2.89 - 3.34 (m, 3 H), 3.34 - 3.70 (m, 2 H), 3.90 - 4.06 (m, 1 H), 4.09 - 4.27 (m, 2 H), 4.30 - 5.00 (m, 5 H), 5.20 - 5.37 (m, 1 H), 7.03 - 7.24 (m, 4 H)

### Stade D: Trichlorhydrate de (3S)-3-[(9aS)-octahydropipérazino[2,1-c]morpholin-8-ylméthyl]-1,2,3,4-tétrahydroisoquinoline

Une solution de HCl 4 M dans le dioxane (45 mL, 180 mmol) est ajoutée au composé du Stade C (6,6 g, 46,0 mmol) refroidi dans un bain de glace. La suspension est ensuite agitée à température ambiante durant 24 h, puis elle est évaporée à sec. Le résidu est co-évaporé avec du MTBE, puis séché sous vide. Le solide ainsi obtenu est mis en suspension dans le tétrahydrofurane (160 mL), puis du LiAlH₄ (3,0 g, 79,1 mmol) est ajouté. La suspension est chauffée à reflux durant 6.5 h, puis elle est refroidie dans un bain de glace. De l'eau (3 mL) est ensuite ajoutée sur une période de 7 minutes. Au bout de 0.5 h, une solution aqueuse 2 N d'hydroxide de sodium (6 mL) est ajoutée. On y additionne à nouveau de l'eau (6 mL) 0.25 h plus tard. Enfin, de la Célite (7 g) et du Na₂SO₄ (25 g) sont ajoutés 0.5 h plus tard. La suspension est filtrée sur Célite et rincée avec du tétrahydrofurane (2 x 100 mL). Le filtrat est concentré à sec. L'huile ainsi obtenue est solubilisée dans le MTBE (50 mL). La solution résultante est filtrée et le filtrat concentré. Le résidu est solubilisé dans le méthanol (60 mL), puis une solution de HCl 4 M dans le dioxane (20 mL) est ajoutée. La solution est réchauffée à 40°C et traitée avec du charbon activé (0,66 g) sous agitation durant 1 h. La suspension est filtrée sur Célite et rincée avec du méthanol chaud. Le filtrat est concentré jusqu'à ce le produit commence à cristalliser. On laisse la cristallisation se poursuivre pendant 16 h à température ambiante. Le solide obtenu est filtré et rincé avec un mélange 2-propanol / MTBE (4/6) (2 x 20 mL), puis avec du MTBE(2 x 20mL). Après séchage, on obtient le composé attendu.
**RMN ¹H** (400 MHz, D₂O δ ppm: 2.28 - 2.44 (m, 1 H), 2.74 - 3.00 (m, 4 H), 3.08 - 3.27 (m, 3 H), 3.27 - 3.42 (m, 2 H), 3.43 - 3.56 (m, 2 H), 3.56 - 3.69 (m, 2 H), 3.76 - 3.95 (m, 2 H), 4.00 - 4.22 (m, 2 H), 4.35 - 4.50 (m, 2 H), 7.20 - 7.41 (m, 4 H)
**RMN ¹³C** (100 MHz, D₂O δ ppm: 29.00, 44.58, 50.30, 51.08, 51.17, 52.75, 53.17, 58.08, 61.60, 64.61, 66.37, 127.14, 127.71, 128.00, 128.77, 129.55, 131.15
**MS (ESI):** [M+H]⁺288.16

### Préparation 8' : (3S)-3-(1-Oxa-6-azaspiro[3.3]hept-6-ylméthyl)-1,2,3,4-tétrahydro isoquinoléine

### Stade A: (3S)-3-(Iodométhyl)-2-[(4-méthylphényl)sulfonyl]-1,2,3,4-tétrahydroisoquinoléine

Le composé du Stade A de la Préparation 6' (4,0 g; 8,48 mmol) dans l'acétonitrile (10 mL) est placé dans un tube à micro-ondes de 27 mL, puis de l'iodure de sodium (1,40 g; 9,33 mmol) est ajouté. Le milieu réactionnel est chauffé pendant 5 h à 100°C par micro-ondes (200 W). Il est ensuite filtré et le solide est lavé avec du dichlorométhane. Le filtrat est évaporé à sec, puis le résidu est purifié par chromatographie sur gel de silice en utilisant l'heptane et l'acétate d'éthyle comme éluants. Le composé du titre est obtenu sous la forme d'une huile.
**RMN ¹H** (400 MHz, dmso-d6) δ ppm: 7.64 (d, 2 H), 7.28 (d, 2 H), 7.15-7 (m, 4 H), 4.5/4.3 (2d, 2 H), 4.14 (m, 1 H), 3.22 (m, 2 H), 2.82 (m, 2 H), 2.31 (s, 3 H)
**IR (ATR)** cm⁻¹: 1897 ν -Ar, 1333 + 1156 ν -SO2

### Stade B : (3S)-2-[(4-Méthylphényl)sulfonyl]-3-(1-oxa-6-azaspiro[3.3]hept-6-ylméthyl)-1,2,3,4-tétrahydroisoquinoléine

Le composé iodé (2,5 g; 5,85 mmol) obtenu au stade précédent est dissous dans l'acétonitrile (50 mL). De l'oxalate de 1-oxa-6-azaspiro[3.3]heptane (1,21 g; 6,36 mmol) est ajouté, suivi de carbonate de potassium (1,61 g; 11,7 mmol). Le milieu réactionnel est chauffé 15 heures à reflux. Le milieu réactionnel est filtré et lavé avec de l'acétonitrile, puis évaporé à sec.
Le composé est purifié par chromatographie sur gel de silice en utilisant le dichlorométhane et le méthanol contenant de l'ammoniac comme éluants. Le composé du titre est obtenu sous la forme d'une huile.
**RMN ¹H** (400 MHz, dmso-d6) δ ppm: 7.68 (d, 2 H), 7.32 (d, 2 H), 7.14-7 (m, 4 H), 4.53/4.2 (dd, 2 H), 4.34 (t, 2 H), 3.95 (m, 1 H), 3.5/3.4/2.98 (3m, 4 H), 2.7 (t, 2 H), 2.68-2.58 (m, 2 H), 2.34 (s, 3 H), 2.31-2.24 (m, 2 H)
**IR (ATR)** cm⁻¹ : 1333 + 1156 ν -SO2

### Stade C : (3S)-3-(1-Oxa-6-azaspiro[3.3]hept-6-ylméthyl)-1,2,3,4-tétrahydroisoquinoléine

Le composé tosylé du stade précédent (1,3 g; 3,26 mmol) est dissous dans 10 mL de méthanol. Le magnésium en poudre (633 mg; 26,08 mmol) est ajouté par portion de 160 mg toutes les 3 heures. Le milieu réactionnel est agité dans un bain à ultrasons pendant 15 h. Il est ensuite filtré sur Célite, lavé abondamment avec du méthanol, puis évaporé à sec. Le composé est purifié par chromatographie sur gel de silice en utilisant le dichlorométhane et le méthanol contenant de l"ammoniac comme éluants. Le composé est obtenu sous la forme d'une huile.
**RMN ¹H** (400 MHz, dmso-d6) δ ppm : 7.01 (m, 4 H), 4.46 (t, 2 H), 3.85 (s, 2 H), 3.51/3.05 (dd, 2 H), 2.73 (t, 2 H), 2.61/2.4 (m, 4 H), 2.4 (m, 1 H), 2.4 (m, 1 H)
**IR (ATR)** cm⁻¹ : 3325 ν >NH

### Préparation 9' : Trichlorhydrate de (3S)-3-[(9aR)-octahydropipérazino[2,1-c]morpholin-8-ylméthyl]-1,2,3,4-tétrahydroisoquinoline

### Stade A : (9aR)-4-Oxo-octahydropipérazino[2,1-c]morpholine-8-carboxylate de tert-butyle

La synthèse de ce composé est décrite dans la littérature (J. Med. Chem. 2012, 55, 5887).

### Stade B : Chlorhydrate de (9aR)-octahydropipéraziuo[2,1-c]morpholin-4-one

Une solution de HCl 4 M dans le dioxane (39 mL, 154 mmol) est ajoutée au composé du Stade A (11,3 g, 44,1 mmol). La solution est ensuite agitée à température ambiante durant 5 h, puis une solution de HCl 4 M dans le dioxane (12 mL, 48 mmol) est ajoutée à nouveau. Le mélange est agité pendant 16 h. La solution est ensuite évaporée à sec pour donner le produit attendu sous la forme d'un solide.
**RMN ¹H** (400 MHz, dmso-d6) δ ppm: 2.79 - 2.93 (m, 2 H), 3.02 (td, *J* = 13.1, 2.7 Hz, 1 H), 3.22 - 3.34 (m, 2 H), 3.59 - 3.67 (m, 1 H), 3.86 - 3.96 (m, 1 H), 3.96 - 4.01 (m, 1 H), 4.05 (AB q, *J* = 13.3 Hz, 2 H), 4.48 (dd, *J* = 14.1, 2.5 Hz, 1 H), 9.71 (br. s, 1 H), 9.91 (br. s, 1 H)

### Stade C : (3S)-3-[(9aR)-4-Oxo-octahydropipérazino[2,1-c]morpholine-8-carbouyl]-1,2,3,4-tétrahydroisoquinoline-2-carboxylate de tert-butyle

De l'EDC (5,17 g , 27,0 mmol) est ajouté à une solution du composé du stade précédent (4,0 g, 20,7 mmol), d'acide (3*S*)-2-[(*tert*-butoxy)carbonyl]-1,2,3,4-tétrahydroisoquinoline-3-carboxylique (6,04 g, 21,8 mmol), de triéthylamine (11,6 mL, 83,1 mmol) et de HOBt (3,65 g, 27,0 mmol) dans le dichlorométhane (100 mL). Le mélange est agité à température ambiante pendant 16 h. Une solution aqueuse de HCl 1 M (70 mL) est ajoutée et le précipité formé est filtré sur Büchner. Les phases du filtrat sont séparées. La phase organique est lavée avec une solution aqueuse saturée en carbonate de potassium puis concentrée sous pression réduite. Le résidu est pré-absorbé sur gel de silice et purifié par chromatographie sur gel de silice en utilisant l'acétate d'éthyle et l'heptane comme éluants pour donner le composé attendu sous la forme d'un solide.
**RMN ¹H** (400 MHz, dmso-d6) δ ppm: 1.25 - 1.58 (m, 9 H), 2.50 - 2.76 (m, 2 H), 2.76 - 3.25 (m, 3 H), 3.37 - 3.76 (m, 2 H), 3.92 - 4.50 (m, 5 H), 4.06 (s, 2 H), 4.66 (d, *J* = 15.6 Hz, 1 H), 4.76 - 5.28 (m, 1 H), 7.05 - 7.31 (m, 4 H)

### Stade D : Trichlorhydrate de (3S)-3-[(9aR)-octahydropipérazino[2,1-c]morpholin-8-ylméthyl]-1,2,3,4-tétrahydroisoquinoline

Une solution de HCl 4 M dans le dioxane (24,0 mL, 96,2 mmol) est ajoutée à une solution du composé du Stade C (8,00 g, 12,25 mmol) dans le dichlorométhane (25 mL). Le mélange est agité à température ambiante pendant 16 h, puis est concentré à sec. Le produit brut obtenu est ajouté à une suspension de LiAlH₄ (1,97 g, 51,91 mmol) dans le tétrahydrofurane (140 mL). Le mélange est chauffé à reflux jusqu'à ce que la réaction (suivie par LC-MS) soit complète, puis il est refroidi à 0°C. De l'eau (2,5 mL) est ajoutée au goutte-à-goutte. Au bout de 10 minutes d'agitation, une solution aqueuse d'hydroxyde de sodium 2 M (5 mL) est ajoutée au goutte-à-goutte. De l'eau (5 mL) est de nouveau ajoutée après 10 minutes d'agitation. On additionne enfin de la Célite (4 g) et du Na₂SO₄ (12 g) après 10 minutes d'agitation supplémentaires. La suspension est filtrée sur Célite et le filtrat concentré à sec. Le résidu brut ainsi obtenu est solubilisé dans le méthanol (80 mL), puis une solution de HCl 4 M dans le dioxane (16.75 mL, 67.0 mmol) est ajoutée. Le mélange est agité à température ambiante pendant 3 h, puis concentré à sec. Le résidu est solubilisé dans un minimum de méthanol chaud (70 mL), puis du MTBE (3-5 mL) est ajouté. La solution est refroidie à 0°C pendant 1 h dans un bain d'eau glacée, et le produit précipite. Un peu de MTBE (2-3 mL) est de nouveau ajouté, puis le mélange est laissé pendant 1 h supplémentaire à 0°C. Le solide obtenu est filtré sur Büchner et séché sous vide pour donner le composé attendu sous la forme d'un solide.
**RMN ¹H** (400 MHz, CD₃OD δ ppm : 2.65 (t, *J* = 11.1 Hz, 1 H), 2.74 - 2.97 (m, 4 H), 3.10 (d, *J* = 12.5 Hz, 1 H), 3.19 (dd, *J* = 17.6, 4.8 Hz, 1 H), 3.25 - 3.55 (m, 5 H), 3.59 - 3.74 (m, 2 H), 3.82 - 4.15 (m, 4 H), 4.45 (AB q, *J* = 15.9 Hz, 2 H), 7.21 - 7.35 (m, 4 H).
**RMN ¹³C** (100 MHz, CD₃OD δ ppm: 30.21, 45.67, 50.22, 51.99, 52.90, 53.58, 53.71, 59.25, 62.58, 65.30, 67.07, 127.76, 128.40, 129.08, 129.36, 130.16, 131.95
**MS (ESI):** [M+H]⁺ 288.2

### Préparation 1" : N-[4-[tert-Butyl(diméthyl)silyl]oxyphényl]-1-méthyl-pyrazol-4-amine

### Stade A : 4-{[tert-Butyl(diméthyl)silyl]oxy}aniline

Le composé du titre est obtenu à partir de la 4-aminophénol dans le THF en présence d'imidazole et de chlorure de *tert*-butyl(diméthyl)silyl selon le protocole décrit dans la littérature (S. Knaggs et al, Organic & Biomolecular Chemistry, 3(21), 4002-4010; 2005**).**
**RMN ¹H :** δ (400 MHz ; dmso-d6 ; 300K) : 6.45-6.55 (dd, 4H, H aromatiques); 4.60 (m, 2H, **NH₂-**Ph) ; 0.90 (s, 9H, Si (CH₂)₂CH(**CH₃**)₂); 0.10 (s, 6H, Si (**CH₂**)₂CH(CH₃)₂)
**IR :** ν : -NH₂⁺ : 3300-3400 cm⁻¹

### Stade B : N-[4-[tert-Butyl(diméthyl)silyl]oxyphényl]-1-méthyl-pyrazol-4-amine

A une solution de 30,8g (0,137 mol) du composé du Stade A dans 525 mL de toluène anhydre, on ajoute successivement 29,8g de *tert*-butylate de sodium (0,310 mol), 4,55 g de Pd₂(dba)₃ (aussi appelé tris(dibenzylideneacétone)dipalladium(0)) (4,96 mmol), 4,81 g de 2-di-*tert*-butylphosphino-2',4',6'-tri-isopropyl-1,1'-biphényle (9,91 mmol) et 12,8 mL de 4-bromo-1-méthyl-1*H*-pyrazole (0,124 mol). L'ensemble est dégazé sous argon durant 30 mn puis chauffé à reflux pendant 3 h. On laisse refroidir. Le milieu réactionnel est concentré à sec, puis repris dans le dichlorométhane, filtré sur célite, puis de nouveau concentré à sec. Le résidu est alors purifié par chromatographie sur gel de silice en utilisant le dichlorométhane et l'acétate d'éthyle comme éluants pour fournir le produit attendu sous la forme d'un solide.
**RMN ¹H :** δ (400 MHz ; dmso-d6 ; 300K) : 7.55 (s, 1H, pyrazole); 7.23 (s, 1H, pyrazole); 7.18 (large s, 1H, **NH₂**-Ph) ;6.64 (m, 4H, H aromatiques); 3.77 (s, 3H, **CH₃**-pyrazole); 0.90 (s, 9H, Si (CH₂)₂CH(**CH₃**)₂); 0.12 (s, 6H, Si (**CH₂**)₂CH(CH₃)₂)
**IR :** ν -NH⁺ : 3275 cm⁻¹; ν Ar et C=N : 1577et 1502 cm⁻¹; ν -Si-C-: 1236 cm-¹ ν -Si-O-: 898 cm⁻¹ ; ν -Si-C-: 828, 774 cm⁻¹

### Préparation 2" : 4-{[tert-Butyl(diméthyl)silyl]oxy}-N-phénylaniline

A une solution de 12 g de 4-anilinophénol (64,7 mmol) dans 200 mL d'acétonitrile sont ajoutés à température ambiante 6,7 g d'imidazole (97,05 mmol) et 11,7 g de chlorure de *tert*-butyl(diméthyl)silyle (77,64 mmol). L'ensemble est mis sous agitation à 70°C pendant 4 heures. Puis, le milieu réactionnel est versé sur de l'eau et extrait avec de l'éther. La phase organique est ensuite séchée sur MgSO₄, puis filtrée et évaporée à sec. Le produit brut ainsi obtenu est alors purifié par chromatographie sur gel de silice en utilisant l'éther de pétrole et le dichlorométhane comme éluants. Le produit du titre est obtenu sous la forme d'une poudre.
**RMN¹H** : δ (400 MHz ; dmso-d6 ; 300K) : 7.84 (s, 1H NH) ; 7.17 (t, 2H aniline) ; 6.98 (d, 2H phénoxy) ; 6.94 (d, 2H aniline) ; 6.76 (d, 2H phénoxy) ; 6.72(t, 1H aniline) ; 0.95 (s, 9H *ter*t-butyl) ; 0.15 (s, 6H diméthyl)
**IR :** ν : >NH : 3403 cm⁻¹ ; ν :>Ar : 1597 cm⁻¹

### Préparation 3" : 5-[(4-{[tert-Butyl(diméthyl)silyl]oxy}phényl)amino-1H-indole-1-carboxylate de tert-butyle

On procède selon le procédé de la Préparation 1" en remplaçant le 4-bromo-1-méthyl-1*H-*pyrazole utilisé au Stade B par le 5-bromo-1*H*-indole-1-carboxylate de *tert*-butyle.
**RMN ¹H :** δ (400 MHz ; dmso-d6 ; 300K): 7.85 (d, 1H); 7.78 (s, 1H); 7.55 (d, 1H); 7.15 (d, 1H); 6.95 (m, 3H); 6.75 (d, 2H); 6.58 (d, 1H); 1.65 (s, 9H); 1.00 (s, 9H); 0.2 (s, 6H)

### Préparation 4" : N-(4{[tert-Butyl(diméthyl)silyl]oxy}phényl)-1-méthyl-1H-indol-5-amine

On procède selon le procédé de la Préparation 1" en remplaçant le 4-bromo-1-méthyl-1H-pyrazole utilisé au Stade B par le 5-bromo-1-méthyl-1*H*-indole.

### Préparation 5" : N-(4{[tert-Butyl(diméthyl)silyl]oxy}phényl)-1-méthyl-1H-indazol-5-amine

On procède selon le procédé de la Préparation 1" en remplaçant le 4-bromo-1-méthyl-1*H-*pyrazole utilisé au Stade B par le 5-bromo-1-méthyl-1*H*-indazole.

### Préparation 6" : N-(4-{[tert-Butyl(diméthyl)silyl]oxy}phényl)-3-fluoro-4-méthylaniline

On procède selon le procédé de la Préparation 1" en remplaçant le 4-bromo-1-méthyl-1*H-*pyrazole utilisé au Stade B par le 4-bromo-2-fluoro-1-méthylbenzène.

### Préparation 7": N-(4-{[tert-Butyl(diméthyl)silyl]oxy}phényl)-3-fluoroaniline

On procède selon le procédé de la Préparation 1" en remplaçant le 4-bromo-1-méthyl-1*H-*pyrazole utilisé au Stade B par le 1-bromo-3-fluorobenzène.

### Préparation 8" : 4-Benzyloxy-N-phényl-aniline

A une solution de 4-hydroxy-N-phényl-aniline (30 g ; 162 mmol) dans de l'acétonitrile (400 mL), on ajoute 58 g de Cs2CO3 (178 mmol) et on agite pendant 15 minutes à température ambiante. Le bromure de benzyle (22.5 mL ; 178 mmol) est ensuite ajouté au goutte-à-goutte puis le milieu réactionnel est chauffé au reflux pendant 4 h. Après filtration et rinçage à l'acétonitrile, le filtrat est concentré et purifié par chromatographie sur gel de silice en utilisant l'éther de pétrole et l'acétate d'éthyle comme éluants. Le produit du titre est alors obtenu sous la forme d'un solide incolore.
**RMN¹H** : δ (400 MHz ; dmso-d6 ; 300K) : 7.80 (m, 1H, NH) ; 7.45 (m, 2H, aryl) ; 7.40 (m, 2H, aryl) ; 7.30 (m, 1H, aryl) ; 7.15 (s, 2H, aryl); 7.05 (d, 2H, aryl) ; 6.9-7.0 (m, 4H, aryl) ; 6.70 (t, 1H, aryl) ; 5.05 (s, 2H, benzyle).
**IR :** ν : >NH : 3408 cm⁻¹

### Préparations 9" : N-(4-{[tert-Butyl(diméthyl)silyl]oxy}phényl)pyridin-4-amine

On procède selon le procédé de la Préparation 1" en remplaçant le 4-bromo-1-méthyl-1*H-*pyrazole utilisé au Stade B par la 4-bromopyridine.
**IR :** ν -NH- : 3200 et 2500 cm⁻¹; ν :-Si-O-: 902 cm⁻¹; ν :-Si-C-: 820 cm⁻¹

### Préparation 10": N-(4-{[tert-Butyl(diméthyl)silyl]oxy}phényl)-4-fluoroaniline

On procède selon le procédé de la Préparation 1" en remplaçant le 4-bromo-1-méthyl-1*H-*pyrazole utilisé au Stade B par le 1-bromo-4-fluorobenzène.

### Préparation 11" : N-(4-{[ter-Butyl(diméthyl)silyl]oxy}phényl)-1-méthyl-1H-pyrrolo[2,3-b1]pyridin-5-amine

On procède selon le procédé de la Préparation 1" en remplaçant le 4-bromo-1-méthyl-1*H-*pyrazole utilisé au Stade B par la 5-bromo-1-méthyl-1*H*-pyrrolo[2,3-*b*]pyridine (obtenue selon un protocole de la littérature : Heterocycles, 60(4), 865, 2003**).**
**IR :** ν :-NH- : 3278 cm⁻¹; ν :-C=C- aromatiques: 1605 cm⁻¹

### Préparation 12" : N-{4-{[tert-Butyl(diméthyl)silyl]oxy}phényl)-2-méthoxypyrimidin-5-amine

On procède selon le procédé de la Préparation 1" en remplaçant le 4-bromo-1-méthyl-1*H-*pyrazole utilisé au Stade B par la 5-bromo-2-méthoxypyrimidine.

### Préparation 13" : N-(4-{[tert-Butyl(diméthyl)silyl]oxy}phényl)-1-méthyl-2,3-dihydro-1H-purrolo[2,3-b]pyridin-5-amine

On procède selon le procédé de la Préparation 1" en remplaçant le 4-bromo-1-méthyl-1H-pyrazole utilisé au Stade B par la 5-bromo-1-méthyl-2,3-dihydro-1*H*-pyrrolo[2,3-*b*]pyridine.

### Préparation 14": N-(4-{[tert-Buty(diméthyl)silyl]oxy}phényl)-1-méthyl-1H-benzimidazol-5-amine

On procède selon le procédé de la Préparation 1" en remplaçant le 4-bromo-1-méthyl-1*H-*pyrazole utilisé au Stade B par le 5-bromo-1-méthyl-1*H*-benzimidazole.

### Préparation 15" : N¹-(4-{[tert-Butyl(diméthyl)silyl]oxy}phénil)-N²,N²-diméthylpyridine-2,4-diamine

On procède selon le procédé de la Préparation 1" en remplaçant le 4-bromo-1-méthyl-1*H-*pyrazole utilisé au Stade B par le 4-bromo-*N*,*N*-diméthylpyridin-2-amine.

### Préparation 16" : N-(4-{[tert-Butyl(diméthyl)silyl]oxy}phényl)pyrazolo[1,5-a]pyrimidin-6-amine

On procède selon le procédé de la Préparation 1" en remplaçant le 4-bromo-1-méthyl-1*H-*pyrazole utilisé au Stade B par le 6-bromopyrazolo[1,5-*a*]pyrimidine.
**IR :** ν -NH- : 3272 cm⁻¹; ν -C=N-: 1634 cm⁻¹; ν -C=C-: 1616 cm⁻¹

### Préparation17" : N-(4-{[tert-Butyl(diméthyl)silyl]oxyphényl]-1-méthil-pyrazolo[3,4-b]pyridin-5-amine

On procède selon le procédé de la Préparation 1" en remplaçant le 4-bromo-1-méthyl-1*H-*pyrazole utilisé au Stade B par la 5-bromo-1-méthyl-pyrazolo[3,4-*b*]pyridine (obtenue selon un protocole de la littérature : WO 2006/052568 à partir de la 2-méthyl-pyrazol-3-amine et du 2-bromopropanedial).

### Préparation 18" : 4-({4-[(tert-ButyldiméthyIsilyl)oxy]phény}amino)-1,5-diméthyl-1H-pyrrole-2-carbonitrile

### Stade A : 4-Bromo-1,5-diméthyl-1H-pyrrole-2-carbonitrile

Une solution de brome (6,58 mL, 0,13 mol) dans l'acide acétique (60 mL) est ajoutée goutte-à-goutte à l'aide d'une ampoule à addition une solution de 1,5-diméthyl-1H-pyrrole-2-carbonitrile (15,0 g, 0,12 mol) dans l'acide acétique (300 mL). L'ensemble est agité à la température ambiante pendant 24 h. Le mélange réactionnel est ensuite versé dans un bécher contenant 300 mL d'eau. Le solide formé est filtré et rincé avec de l'eau. Puis, il est solubilisé dans le dichlorométhane (300 mL) et la phase organique est lavée avec de la saumure, séchée sur Na₂SO₄, filtrée et concentrée sous vide pour donner le produit attendu sous la forme d'un solide.
**RMN ¹H** (CDCl₃) δ ppm : 2.25 (s, 3 H), 3.67 (s, 3 H), 6.74 (s, 1 H)

### Stade B : 4-({4-[(tert-Butyldiméthylsilyl)oxy]phényl}amino)-1,5-diméthyl-1H-pyrrole-2-carbonitrile

Une solution du composé du stade précédent (1,5 g, 7,53 mmol), de 4*-*[(*tert-*butyldiméthylsilyl)oxy]aniline (2,02 g, 9,04 mmol), de *tert*-butylate de sodium (1,45 g, 15,06 mmol) et de 2-di-*tert*-butylphosphino-2',4',6'-triisopropylbiphényle (0,13 g, 0,30 mmol) dans le toluène (20 mL) est purgée à l'azote. Le tris(dibenzylideneacétone)dipalladium(0) (0,28 g, 0,30 mmol) est ajouté, puis le mélange réactionnel est chauffé à 90°C jusqu'à ce que la réaction soit complète (suivi par CCM). Le chauffage est arrêté et on laisse le mélange revenir à la température ambiante. De l'eau (75 mL) est ajoutée et le mélange est extrait avec de l'acétate d'éthyle (3 x 75 mL). Les phases organiques réunies sont lavées avec de la saumure puis concentrées. Le produit brut est absorbé sur gel de silice et purifié par chromatographie sur gel de silice en utilisant l'acétate d'éthyle et l'heptane comme éluants). Le produit ainsi obtenu est solubilisé à chaud dans l'heptane et est laissé précipiter sous agitation à température ambiante, puis à 0°C. Le solide est filtré et l'opération est répétée sur le filtrat pour donner le composé attendu sous la forme d'un solide.
**RMN ¹H** (400 MHz, CDCl₃) δ ppm : 0.15 (s, 6 H), 0.97 (s, 9 H), 2.13 (s, 3 H), 3.66 (s, 3 H), 4.68 (br. s, 1 H), 6.49 (d, *J* = 8.5 Hz, 2 H), 6.64 (s, 1 H), 6.66 (d, *J* = 8.7 Hz, 2 H)
**RMN ¹³C** (100 MHz, CDCl₃ δ ppm: 4.34, 9.72, 18.30, 25.88, 32.94, 101.27, 114.37, 114.70, 116.41, 120.73, 124.52, 131.23, 141.54, 148.27
**MS (ESI+):** [M+H]⁺ mesuré : 342.3

### Préparation 19" : 4-[(4-{[tert-Butyl(diméthyl)silyl]oxy}phényl)amino]-1-méthyl-1H-pyrrole-2-carbonitrile

### Stade A : 1-Méthyl-1H-pyrrole-2-carbonitrile

Du *N*,*N*-diméthylformamide (3 mL) et du 1,4-diazabicyclo[2.2.2]octane (0,49 g, 4,3 mmol) sont ajoutés à une solution de pyrrole-2-carbonitrile (4 g, 43,4 mmol) dans le diméthylcarbonate (56 mL). La solution est agitée à 90°C pendant 15 h, puis chauffée à 110°C pendant 8 h. Le mélange est refroidi jusqu'à la température ambiante, puis de l'acétate d'éthyle (80 mL) est ajouté. Les phases sont séparées et la phase organique est lavée avec de l'eau (2 x 80 mL) et une solution aqueuse de HCl 1 N (1 x 80 mL). Les phases aqueuses combinées sont extraites à nouveau avec de l'acétate d'éthyle (1 x 80 mL). Les phases organiques combinées sont lavées avec de la saumure (1 x 80 mL), séchées sur MgSO₄, filtrées et concentrées sous vide pour obtenir le produit attendu sous la forme d'un liquide.
**RMN ¹H** (400 MHz, CDCl₃) δ ppm : 3.78 (m, 2 **H**), 6.12 - 6.18 (m, 1 H), 6.74 - 6.82 (m, 1 H)

### Stade B : 4-Bromo-1-méthyl-1H-pyrrole-2-carbonitrile

Du *N*-bromosuccinimide (6,2 g, 34,9 mmol) est ajouté à une solution de 1-méthyl-1*H-*pyrrole-2-carbonitrile (3,7 g, 34,9 mmol) dans le *N,N-*diméthylformamide (150 mL). La solution est agitée pendant 15 h à température ambiante. Une nouvelle quantité de *N-*bromosuccinimide (2,0 g, 11 mmol) est ajoutée et le mélange est agité pendant 3 h. De la silice (7 g) est ensuite ajoutée, puis la suspension est évaporée à sec. La pré-absorption sur silice est déposée sur une colonne de gel de silice et le produit est purifié par chromatographie sur gel de silice en utilisant l'acétate d'éthyle et l'heptane comme éluants pour obtenir le produit attendu sous la forme d'un solide.
**RMN ¹H** (400 MHz, CDCl₃ δ ppm : 3.77 (s, 3 H), 6.75 (d, *J* = 1.7 Hz, 1 H), 6.80 (d, *J* = 1.7 Hz, 1 H)

### Stade C : 4-[(tert-Butyldiméthylsilyl)oxy]phényl}amino)-1-méthyl-1H-pyrrole-2-carbonitrile

On laisse buller de l'azote pendant 5 minutes dans une solution de 4-bromo-1-méthyl-1H-pyrrole-2-carbonitrile (2,82 g, 15,2 mmol) et de 4-[(*tert*-butyldiméthylsilyl)oxy]aniline (4,08 g, 18,3 mmol) dans le toluène (55 mL). Du *tert*-butylate de sodium (2.92 g, 30.4 mmol), du tris(dibenzylideneacétone)dipalladium(0) (556 mg, 0,6 mmol) et du 2-di-*tert-*butylphosphino-2',4',6'-trüsopropylbiphényle (255 mg, 0.6 mmol) sont ensuite ajoutés au mélange réactionnel. Le milieu est agité 1 h à 80°C sous azote. La suspension est ensuite refroidie jusqu'à la température ambiante et filtrée sur Célite. Le tampon de Célite est ensuite rincé avec de l'acétate d'éthyle. Le filtrat est lavé avec de l'eau puis de la saumure. La phase organique est séchée sur MgSO₄, filtrée et concentrée sous vide. Le produit est purifié deux fois par chromatographie sur gel de silice en utilisant l'acétate d'éthyle et l'heptane comme éluants, puis ensuite par trituration dans l'heptane pour obtenir le produit attendu sous la forme d'un solide.
**RMN ¹H** (400 MHz, CDCl₃) δ ppm: 0.16 (s, 6 H), 0.97 (s, 9 H), 3.73 (s, 3 H), 6.57 (d, *J* = 1.9 Hz, 1 H), 6.64 - 6.66 (m, 1 H), 6.70 (s, 4 H); RMN
**RMN ¹³C** (100 MHz, CDCl₃ δ ppm: -4.48, 18.17, 25.72, 35.46, 103.01, 113.56, 113.69, 115.92, 119.55, 120.67, 129.04, 139.94, 148.85
**MS (ESI+):** [M+H]⁺ 328.25

### Préparation 20" : N-[4-[tert-Butyl(diméthyl)silyl]oxy-3-fluoro-phényl]-1-méthyl-1H-pyrazol-4-amine

On procède selon le protocole de la Préparation 1" en remplaçant le 4-aminophénol utilisé au Stade A par le 2-fluoro-4-aminophénol.
**RMN ¹H** (400 MHz, dmso-d6) δ ppm: 7.59 (sl, 1 H), 7.39 (m, 1 H), 7.24 (d, 1 H), 6.74 (dd, 1 H), 6.52 (dd, 1 H), 6.42 (ddd, 1 H), 3.76 (s, 3 H), 0.92 (s, 9 H), 0.1 (d, 6 H)

### Préparation 21" : 2-({4-[(tert-Butyldiméthylsilyl)oxy]phényl}amino)pyridine-4-carbonitrile

Une solution constituée de 2-bromo-4-pyridinecarbonitrile (5,00 g, 36,1 mmol), de 4-[(*tert-*butyldiméthylsilyl)oxy]aniline (8,06 g, 36,1 mmol), de *tert*-butylate de sodium (4,50 g, 46,9 mmol) et de 2-di-*tert*-butylphosphino-2',4',6'-triisopropylbiphényle (0,458 g, 1,08 mmol) dans le toluène (50 mL) est purgée avec de l'azote. Le tris(dibenzylideneacétone)dipalladium(0) (0,99 g, 1,08 mmol) est ensuite ajouté au mélange réactionnel, puis l'ensemble est chauffé à 50°C pendant 1.5 h. On laisse ensuite le milieu se refroidir jusqu'à la température ambiante. De l'eau est ajoutée et le mélange réactionnel est extrait avec de l'acétate d'éthyle (3 x 20 mL). Les phases organiques réunies sont lavées avec de la saumure, puis concentrées sous pression réduite. Le produit brut est absorbé sur gel de silice et purifié par chromatographie sur gel de silice en utilisant l'acétate d'éthyle et l'heptane comme éluants. Le produit obtenu est solubilisé à chaud dans l'heptane et précipite sous agitation à la température ambiante, puis à 0°C. Après filtration, on obtient le composé attendu sous la forme d'un solide.
**RMN ¹H** (400 MHz, CDCl₃ δ ppm : 0.22 (s, 6 H), 1.00 (s, 9 H), 6.61 (br. s, 1 H), 6.81 - 6.84 (m, 2 H), 6.84 - 6.89 (m, 2 H), 7.12 - 7.17 (m, 2 H), 8.26 (dd, *J* = 5.1, 0.9 Hz, 1 H) **RMN ¹³C** (100 MHz, CDCl₃) δ ppm : -4.29, 18.31, 25.78, 109.11, 114.73, 117.23, 121.17, 121.74, 124.93, 132.12, 149.79, 153.45, 158.00
**MS (ESI+):** [M+H]⁺ 326.19

### Préparation 22" : N-[4-[tert-Butyl(diméthyl)silyl]oxyphényl]-1-tétrahydrofuran-3-yl-pyrazol-4-amine

La 4-[*tert*-butyl(diméthyl)silyl]oxyaniline (0,92 g, 3,48 mmol) et le 4-iodo-1-tétrahydrofuran-3-yl-pyrazole (0,78 g, 3,48 mmol) en solution dans du tétrahydrofurane anhydre (20 mL) sont agités une heure à température ambiante en présence de *tert*-butylate de sodium (1,7 mL, solution 2M dans le THF) et de chloro(2-di-tert-butylphosphino-2',4',6'-trüsopropyl-1,1'-biphényl)[2-(2-aminoéthyl)phényl]palladium(II) (84 mg, 0,122 mmol). Le milieu réactionnel est filtré sur Célite puis évaporé à sec. Le résidu est cristallisé dans un mélange heptane/acétate d'éthyle, filtré et lavé à l'heptane puis purifié par chromatographie sur gel de silice en utilisant le dichlorométhane et le méthanol comme éluants pour conduire au produit attendu.
RMN **¹H** (500 MHz, dmso-d6, 300 K) δ ppm : 7.62 (d, 1H, pyrazole-H5), 7.29 (d, 1H, pyrazole-H3), 7.26 (s, 1H, NH), 6.68 (d, 2H, Ar-H), 6.64 (d, 2H, Ar-H), 4.93 (m, 1H, THF-3'H), 3.95 (m, 1H, THF-5'H), 3.94 (m, 1H, THF-2'H), 3.87 (m, 1H, THF-2'H), 3.80 (m, 1H, THF-5'H), 2.33 (m, 1H, THF-4'H), 2.27 (m, 1H, THF-4'H), 0.93 (s, 9H, ^{t}Bu), 0.12 (s, 6H, Me)
IR: ν C-H: 2857 cm⁻¹; ν aromatique: 1505 cm⁻¹; ν Si-C: 1249 cm⁻¹

### Préparation 23" : 6-({4-[(tert-Butyldiméthylsilyl)oxy]phényl}amino)pyridine-2-carbonitrile

Du 4-aminophénol (3,3 g, 30,2 mmol) est ajouté à une solution de 6-chloropyridine-2-carbonitrile (3,5 g, 25,3 mmol) dans le 1-méthyl-2-pyrrolidinone (70 mL). Le mélange réactionnel est chauffé à 140-150°C pendant 16 h dans un ballon scellé. Le tout est ensuite refroidi à la température ambiante. De l'imidazole (3,4 g, 49,9 mmol) et du chlorure de *tert*-butyl(diméthyl)silyle (7,6 g, 50,4 mmol) sont par la suite ajoutés et le mélange est agité pendant 16 h à la température ambiante. Le mélange est dilué avec de l'eau (140 mL) et le produit est extrait avec AcOEt (4 x 50 mL). Les phases organiques sont combinées et lavées avec de l'eau (3 x 50 mL), puis de la saumure (1 x 50 mL). La phase organique est ensuite séchée sur MgSO₄, filtrée et concentrée sous vide. Le produit brut est purifié par chromatographie sur gel de silice en utilisant l'acétate d'éthyle et heptane comme éluants pour obtenir le produit du titre.
**RMN ¹H** (400 MHz, CDCl₃ δ ppm : 0.20 (s, 6 H), 0.99 (s, 9 H), 6.74 (dd, *J* = 7.6, 4.9 Hz, 1 H), 6.81 - 6.88 (m, 3 H), 7.36 - 7.42 (m, 2 H), 7.74 (dd, *J* = 7.6, 1.9 Hz, 1 H), 8.34 (dd, *J* = 4.9, 1.9 Hz, 1 H)
**RMN ¹³C** (100 MHz, CDCl₃) δ ppm : -4.38, 18.21, 25.73, 92.58, 113.50, 116.53, 120.30, 123.20, 131.97, 141.67, 152.42, 152.45, 156.51
**MS (ESI):** [M+H]⁺ 326.24

### Préparation 24" : 4-({4-[(tert-Butyldiméthylsilyl)oxy]phényl}amino)pyrimidine-2-carbonitrile

### Stade A: N-{4-[(tert-Butyldiméthylsilyl)oxy]phényl)-2-chloropyrimidin-4-amine

Du 4-aminophénol (8,8 g, 80,6 mmol) et de la triéthylamine (18,6 mL, 133,4 mmol) sont ajoutés à une solution de 2,4-dichloropyrimidine (10,0 g, 67,1 mmol) dans l'éthanol (150 mL). Le mélange réactionnel est chauffé à 150°C pendant 14 h dans un ballon scellé. Le tout est ensuite refroidi à la température ambiante et le solvant est évaporé sous vide. Du dichlorométhane (200 mL) est ajouté au résidu, puis de l'imidazole (9,1 g, 133,7 mmol) et du chlorure de *tert*-butyl(diméthyl)silyle (12,1 g, 80,3 mmol) sont additionnés. Le mélange est agité pendant 15 h à la température ambiante. La réaction est diluée avec de l'eau (200 mL). Les phases sont séparées et la phase organique est lavée avec de la saumure (1 x 100 mL). Elle est ensuite séchée sur MgSO₄, filtrée et concentrée sous vide. Le résidu est purifié par chromatographie sur gel de silice en utilisant l'acétate d'éthyle et l'heptane comme éluants pour obtenir un solide. Ce dernier est trituré dans l'heptane, filtré et rincé à l'heptane pour donner le composé attendu sous la forme d'un solide.
**RMN ¹H** (400 MHz, dmso-d6) δ ppm: 0.22 (s, 6 H), 0.99 (s, 9 H), 6.42 (d, *J* = 5.9 Hz, 1 H), 6.81 (br. s, 1 H), 6.85 - 6.90 (m, 1 H), 7.13 (d, *J* = 8.7 Hz, 2 H), 8.07 (d, *J* = 5.9 Hz, 2 H)

### Stade B: 4-({4-[(tert-Butyldiméthylsilyl)oxy]phényl}amino)pyrimidine-2-carbonitrile

Du *N*,*N*-diméthylformamide anhydre (10 mL) est placé sous azote dans un ballon, puis composé du Stade A (670 mg, 2,0 mmol) est ajouté. Du cyanure de zinc (468 mg, 4,0 mmol) et du tétrakis(triphénylphosphine) palladium(0) (404 mg, 0,3 mmol) sont additionnés par la suite. On laisse buller de l'azote dans la solution pendant 5 min, puis le mélange réactionnel est agité à 120°C pendant 2 h sous atmosphère d'azote. La réaction, suivie par LC-MS, est complète. Le mélange est refroidi à la température ambiante, puis de l'eau (15 mL) y est ajoutée. Le produit est extrait avec de l'AcOEt (3 x 25 mL). Les phases organiques sont combinées et lavées avec de l'eau (4 x 25 mL), puis de la saumure (1 x 25 mL). La phase organique est séchée sur MgSO₄, filtrée et concentrée sous vide. Le résidu est purifié par chromatographie sur gel de silice en utilisant l'acétate d'éthyle et l'heptane comme éluants pour obtenir le composé attendu sous la forme d'un solide.
**RMN ¹H** (400 MHz, CDCl₃) δ ppm : 0.22 (s, 6 H), 0.99 (s, 9 H), 6.63 (d, *J* = 6.1 Hz, 1 H), 6.86 - 6.92 (m, 2 H), 7.03 (br. s, 1 H), 7.17 (d, *J* = 8.5 Hz, 2 H), 8.22 (d, *J* = 6.1 Hz, 1 H) **RMN ¹³C** (100 MHz, CDCl₃ δ ppm : -4.51, 18.10, 25.55, 106.32, 115.92, 121.00, 125.22, 129.73, 144.55, 154.16, 156.07, 161.56

### Préparation 25" : N-{4-[(tert-Butyldiméthylsilyl)oxy]phényl}-1-trideutériométhyl-1H-pyrazol-4-amine

### Stade A: 4-Bromo-1-trideutériométhyl-1H-pyrazole

Le 4-bromo-1*H*-pyrazole (9,05 g, 61,6 mmol) est ajouté par portions à une suspension de NaH (60% dans l'huile) (2,83 g, 70,8 mmol) dans du tétrahydrofurane (90 mL) refroidie dans un bain de glace. Après avoir retiré le bain de glace, la solution est agitée à la température ambiante pendant 0,5 h. Celle-ci est à nouveau refroidie dans un bain de glace et de l'iodométhane-*d*₃ (5,0 mL, 80,3 mmol) est ajouté. La solution est agitée à la température ambiante durant 19 h. La suspension est ensuite concentrée. Le résidu d'évaporation est trituré avec du MTBE (90 mL) et filtré. Le filtrat est concentré sous vide pour obtenir le composé attendu sous la forme d'une huile.
**RMN ¹H** (400 MHz, CDCl₃) δ ppm: 7.37 (s, 1 H), 7.43 (s, 1 H)

### Stade B: N-{4-[(tert-Butyldiméthylsilyl)oxy]phényl}-1-trideutériométhyl-1H-pyrazol-4-amine

Du 4-bromo-1-trideutériométhyl-1*H*-pyrazole (9,6 g, 58,5 mmol), de la *4-*[(*tert-*butyldiméthylsilyl)oxy]aniline (14,4 g, 64,6 mmol) et du toluène (150 mL) sont ajoutés dans un tricol de 500 mL. La solution est dégazée avec de l'azote durant 15 minutes, puis du *tert*-butylate de sodium (11,4 g, 0,12 mol), du 2-di-*tert*-butylphosphino-2',4',6'-triisopropylbiphényle (0,77 g, 1,81 mmol) et du tris(dibenzylideneacétone)dipalladium(0) (1,64 g, 1,79 mmol) sont successivement ajoutés. La suspension est chauffée à 85°C durant 1.5 h. Le mélange réactionnel est ensuite refroidi à la température ambiante et de l'eau (270 mL) est ajoutée. Le mélange est agité durant 30 minutes. Puis, on additionne de la Célite (30 g) et la suspension est filtrée sur un lit de Célite. Les phases du filtrat sont séparées et la phase aqueuse est extraite avec de l'acétate d'éthyle (3 x 200 mL). Les phases organiques combinées sont séchées sur Na₂SO₄et filtrées. De la silice (36 g) est ajoutée au filtrat, et le tout est évaporé à sec. Le produit est purifié par chromatographie sur gel de silice en utilisant l'acétate d'éthyle et l'heptane comme éluants. Le produit obtenu est recristallisé dans l'heptane (80 mL) pour obtenir le composé attendu.
**RMN ¹H** (400 MHz, CDCl₃) δ ppm: 0.16 (s, 6 H), 0.97 (s, 9 H), 4.92 (s, 1 H), 6.61 - 6.73 (m, 4 H), 7.25 (s, 1 H), 7.36 (s, 1 H)
**RMN ¹³C** (100 MHz, CDCl₃) δ ppm: -4.37, 18.28, 25.86, 38.67 (sept., ¹*J*_{C-D} = 21.0 Hz), 115.12, 120.73, 123.76, 126.52, 134.74, 141.07, 148.43
**MS (ESI):** [M+H]⁺ 307.08

### Préparation 26" : N-[4-[tert-Butyl(diméthyl)silyl]oxyphényl]-1-(oxétan-3-yl)-1H-pyrazol-4-amine

### Stade A: 4-Bromo-1-(oxétan-3-yl)-1H-pyrazole

Le 4-bromo-1*H*-pyrazole (1,53 g, 10,7 mmol) est dissous dans du diméthylformamide anhydre (15 mL). On y ajoute successivement du 3-bromooxétane (2,0 g, 14,6 mmol) et du carbonate de césium (4,7 g, 14 mmol). Le milieu réactionnel est chauffé pendant 8 heures à 130°C dans un flacon scellé. A la fin de la réaction, le solvant est évaporé sous vide et le résidu est purifié par chromatographie sur gel de silice en utilisant le dichlorométhane contenant de la diéthylamine et le méthanol comme éluants pour conduire au composé attendu.

### Stade B: N-[4-[tert-Butyl(diméthyl)silyl]oxyphényl]-1-(oxétan-3-yl)-1H-pyrazol-4-amine

De la 4-[*tert*-butyl(diméthyl)silyl]oxyaniline (1,5 g, 7,2 mmol) et du 4-bromo-1-(oxétan-3-yl)pyrazole (1,6 g, 7,2 mmol) en solution dans le tétrahydrofurane anhydre (25 mL) sont agités pendant 3 heures à température ambiante en présence de *tert*-butylate de sodium (3,7 mL, solution 2M dans le THF) et de chloro(2-di-*tert*-butylphosphino-2',4',6'-triisopropyl-1,1'-biphényl)[2-(2-aminoéthyl)phényl]palladium(II) (101 mg, 0,145 mmol). Le milieu réactionnel est filtré sur Célite, puis évaporé à sec. Le résidu est purifié par chromatographie sur gel de silice en utilisant le dichlorométhane contenant de la diéthylamine et l'acétate d'éthyle comme éluants pour conduire au produit attendu.
**RMN ¹H** (500 MHz, dmso-d6, 300 K) δ ppm: 7.76 (s, 1H, pyrazole-5'H), 7.40 (s, 1H, pyrazole-3'H), 7.34 (br s, 1H, NH), 6.70 (d, 2H, Ar-H), 6.65 (d, 2H, Ar-H), 5.49 (m, 1H, oxétane), 4.89 (d, 4H, oxétane), 0.93 (s, 9H, ^{t}Bu), 0.13 (s, 6H, Me)
**IR :** v: C-H: 2955 cm⁻¹; aromatique: 1505 cm⁻¹; Si-C: 1237 cm⁻¹

### Préparation 27" : Mélange de N-(4-{[tert-butyl(diméthyl)silyl]oxy}phényl)-1,5-diméthyl:1H-pyrazol-4-amine et de N-(4-{[tert-butyl(diméthyl)silyl]oxy}phényl)-1,3-diméthyl-1H-pyrazol-4-amine

### Stade A : Mélange de 4-bromo-1,5-diméthyl-1H-pyrazole et de 4-bromo-1,3-diméthyl-1H-pyrazole

A une suspension de NaH à 60% dans l'huile (0,3g ; 7.45 mmol) dans du tétrahydrofurane (150 mL) est ajouté à 10°C le 4-bromo-3-méthyl-1*H*-pyrazole en solution dans 15 mL de tétrahydrofuranne au goutte à goutte en 15 minutes. Après 40 minutes d'agitation à température ambiante, l'iodométhane (0,45 mL ; 7,45 mmol) est ajouté au goutte à goutte, puis le milieu réactionnel est laissé sous agitation une nuit. Après addition d'eau, le milieu réactionnel est évaporé et repris au dichlorométhane. La phase organique est séparée et séchée sur MgSO₄, filtrée et concentrée à sec. Le résidu est purifié par chromatographie sur gel de silice pour donner un mélange des composés du titre (4-bromo-1,3-diméthyl-pyrazole et de 4-bromo-1,5-diméthyl-pyrazole respectivement dans un rapport 4 :6).

### 4-bromo-1,5-diméthyl-1H-pyrazole:

**RMN ¹H** (500 MHz, dmso-d6) δ ppm: 7.41 (s, 1 H), 3.76 (s, 3 H), 2.22 (s, 3 H)
**4-bromo-1,3-diméthyl-1*H*-pyrazole** :
**RMN ¹H** (500 MHz, dmso-d6) δ ppm: 7.81 (s, 1 H), 3.74 (s, 3 H), 2.09 (s, 3 H)

### Stade B : Mélange de N-(4-{[tert-butyl(diméthyl)silyl]oxy}phényl)-1,5-diméthy1-1H-pyrazol-4-amine et de N-(4-{[tert-butyl(dimethyl)silyl]oxy}phenyl)-1,3-dimethyl-1H-pyrazol-4-amine

On procède selon le Stade B de la Préparation 1" en remplaçant le 4-bromo-1-méthyl-1*H-*pyrazole par le mélange d'isomères issu du Stade A. On obtient un mélange d'isomères dans un rapport 4 :6 (respectivement *N*-(4-{[*tert*-butyl(diméthyl)silyl]oxy}phényl)-1,5-diméthyl-1*H*-pyrazol-4-amine et de *N*-(4-{[*tert*-butyl(dimethyl)silyl]oxy}phenyl)-1,3-dimethyl-1*H*-pyrazol-4-amine).

### Préparation 28": N-[4-[tert-Butyl(diméthyl)silyl]oxyphényl]-1-cyclopropyl-1H-pyrazol-4-amine

### Stade A : 4-Bromo-1-cyclopropyl-1H-pyrazole

Le 4-bromo-1*H*-pyrazole (1,76 g, 12 mmol) est dissous dans du diméthylformamide anhydre (15 mL). On y ajoute successivement le bromure de cyclopropyle (2,9 mL, 36 mmol) et le carbonate de césium (7,8 g, 24 mmol). Le milieu réactionnel est chauffé pendant 15 heures à 160°C dans un flacon scellé. A la fin de la réaction, le solvant est évaporé sous vide et le résidu est purifié par chromatographie sur gel de silice en utilisant l'heptane et le dichlorométhane comme éluants pour conduire au composé attendu.

### Stade B : N-[4-[tert-Butyl(diméthyl)silyl]oxyphényl]-1-cyclopropyl-1H-pyrazol-4-amine

Le 4-[*tert*-butyl(diméthyl)silyl]oxyaniline (1,64 g, 7,3 mmol) et le 4-bromo-1-cyclopropyl-1*H*-pyrazole (1,4 g, 7,3 mmol) en solution dans du tétrahydrofurane anhydre (30 mL) sont agités pendant 3 h à température ambiante en présence de *tert*-butylate de sodium (3,7 mL, solution 2M dans le THF) et de chloro(2-di-*tert*-butylphosphino-2',4',6'-trüsopropyl-1,1'-biphényl)[2-(2-aminoéthyl)phényl]palladium(II) (101 mg, 0,146 mmol). Le milieu réactionnel est filtré sur Célite, puis évaporé à sec. Le résidu est purifié par chromatographie sur gel de silice en utilisant l'heptane et l'acétate d'éthyle comme éluants pour conduire au produit attendu.
**RMN ¹H** (500 MHz, dmso-d6, 300 K) δ ppm: 7.61 (s, 1H, pyrazole-5'H), 7.23 (s, 1H, pyrazole-3'H), 7.22 (br s, 1H, NH), 6.65 (d, 2H, Ar-H), 6.63 (d, 2H, Ar-H), 3.64 (m, 1H, Cp-H), 1.00 - 0.91 (m, 4H, Cp), 0.93 (s, 9H, ^{t}Bu), 0.12 (s, 6H, Me)
**IR :** v: C-H: 2930 cm⁻¹; aromatique: 1504 cm⁻¹; Si-C: 1237 cm⁻¹

### Masse haute résolution (ESI+) :

Formule brute : C₁₈H₂₇N₃OSi
[M+H]⁺ calculé : 330.2003
[M+H]⁺ mesuré : 330.1989

### Préparation 29" : 1,5-Diméthyl-4-(phénylamino)-1H-pyrrole-2-carbonitrile

On procède selon le protocole de la Préparation 1" en remplaçant le 4-{[*tert-*butyl(diméthyl)silyl]oxy}aniline utilisé au Stade B par l'aniline.
**RMN ¹H** (400 MHz, dmso-d6) δ ppm: 7.19 (s, 1 H), 7.05 (t, 2 H), 6.79 (s, 1 H), 6.6 (m, 3 H), 3.61 (s, 3 H), 2.1 (s, 3 H)

### Préparation 30" : 1-Méthyl-N-phényl-1H-pyrrolo[2,3-b]pyridin-6-amine

On procède selon le protocole du Stade B de la Préparation 1" en utilisant l'aniline et la 5-bromo-1-méthyl-1*H*-pyrrolo[2,3-*b*]pyridine (obtenue selon un protocole de la littérature : Heterocycles, 60(4), 865, 2003**).**
**RMN ¹H** (400 MHz, dmso-d6) δ ppm: 8.1 (d, 1 H), 7.9 (s, 1 H), 7.7 (d, 1 H), 7.45 (d, 1 H), 7.17 (t, 2 H), 6.9 (d, 2 H), 6.7 (t, 1 H), 6.38 (d, 1 H), 3.8 (s, 3 H)

### Préparation 31" : N-(4-{[tert-Butyl(diméthyl)silyl]oxy}phényl)-1-trideutériométhyl-1H-pyrrolo[2,3-b]pyridin-5-amine

On procède selon le procédé de la Préparation 1" en remplaçant le 4-bromo-1-méthyl-1*H-*pyrazole utilisé au Stade B par la 5-bromo-1-(trideutériométhyl)-1*H*-pyrrolo[2,3-*b*]pyridine (obtenue selon un protocole de la littérature Heterocycles, 60(4), 865, 2003 en remplaçant l'iodure de méthyle par l'iodure de méthyle trideutéré).
**RMN ¹H** (400 MHz, dmso-d6) δ ppm: 8.05 (d, 1 H), 7.6 (m+d, 2 H), 7.4 (d, 1 H), 6.85/6.7 (2d, 4 H), 6.3 (d, 1 H), 0.95 (s, 9 H), 0.15 (s, 6 H)

### Préparation 32" : 4-({4-[(tert-Butyldiméthylsilyl)oxy]phényl}amino)-1-trideutériométhyl-1H-pyrrole-2-carbonitrile

### Stade A : 1-Trideutériométhyl-1H-pyrrole-2-carbonitrile

NaH (60% dans l'huile) (2,61 g, 65,2 mmol) est mis en suspension dans du tétrahydrofurane (110 mL) à 0°C. Du 1*H*-pyrrole-2-carbonitrile (5 g, 54,3 mmol) est ajouté goutte-à-goutte sur 10 minutes. Le milieu réactionnel est ensuite réchauffé jusqu'à la température ambiante sur 30 minutes. Il est de nouveau refroidi à 0°C, puis de l'iodométhane-*d*₃ (10,23 g, 70,6 mmol) est ajouté. La réaction est agitée pendant 16 h sous azote à la température ambiante. Le milieu réactionnel est ensuite évaporé sous pression réduite, puis de l'acétate d'éthyle (200 mL) et de l'eau (200 mL) sont ajoutés. Les phases sont séparées, et la phase aqueuse est extraite avec de l'acétate d'éthyle (2 x 200 mL). Les phases organiques combinées sont lavées avec de la saumure (1 x 80 mL), séchées sur MgSO₄, filtrées et concentrées sous vide. Le résidu est purifié par chromatographie sur gel de silice en utilisant l'acétate d'éthyle et l'heptane comme éluants. Les fractions sont combinées et évaporées sous vide pour obtenir le composé attendu.
**RMN ¹H** (400 MHz, CDCl₃) δ ppm : 6.13 (dd, *J* = 2.7, 3.9 Hz, 1 H), 6.75 (dd, *J* = 1.5, 4.1 Hz, 1 H), 6.79 (dd, *J* = 1.7, 2.6 Hz, 1 H)

### Stade B : 4-Bromo-1-trideutériométhyl-1H-pyrrole-2-carbonitrile

Du N-bromosuccinimide (6,68 g, 37,5 mmol) est ajouté à une solution du composé du stade précédent (4,09 g, 37,5 mmol) dans le *N*,*N*-diméthylformamide (188 mL). La solution est agitée pendant 16 h à température ambiante- Le résidu est purifié par chromatographie en utilisant l'acétate d'éthyle et l'heptane comme éluants pour obtenir le composé attendu sous la forme d'un solide.
**RMN ¹H** (400 MHz, CDCl₃) δ ppm : 6.75 (d, *J* = 1.7 Hz, 1H), 6.80 (d, *J* = 1.7 Hz, 1 H)

### Stade C : 4-({4-[(tert-Butyldiméthylsilyl)oxy]phényl}amino)-1-trideutériométhyl-1H-pyrrole-2-carbonitrile

On laisse buller de l'azote pendant 5 minutes dans une solution constituée du composé obtenu au stade précédent (5,85 g, 31,1 mmol), de 4-[(*tert*-butyldiméthylsilyl)oxy]aniline (8 g, 35,8 mmol), de *tert*-butylate de sodium (3,88 g, 40,4 mmol) et de 2-di-*tert-*butylphosphino-2',4',6'-trüsopropylbiphényle (662 mg, 1,56 mmol) dans le toluène (260 mL). Du tris(dibenzylideneacétone)dipalladium(0) (1,43 g, 1,56 mmol) est ensuite ajouté. Le mélange est agité pendant 1 h à 70°C sous azote. La suspension est ensuite refroidie jusqu'à la température ambiante, diluée avec de l'acétate d'éthyle et filtrée sur Célite. Le tampon de Célite est ensuite rincé avec de l'acétate d'éthyle. Le filtrat est lavé avec de l'eau (3 fois), puis de la saumure (1 fois). La phase organique est séchée sur Na₂SO₄, filtrée et concentrée sous vide. Le produit est purifié deux fois par chromatographie sur gel de silice en utilisant l'acétate d'éthyle et l'heptane comme éluants, puis ensuite par chromatographie en phase inverse en utilisant le méthanol et l'eau comme éluants pour obtenir le composé attendu sous la forme d'une poudre.
**RMN ¹H** (400 MHz, CDCl₃) δ ppm: 0.17 (s, 6 H), 0.98 (s, 9 H), 4.96 (br. s, 1 H), 6.57 (d, *J* = 1.9 Hz, 1 H), 6.64 (d, *J* = 1.8 Hz, 1 H), 6.70 (br. s, 4 H)
**RMN ¹³C** (100 MHz, CDCl₃ δ ppm: -4.38, 18.26, 25.83, 34.83, 102.96, 113.69, 113.71, 115.95, 119.58, 120.75, 129.14, 140.10, 148.84
**MS (ESI):** [M+H]⁺ 331.09

### Préparation 33" : 4-({4-[(tert-Butyldiméthylsilyl)oxy]phényl}amino)-1-trideutériométhyl-5-méthyl-1H-pyrrole-2-carbonitrile

### Stade A : 5-Méthyl-1Hpyrrole-2-carbonitrile

Le composé est préparé selon le protocole décrit dans Heterocycles 2011, 82, 1503.

### Stade B : 1-Trideutériométhyl-5-méthyl-1H-pyrrole-2-carbonitrile

Une solution de 5-méthyl-1*H*-pyrrole-2-carbonitrile (0,30 g, 2,82 mmol) dans du *N,N-*diméthylformamide (5 mL) est refroidie à 0°C. NaH (60% dans l'huile) (0,118 g, 2,96 mmol) est ajouté par portions, et le mélange réactionnel est agité à 0°C pendant 30 minutes. De l'iodométhane-*d*₃ (4,15 mL, 67,2 mmol) est ajouté en une seule portion, et le mélange réactionnel est agité à température ambiante jusqu'à ce que la réaction soit complète. Il est ensuite dilué avec de l'eau (30 mL) et de l'acétate d'éthyle (15 mL). Les phases sont séparées et la phase aqueuse est extraite une seconde fois avec de l'acétate d'éthyle (15 mL). Les phases organiques réunies sont lavées avec de l'eau (1 x 50 mL), puis de la saumure, et séchées sur Na₂SO₄. Après filtration et concentration sous pression réduite, le résidu est purifié par chromatographie sur gel de silice en utilisant l'acétate d'éthyle et l'heptane comme éluants pour donner le composé attendu sous la forme d'un solide.
**RMN ¹H** (400 MHz, CDCl₃) δ ppm : 2.25 (s, 3 H), 5.91 (dd, *J* = 3.9, 0.6 Hz, 1 H), 6.69 (d, *J* = 3.9 Hz, 1 H)

### Stade C : 4-Bromo-1-trideutériométhyl-5-méthyl-1H-pyrrole-2-carbonitrile

Une solution de brome (0,133 mL, 2,60 mmol) dans l'acide acétique (1,5 mL) est ajoutée goutte-à-goutte à une solution du composé obtenu au stade précédent (0,305 g, 2,48 mmol) dans l'acide acétique (5.5 mL) préalablement refroidie à 0°C. Le mélange réactionnel est agité et réchauffé progressivement jusqu'à la température ambiante sur une période de 20 h. Le mélange réactionnel est versé dans l'eau (50 mL) et le mélange est extrait avec du dichlorométhane (2 x 50 mL). La phase organique est séchée sur Na₂sO₄, filtrée et concentrée sous pression réduite pour donner le composé attendu sous la forme d'un solide.
**RMN ¹H** (400 MHz, CDCl₃) δ ppm : 2.24 (s, 3 H), 6.73 (s, 1 H)

### Stade D : 4-({4-[(tert-Butyldiméthylsilyl)oxy]phényl}amino)-1-trideutériométhyl-5-méthyl-1H-pyrrole-2-carbonitrile

Une solution constituée du composé obtenu au stade précédent (7,00 g, 34,6 mmol), de 4-[(*tert*-butyldiméthylsilyl)oxy]aniline (8,90 g, 39,8 mmol), de *tert*-butylate de sodium (4,33 g, 45,0 mmol) et de 2-di-*tert*-butylphosphino-2',4',6'-triisopropylbiphényle (0,441 g, 1,04 mmol) dans le toluène (70 mL) est purgée avec de l'azote. Du tris(dibenzylideneacétone)dipalladium(0) (0,951 g, 1,04 mmol) est ajouté, puis le mélange réactionnel est chauffé à 65°C jusqu'à ce que la réaction, suivie par CCM, soit complète. Le chauffage est arrêté et le mélange est refroidi jusqu'à la température ambiante. De l'eau (200 mL) est ajoutée, et le mélange est extrait avec de l'acétate d'éthyle (3 fois). Les phases organiques réunies sont lavées avec de la saumure, puis concentrées. Le produit brut est purifié par chromatographie sur gel de silice en utilisant l'acétate d'éthyle et l'heptane comme éluants. Le produit obtenu est solubilisé à chaud dans l'heptane ; on le laisse précipiter à température ambiante, puis à 0°C pour donner produit attendu sous forme de cristaux.
**RMN ¹H** (400 MHz, CDCl₃) δ ppm: 0.15 (s, 6 H), 0.96 (s, 9 H), 2.12 (s, 3 H), 4.66 (br. s, 1 H), 6.46 - 6.51 (m, 2 H), 6.64 (s, 1 H), 6.64 - 6.69 (m, 2 H)
**RMN ¹³C** (100 MHz, CDCl₃ δ ppm: -4.37, 9.64, 18.26, 25.84, 31.62 - 32.87 (m), 101.14, 114.35, 114.66, 116.33, 120.68, 124.51, 131.17, 141.53, 148.18
**MS (ESI):** [M+H]⁺ 345.13

### Préparation 34" : 5-[(4-{[tert-Butyl(diméthyl)silyl]oxy}phényl)amino]-1-méthyl-1H-pyrazole-3-carbonitrile

### Stade A : 5-Amino-1-méthyl-1H-pyrazole-3-carbonitrile

A une suspension de 1-méthyl-5-nitro-1*H*-pyrazole-3-carbonitrile (2 g ; 13,1 mmol) dans un mélange d'eau (14 mL) et d'éthanol (120 mL) sont ajoutés du HCl à 37% (170 µL) et de la limaille de fer (5,1 g ; 91 mmol). Le milieu réactionnel est chauffé pendant 5 h à 50°C. Après refroidissement jusqu' à la température ambiante, le milieu réactionnel est filtré. Le filtrat est concentré à sec, puis purifié par chromatographie sur gel de silice en utilisant le dichlorométhane et l'acétate d'éthyle comme éluants pour conduire au composé attendu sous la forme d'un solide.
**RMN ¹H** (400 MHz, dmso-d6) δ ppm: 5.8 (s, 1 H), 5.7 (m, 2 H), 3.6 (s, 3 H)

### Stade B: 5-[(4-{[tert-Butyl(diméthyl)silyl]oxy}phényl)amino]-1-méthyl-1H-pyrazole-3-carbonitrile

On procède selon le protocole du Stade B de la Préparation 1 " en remplaçant le 4-bromo-1-méthyl-1*H*-pyrazole par la (4-bromophénoxy)-(*tert*-butyl)diméthyl-silane et la 4-{[*tert-*butyl(diméthyl)silyl]oxy}aniline par le composé issu du Stade A.
**RMN ¹H** (400 MHz, dmso-d6) δ ppm: 8.15 (s, 1 H), 6.9 (d, 2 H), 6.75 (d, 2 H), 6.45 (s, 1 H), 3.75 (s, 3 H), 0.95 (s, 9 H), 0.15 (s, 6 H)

### Préparation 35": 4-[(4-{[tert-Butyl(diméthyl)silyl]oxy}phényl)amino]-5-méthyl-1-[2-(morpholin-4-yl)éthyl]-1H-pyrrole-2-carbonitrile

On procède selon le procédé de la Préparation 33" en remplaçant au Stade B l'iodométhane-*d*₃ par le chlorhydrate de 2-(chloroéthyl)morpholine.
**RMN ¹H** (400 MHz, dmso-d6) δ ppm: 6.85 (s, 1 H), 6.75 (s, 1 H), 6.6 (d, 2 H), 6.5 (d, 2 H), 4.1 (t, 2 H), 3.55 (t, 4 H), 2.6 (t, 2 H), 2.4 (t, 4 H), 2.1 (s, 3 H), 0.9 (s, 9 H), 0.1 (s, 6 H)

### Préparation 36" : N-(4-{[tert-Butyl(diméthyl)silyl]oxy}phényl)-2-[2-(morpholin-4-yl)éthoxy]pyrimidin-5-amine

### Stade A : 4-[2-(5-Bromopyrimidin-2-yl)oxyéthyl]morpholine

NaH (1,0 g, 25,0 mmol, 60% dans l'huile) en suspension dans du tétrahydrofurane anhydre est refroidi à 0°C dans un bain de glace sous argon, puis le 2-morpholinoéthanol (2,7 g, 20,7 mmol) est ajouté goutte à goutte. Le bain de glace est retiré et la suspension est agitée pendant 1heure à température ambiante. Puis, le 5-bromo-2-chloro-pyrimidine (4,0 g, 20,7 mmol) est ajouté à température ambiante et la réaction est agitée pendant 16 heures à température ambiante. Une solution aqueuse saturée en chlorure d'ammonium (10 mL) et de l'eau (10 mL) sont ajoutés au milieu réactionnel ; le pH est ajusté à 9 par addition d'une solution aqueuse saturée en NaHCO₃. Cette solution est extraite 3 fois avec de l'acétate d'éthyle, la phase organique est ensuite lavée avec de la saumure, séchée sur MgSO₄, puis évaporée à sec. Le composé attendu précipite par addition d'éther de pétrole.
**RMN ¹H** (400 MHz, dmso-d6) δ ppm: 8.75 (s, 2 H), 4.4 (t, 2 H), 3.55 (t, 4 H), 2.7 (t, 2 H), 2.45 (t, 4 H)
**IR (ATR)** cm⁻¹: 1562 ν >C=C< et C=N, 787 ν -C-H Ar

### Stade B : N-(4-{[tert-Butyl(diméthyl)silyl]oxy}phényl)-2-[2-(morpholin-4-yl)éthoxy]pyrimidin-5-amine

On procède selon le protocole du Stade B de la Préparation 1 " en remplaçant le 4-bromo-1-méthyl-1*H*-pyrazole par le composé issu du Stade A.
**RMN ¹H** (400 MHz, dmso-d6) δ ppm: 8.3 (s, 2 H), 7.8 (s, 1 H), 6.9/6.75 (2d, 4 H), 4.35 (t, 2 H), 3.55 (t, 4 H), 2.7 (t, 2 H), 2.45 (t, 4 H), 0.95 (s, 9 H), 0.15 (s, 6 H)
**IR (ATR)** cm⁻¹: 3300 ν -NH, 1506 ν -NH, 837 ν -Si-Me, 837 et 778 ν -CH Ar

### Préparation 37": 4-({4-[(tert-Butyldiméthylsilyl)oxy]phényl}amino)-1,3-diméthyl-1H-pyrrole-2-carbonitrile

### Stade A : 4-Méthyl-N-(prop-2-én-1-yl)beuzène-1-sulfonamide

De l'allylamine (10,6 mL, 0,14 mol) est ajoutée sur une période de 4 minutes à une solution de chlorure de tosyle (25,1 g, 0,13 mol) dans le dichlorométhane (250 mL) refroidie dans un bain de glace. De la triéthylamine (24 mL, 0,18 mol) est additionnée, puis la solution est agitée à la température ambiante durant 1.25 h. Une solution aqueuse de HCl 3 N (60 mL) est ajoutée puis les phases sont séparées. La phase organique est lavée avec un autre volume de solution aqueuse de HCl 3 N (60 mL), puis avec une solution aqueuse 5% de bicarbonate de sodium (60 mL). La phase organique est séchée sur Na₂sO₄, filtrée et concentrée pour obtenir le composé attendu sous la forme d'un solide.
**RMN ¹H** (400 MHz, CDCl₃) δ ppm : 2.43 (s, 3 H), 3.54 - 3.63 (m, 2 H), 4.50 (1s, 1 H), 5.05 - 5.22 (m, 2 H), 5.66 - 5.79 (m, 1H), 7.31 (d, *J* = 8.1 Hz, 2 H), 7.76 (d, *J* = 8.1 Hz, 2 H)

### Stade B : 4-Méthyl-N-(2-méthylprop-2-én-1-yl)-N-(prop-2-én-1-yl)benzène-1-sulfonamide

Du 3-chloro-2-méthylpropene (20 mL, 0,20 mol) est ajouté sur une période de 5 minutes à une suspension de 4-méthyl-*N*-(prop-2-én-1-yl)benzène-1-sulfonamide (27,9 g, 0,13 mol) et de carbonate de potassium (28,1 g, 0,20 mol) dans le *N,N*-diméthylformamide (200 mL) refroidie dans un bain de glace. Au bout de 20 minutes, la suspension est agitée à température ambiante durant 18 h. La suspension est concentrée à sec. Le résidu est repris avec de l'acétate d'éthyle (250 mL) et de l'eau (110 mL). La phase aqueuse est extraite avec de l'acétate d'éthyle (2 x 50 mL). Les phases organiques combinées sont lavées successivement avec une solution aqueuse de HCl 3 N (50 mL), de l'eau (3 x 50 mL), une solution aqueuse à 5% de bicarbonate de potassium (50 mL), et enfin de la saumure (50 mL). La phase organique est séchée sur Na₂SO₄, filtrée et concentrée pour obtenir le composé attendu sous la forme d'une huile.
**RMN ¹H** (400 MHz, CDCl₃) δ ppm : 1.69 (s, 3 H), 2.43 (s, 3 H), 3.70 (s, 2H), 3.75 - 3.79 (m, 2 H), 4.87 (d, *J* = 25.1 Hz, 1 H), 5.04 - 5.09 (m, 1 H), 5.09 - 5.12 (m, 1 H), 5.45 - 5.59 (m, 1H), 7.29 (d, *J* = 8.1 Hz, 2 H), 7.71 (d, *J* = 8.1 Hz, 2 H)

### Stade C : 3-Méthyl-1-(4-méthylbenzènesulfonyl)-1H-pyrrole

Une solution du composé obtenu au Stade B (15,2 g, 57,3 mmol) dans le toluène (550 mL) est chauffée à 80°C en présence de (1,3-bis(2,4,6-triméthylphényl)-2-imidazolidinylidene)dichloro(phénylméthylene)(tricyclohexylphosphine) ruthénium (catalyseur de Grubbs, 2^{e} génération) (150 mg, 0,18 mmol) durant 1 h. Puis, du 2,3-dichloro-5,6-dicyano-*p*-benzoquinone (16,1 g, 70,9 mmol) est ajouté en une portion, et la solution est chauffée à 80°C durant 24 h. La solution est filtrée sur Célite, et le filtrat est concentré sous vide. Le produit est purifié par chromatographie sur gel de silice en utilisant l'acétate d'éthyle et l'heptane comme éluants pour obtenir le composé attendu sous la forme d'un solide.
**RMN ¹H** (400 MHz, CDCl₃) δ ppm: 2.02 (d, *J* = 1.0 Hz, 3 H), 2.39 (s, 3 H), 6.09 - 6.13 (m, 1H), 6.85 - 6.90 (m, 1 H), 7.03 - 7.07 (m, 1 H), 7.27 (d, *J* = 8.4 Hz, 2 H), 7.72 (d, *J* = 8.4 Hz, 2 H)

### Stade D : 3-Méthyl-1-(4-méthylbenzènesulfonyl)-1H-pyrrole-2-carbonitrile

Du chlorure d'aluminum (19,4 g, 0,15 mol) est ajouté d'une seule traite à une solution de 3-méthyl-1-(4-méthylbenzènesulfonyl)-1*H*-pyrrole (13,0 g, 55,3 mmol) dans le 1,2-dichloroéthane (230 mL) à température ambiante. Après 20 minutes d'agitation, du bromure cyanique (11,11 g, 0,10 mol) est ajouté par portions sur une période de 20 minutes. Après 4.5 h, une quantité supplémentaire de bromure cyanique (1,94 g, 18,3 mmol) est ajoutée. Après 17 h d'agitation à température ambiante, le milieu réactionnel est versé lentement dans un mélange de dichlorométhane (300 mL) et d'eau (600 mL) refroidi à 0°C. Le mélange résultant est agité durant 1 h. Par la suite, les phases sont séparées et la phase aqueuse est extraite avec du dichlorométhane (3 x 150 mL). Les phases organiques combinées sont lavées avec de l'eau (2 x 150 mL) et de la saumure (150 mL). La phase organique est séchée sur Na₂SO₄, filtrée et concentrée sous vide pour obtenir le composé attendu sous la forme d'un solide.
**RMN ¹H** (400 MHz, CDCl₃) δ ppm : 2.18 (s, 3 H), 2.43 (s, 3 H), 6.17 (d, *J* = 3.2 Hz, 1 H), 7.33 - 7.39 (m, 3 H), 7.90 (d, *J* = 8.5 Hz, 2 H)

### Stade E : 4-Bromo-3-méthyl-1-(4-méthylbenzènesulfonyl)-1H-pyrrole-2-carbonitrile

Du N-bromosuccinimide (12,0 g, 67,4 mmol) est ajouté d'une seule traite à une suspension de 3-méthyl-1-(4-méthylbenzènesulfonyl)-1*H*-pyrrole-2-carbonitrile (14,45 g, 55,6 mmol) dans le *N,N*-diméthylformamide (60 mL) à la température ambiante. Le mélange est agité à la température ambiante durant 29 h, puis est refroidi dans un bain de glace. Une solution aqueuse saturée en bisulfite de sodium (90 mL), de l'eau (90 mL) et de l'acétate d'éthyle (250 mL) sont ensuite ajoutés. Les phases sont séparées, puis la phase aqueuse est extraite avec de l'acétate d'éthyle (2 x 70 mL). Les phases organiques combinées sont lavées avec une solution aqueuse 5% de bicarbonate de potassium (90 mL), de l'eau (3 x 90 mL), puis de la saumure (3 x 90 mL). La phase organique est séchée sur Na₂SO₄, filtrée et concentrée sous vide. Le produit brut est purifié par chromatographie sur gel de silice en utilisant le toluène et l'heptane comme éluants pour obtenir le composé attendu sous la forme d'un solide.
**RMN ¹H** (400 MHz, CDCl₃) δ ppm : 2.13 (s, 3 H), 2.45 (s, 3 H), 7.38 (d, *J* = 8.4 Hz, 2 H), 7.43 (s, 1 H), 7.92 (d, *J* = 8.4 Hz, 2 H)

### Stade F : 4-Bromo-1,3-diméthyl-1H-pyrrole-2-carbonitrile

De l'hydroxyde de potassium (3,65 g, 65,1 mmol) est ajouté d'une seule traite à une suspension de 4-bromo-3-méthyl-1-(4-méthylbenzènesulfonyl)-1*H*-pyrrole-2-carbonitrile (4,66 g, 13,7 mmol) dans du méthanol (95 mL) refroidie avec un bain de glace. Après 15 minutes, la suspension est agitée à température ambiante durant 17 h. Le méthanol est évaporé à sec. Le résidu d'évaporation est repris avec du MTBE (25 mL) et lavé avec de l'eau (25 mL). La phase aqueuse est extraite avec du MTBE (2 x 25 mL). Les phases organiques combinées sont séchées sur Na₂SO₄, filtrées et concentrées sous vide. Le résidu obtenu est solubilisé dans le tétrahydrofurane (50 mL) et la solution est refroidie avec un bain de glace. NaH (60% dans l'huile) (1,02 g, 25,5 mmol) est ajouté. Après 10 minutes, de l'iodométhane (2,4 mL, 38,6 mmol) est également ajouté. Le mélange est agité à la température ambiante durant 1.5 h. Le tétrahydrofurane est évaporé à sec. Le résidu est repris avec du dichlorométhane et lavé avec de l'eau. La phase aqueuse est extraite avec du dichlorométhane. Les phases organiques combinées sont séchées sur Na₂sO₄, filtrées et concentrées sous vide pour obtenir le composé attendu sous la forme d'un solide.
**RMN ¹H** (400 MHz, CDCl₃) δ ppm: 2.16 (s, 3 H), 3.71 (s, 3 H), 6.74 (s, 1 H)

### Stade G : 4-({4-[(tert-Butyldiméthylsilyl)oxy]phényl}amino)-1,3-diméthyl-1H-pyrrole-2-carbonitrile

Du 4-bromo-1,3-diméthyl-1*H*-pyrrole-2-carbonitrile (2,03 g, 10,2 mmol) et de la 4-[(*tert-*butyldiméthylsilyl)oxy]aniline (3,41 g, 15,3 mmol) sont solubilisés dans du toluène (40 mL). La solution est dégazée avec de l'azote pendant 10 minutes. Du *tert*-butylate de sodium (1,18 g, 12,2 mmol), du 2-di-*tert*-butylphosphino-2',4',6'-trüsopropylbiphényle (0.17 g, 0.41 mmol) et du tris(dibenzylideneacétone)dipalladium(0) (0,187 g, 0,2 mmol) sont ensuite ajoutés. Le mélange est chauffé à 100°C pendant 30 minutes, puis refroidi jusqu'à la température ambiante. De l'eau (50 mL) et de la Célite (6 g) sont ajoutés. La suspension est filtrée sur Célite, et le filtrat est dilué avec du MTBE. Les phases sont séparées et la phase aqueuse est extraite avec du MTBE (2 x 50 mL). Les phases organiques combinées sont séchées sur Na₂SO₄et filtrées. Le résidu est purifié par chromatographie sur gel de silice en utilisant l'acétate d'éthyle et l'heptane comme éluants suivi par une chromatographie en phase inverse en utilisant le méthanol et l'eaucomme éluants. Le produit obtenu est lyophilisé pour fournir le composé attendu sous la forme d'un solide.
**RMN ¹H** (400 MHz, CDCl₃) δ ppm: 0.15 (s, 6 H), 0.98 (s, 9 H), 2.05 (s, 3 H), 3.70 (s, 3 H), 4.69 (br. s, 1 H), 6.55 - 6.57 (m, 2 H), 6.64 - 6.69 (m, 3 H)
**RMN ¹³C** (100 MHz, CDCl₃ δ ppm: -4.37, 9.42, 18,27, 25.84, 35.50, 102.59, 113.77, 115.13, 120.72, 121.91, 126.77, 126.94, 140.98, 148.39

### Préparation 38" : 4-[(4-{[tert-Butyl(diméthyl)silyl]oxy}phényl)amino]-1-éthyl-5-méthyl-1H-pyrrole-2-carbonitrile

On procède selon le procédé de la Préparation 33" en remplaçant au Stade B l'iodométhane-*d*₃ par le iodoéthane.
**RMN ¹H** (400 MHz, dmso-d6) δ ppm: 6.85 (s, 1 H), 6.75 (s, 1 H), 6.6/6.5 (2d, 4 H), 4 (quad, 2 H), 2.1 (s, 3 H), 1.3 (s, 3 H), 0.9 (s, 9 H), 0.1 (s, 6 H)

### Préparation 39" : N-[4-[tert-Butyl(diméthyl)silyl]oxyphényl]-2-éthoxy-pyrimidin-5-amine

### Stade A : 5-Bromo-2-éthoxy pyrimidine

Le 5-bromo-2-chloro-pyrimidine (5,0 g, 25 mmol) est dissous dans l'éthanol (55 mL) et l'éthylate de sodium (1,81 g, 26,6 mmol) est ajouté par portion. Le milieu réactionnel est agité pendant 15 heures à température ambiante. Quand la réaction est complète, le solvant est évaporé, de l'eau (200 mL) est ajoutée, puis le milieu réactionnel est extrait avec du dichlorométhane (2x 100mL). La phase organique est séchée sur Na₂sO₄, puis évaporée à sec pour conduire au 5-bromo-2-éthoxy-pyrimidine.

### Stade B : N-[4-[tert-Butyl(diméthyl)silyl]oxyphényl]-2-éthoxy-pyrimidin-5-amine

Le 4-[*tert*-butyl(diméthyl)silyl]oxyaniline (1,8 g, 8 mmol) et le 5-bromo-2-éthoxy-pyrimidine (1,6 g, 8 mmol) en solution dans du tétrahydrofurane anhydre (30mL) sont agités pendant 1 h à température ambiante en présence de *tert*-butylate de sodium (4 mL, solution 2M dans le THF) et de chloro(2-di-*tert*-butylphosphino-2',4',6'-triisopropyl-1,1'-biphényl)[2-(2-aminoéthyl) phényl]palladium(II) (111 mg, 0.16 mmol). Le milieu réactionnel est filtré sur Célite, puis évaporé à sec. Le résidu est trituré dans l'heptane pour obtenir le composé attendu après filtration.
**RMN ¹H** (500 MHz, dmso-d6, 300 K) δ ppm: 8.31 (s, 2H, pyrimidine-H), 7.80 (s, 1H, NH), 6.73 (d, 2H, Ar-H), 6.68 (d, 2H, Ar-H), 4.26 (q, 2H, CH₂CH₃), 1.31 (t, 3H, CH₂CH₃), 0.94 (s, 9H, ^{t}Bu), 0.15 (s, 6H, Me)
**IR :** ν : aromatique: 1504 cm⁻¹; Si-C: 1247 cm⁻¹; C-O-C: 1057 cm⁻¹

### Préparation 40" : Carbonate de tert-butyle et de 6-[(1-méthyl-1H-pyrrolo[2,3-b]pyridin-5-yl)amino]pyridin-3-yle

### Stade A : Carbonate de 6-bromopyridin-3-yle et de tert-butyle

A une solution de 5 g de 6-bromopyridin-3-ol (28,7 mmol) et de 7,53 g de dicarbonate de di-*tert*-butyle (34,5 mmol) dans 50 mL de tétrahydrofurane, est additionné 0,18 g de 4-diméthylaminopyridine (1,4 mmol). Le mélange est agité à température ambiante pendant 6 heures puis concentré. Le résidu obtenu est dissous dans un mélange d'éther éthylique et d'eau. Après décantation, la phase organique est séparée, séchée sur MgSO₄ et concentrée à sec. Le produit du titre est obtenu sous forme d'un solide qui est utilisé au stade suivant sans autre purification.
**RMN ¹H** (400 MHz, dmso-d6) δ ppm: 8.4 (s, 1 H), 7.71 (s, 2 H), 1.5 (s, 9 H)

### Stade B : Carbonate de tert-butyle et de 6-[(1-méthyl-1H-pyrrolo[2,3-b]pyridin-5-yl)amino]pyridin-3-yle

A une solution de 2,79 g du composé obtenu au Stade A (10,2 mmol) dans 15 mL de toluène et 15 mL de tétrahydrofurane, on ajoute 1,18 g de *tert*-butylate de sodium (12,2 mmol), puis l'ensemble est agité sous argon pendant 15 minutes. On ajoute alors 0,35 g de catalyseur au palladium (0,5 mmol). Le milieu réactionnel est ensuite agité à température ambiante pendant 16 heures puis filtré. Le filtrat est concentré et repris dans un mélange de dichlorométhane et d'eau. La phase organique est séparée puis lavée à l'eau, séchée sur MgSO₄, filtrée et concentrée à sec. Le produit brut ainsi obtenu est purifié par chromatographie sur gel de silice en utilisant le dichlorométhane et l'acétate d'éthyle comme éluants. Le résidu est ensuite repris dans un minimum d'éther isopropylique. Le solide alors obtenu est filtré, lavé à l'éther, puis séché. Le produit du titre est obtenu sous la forme d'un solide, qui est utilisé pour la suite sans autre purification.
**RMN ¹H** (400 MHz, dmso-d6) δ ppm: 9 (s, 1 H), 8.35 (df, 1 H), 8.3 (df, 1 H), 8 (df, 1 H), 7.45 (dd, 1 H), 7.45 (df, 1 H), 6.8 (d, 1 H), 6.4 (df, 1 H), 3.8 (s, 3 H), 1.5 (s, 9 H)

### Préparation 41" : 3-[4-[tert-butyl(diméthyl)silyl]oxyanilino]benzonitrile

On procède selon le procédé de la Préparation 1" en remplaçant le 4-bromo-1-méthyl-1*H-*pyrazole utilisé au Stade B par le 3-bromobenzonitrile.
**RMN ¹H** (400 MHz, dmso-d6) δ ppm: 8.3 (s, 1 H), 7.3 (t, 1 H), 7.2/7.1 (2 dd, 2 H), 7.15 (t, 1 H), 7.05 (d, 2 H), 6.8 (d, 2 H), 0.95 (s, 9 H), 0.2 (s, 6 H)

### Préparation 42" : 4-[4-[tert-butyl(diméthyl)silyl]oxyanilino]thiophène-2-carbonitrile

On procède selon le procédé de la Préparation 1" en remplaçant le 4-bromo-1-méthyl-1*H-*pyrazole utilisé au Stade B par le 4-bromothiophène-2-carbonitrile.
**RMN ¹H** (400 MHz, dmso-d6) δ ppm: 8.35 (s, 1 H), 7.59 (df, 1 H), 7.08 (df, 1 H), 6.95 (d, 2 H), 6.75 (d, 2 H), 0.94 (s, 9 H), 0.16 (s, 6 H)

Les amines NHR₃R₄ dans lesquelles R₃ et R₄ représentent indépendamment l'une de l'autre un groupement aryle ou hétéroaryle sont obtenues selon les procédés décrits dans la littérature (Surry D.S et al., Chemical Science, 2011, 2, 27-50, Charles M.D. et al., Organic Letters, 2005, 7, 3965-3968). La réaction de protection de la fonction hydroxy du 4-anilinophénol décrite à la Préparation 2" peut être appliquée à diverses amines secondaires NHR₃R₄ (telles que définies précédemment), comportant une ou plusieurs fonctions hydroxy, lorsque celles-ci sont disponibles commercialement. Alternativement, les amines secondaires comportant au moins un substituant hydroxy peuvent être synthétisées directement sous une forme protégée, i.e. à partir de réactifs dont la fonction hydroxy a été préalablement protégée. Parmi les groupements protecteurs, le *tert*-butyl(diméthyl)silyloxy et le benzyloxy sont particulièrement préférés.

Parmi les amines NHR₃R₄ comportant un substituant hydroxy qui sont utilisées pour synthétiser les composés de l'invention, on peut citer : le 4-(4-toluidino)phénol, le 4-(4-chloroanilino)phénol, le 4-(3-fluoro-4-méthylanilino)phénol, le 4-[4-(trifluorométhoxy)anilino]phénol, le 4-[4-hydroxyanilino]phénol, le {4-[(1-méthyl-1*H-*indol-6-yl)amino]phényl}méthanol, le 4-(2,3-dihydro-1H-indol-6-ylamino)phénol, le 4-[(1-méthyl-2,3-dihydro-1*H*-indol-6-yl)amino]phénol, 4-[(1-méthyl-1*H*-indol-6-yl)amino]phénol, le 4-[(1-méthyl-1*H*-indol-6-yl)amino]cyclohexanol, le 4-[(1-méthyl-1,2,3,4-tétrahydro-6-quinolinyl)amino]phénol, le 4-[(4-méthyl-3,4-dihydro-2*H*-1,4-benzoxazin-7-yl)amino]phénol, le 4-[4-(diéthylamino)anilino]phénol, le 4-(2,3-dihydro-1*H*-indén-5-ylamino)phénol, le 4-[(1-méthyl-1*H*-indazol-5-yl)amino]phénol, le 4-[(1'-méthyl-1',2'-dihydrospiro[cyclopropane-1,3'-indol]-5'-yl)amino]phénol, le 4-[(1,3,3-triméthyl-2,3-dihydro-1*H*-indol-5-yl)amino]phénol, le 4-[4-méthoxy-3-(trifluorométhyl)anilino]phénol, le 4-[4-(méthylsulfanyl)-3-(trifluorométhyl) anilino]phénol, le 2-fluoro-4-[(1-méthyl-1H-indol-5-yl)amino]phénol, le 4-[(1-éthyl-1*H-*indol-5-yl)amino]phénol, le 4-[(1-éthyl-2,3-dihydro-1*H*-indol-5-yl)amino]phénol, le 4-[(1-isopropyl-2,3-dihydro-1*H*-indol-5-yl)amino]phénol, le 4-(butylamino)phénol, le 3-[(1-méthyl-1*H*-indol-5-yl)amino]-1-propanol, le 4-[(1-méthyl-1*H*-indol-5-yl)amino]-1-butanol, le 4-[(3-fluoro-4-méthylphényl)amino]phénol, 4-[(3-chloro-4-méthylphényl)amino]phénol, 4-[(4-fluorophényl)amino]phénol, 4-[(1-méthyl-1*H*-pyrrolo[2,3-*b*]pyridin-5-yl)amino]phénol, 4-[(4-fluorophényl)amino]phénol, 4-[(2-fluorophényl)amino]phénol, 4-[(3-fluorophényl)amino]phénol, 4-[(2,4-difluorophényl) amino]phénol, 4-[(3,4-difluorophényl)amino]phénol, 3-[(4-hydroxyphényl)amino] benzonitrile, 4-[(3-méthoxyphényl)amino]phénol, 4-[(3,5-difluorophényl)amino]phénol, 4-[(3-méthylphényl)amino]phénol, 4-[(4-hydroxyphényl)amino]benzonitrile, 4-[(3-chlorophényl)amino]phénol, 4-(pyrimidin-2-ylamino)phénol, 4-[(cyclobutylméthyl) amino]phénol, 2-[(4-hydroxyphényl)amino]benzonitrile, 4-{[(1-méthyl-1*H*-pyrazol-4-yl)méthyl]amino}phénol, 4-[(cyclopropylméthyl)amino]phénol, 4-{[(1-méthyl-1*H-*pyrazol-3-yl)méthyl]amino}phénol, 4-(but-2-yn-1-ylamino)phénol, 4-(pyrazin-2-ylamino) phénol, 4-(pyridin-2-ylamino)phénol, 4-(pyridazin-3-ylamino)phénol, 4-(pyrimidin-5-ylamino)phénol, 4-(pyridin-3-ylamino)phénol, 4-[(3,5-difluoro-4-méthoxyphényl) amino]phénol, 4-(pyridin-4-ylamino)phénol, 4-[(3-fluoro-4-méthoxyphényl)amino] phénol, 2-(phénylamino)pyrimidin-5-ol, 5-[(4-hydroxyphényl)amino]-2-méthoxy benzonitrile, 4-{[3-(trifluorométhyl)phényl]amino}phénol, le 4-(méthylamino)phénol.

La ou les fonction(s) hydroxy des amines secondaires listées ci-dessus est (sont) préalablement protégée(s) par un groupement protecteur adapté avant tout couplage à un dérivé d'acide du composé de formule (VII) tel que défini dans le procédé général précédent.

### Préparation I: [2-(3-Iodo-4-{[(3R)-3-méthyl-3,4-dihydroisoquinolin-2(1H)-yl]carbonyl}phényl)éthyl]carbamate de tert-butyle

### Stade A : Acide 4-[2-(tert-butoxycarbonylamino)éthyl]benzoïque

Le dicarbonate de *tert*-butyle (1,64 g ;7,5 mmol) est dissous dans du 1,4-dioxanne (5 mL) et la solution est ajoutée à une solution bien agitée d'acide 4-(2-aminoéthyl)benzoïque (1,0 g ; 5 mmol) dans une solution aqueuse d'hydroxyde de sodium à 1 M (25 mL) et de 1,4-dioxanne (10 mL), puis le mélange est agité à température ambiante pendant 3 heures. Le 1,4-dioxanne est éliminé par évaporation sous vide, puis l'acétate d'éthyle (200 mL) est ajouté au résidu. Le pH de la phase aqueuse est ajusté à 1,5 au moyen d'une solution aqueuse de HCl 2 M. La phase organique est séparée, puis la phase aqueuse est extraite avec de l'acétate d'éthyle (100 mL). Les extraits organiques combinés sont lavés avec de l'eau, séchés sur Na₂SO₄ et évaporés jusqu'à un volume de 10 mL. De l'heptane est ajouté au résidu, puis le précipité solide est filtré pour conduire au composé du titre.
**¹H-NMR (500 MHz, dmso-d6, 300K):** 12.81 (br s, 1H, COOH), 7.85 (m, 2H, Ar-2' and 6'H), 7.31 (m, 2H, Ar-3' and 5'H), 6.91 and 6.51 (2x br s, 1H, NH), 3.16 (m, 2H, CH₂), 2.75 (t, 2H, CH₂), 1.35 and 1.31 (2 x br s, 9H, Bu^{t}); **¹³C**-**NMR (125 MHz, dmso-d6, 300 K):** 167.8 (q), 156.0 (CH), 145.3 (q), 129.8 (2 x CH), 129.3 (2 xCH), 129.1 (q), 78.0 (q), 41.5 (CH₂), 35.9 (CH₂), 28.7 (CH₃)

### Stade B : Acide 4-[2-(tert-butoxycarbonylamino)éthyl]-2-iodo-benzoïque

Le composé du stade précédent (0,88 g ; 3,31 mmol), du diacétate d'iodobenzène (1,07 g ; 3,31 mmol), de l'iodure de tétrabutylammonium (1,22 g ; 3,31 mmol), de l'iode (0,84 g ; 3,31 mmol) et du diacétoxypalladium (0,04 g ;0,165 mmol) sont dissous dans du 1,2-dichloroéthane (8 mL), puis le mélange est chauffé dans un réacteur à micro-ondes à 80 °C pendant 90 minutes. Le mélange refroidi est partagé entre une solution aqueuse saturée en Na₂CO₃ (30 mL) et le solvant d'origine. La phase organique est séparée et le résidu aqueux est extrait avec du diéthyléther (2 x 15 mL). Le pH de la solution aqueuse résultante est ajusté à 2 par une solution aqueuse de HCl 2 M, puis le produit est extrait avec de l'acétate d'éthyle (2 x 75 mL). La phase organique est séchée sur Na₂SO₄ et évaporée. Le produit brut est purifié par chromatographie sur gel de silicepour donner le mélange de régioisomères qui sont séparés par CLHP préparative en utilisant eau-TFA et acétonitrile comme éluants. Le pH des fractions appropriées combinées est ajusté à 5 avec du NaHCO₃, puis l'acétonitrile est évaporé sous pression réduite. Le précipité est récupéré par filtration, puis séché pour conduire au composé du titre.
**RMN-¹H (500 MHz, dmso-d6, 300K) :** 13,16 (s large, 1H, COOH), 7,81 (s, 1H, Ar-3'H), 7,66 (d, 1H, Ar-6'H), 7,29 (d, 1H, Ar-5'H), 6,89 (t, 1H, NH), 3,14 (t, 1H, CH₂), 2,69 (t, 1H , CH₂), 1,35 (s, 9H, Boc)

### Stade C : [2-(3-Iodo-4-{[(3R)-3-méthyl-3,4-dihydroisoquinolin-2(1H)-yl]carbonyl phényl)éthyl]carbamate de tert-butyle

Le composé du stade précédent (0,714 g ;1,82 mmol), du TBTU (0,73 g ; 2,28 mmol), de la N-éthyl-N-isopropyl-propan-2-amine (0,94 mL ; 5,46 mmol) et de la *N,N-*diméthylpyridin-4-amine (22 mg) sont agités dans du dichlorométhane (20 mL) pendant 5 minutes, puis de la (3*R*)-3-méthyl-1,2,3,4-tétrahydroisoquinoline (0,282 g ; 1,92 mmol) *(cf.* Préparation 1') est ajoutée. Le mélange est agité pendant trois heures supplémentaires. Le mélange réactionnel est dilué avec du dichlorométhane (150 mL), puis lavé avec de l'eau (25 mL), séché sur Na₂SO₄ et évaporé. Le produit brut est purifié par chromatographie sur gel de silice en utilisant du dichlorométhane et du méthanol comme éluants pour conduire au composé du titre.

### Masse haute résolution (ESI+) :

Formule brute: C24H₂₉IN₂O₃
[M+H]⁺ calculé: 521.1303
[M+H]⁺ mesuré: 521.1282
**IR:** ν : C-H: 2931 cm⁻¹ ; >C=O: 1706, 1627 cm⁻¹ amide: 1505 cm⁻¹ C-O-C: 1248, 1166 cm⁻¹

### Préparation II: (4-Iodo-3-{[(3R)-3-méthyl-3,4-dihydroisoquinolin-2(1H)-yl]carbonyl}benzyl)carbamate de tert-butyle

### Stade A : Acide 2-iodo-5-méthyl-benzoïque

De l'acide 3-méthylbenzoïque (6,12 g ; 45 mmol), du diacétate d'iodobenzène (14,49 g ;45 mmol), de l'iodure de tétrabutylammonium (16,62 g, 45 mmol), de l'iode (11,43 g ; 45 mmol) et du diacétoxypalladium (0,5 g ; 2,2 mmol) sont dissous dans du 1,2-dichloroéthane, puis le mélange est chauffé dans un tube scellé à 85°C pendant 1 heure et 45 minutes. La solution refroidie est purifiée par chromatographie sur gel de silice en utilisant le dichlorométhane et le méthanolcomme éluants, pour conduire au composé du titre.

### Stade B : 2-Iodo-5-méthyl-benzoate de méthyle

Le composé du stade précédent (6,25 g, 23,9 mmol) est dissous dans du méthanol anhydre (100 mL), puis du SOCl₂ (3,6 mL, 49 mmol) est ajouté goutte-à-goutte à la solution bien agitée. La solution est chauffée à reflux pendant 18 heures, puis évaporée jusqu'à un volume de 25 mL qui est ensuite versé dans de la glace pillée (100 g). Le mélange résultant est extrait avec du diéthyléther (2 x 75 mL). La phase organique est lavée avec une solution aqueuse saturée en NaHCO₃ puis de la saumure, séchée sur Na₂SO₄ et évaporée à sec pour conduire au composé du titre.

### Stade C : 5-(Bromométhyl)-2-iodo-benzoate de méthyle

Le composé du stade précédent (5,7 g ; 20,7 mmol), du N-bromo-succinimide (3,67 g ; 20,7 mmol) et du dibenzoylperoxyde (0,24 g ; 1 mmol) sont dissous dans du tétrachlorure de carbone (40 mL) puis chauffés à reflux pendant 5 heures. Ensuite, une nouvelle portion de N-bromosuccinimide (1,0 g ; 5,6 mmol) et de dibenzoylperoxyde (0,05 g ; 0,2 mmol) sont ajoutées et le chauffage poursuivi pendant 4 heures supplémentaires. Le mélange réactionnel est refroidi jusqu'à température ambiante. Les parties insolubles sont retirées par filtration, puis le filtrat concentré est purifié par chromatographie sur gel de silice en utilisant l'heptane et l'acétate d'éthyle comme éluants, pour conduire au composé du titre.

### Stade D : 5-(Aminométhyl)-2-iodo-benzoate de méthyle

Le composé du stade précédent (0,53 g ; 1,5 mmol) est dissous dans de l'ammoniac méthanolique 7 M (20 mL), puis laissé sous agitation à température ambiante pendant 1 heure. La solution est évaporée à sec pour conduire au composé du titre qui a été utilisé dans l'étape suivante sans purification.

### Stade E : Acide 5-[(tert-butoxycarbonylamino)méthyl]-2-iodo-benzoïque

Du bromhydrate de 5-(aminométhyl)-2-iodo-benzoate de méthyle (0,56 g ; 1,5 mmol) a été dissous dans de la pyridine (10 mL), puis du dicarbonate de di-*tert*-butyle (0,80 g ; 3,6 mmol) est ajouté et le mélange agité à température ambiante pendant 30 minutes, puis il est évaporé à sec. Le résidu est redissous dans du méthanol (10 mL), puis une solution aqueuse de NaOH 2 M (3 mL) et 2 mL d'eau sont ajoutés. La solution résultante est agité pendant 2 heures à 50 °C, puis elle est diluée avec de l'eau (20 mL) et le méthanol évaporé sous pression réduite. Le pH de la solution aqueuse résultante est ajusté à 3 au moyen d'une solution aqueuse deHCl 2 M, puis le produit est extrait avec de l'acétate d'éthyle. La phase organique est lavée avec de la saumure, puis séchée sur Na₂SO₄ et évaporée à sec. Le produit brut est trituré avec du DCM. Le solide formé est récupéré par filtration pour conduire au composé du titre.
**RMN-¹H (500 MHz, dmso-d6, 300 K) :** 13,24 (s large, 1H, COOH), 7,91 (d, 1H, Ar-3'H), 7,58 (d, 1H, Ar-6'H), 7,47 (t, 1H, NH), 7,09 (d, 1H, (Ar-5'H), 4,09 (d, 2H, CH2), 1,38 (s, 9H, Boc) ; RMN-¹³C (125 MHz, dmso-d6, 300K) : 168,6 (q), 156,3 (q), 141,1 (q), 140,9 (CH), 137,1 (q), 131,6 (CH), 129,1 (CH), 92,2 (q), 78,5 (q), 28,7 (CH₃)

### Stade F : (4-Iodo-3-{[(3R)-3-méthyl-3,4-dihydroisoquinolin-2(1H)-yl]carbonyl} benzyl)carbamate de tert-butyle

Le composé du stade précédent (1,62 g ; 4,3 mmol), du TBTU (2,76 g ; 8,59 mmol), de la *N*-éthyl-*N*-isopropyl-propan-2-amine (1,48 mL ; 8,59 mmol) et de la *N,N*-diméthylpyridin-4-amine (10 mg) sont laissés sous agitation dans du DCM (50 mL) pendant 5 minutes, puis de la (3R)-3-méthyl-1,2,3,4-tétrahydroisoquinoline (0,76 g ; 5,15 mmol) (*cf.* Préparation 1') est ajoutée et le mélange agité pendant 15 minutes supplémentaires. Les parties insolubles sont retirées par filtration, puis le filtrat concentré est purifié par chromatographie sur gel de silice en utilisant le dichlorométhane et le méthanol comme éluants, pour conduire au composé du titre.

### Préparatîon III : (3-Bromo-4-1{[(3R)-3-méthyl-3,4-dihydroisoquinolin-2,(1H)-yl]carbonyl}benzyl)carbamate de tert-butyle

### Stade A : 4-(Aminométhyl)-2-bromo-benzoate de méthyle

Une solution de 2-bromo-4-(bromométhyl)benzoate de méthyle (4,57 g ; 14,84 mmol) dans 50 mL de méthanol estajoutée goutte-à-goutte à une solution bien agitée d'ammoniac dans le méthanol (7 M ; 315 mL) à température ambiante, puis la solution est agitée pendant 3 heures. Tous les solvants sont évaporés et le résidu est purifié par chromatographie sur gel de silice en utilisant le dichlorométhane méthanol comme éluants, pour conduire au composé du titre.

### Stade B : Acide 2-bromo-4-[(tert-butoxycarbonylamino)méthyl]benzoïque

Le composé du stade précédent (4,04 g ; 16,6 mmol) est dissous dans de la pyridine (55 mL), puis du dicarbonate de di-*tert*-butyle (5,43 g ; 24,9 mmol) est ajouté et le mélange est agité à température ambiante pendant 16 heures, puis il es évaporé à sec. Le résidu est redissous dans du méthanol (100 mL), puis une solution aqueuse de NaOH 1 M (54 mL) est ajoutée. La solution résultante est agitée pendant 1,5 heure à 50 °C, puis elle est laissée refroidir jusqu'à température ambiante. Le pH de la solution est ajusté à 7 au moyen d'une solution aqueuse de HCl 2 M, puis le méthanol est évaporé sous pression réduite. La solution aqueuse résultante est diluée avec de l'eau (50 mL), puis le pH est ajusté à 3 au moyen d'une solution aqueuse de HCl 2 M. Le produit est extrait avec du dichlorométhane (2 x 100 mL). La phase organique est lavée avec de la saumure, puis séchée sur Na₂SO₄ et évaporée à sec pour conduire au composé du titre qui est utilisé dans l'étape suivante sans purification.
**IR:** ν : N-H: 3359 cm⁻¹; C-H: 2983 cm⁻¹; >C=O: 1685, 1603 cm⁻¹; amide: 1519 cm⁻¹; C-OC: 1248, 1162, 1057 cm⁻¹

### Stade C : (3-bromo-4-{[(3R)-3-méthyl-3,4-dihydroisoquinolin-2(1H)-yl]carbonyl} benzyl)carbamate de tert-butyle

Le composé du stade précédent (3,7 g ; 11,2 mmol), du TBTU (7,19 g ; 22,4 mmol), de la N-éthyl-N-isopropyl-propan-2-amine (3,86 mL ; 22,4 mmol) et de la *N,N*-diméthylpyridin-4-amine (48 mg) sont agités dans du dichlorométhane (100 mL) pendant 5 minutes, puis de la (3R)-3-méthyl-1,2,3,4-tétrahydroisoquinoline (1,98 g, 13,44 mmol) (cf. Préparation 1') est ajoutée, et le mélange agité pendant 1 heure supplémentaire. Les parties insolubles sont retirées par filtration, puis le filtrat concentré est purifié par chromatographie sur gel de silice en utilisant le dichlorométhane et le méthanol comme éluants, pour conduire au composé du titre.

### Masse haute résolution (ESI+) :

Formule empirique : C₂₃H₂₇BrN₂O₃
[M+H]⁺ calculé : 459,1285
[M+H]⁺ mesuré : 459,1282

**RMN-¹H** (500 MHz, dmso-d6, 300K, présence de rotamères d'amide) : 7,66-6,87 (m, 7H, aromatique), 5,34 et 4,12 (d, 2H, CH₂-isoquinoline), 5,02 et 4,97 et 3,87 et 3,83 (m, 1H, CH₂-isoquinoline), 4,17 (d, 2H, CH₂-benzyle), 3,18-2,48 (m, 1H, CH₂-isoquinoline), 1,16 et 1,14 et 1,10 et 0,98 (d, 3H, isoquinoline CH₃), 1,31 et 1,41 (s, 9H, Boc).

### Exemple 1. 5-(5-Chloro-2-{[(3R)-3-méthyl-3,4-dihydroisoquinotin-2(1H)-yl]carbonyl}phényl)-N-(4-hydroxyphényl)-1,2-diméthyl-N-(1-méthyl-1H-pyrazol-4-yl)-1H-pyrrole-3-carboxamide

### Stade A : 5-(5-Chloro-2-{[(3R)-3-méthyl-3,4-dihydroisoquinolin-2(1H)-yl]carbonyl} phényl)-1,2-diméthyl-1H-pyrrole-3-carboxylate d'éthyle

A une solution de 1,4 g de composé obtenu à la Préparation 1 (4,35 mmol) dans 50 mL de dichlorométhane, on ajoute successivement 0,7 g du composé obtenu à la Préparation 1' (4,79 mmol), 0,7 g de HOBT (5,22 mmol), 0,81 g d'EDC (5,22 mmol) et 1,6 mL de triéthylamine (21,7 mmol). L'ensemble est ensuite agité pendant une nuit à température ambiante. Puis, le milieu réactionnel est dilué avec du dichlorométhane, lavé 3 fois avec une solution aqueuse saturée en NaHCO₃. La phase organique est alors séchée sur MgSO₄, filtrée, concentrée à sec, puis purifiée par chromatographie sur gel de silice en utilisant le dichlorométhane et le méthanol comme éluants).
**RMN¹H** : δ (500 MHz ; dmso-d6; 300K) : 7.6-7.3(m, 3H, H aromatiques, 4-chlorophényl); 7,2-6,85 (m, 4H, H aromatiques, tétrahydroisoquinoline); 6.45-6.3 (m, 1H, H pyrrole) ; 5.0-4.8-3.8 (m, 1H, H aliphatique, tétrahydroisoquinoline) ; 5.3-3.75 (dd, 2H, H aliphatiques tétrahydroisoquinoline); 4.2-4.0 (m, 2H, O**CH₂**CH₃) ; 3.25 (s, 3H, **CH₃**-N-pyrrole); 3.0-2.2 (m, 2H, H aliphatiques, tétrahydroisoquinoline); 2.5 (s, 3H, **CH₃**-pyrrole) ; 1.25 (t, 3H, OCH₂**CH₃**); 1.05 (d, 3H, tétrahydroisoquinoline-**CH₃**)
**IR :** ν : >C=O : 1693 cm⁻¹ ester ; ν : >C=O : 1625 cm⁻¹ amide

### Stade B : Acide 5-(5-chloro-2-{[(3R)-3-méthyl-3,4-dihydroisoquinolin-2(1H)-yl]carbonyl}phényl)-1,2-diméthyl-1H-pyrrole-3-carboxylique

A une solution de 1,7 g du composé obtenu au Stade A (3,77 mmol) dans 5 mL de dioxane, on ajoute 0,317 g de LiOH (7,5 mmol) en solution dans 5 mL d'eau. L'ensemble est chauffé dans un appareil micro-ondes pendant 4 h à 100°C (puissance 140 W). Le milieu réactionnel est ensuite filtré et concentré à sec. Le résidu ainsi obtenu est repris dans le dichlorométhane (50 mL), puis on y ajoute une solution aqueuse saturée en NH₄Cl. La phase organique est lavée à l'eau, puis avec de la saumure, séchée sur MgSO₄, filtrée et concentrée à sec. Le produit du titre est obtenu sous la forme d'un solide et est utilisé directement pour l'étape suivante.
**RMN¹H :** δ (500 MHz ; dmso-d6 ; 300K) : 11.05 (large s, 1H, COOH), 7.5-7.2 (m, 3H, H aromatiques, 4-Chlorophényl) ; 7.2-6.9 (m, 4H, H aromatiques, tétrahydroisoquinoline); 6.45-6.2 (m, 1H, H aliphatique, H pyrrole) ; 5.3-3.75 (dd, 2H, H aliphatiques, tétrahydroisoquinoline); 5.0-4.8-3.8 (m, 1H, H aliphatique, tétrahydroisoquinoline) ; 3.5-3.2 (s, 3H, **CH₃**-N-pyrrole); 3.0-2.1 (H aliphatiques, **CH₃-**tétraisoquinoline); 2.5-2.4-1.98 (m, 3H, **CH₃**-pyrrole) ; 1.05-0.52 (m, 3H, H aliphatiques, **CH₃**-tétraisoquinoline)
**IR :** ν : -OH : 3500-2000 cm⁻¹ acide carboxylique ; ν : >C=O : 1699 + 1658 cm⁻¹ acide carboxylique ; ν : >C=N- : 1625 cm⁻¹ amide

### Stade C : N-(4-{[tert-Butyl(diméthyl)silyl]oxy}phényl)-5-(5-chloro-2-{[(3R)-3-méthyl-3,4-dihydroisoquinolin-2(1H)-yl]carbonyl}phényl)-1,2-diméthyl-N-(1-méthyl-1H-pyrazol-4-yl)-1H-pyrrole-3-carboxamide

A une solution de 0,65 g du composé obtenu au Stade B (1,54 mmol) dans 15 mL de dichloroéthane est ajouté, au goutte à goutte, 0,244 mL de 1-chloro-*N,N*,2-triméthyl-prop-1-èn-1-amine. Le milieu réactionnel est agité à température ambiante pendant 1 heure, puis on ajoute 0,56 g du composé de la Préparation 1" (1,84 mmol), 10 mL de dichloroéthane et 0,376 g de 4-diméthylaminopyridine (DMAP) (3,07 mmol). L'ensemble est agité à 110°C pendant une nuit. Le milieu réactionnel est concentré, mis en solution dans le dichlorométhane et lavé par une solution aqueuse saturée en NaHCO₃. Le produit du titre est obtenu sous forme d'une huile et est utilisé directement pour l'étape suivante.

### Stade D : 5-(5-Chloro-2-{[(3R)-3-méthyl-3,4-dihydroisoquinolin-2(1H)-yl]carbonyl} phényl)-N-(4-hydroxyphényl)-1,2-diméthyl-N-(1-méthyl-1H-pyrazol-4-yl)-1H-pyrrole-3-carboxamide

A une solution de 0,8 g du composé obtenu au Stade C (1,03 mmol) dans 2 mL de méthanol, on ajoute 1,55 mL d'une solution méthanolique d'hydroxyde de potassium 1M (1,55 mmol). L'ensemble est agité à température ambiante pendant 30 min. Le milieu réactionnel est ensuite dilué dans le dichlorométhane et lavé successivement avec une solution aqueuse de HCl 1M, une solution aqueuse saturée en NaHCO₃ , puis de la saumure jusqu'à neutralité. La phase organique est ensuite séchée sur MgSO₄, filtrée et évaporée. Le produit brut ainsi obtenu est purifié par chromatographie sur gel de silice en utilisant le dichlorométhane et le méthanol comme éluants. Le solide ainsi obtenu est dissous dans un mélange eau / acétonitrile jusqu'à solubilisation totale, filtré, puis est lyophilisé.
***Microanalyse élémentaire :* (% *théorique : mesuré)***
%C=68.74:68.25; %H=5.43:5.69; %N=11.79:11.66; % Cl=5.97:5.95
***Masse haute résolution (ESI*+*) :***
Formule brute : C₃₄H₃₂ClN₅O₃
[M+H]⁺calculé : 594.2266
[M+H]⁺ mesuré : 594.2289

### Exemple 2. 5-(2-{[3S)-3-(Aminométhyl)-3,4-dihydroisoquinolin-2(1H)-yl]carbonyl}phényl)-1-méthyl-N,N-diphényl-1H-pyrrole-3-carboxamide

### Stade A : {[(3S)-2-{2-[4-(Diphénylcarbamoyl)-1-méthyl-1H-pyrrol-2-yl]benzoyl}-1,2,3,4-tétrahydroisoquinolin-3-yl]méthyl}carbamate de tert-butyle

Le composé du titre est obtenu selon le procédé de l'Exemple 1 en utilisant l'acide de la Préparation 2 et le [(3*S*)-1,2,3,4-tétrahydroisoquinolin-3-ylméthyl]carbamate de *tert*-butyle (voir Préparation 2') au Stade A, ainsi que la N-phénylaniline au Stade C.

### Stade B : 5-(2-{[(3S)-3-(Aminométhyl)-3,4-dihydroisoquinolin-2(1H)-yl]carbonyl} phényl)-1-méthyl-N,N-diphényl-1H-pyrrole-3-carboxamide

Une solution du dérivé NH-Boc du Stade A dans le dichlorométhane est placée à 0°C. On y ajoute, goutte à goutte, 10 équivalents molaires d'acide trifluoroacétique. L'ensemble est agité à température ambiante pendant 4 h jusqu'à totale disparition du produit de départ. Le milieu réactionnel est ensuite concentré à sec, repris et co-évaporé 2 fois avec du toluène, puis repris avec un mélange acétonitrile/H₂O et enfin lyophilisé. Le produit du titre est ensuite obtenu après une étape de neutralisation.
***Microanalyse élémentaire :* (% *théorique : mesuré)***
%C=77.75:77.27; %H=5.97:5.73; %N=10.36:10.44

### Exemple 3. 5-(5-Chloro-2-{[(3S)-3-[(4-méthylpipérazin-1-yl]-3,4-dihydroisoquinolin-2(1H)-yl]carbonyl}phényl)-N-(4-hydroxyphényl)-N-(1H-inodol-5-yl)-1-méthyl-1H-pyrrole-3-carboxamide

### Stade A : 5-[(4-{[tert-Butyl(diméthyl)silyl]oxy}phényl){[5-(5-chloro-2-{[(3S)-3-[(4-méthylpipérazin-1-yl)méthyl]-3,4-dihydroisoquinolin-2(1H)-yl]carbonyl}phényl)-1-méthyl-1H-pyrrol-3-yl]carbonyl}amino]-1H-indole-1-carboxylate de tert-butyle

Le composé du titre est obtenu selon le procédé de l'Exemple 1 en utilisant l'acide de la Préparation 3 et la (3*S*)-3-[(4-méthyl-1-pipérazinyl)méthyl]-1,2,3,4-tétrahydroisoquinoline (voir Préparation 4') au Stade A, ainsi que le composé de la Préparation 3" au Stade C.

### Stade B : 5-(5-Chloro-2-{[(3S)-3-[(4-méthylpipérazin-1-yl)méthyl]-3,4-dihydroisoquinolin-2(1H)-yl]carbonyl}phényl)-N-(4-hydroxyphényl)-N-(1H-indol-5-yl)-1-méthyl-1H-pyrrole-3-carboxamide

A une solution de 455 mg (0,49 mmol) du produit obtenu au Stade A dans 5 mL de méthanol, est ajouté 135 mg (2,5 mmol) de KOH. Après 3 h d'agitation à température ambiante, le milieu réactionnel est concentré, traité par une solution aqueuse saturée en NaHCO₃ et extrait au chlorure de méthylène. La phase organique est ensuite séchée sur MgSO₄, filtrée et évaporée à sec. Le produit brut ainsi obtenu est alors purifié par chromatographie sur gel de silice en utilisant le dichlorométhane et le méthanol comme éluants pour donner le produit attendu sous la forme d'une mousse.
***Microanalyse élémentaire :* (% *théorique : mesuré)***
%C=70.72:68.54; %H=5.79:5.37; %N=11.78:11.41; %Cl=4.97:4.79
***Masse haute résolution (ESI+)** :*
Formule brute : C₄₂H₄₁ClN₆O₃
[M+H]⁺ calculé : 713.3007
[M+H]⁺ mesuré : 713.2973

### Exemple 4. N-(4-Hydroxyphényl)-N-(1H-indol-5-yl)-1-méthyl-5-(6-{[(3S)-3-[(4-méthylpipérazin-1-yl)méthyl]-3,4-dihydroisoquinolin-2(1H)-yl]carbonyl}-1,3-benzodioxol-5-yl)-1H-pyrrole-3-carboxamide

Le composé du titre est obtenu selon le procédé de l'Exemple 3 en utilisant le composé de la Préparation 4 au Stade A.
***Microanalyse élémentaire** :* **(*% théorique : mesuré)***
%C=71.45:69.32; %H=5.86:5.22; %N=11.63:11.08
***Masse haute résolution (ESI+)** :*
Formule brute : C₄₃H₄₂N₆O₅
[M+H]⁺ calculé : 723.3295
[M+H]⁺ mesuré : 723.3262

**Sauf mention contraire, les composés des Exemples suivants sont synthétisés selon le procédé de l'Exemple 1 en utilisant au Stade A : (i) l'acide approprié obtenu selon l'une des Préparation 1 à 32 et (ii) le dérivé de tétrahydroisoquinoline approprié obtenu selon l'une des Préparations 1' à 9', ainsi qu'au Stade C : (iii) l'amine NHR₃R₄ adéquate (une liste non exhaustive est proposée aux Préparations 1" à 42". Les composés ainsi obtenus sont optionnellement soumis à une étape de salification en présence de HCl dans l'éther. Après filtration et lyophilisation dans un mélange acétonitrile/eau, on obtient le chlorhydrate du composé attendu.**

### Exemple 5. Chlorhydrate de 5-(5-chloro-2-{[(3S)-3-[(4-méthylpipérazin-1-yl)méthyl]-3,4-dihydroisoquinolin-2(1H)-yl]carbonyl}phényl)-N-(4-hydroxyphényl)-1-méthyl-N-(1-méthyl-1H-indol-5-yl)-1H-pyrrole-3-carboxamide

### Microanalyse élémentaire : (% théorique : mesuré)

%C=64.54:64.9; %H=5.67:5.49; %N=10.5:10.4; %Cl=13.29:12.52; %Cl-=8.86:7.39

### Exemple 6. 5-(5-Chloro-2-{(3S)-3-[(4-méthylpipérazin-1-yl)méthyl]-3,4-dihydroisoquinolin-2(1H)-yl]carbonyl}phényl)-1-éthyl-N-(4-hydroxyphényl)-N-(1-méthyl-1H-indol-5-yl)-1H-pyrrole-3-carboxamide

### Masse haute résolution (ESI+) :

Formule brute : C₄₄H₄₅ClN₆O₃
[M+H]⁺ calculé : 741.3320
[M+H]⁺ mesuré : 741.3326

### Exemple 7. 5-(5-Chloro-2-{[(3S)-3-[(4-méthylpipérazin-1-yl)méthyl]-3,4-dihydroisoquinolin-2(1H)-yl]carbonyl}phényl)-1-cyclopropyl-N-(4-hydroxyphényl)-N-(1-méthyl-1H-indol-5-yl)-1H-pyrrole-3-carboxamide

### Masse haute résolution (ESI+) :

Formule brute : C₄₅H₄₅ClN₆O₃
[M+H]⁺ calculé : 753.3320
[M+H]⁺ mesuré : 753.3306

### Exemple 8. 5-(5-Chloro-2-{[(3S)-3-[(4-méthylpipérazin-1-yl)méthyl]-3,4-dihydroisoquinolin-2(1H)-yl]carbonyl}phényl)-N-(4-hydroxyphényl)-N-(1-méthyl-1H-indol-5-yl)-1-(propan-2-yl)-1H-pyrrole-3-carboxamide

### Masse haute résolution (ESI+) :

Formule brute : C₄₅H₄₇ClN₆O₃
[M+H]⁺ calculé : 755.3476
[M+H]⁺ mesuré : 755.3466

### Exemple 9. Chlorhydrate de N-(4-Hydroxyphényl)-1-méthyl-1H-indole-5-yl)-5-(6-{[(3S)-3-(4-méthylpipérazin-1-yl)méthyl]-3,4-dyhydroisoquinolin-2(1H)-yl]carbonyl}-1,3-benzodioxol-5-yl)-1H-pyrrole-3-carboxamide

### Microanalyse élémentaire : (% théorique : mesuré)

%C=65.26:65.8; %H=5.73:5.47; %N=10.38:10.48; %Cl=8.76:8.46; %Cl-=8.76:8.02

### Exemple 10. Chlorhydrate de 5-(5-fluoro-2-{[(3S)-3-[(4-morpholin-4-ylméthyl)-3,4-dihydroisoquinolin-2(1H)-yl]carbonyl}phényl)-N-(4-hydroxyphényl)-1,2-diméthyl-N-phényl-1H-pyrrole-3-carboxamide

### Microanalyse élémentaire : (% théorique : mesuré)

%C=69.11:68.56; %H=5.8:5.31; %N=8.06:8.13; %Cl-=5.1:4.68

### Exemple 11. Chlorhydrate de 5-(5-fuoro-2-{[(3S)-3-(morpholin-4ylméthyl)-3,4-dihydroisoquinolin-2(1H)yl]carbonyl}phényl)-N-(4-hydroxyphényl)-1-(3-hydroxypropyl)-2-méthyl-N-phényl-1H-phényl-1H-pyrrole-3-carboxamide

### Stade A : 1-(3-(Benzyloxy)propyl)-5-(5-fluoro-2-{[(3S)-3-(morpholin-4-ylméthyl)-3,4-dihydroisoquinolin-2(1H)-yl]carbonyl}phényl)-N-(4-hydroxyphényl)-2-méthyl-N-phényl-1H-pyrrole-3-carboxamide

Le composé du titre est obtenu selon le procédé de l'Exemple 1 en utilisant l'acide de la Préparation 10 et le composé de la Préparation 3' au Stade A, ainsi que le composé de la Préparation 2" au Stade C.

### Stade B : Chlorhydrate de 5-(5-fluoro-2-{[(3S)-3-(morpholin-4-ylméthyl)-3,4-dihydroisoquinolin-2(1H)-yl]carbonyl}phényl)-N-(4-hydroxyphényl)-1-(3-hydroxypropyl)-2-méthyl-N-phényl-1H-pyrrole-3-carboxamide

Le composé du Stade A est soumis à une réaction de déprotection selon un protocole analogue à celui décrit au Stade B de l'Exemple 23.

### Microanalyse élémentaire : (% théorique : mesuré)

%C=68.24:67.96; %H=6:5.79; %N=7.58:7.61; %Cl-=4.8:4.75

### Masse haute résolution (ESI+) :

Formule brute : C₄₂H₄₃FN₄O₅
[M+H]⁺ calculé : 703.3296
[M+H]⁺ mesuré : 703.33294

### Exemple 12. Chlorhydrate de 5-(5-fluoro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(1-méthyl-1H-indazol-5-yl)-1H-pyrrole-3-carboxamide

### Microanalyse élémentaire : (% théorique : mesuré)

%C=67.33:67.39; %H=5.65:5.18; %N=11.22:11.52; %Cl-=4.73:3.82

### Exemple 13. Chlorhydrate de N-(3-fluoro-4-méthylphényl)-N-(4-hydroxyphényl)-1,2-diméthyl-5-(6-{[(3S)-3-(morpholin-4-ylméthyl)-3,4-dihydroisoquinolin-2(1H)-yl]carbonyl}-1,3-benzodioxol-5-yl)-1H-pyrrole-3carboxamide

### Microanalyse élémentaire : (% théorique : mesuré)

%C=66.97:66.72; %H=5.62:5.21; %N=7.44:7.59; %Cl-=4.71:4.54

### Exemple 14. Chlorhydrate de N-(4-hydroxyphényl)-1,2-diméthyl-N-(1-méthyl-1H-indazol-5-yl)-5-(6-{[(3S)-3-(morpholin-4-ylméthyl)-3,4-dihydroisoquinolin-2(1H)-yl]carbonyl}-1,3-benzodioxol-5-yl)-1H-pyrrole-3-carboxamide

### Microanalyse élémentaire : (% théorique : mesuré)

%C=66.62:66.59; %H=5.59:4.67; %N=10.84:10.85; %Cl-=4.57:4.24

### Exemple 15. Chlorhydrate de N-(4-hydroxyphényl)-1,2-diméthyl-5-(6-{[(3S)-3-(morpholin-4-ylméthyl)-3,4-dihydroisoquinolin-2(1H)-yl]carbonyl}-1,3-benzodioxol-5-yl)-N-phényl-1H-pyrrole-3-carboxamide

### Microanalyse élémentaire : (% théorique : mesuré)

%C=68.28:67.07; %H=5.73:5.09; %N=7.77:7.75; %Cl-=4.92:5.71

### Masse haute résolution (ESI+):

Formule brute : C₄₁H₄₀N₄O₆
[M+H]⁺ calculé : 685.3026
[M+H]⁺ mesuré : 685.3033

### Exemple 16. Chlorhydrate de N-(3-fluorophényl)-N-(4-hydroxyphényl)-1,2-diméthyl-5-(6-{[(3S)-3-(morpholin-4-ylméthyl)-3,4-dihydroisoquinolin-2(1H)-yl]carbonyl}-1,3-benzodioxol-5-yl)-1H-pyrrole-3-carboxamide

### Microanalyse élémentaire : (% théorique : mesuré)

%C=66.62:66.11; %H=5.45:5.2; %N=7.58:7.89; %Cl-=4.8:5.59

### Exemple 17. Chlorhydrate de 5-(5-chloro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-phényl-1H-pyrrole-3-carboxamide

### Microanalyse élémentaire : (% théorique : mesuré)

%C=67.51:66.91; %H=5.66:5.05; %N=7.87:7.88; %Cl-=4.98:5.75

### Exemple 18. Chlorhydrate de 1-(2-hydroxyéthyl)-N-(4-hydroxyphényl)-2-méthyl-5-(6-{[(3S)-3-[2-(morpholin-4-yl)éthyl]-3,4-dihydroisoquinolin-2(1H)-yl]carbonyl}-1,3-benzodioxol-5-yl)-N-phényl-1H-pyrrole-3-carboxamide

### Stade A : 1-[2-(Benzyloxy)éthyl]-N-[4-(benzyloxy)phényl]-2-méthyl-5-(6-{[(3S)-3-[2-(morpholin-4-yl)éthyl]-3,4-dihydroisoquinolin-2(1H)-yl]carbonyl}-1,3-benzodioxol-5-yl)-N-phényl-1H-pyrrole-3-carboxamide

Le composé du titre est obtenu selon le procédé de l'Exemple 1 en utilisant l'acide de la Préparation 9 et la (3*S*)-3-[2-(morpholin-4-yl)éthyl]-1,2,3,4-tétrahydroisoquinoline (voir Préparation 5') au Stade A, ainsi que la 4-(benzyloxy)-*N*-phénylaniline (voir Préparation 8") au Stade C.

### Stade B : Chlorhydrate de 1-(2-hydroxyéthyl)-N-(4-hydroxyphényl)-2-méthyl-5-(6-{[(3S)-3-[2-(morpholin-4-yl)éthyl]-3,4-dihydroisoquinolin-2(1H)-yl]carbonyl}-1,3-benzodioxol-5-yl)-N-phényl-1H-pyrrole-3-carboxamide

Le composé du Stade A est soumis à une réaction de déprotection selon un protocole analogue à celui décrit au Stade B de l'Exemple 23.

### Masse haute résolution (ESI+) :

Formule brute : C₃₈H₄₀N₆O₅
[M+H]⁺ calculé : 729.3283
[M+H]⁺ mesuré : 729.3282

### Exemple 19. Chlorhydrate de N-(4-hydroxyphényl)-5-(.5-méthoxy-2-{[(3S)-3-(morpholin-4-ylméthyl)-3,4-dihydroisoquinolin-2(1H)-yl]carbonyl}phényl)-1,2-diméthyl-N-phényl-1H-pyrrole-3-carboxamide

### Microanalyse élémentaire : (% théorique : mesuré)

%C=69.63:69.45; %H=6.13:5.94; %N=7.92:7.79; %Cl-=5.01:4.58

### Exemple 20. Chlorhydrate de N-(4-hydroxyphényl)-5-(5-méthoxy-2-{[(3R)-3-méthyl-3,4-dihydroisoquinolin-2(1H)-yl]carbonyl}phényl)-1,2-diméthyl-N-(pyridin-4-yl)-1H-pyrrole-3-carboxamide

### Microanalyse élémentaire: (% théorique : mesuré)

%C=69.39:69.66; %H=5.66:5.46; %N=8.99:8.92; %Cl-=5.69:5.29

### Masse haute résolution (ESI+) :

Formule brute : C₃₆H₃₅N₄O₄
[M+H]⁺ calculé : 587.2653
[M+H]⁺ mesuré : 587.2649

### Exemple 21. Chlorhydrate de N-(4-hydroxyphényl)-1,2-dyméthyl-N-(1-méthyl-1H-pyrazol-4-yl)-5-(7-{[(1S)-3-(morpholin-4-ylméthyl)-3,4-dihydroisoquinolin-2(1H)-yl]carbonyl}-2,3-dihydro-1,4-benzodioxin-6-yl)-1H-pyrrole-3-carboxamide

### Microanalyse élémentaire : (% théorique : mesuré)

%C=64.99:64.67; %H=5.86:5.67; %N=11.37:11.27; %Cl-=4.8:4.71

### Masse haute résolution (ESI+) :

Formule brute : C₄₀H₄₃N₆O₆
[M+H]⁺ calculé : 703.3236
[M+H]⁺ mesuré : 703.3239

### Exemple 22. Chlorhydrate de N-(4-hydroxyphényl)-1,2-diméthyl-5-(7-{[(3S)-3-(morpholin-4-ylméthyl)-3,4-dihydroisoquinolin-2(1H)-yl]carbonyl}-2,3-dihydro-1,4-benzodioxin-6-yl)-N-phényl-1H-pyrrole-3-carboxamide

### Microanalyse élémentaire : (% théorique : mesuré)

%C=68.61:67.97; %H=5.89:5.59; %N=7.62:7.55; %Cl-=4.82:4.27

### Exemple 23. 1-(2-Hydroxyéthyl)-N-(-4-hydroxyphényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

### Stade A : 1-[2-(Benzyloxy)éthyl]-N-(4-{[tert-butyl(diméthyl)silyl]oxy}phényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

Le composé du titre est obtenu selon le procédé de l'Exemple 1 en utilisant l'acide de la Préparation 9 et le composé de la Préparation 1" au Stade C.

### Stade B : N-(4-{[tert-Butyl(diméthyl)silyl]oxy}phényl)-1-(2-hydroxyéthyl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

A une solution de 6,86 g (8,19 mmol) du composé du Stade A dans 70 mL de méthanol anhydre, on ajoute 1,37 g de Pd/C 10%. L'ensemble est dégazé pendant 0,5 h, puis agité à température ambiante sous pression de dihydrogène (1,5 bar) pendant 12 h. Le milieu réactionnel est ensuite filtré puis concentré à sec. On obtient le produit attendu sous la forme d'un solide.
**RMN¹H**: δ (500 MHz ; dmso-d6 ; 300K) : 7.7-7.4 (s, 1H, H pyrrazole); 7.1-6.8 (s, 1H, H pyrrazole); 7.3-6.9 (m, 4H, H aromatiques, H tétrahydroisoquinoline); 7.0-6.7 (m, 2H, H aromatiques) ; 6.9-6.4 (m, 2H, H aromatiques) ; 6.8-6.4 (m, 2H, H aromatiques) ; 6.1 (m, 2H, **OCH₂O**) ; 5.2 , 5.0, 4.7 (4s, H, H-pyrrole (présence de conformères) ; 5.15 (d, 1H, H aliphatique, H tétrahydroisoquinoline); 4.9 (m, 1H, H aliphalitique, H tétrahydroisoquinoline); 4.9-4.8 (m, 1H, CH₂**OH**) ; 4.7 (m, 1H, H aliphalitique, H tétrahydroisoquinoline); 3.9 (d, 1H, H aliphalitique, H tétrahydroisoquinoline) ; 3.85 (m, 1H, H aliphalitique, H tétrahydroisoquinoline); 3.7 (m, 2H, H aliphatiques, **CH**₂CH₂OH) ; 3.75 (2s, 3H, ) ; 3.5-3.2 (m, 2H, H aliphatiques, CH₂**CH₂O**H) ; 3.0-2.4 (m, 2H, H aliphalitique, H tétrahydroisoquinoline); 2.4-2.3 (4s, 3H, **CH₃**-pyrrole) ; 1.5, 0.95, 0.75 (4 d, 3H, **CH₃**-THIQ) ; 0.85 (large s, 9H, Si(CH₃)₂(**CH(CH₃)**₂) ; 0.15 (m, 6H, Si(**CH₃**)**₂**(CH(CH₃)₂)
IR : ν : -OH : 3346 cm⁻¹ acide carboxylique ; ν : >C=O : 1621 cm⁻¹

### Stade C : 1-(2-Hydroxyéthyl)-N-(4-hydroxyphényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

La fonction phénol du composé du Stade B est déprotégée suivant le procédé du Stade D de l'Exemple 1.
*Microanalyse **élémentaire** :* **(%** *théorique : mesuré)*
%C=68.23:68.12; %H=5.57:5.29; %N=11.05:10.79
***Masse haute résolution (ESI*+*****)** :*
Formule brute : C₃₆H₃₆N₅O₆
[M+H]⁺ calculé : 634.2662
[M+H]⁺ mesuré : 634.2660

### Exemple 24. Chlorhydrate de N-(4-hydroxyphényl)-5-(5-méthoxy-2-{[(3S)-3-(morpholin-4-ylméthyl)-3,4-dihydroisoquiinolin-2(1H)-yl]carbonyl}phényl)-1,2-diméthyl-N-(1-méthyl-1H-pyrazol-4-yl)-1H-pyrrole-3-carboxamide

### Microanalyse élémentaire : (% théorique : mesuré)

%C=65.86:64.47; %H=6.09:5.88; %N=11.82:11.45; %Cl-=4.98:6.7

### Masse haute résolution (ESI+) :

Formule brute : C₃₉H₄₂N₆O₅
[M+H]⁺ calculé : 675.3289
[M+H]⁺ mesuré : 675.3287

### Exemple 25. Chlorhydrate de 5-(5-chloro-2-{[(3S)-3-(morpholin-4ylméthyl)-3,4-dihydroisoquinolin-2(1H)-yl]carbonyl}phényl)-N-(4-hydroxyphényl)-1,2-diméthyl-N-(1-méthyl-1H-pyrazol-4-yl)-1H-pyrrole-3-carboxamide

### Microanalyse élémentaire : (% théorique : mesuré)

%C=63.77:62.83; %H=5.63:5.83; %N=11.74:11.29; %Cl-=4.95:5.42

### Exemple 26. Chlorhydrate de N-(4-hydroxyphényl)-2-méthyl-1-[2-(méthylamino)éthyl]-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

On procède selon les Stades A, B et C du procédé de l'Exemple 30 en remplaçant l'azoture de sodium utilisé au Stade B par la méthylamine. Après purification sur gel de silice (gradient CH₂Cl₂/MeOH/NH₃), le dérivé obtenu 0,3 g (0,46 mmol) est solubilisé dans 5 mL de méthanol anhydre, et on ajoute, goutte à goutte, 0,464 mL (0,47 mmol) d'une solution d'éther chlorhydrique 1M. L'ensemble est agité pendant 0,5 h à température ambiante. Le précipité ainsi obtenu est filtré et séché, puis solubilisé dans un mélange CH₃CN/H₂O avant d'être lyophilisé à basse température. On obtient le produit attendu sous la forme d'un solide.
***Microanalyse élémentaire** :* (% ***théorique : mesuré)***
%C=65.05:64.64; %H=5.75:5.43; %N=12.3:12.3; %Cl-=5.19:5.39
***Masse haute résolution (ESI*+*****)** :*
Formule brute : C₃₇H₃₈N₆O₅
[M+H]⁺ calculé : 647.2903
[M+H]⁺ mesuré : 647.2922

### Exemple 27. Chlorhydrate de 1-[2-(diméthylamino)éthyl]-N-(4-hydroxyphényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl]-1H-pyrrole-3-carboxamide

On procède selon le procédé de l'Exemple 26 en remplaçant la méthylamine au Stade B par la diméthylamine.
***Microanalyse élémentaire :* (% *théorique : mesuré)***
%C=65.46:65.07; %H=5.93:5.87; %N=12.05:12.06; %Cl-=5.08:5.55
***Masse haute révolution (ESI*+*****)** :*
Formule brute : C₃₈H₄₀N₆O₅
[M+H]⁺ calculé : 661.3060
[M+H]⁺ mesuré : 661.3045

### Exemple 28. Chlorhydrate de 5-(5-chloro-2-{[(3R)-3-méthyl-3,4-dyhydroisoquinolin-2(1H)-yl]carbonyl}phényl)-N-(4-hydroxyphényl)-1,2-diméthyl-N-(pyridin-4yl)-1H-pyrrole-3-carboxamide

### Microanalyse élémentaire : (% théorique : mesuré)

%C=66.99:66.88; %H=5.14:5.28; %N=8.93:8.87; %Cl-=5.65:4.98

### Masse haute révolution (ESI+) :

Formule brute : C₃₅H₃₂ClN₄O₃
[M+H]⁺ calculé : 591.2157
[M+H]⁺ mesuré : 591.2178

### Exemple 29. Chlorhydrate de 5-(5-chloro-2-{[(3S)3-{[2-(morpholin-4-yl)éthoxy] méthyl}-3,4-dihydrosoiquinolin-2(1H)-yl]carbonyl}phényl)-N-(4-hydroxyphényl)-1,2-diméthyl-N-phényl-1H-pyrrole-3-carboxamide

### Stade A: 5-(5-Chloro-2-{[(3S)-3-(hydroxyméthyl)-3,4-dihydroisoquinolin-2(1H)-yl]carbonyl}phényl)-1,2-diméthyl-1H-pyrrole-3-carboxylate de méthyle

A une solution de 4,9 g du composé de la Préparation 1 dans un mélange de 40 mL de diméthylformamide et 40 mL de tétrahydrofurane sont ajoutés 2,73 g (1,1 équivalent) de (3*S*)-3-hydroxyméthyl-1,2,3,4-tétrahydroisoquinoline, 7,95 mL de diisopropyléthylamine, et 9,84 g (1,7 équivalent) de HATU. Après 2 h d'agitation à température ambiante, le milieu réactionnel est mis à sec, repris dans l'acétate d'éthyle, puis lavé avec une solution aqueuse d'acide citrique à 10% et de l'eau. Les phases aqueuses réunies sont extraites à l'acétate d'éthyle. Les phases organiques ainsi obtenues sont réunies, séchées sur Na₂SO₄, filtrées et concentrées sous pression réduite. Le produit brut obtenu est purifié par chromatographie sur gel de silice en utilisant le dichlorométhane et l'éthanol comme éluants pour conduire au composé du titre.
**RMN¹H** : δ (500 MHz ; dmso-d6 ; 300K) : 7,6-7,4 (m, 3H, Cl-Ph) ; 7,2-6,9 (m, 4H, Ar(THIQ) ; 6,5-6,15 (4s large, 1H, pyrrole) ; 4,9-3,8 (6 d, 2H, NCH₂ THIQ) ; 4,85-3,6 (m , 1H, NCH THIQ) ; 4,2-4,0 (m, 2H, OCH₂ ester), 3,45-3,2 (3s, 3H, N-CH₃) ; 3,35-3,0 (3m, 2H, HO**CH₂**); 3,0-2,0 (m, 2H, THIQ); 2,5-2,1 (m, 3H, CH₃ pyrrole); 1,25-1,1 (m, 3H, CH₃ ester)
**IR :** ν -OH : 3388 cm⁻¹, ν >C=O : 1693 cm⁻¹ (ester conjugué), ν >C=O : 1620 cm⁻¹ (amide)

### Stade B: 5-(5-Chloro-2-{[(3S)-3-{[2-(morpholin-4-yl)éthoxy]méthyl}-3,4-dihydroisoquinolin-2(1H)-yl]carbonyl}phényl)-1,2-diméthyl-1H-pyrrole-3-carboxylate de méthyle

Une solution de 1,7 g (3,2 mmol) du composé obtenu au stade précédent dans 20 mL de diméthylformamide est additionnée, goutte à goutte à 0°C, sur une suspension de 0,280 g de NaH à 60% dans l'huile (2,2 équivalents) dans 10 mL de diméthylformamide. Après 5 min d'agitation à température ambiante, une suspension de 0,68 g de bromhydrate de 4-(2-bromoéthyl)morpholine (1,1 équivalent) est ajoutée au goutte à goutte. Le milieu réactionnel est agité à température ambiante pendant 45 min. Puis, on ajoute, en deux étapes sur 20 h, 2,2 équivalents de bromhydrate de 4-(2-bromoéthyl)morpholine, puis 3 équivalents de NaH à 60% dans l'huile. Le milieu réactionnel est alors versé sur un mélange de chlorure d'ammonium aqueux à 10% et d'acétate d'éthyle. La phase organique résultante est lavée successivement avec de l'eau, puis avec une solution aqueuse saturée en LiCl et de la saumure. Elle est ensuite séchée sur Na₂SO₄, filtrée et concentrée sous pression réduite. L'huile obtenue est purifiée par chromatographie sur gel de silice en utilisant le dichlorométhane et l'éthanol comme éluants pour fournir le produit attendu.
**RMN¹H:** δ (500 MHz ; dmso-d6 ; 300K) : 7,6-7,3 (m, 3H, Cl-Ph) ; 7,2-6,85 (m, 4H, Ar(THIQ) ; 6,5-6,15 (s large, 1H, pyrrole) ; 5,25-3,8 (m, 2H, NCH₂ THIQ) ; 5,05-3,75 (m , 1H, NCH THIQ) ; 4,2-4,0 (m, 2H, OCH₂ ester), 3,5-3,25 (m, 3H, N-CH₃) ; 3,6-2,75 (m, 8H, OCH₂ morpholinoéthoxyméthyl); 2,95-2,05 (m, 2H, THIQ); 2,55-2,15 (m, 6H, NCH₂ morpholinoéthoxyméthyl); 2,55-2,05 (m, 3H, CH₃ pyrrole), 1,25-1,1 (m, 3H, CH₃ ester) **IR :** ν >C=O : 1694 cm⁻¹ (ester conjugué), ν >C=O : 1629 cm⁻¹ (amide)

### Stade C: Acide 5-(5-Chloro-2-{[(3S)-3-{[2-(morpholin-4-yl)éthoxy]méthyl}-3,4-dihydroisoquinolin-2(1H)-yl]carbonyl}phényl)-1,2-diméthyl-1H-pyrrole-3-carboxylique

A une solution de 1,46 g du composé du stade précédent dans 5 mL de dioxane sont ajoutés 5 mL d'une solution aqueuse de LiOH 1 M. L'ensemble est chauffé dans un appareil à micro-ondes (100 W) à 100°C pendant 2 h. Le milieu réactionnel est versé sur de l'eau, puis extrait à l'éther éthylique. La phase éthérée est extraite de nouveau avec 10 mL d'eau. Les phases aqueuses sont acidifiées à pH 5-6 par ajout d'une solution aqueuse saturée en chlorure d'ammonium, puis extraites 2 fois avec du dichlorométhane. La phase dans le dichlorométhane est séchée sur Na₂SO₄, filtrée et concentrée à sec. Le produit du titre est obtenu sous la forme d'une meringue.
**RMN¹H :** δ (500 MHz ; dmso-d6 ; 300K) : 11,4 (s large, 1H, CO₂H), 7,6-7,3 (m, 3H, Cl-Ph) ; 7,2-6,8 (m, 4H, Ar(THIQ) ; 6,5-6,2 (4s larges, 1H, pyrrole) ; 5,25-3,75 (m, 2H, NCH₂ THIQ) ; 5,05-3,7 (3m , 1H, NCH THIQ) ; 3,5-3,2 (3s, 3H, N-CH₃) ; 3,6-2,7 (m, 8H, OCH₂ morpholinoéthoxyméthyl); 2,5-2,2 (m, 6H, NCH₂ morpholinoéthoxyméthyl); 3,0-2,0 (m, 2H, THIQ), 2,5-2,0 (3s, 3H, CH₃ pyrrole)
**IR:** ν -OH : 3300-2200 cm⁻¹, ν >C=O : 1697-1662 cm⁻¹ (bande dédoublée, acide carboxylique), ν >C=O : 1628 cm⁻¹ (amide)

### StadeD: N-(4-{[tert-Butyl(diméthyl)silyl]oxy}phényl)-5-(5-chloro-2-{[(3S)-3-{[2-(morpholin-4-yl)éthoxy]méthyl}-3,4-dihydroisoquinolin-2(1H)-yl]carbonyl}phényl)-1,2-diméthyl-N-phényl-1H-pyrrole-3-carboxamide

L'acide obtenu au Stade C (1,48 g) est mis en suspension dans 15 mL de 1,2-dichloroéthane. On y ajoute 1,1 équivalent de 1-chloro-*N,N*,2-triméthylpropènylamine. Après 1 h d'agitation à température ambiante, sont ajoutés 15 mL de toluène et 1,05 équivalent du composé la Préparation 2". Le milieu réactionnel est chauffé pendant 14 h à 110°C, puis mis à sec. Le produit brut ainsi obtenu est purifié par chromatographie sur gel de silice en utilisant le dichlorométhane et l'éthanol comme éluants pour conduire au produit attendu.
RMN¹H : δ (500 MHz ; dmso-d6 ; 300K) : 7,55-7,25 (m, 3H, Cl-Ph) ; 7,25-6,6 (m, 9H, Ar(THIQ)+phényl) ; 6,8-6,5 (m, 2H, phénoxy) ; 7,0-6,6 (m, 2H, phénoxy) ; 5,7-5,05 (4s larges, 1H, pyrrole) ; 5,2-3,6 (8d, 2H, THIQ) ; 5,05-3,6 (4m, 1H, NCH THIQ), 3,6-2,9 (m, 8H, aliphatiques morpholinoéthoxyméthyl); 3,4-3,2 (3s larges, 3H, N-CH₃); 3,0-2,1 (m, 2H, THIQ), 2,35 (m, 2H, NCH₂ morpholinoéthoxyméthyl); 2,4-2,2 (m, 4H, NCH₂ morpholine); 2,4-2,15 (3s larges, 3H, CH₃ pyrrole);.0,8 (2s larges, 9H, SiC(CH₃)₃); 0,1 (4s, 6 H, SiCH₃)
IR : ν >C=O : 1635-1595 cm⁻¹ (bande dédoublée), ν Si-O : 1117 cm⁻¹, ν Si-C : 837 cm⁻¹

### Stade E : Chlorhydrate de 5-(5-chloro-2-{[(3S)-3-{[2-(morpholin-4-yl)éthoxy)méthyl-3,4-dihydroisoquinolin-2(1H)-yl]carbonyl}phényl)-N-(4-hydroxyphényl)-1,2-diméthyl-N-phényl-1H-pyrrole-3-carboxamide

A une solution de 0,7 g du composé du Stade D dans 5 mL de méthanol est ajoutée 0,92 mL d'une solution de potasse 1M dans le méthanol. Après 1h10 d'agitation à température ambiante, une solution aqueuse saturée en bicarbonate de sodium est additionnée. Le produit qui précipite est extrait à l'acétate d'éthyle, puis lavé avec de l'eau et de la saumure. Les phases aqueuses résultantes sont extraites de nouveau à l'acétate d'éthyle. Les phases organiques ainsi obtenues sont séchées sur Na₂SO₄, filtrées et concentrées sous pression réduite. Le produit brut obtenu est purifié par chromatographie sur gel de silice en utilisant le dichlorométhane et l'éthanol contenant de l'ammoniac comme éluants pour fournir 0,536 g du produit du titre, sous sa forme base. Cette dernière est solubilisée dans l'acétonitrile et salifiée à l'aide d'une solution aqueuse de HCl 1 M . Après une étape de lyophilisation, on obtient le produit attendu sous la forme d'un solide.
***Microanalyse élémentaire : (%mesuré (théorique))***
%C=66.4(66.75);%H=5.98(5.87);%N=7.38(7.41);%Cl=9.42(9.38);%Cl⁻=4.57(4.69)

### Exemple 30. Chlorhydrate de 1-(2-aminoéthyl)-N-(4-hydroxyphényl)-2-méthyl-5(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

### Stade A : 2-{3-[(4-{[tert-Butyl(diméthyl)silyl]oxy}phényl)(1-méthyl-1H-pyrazol-4-yl)carbamoyl]-2-méthyl-5-(6-{[(3R)-3-méthyl-3,4-dihydroisoquinolin-2(1H)-yl]carbonyl}-1,3-benzodioxol-5-yl)-1H-pyrrol-1-yl}éthyl méthanesulfonate

Une solution 4,65 g du composé obtenu au Stade B de l'Exemple 23 (6,22 mmol) dans 100 mL de THF anhydre est placée à 0°C. On y ajoute successivement 3,5 mL (12,44 mmol) de triéthylamine, puis, goutte à goutte, 0,722 mL de chlorure de méthane sulfonique (9,33 mmol) en solution dans 20 mL de THF. L'ensemble est ensuite agité à température ambiante pendant 2 h. Le milieu réactionnel est hydrolysé par addition d'une solution aqueuse saturée en NaHCO₃, puis extraite 2 fois avec l'acétate d'éthyle. Les phases organiques sont regroupées, séchées sur MgSO₄ et concentrées à sec. On obtient le composé attendu sous la forme d'un solide vitreux.
**RMN¹H** : δ (500 MHz ; dmso-d6 ; 300K) : 7.7-7.45 (s, 1H, H pyrrazole); 7.1-6.9 (s, 1H, H pyrrazole); 7.2-6.4 (m, 8H, H aromatiques); 6.95-6.65 (m, 2H, H aromatiques) ; 6.1 (m, 2H, O**CH₂**O) ; 5.2 , 4.6 (4s, H, H-pyrrole (présence de conformères) ; 4.9, 4.6, 3.9, 3.8 (m, 1H), 5.05-3.75 (m, 1H, H aliphalitique, H tétrahydroisoquinoline); 4.3-4.05 (m, 2H, H aliphatiques, CH₂**CH**₂OSO₂CH₃) ;4.2-3.95 (m, 2H, H aliphatiques, **CH₂**CH₂OSO₂CH₃**) ;** 3.75-2.7 (2s, 3H, **CH₃**-pyrazole) ; 3.0 (plusieurs s, 3H, CH₂OSO₂**CH₃**) ; 3.05-2.5 (plusieurs m, 2H, H aliphalitiques, H tétrahydroisoquinoline); 2.45-2.3 (plusieurs s, 3H, **CH₃**-pyrrole) ; 1.05, 0.75 (plusieurs d, 3H, **CH₃**-tétraisoquinoline) ; 0.9 (plusieurs s, 9H, Si(CH₃)₂(**CH(CH₃)**₂) ; 0.1 (m, 6H, Si(**CH₃**)₂(CH(CH₃)₂)
**IR :** ν : >C=O : 1626 cm⁻¹, ν : -SO₂ : 1349 et 1172 cm⁻¹

### Stade B : 1-(2-Azidoéthyl)-N-(4-{[tert-butyl(diméthyl)silyl]oxy}phényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

A une solution de 2 g (2,42 mmol) de composé du Stade A dans 20 mL de DMF anhydre, on ajoute 0,472 g (7,26 mmol) d'azoture de sodium. L'ensemble est agité à 65°C pendant 4h30. Le milieu réactionnel est versé dans une solution aqueuse saturée en NaHCO₃, puis extrait 2 fois avec de l'acétate d'éthyle. Les phases organiques sont regroupées et lavées avec une solution aqueuse saturée en LiCl, séchées sur MgSO₄ et concentrées à sec. Après purification sur gel de silice en utilisant le dichloroméyhane et le méthanol contenant de l'ammoniac comme éluants, on obtient le produit attendu sous la forme d'une mousse.
**RMN¹H** : δ (500 MHz ; dmso-d6 ; 300K) : 7.65-7.45 (plusieurs s, 1H, H pyrrazole); 7.1-6.9 (plusieurs s, 1H, H pyrrazole); 7.3-6.4 (plusieurs m, 6H, H aromatiques); 7.1-6.9 (plusieurs s, 1H, H aromatiques) ; 6.8, 6.5 (2m, 2H, H aromatiques) ; 6.1 (plusieurs s, 2H, **OCH₂**O) ; 5.25- 4.65 (plusieurs s, 1H, H-pyrrole (présence de conformères) ; 4.9, 4.6,3.8 (plusieurs m, 1H) ; 5.05-3.7 (plusieurs m, 4H, H aliphatiques, H-THIQ + **CH₂**CH₂N₃) ; 3.7 (plusieurs s, 3H, H **CH₃**-pyrazole) ; 3.5-3.25 (m, 2H, CH₂**CH₂**N₃) ; 3.1-2.4 (plusieurs m, 3H, **CH₃**-pyrrole) ; 2.45-2.3 (plusieurs s, 3H, **CH₃**-pyrrole) ; 1.0-0.75 (plusieurs d, 3H, **CH₃**-THIQ) ; 0.9 (plusieurs s, 9H, Si(CH₃)₂**(CH(CH₃)₂)** ; 0.1 (plusieurs s, 6H, Si(**CH₃)₂**(CH(CH₃)₂)
**IR :** ν :-N=N=N : 2100 cm⁻¹, ν : >C=O : 1630 cm⁻¹, ν : -Si-O- : 1035 cm⁻¹

### Stade C : 1-(2-Azidoéthyl)-N-(4-hydroxyphényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

A une solution de 1,12 g du composé obtenu au Stade B (14,49 mmol) dans 5 mL de méthanol, on ajoute, goutte à goutte, 7,24 mL d'une solution méthanolique 1 M d'hydroxyde de potassium (72,45 mmol). L'ensemble est agité à température ambiante pendant 3h. Le milieu réactionnel est ensuite concentré à sec, repris avec du dichlorométhane, hydrolysé avec une solution aqueuse saturée en NaHCO₃, puis extrait 2 fois avec du dichlorométhane. Les phases organiques sont alors regroupées, lavées avec de l'eau, séchées sur MgSO₄ et concentrées à sec. On obtient le produit attendu sous la forme d'une mousse, utilisée directement pour l'étape suivante.

**RMN¹H** : δ (500 MHz; dmso-d6 ; 300K) : 9.60((s, 1H, CH₂CH₂**OH)** ; 7.65-7.45 (plusieurs s, 1H, H pyrrazole); 7.1-6.9 (plusieurs s, 1H, H pyrrazole); 7.3-6.4 (plusieurs m, 6H, H aromatiques); 7.1-6.9 (plusieurs s, 1H, H aromatiques) ; 6.8, 6.5 (2m, 2H, H aromatiques) ; 6.15 (plusieurs s, 2H, **OCH₂**O) 5.35- 4.8 (plusieurs s, 1H, H-pyrrole (présence de conformères) ; 4.9, 4.6,3.8 (plusieurs m, 1H, H aliphatique, H tétrahydroisoquinoline) ; 3.7 (plusieurs s, 3H, H **CH₃**-pyrazole) ; 3.5-3.25 (m, 2H, CH₂**CH₂**N₃) ; 3.1-2.4 (plusieurs m, 3H, **CH₃**-pyrrole) ; 2.45-2.3 (plusieurs s, 3H, H THIQ) ; 2.45-2.3 (plusieurs s, 3H, **CH₃**-pyrrole) ; 1.0-0.75 (plusieurs d, 3H, **CH₃-**THIQ) IR : ν :-OH : 3171 cm⁻¹, ν :-N=N=N : 2100 cm⁻¹, ν : >C=O : 1617 cm⁻¹

### Stade D : Chlorhydrate de 1-(2-aminoéthyl)-N-(4-hydroxyphényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

A une solution de 1,27 g du composé obtenu au stade précédent (1.93 mmol) dans 15 mL d'éthanol anhydre, on ajoute 0,154 g de Pd/C 10%. L'ensemble est dégazé pendant 0,5 h. Il est ensuite agité pendant 12 h à température ambiante sous pression d'hydrogène (1,5 bar). Le milieu réactionnel est ensuite filtré puis concentré à sec. Après purification par chromatographie sur gel de silice (gradient CH₂Cl₂/ MeOH / NH₃), on obtient un résidu huileux. Ce dernier est mis en solution dans 10 mL d'éthanol anhydre, puis salifié par l'addition de deux équivalents molaires d'une solution éthanolique de HCl 1N. Le produit est ensuite mis à sec avant d'être solubilisé dans le minimum d'un mélange d'eau et d'acétonitrile. Après une étape de lyophilisation à basse température, on obtient le produit attendu sous la forme d'un solide.
***Microanalyse élémentaire** :* **(% *théorique : mesuré)***
%C=64.62:63.84; %H=5.57:5.55; %N=12.56:12.36; %Cl-=5.3:5.56
***Masse haute résolution (ESI***+*) :*
Formule brute : C₃₆H₃₆N₆O₅
[M+H]⁺ calculé : 633.2820
[M+H]⁺ mesuré : 633.2808

### Exemple 31. N-(4-Hydroxyphényl)-5-(5-méthoxy-2{[(3R)-3-méthyl-3,4-dyhydroisoquinolin-2(1H)-yl]carbonyl}phényl)-1,2-diméthyl-N-(1-méthyl-1H-pyrazol-4-yl)-1H-pyrrole-3-carboxamide

### Microanalyse élémentaire (% théorique : mesuré)

%C=71.29:70.17; %H=5.98:5.98; %N=11.88:11.49

### Masse haute résolution (ESI+) :

Formule brute : C₃₅H₃₆N₅O₄
[M+H]⁺ calculé : 590.2762
[M+H]⁺ mesuré : 590.2778

### Exemple 32. (exemple de référence) (4-{4-[(4-Hydroxyphényl)(méthyl)carbamoyl]-1,5-diméthyl-1H-pyrrol-2-yl}-3-{[(3R)-3-méthyl-3.4-dihydroisoquinolin-2(1H)-yl]carbonyl}benzy)carbamate de phényle

### Stade A : N-(4-Benzyloxyphényl)-N,1,2-triméthyl-pyrrole-3-carboxamide

Le procédé est analogue à celui décrit au Stade A de l'Exemple 45.

### Stade B : [4-(4-{(4-(benzyloxy)phényl)(méthyl)carbamoyl}-1,5-diméthyl-1H-pyrrol-2yl)-3-{[(3R)-3-méthyl-3,4-dihydroisoquinolin-2(1H)-yl]carbonyl}benzyl]carbamate de tert-butyle

Le composé de la Préparation II (1,5 g ; 2,96 mmol) et le composé du Stade A (1,16 g ; 3,44 mmol) est dissous dans du diméthylacétamide (25 mL), puis de l'azote est mis à barboter dans la solution pendant 5 minutes. De l'acétate de potassium (0,586 g ; 5,92 mmol) et du dichlorure de bis(triphénylphosphine)palladium(II) (0,2 g ; 0,295 mmol) sont ajoutés au mélange qui est ensuite chauffé jusqu'à 100 °C et, après 20 minutes d'agitation, de l'eau (10 µL) est ajoutée. L'agitation à 100 °C sous une atmosphère d'azote est poursuivie pendant 2 heures supplémentaires. Le mélange réactionnel est laissé à refroidir jusqu'à température ambiante, puis évaporé jusqu'à un volume de 15 mL. Le mélange résultant est filtré sur une courte colonne de célite, puis purifié par CLHP préparative en utilisant de l'eau-TFA et de l'acétonitrile comme éluants . Le pH des fractions appropriées est ajusté à 7 avec du NaHCO₃, puis l'acétonitrile est évaporé sous pression réduite. Le résidu aqueux est extrait avec du DCM. La phase organique est lavée avec de la saumure, séchée sur Na₂SO₄ et évaporée à sec pour conduire au composé du titre.
**Masse haute résolution (ESI+)** :
Formule empirique : C₄₀H₄₁N₅O₄
[M+H]⁺ calculé : 713,3705
[M+H]⁺ mesuré : 713,3709

### Stade C : Chlorhydrate de 5-[4-(aminométhyl)-2-{[(3R)-3-méthyl-3,4-dihydroisoquinolin-2(1H)-yl]carbonyl}phényl]-N-[4-(benzyloxy)phényl]-N,1,2-triméthyl-1H-pyrrole-3-carboxamide

Le composé du stade précédent (1,18 g, 1,66 mmol) est dissous / mis en suspension dans du HCl à 4 M dans le dioxanne (20 mL). Le mélange hétérogène est laissé sous agitation pendant 30 minutes à température ambiante, puis évaporé à sec pour donner 1,19 g du composé du titre qui est utilisé dans l'étape suivante sans purification.

### Stade D : (4-{4-[(4-Hydroxyphényl)(méthyl)carbamoyl]-1,5-diméthyl-1H-pyrrol-2-yl}-3-{[(3R)-3-méthyl-3,4-dihydroisoquinolin-2(1H)-yl]carbonyl}benzyl)carbamate de phényle

Le composé du titre est obtenu à partir du composé du stade précédent selon le procédé du Stade D de l'Exemple 45.
***Microanalyse élémen**t**aire :*** (%***mesuré*** (***théorique***))
%C=72.83(72.88);%H=6.01(5.96);%N=8.02(8.72)
***Masse haute résolution (ESI*+*) :***
Formule brute : C₃₉H₃₈N₄O₅
[M+H]⁺ calculé : 643.2922
[M+H]⁺ mesuré : 643.2916

### Exemple 33. Chlorhydrate de 5-(5-chloro-2-{[(3S)-3-(morpholin-4-ylméthyl)-3,4-dihydroisoquinolin-2(1H)-yl]carbonyl}phényl)-N-(4-fluorophényl)-N-(4-hydroxyphényl)-1,2-diméthyl-1H-pyrrole-3-carboxamide

### Microanalyse élémentaire : (%mesuré (théorique))

%C=65.75(65.84);%H=5.38(5.39);%N=7.62(7.68);%Cl=9.77(9.72);%Cl-=4.84(4.86)

### Exemple 34. Chlorhydrate de 5-(5-chloro-2-{[(3S)-3-(morpholin-4-ylméthyl)-3,4-dihydroisoquinolin-2(1H)-yl]carbonyl}phényl)-N-(3-fluorophényl)-N-(4-hydroxyphényl)-1,2-diméthyl-1H-pyrrole-3-carboxamide

### Microanalyse élémentaire : (%mesuré (théorique))

%C=65.61(65.84);%H=5.09(5.39);%N=7.76(7.68);%Cl-=4.83(4.86)

### Exemple 35. (exemple de référence) N-(4-Hydroxyphényl)-N,1,2-triméthyl-5-(2-{[(3R)-3-méthyl-3,4-dihydroisoquinolin-2(1H)-yl]carbonyl}-4-{[(phénoxyacétyl)amino]méthyl}phényl)-1H-pyrrole-3-carboxamide

Le composé du titre est obtenu selon le procédé de l'Exemple 32 en utilisant au Stade D le chlorure de 2-phénoxyacétyle comme agent acylant.
***Microanalyse élémentaire :*** (%***mesuré*** (***théorique***))
%C=72.53(73.15);%H=5.76(6.14);%N=8.31(8.53)
***Masse haute résolution (ESI*+*) :***
Formule brute : C₄₀H₄₀N₄O₅
[M+H]⁺ calculé : 657.3079
[M+H]⁺ mesuré : 657.3061

### Exemple 36. (exemple de référence) 5-(4-{[(Éthylcarbamoyl)amino]méthyl}-2-{[(3R)-3-méthyl-3,4-dihydroisoquinolin-2(1H)-yl]carbonyl}phényl)-N-(4-hydroxyphényl)-N,1,2-triméthyl-1H-pyrrole-3-carboxamide

Le composé du titre est obtenu selon le procédé de l'Exemple 32 en utilisant au Stade D l'isocyanate d'éthyle comme agent acylant.
***Microanalyse élémentaire :*** (%***mesuré*** (***théorique***))
%C=70.88(70.8);%H=6.02(6.62);%N=11.17(11.8)
***Masse haute résolution (ESI*+*****)** :*
Formule brute : C₃₅H₃₉N₅O₄
[M+H]⁺ calculé : 594.3100
[M+H]⁺ mesuré : 594.3083

### Exemple 37. (exemple de référence) 5-(4-{[(Benzylcarbamoyl)amino]méthyl}-2-{[(3R)-3-méthyl-3,4-dihydroisoquinolin-2(1H)-yl]carbonyl}phényl)-N-(4-hydroxyphényl)-N-1,2-triméthyl-1H-pyrrole-3-carboxamide

Le composé du titre est obtenu selon le procédé de l'Exemple 32 en utilisant au Stade D l'isocyanate de benzyle comme agent acylant.
***Microanalyse élémentaire** : **(%mesuré*** (***théorique***))
%C=73.21(73.26);%H=5.98(6.3);%N=10.23(10.68)
***Masse haute résolution (ESI*+*****)** :*
Formule brute : C₄₀H₄₁N₅O₄
[M+H]⁺ calculé : 656.3239
[M+H]⁺ mesuré : 656.3256

### Exemple 38. 5-(5-Chloro-2-{[(3S)-3-(hydroxyméthyl)-3,4-dihydroisoquinolin-2(1H)-yl]carbonyl}phényl)-N-(4-hydroxyphényl)-1,2-diméthyl-N-phényl-1H-pyrrole-3-carboxamide

### Microanalyse élémentaire : (%mesuré (théorique))

%C=70.97(71.34);%H=5.2(5.32);%N=6.96(6.93)

### Exemple 39. N-(4-Hydroxyphényl)-1,2-diméthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1H-pyrrole-3-carboxamide

### Microanalyse élémentaire : (%mesuré (théorique))

%C=69.81(69.64);%H=5.6(5.51);%N=11.55(11.6)

### Masse haute résolution (ESI+) :

Formule brute : C₃₅H₃₄N₅O₅
[M+H]⁺ calculé : 604.2554
[M+H]⁺ mesuré : 604.2565

### Exemple 40. (exemple de référence) (3-{4-[(4-Hydroxyphényl)(méthyl)carbamoyl]-1,5-diméthyl-1H-pyrrol-2-yl}-4-{[(3R)-3-méthyl-3,4-dihydroisoquinolin(1H)-yl]carbonyl}benzyl)carbamate de phényle

### Stade A : N-(4-Benzyloxyphényl)-N,1,2-triméthyl-pyrrole-3-carboxamide

Le procédé est analogue à celui décrit au Stade A de l'Exemple 45.

### Stade B : Chlorhydrate de 5-[5-(aminométhyl)-2-{[(3R)-3-méthyl-3,4-dihydroisoquinolin-2(1H)-yl]carbonyl}phényl]-N-[4-(benzyloxy)phényl]-N,1,2-triméthyl-1H-pyrrole-3-carboxamide

Le composé de la Préparation III (1,84 g ; 3,99 mmol) et le composé du Stade A (1,60 g ; 4,48 mmol) sont dissous dans du diméthylacétamide (20 mL), puis de l'azote est mis à barboter dans la solution pendant 5 minutes. De l'acétate de potassium (0,78 g ; 7,48 mmol) et du dichlorure de bis(triphénylphosphine)palladium(II) (0,28 g ; 0,4 mmol) sont ajoutés au mélange qui est ensuite chauffé jusqu'à 105 °C et, après 10 minutes d'agitation, de l'eau (11 µL) est ajoutée. L'agitation à 105 °C sous une atmosphère d'azote est poursuivie pendant 6 heures supplémentaires. Le mélange réactionnel est laissé à refroidir jusqu'à température ambiante, le mélange résultant est filtré sur une courte colonne de célite puis purifié par CLHP préparative en utilisant de l'eau-TFA et de l'acétonitrile comme éluants. Le pH des fractions appropriées est ajusté à 7 avec du NaHCO₃, puis l'acétonitrile est évaporé sous pression réduite. Le solide précipité est retiré par filtration puis séché (5 mbar, 45 °C, 16 heures) pour former le [3-(4-{[4-**(benzyloxy)phényl](méthyl)carbamoyl}-1,5-diméthyl-1*H*-pyrrol-2-yl)-4-{[(3*R*)-3-méthyl-**3,4-dihydroisoquinolin-2(1*H*)-yl]carbonyl}benzyl]carbamate de *tert*-butyle qui est dissous / mis en suspension dans du HCl 4 M dans le dioxanne (35 mL). Le mélange hétérogène est agité pendant 30 minutes à température ambiante, puis évaporé à sec pour conduire au composé du titre qui est utilisé dans l'étape suivante sans purification.
**Masse haute résolution** (**ESI**+) **:**
Formule empirique : C₃₉H₄₀N₄O₃
[M+H]⁺ calculé : 613,3180
[M+H]⁺ mesuré : 613,3194
**IR:** ν : N-H+: 2854 cm⁻¹; >C=O: 1621 cm⁻¹; C-O-C: 1234, 1120, 1011 cm⁻¹

### Stade C : (3-{4-[(4-Hydroxyphényl)(méthyl)carbamoyl]-1,5-diméthyl-1H-pyrrol-2-yl}-4-{[(3R)-3-méthyl-3,4-dihydroisoquinolin-2(1H)-yl]carbonyl}benzyl)carbamate de phényle

Le composé du titre est obtenu à partir du composé du stade précédent selon le procédé du Stade D de l'Exemple 45.
***Microanalyse élémentaire** :* (%***mesuré (théorique))***
%C=73.2(72.88);%H=5.83(5.96);%N=8.13(8.72)
***Masse haute résolution (ESI*+*****)** :*
Formule brute : C₃₉H₃₈N₄O₅
[M+H]⁺ calculé : 643.2922
[M+H]⁺ mesuré : 643.2902

### Exemple 41. N-(3-Fluorophényl)-N-(4-hydroxyphényl)-1,2-diméthyl-5-(6-{[(3R)-3-méthyl-3,4-dihydroisoquinolin-3(1H)-yl]carbonyl}-1,3-benzodioxol-5-yl)-1H-pyrrole-3-carboxamide

### Microanalyse élémentaire : (%mesuré (théorique))

%C=71.44(71.95);%H=4.95(5.22);%N=6.8(6.8)

### Masse haute résolution (ESI+) :

Formule brute : C₃₇H₃₃FN₃O₅
[M+H]⁺ calculé : 618.2399
[M+H]⁺ mesuré : 618.2392

### Exemple 42. N-(4-Hydroxyphényl)-1,2-diméthyl-5-(6-{[(3R)-3,4-dihydroisoquinolin-2(1H)-yl]carbonyl}-1,3-benzodioxol-5-yl)-N-(1-méthyl-1H-pyrrolo[2,3-b]pyridin-5-yl)-1H-pyrrole-3-carboxamide

### Microanalyse élémentaire : (%mesuré (théorique))

%C=71.32(71.65);%H=5.17(5.4);%N=10.67(10.71)

### Masse haute résolution (ESI+) :

Formule brute : C₃₉H₃₆N₅O₅
[M+H]⁺ calculé : 654.2711
[M+H]⁺ mesuré : 654.2710

### Exemple 43. N-(4-Hydroxyphényl)-1,2-diméthyl-5-(6-{[(3R)-3-méthyl-5-(6-{[(3R)-dihydroisoquinolin-2(1H)-yl]carbonyl}-1,3-benzodioxol-5-yl)-N-phényl-1H-pyrrole-3-carboxamide

### Microanalyse élémentaire : (%mesuré (théorique))

%C=73.9(74.11);%H=5.16(5.55);%N=6.81(7.01)

### Masse haute résolution (ESI+) :

Formule brute : C₃₇H₃₄N₃O₅
[M+H]⁺ calculé : 600.2493
[M+H]⁺ mesuré : 600.2495

### Exemple 44. Chlorhydrate de N-(4-hydroxyphényl)1,2-diméthyl-N-(1-méthyl-1H)-pyrrolo[2,3-b]pyridin-5-yl)-5-(6-{[(3S)-3-(morpholin-4-ylméthyl)-3,4-dihydroisoquinolin-2(1H)-yl]carbonyl}-1,3-benzodioxol-5-yl)-1H-pyrrole-3-carboxamide

### Microanalyse élémentaire : (%mesuré (théorique))

%C=66.41(66.62);%H=5.08(5.59);%N=10.85(10.84);%Cl-=4.68(4.57)

### Masse haute résolution (ESI+) :

Formule brute : C₄₃H₄₂N₆O₆
[M+H]⁺ calculé : 739.3239
[M+H]⁺ mesuré : 739.3246

### Exemple 45. (exemple de référence) [2-(3-{4-[(4-Hydroxyphényl)(méthyl)carbamoyl]-1,5-diméthyl-1H-pyrrol-2-yl}-4-{[(3R)-3-méthyl-3,4-dihydroisoquinolin-2(1H)-yl]carbonyl}phényl)éthyl]carbamate de phényle

### Stade A : N-(4-Benzyloxyphényl)-N,1,2-triméthyl-pyrrole-3-carboxamide

A une suspension d'acide 1,2-diméthylpyrrole-3-carboxylique (2,085 g ; 15 mmol) dans le 1,2-dichloroéthane (40 mL), 1a1-chloro-*N,N*,2-triméthyl-prop-1-èn-1-amine (2,37 mL ; 17,9 mmol) est ajoutée, et la solution formée est agitée pendant 1 heure. A cette solution, une solution glacée de 4-benzyloxy-N-méthyl-aniline (3,73 g ;15 mmol) et de N-éthyl-N-isopropyl-propan-2-amine (7,75 mL ; 45 mmol) dans le 1,2-dichloroéthane (40 mL) est ajoutée goutte-à-goutte. Le mélange réactionnel est agité à température ambiante pendant 1 heure, puis il est dilué avec du dichlorométhane (250 mL) et lavé avec de l'eau (2 x 30 mL), séché sur Na₂SO₄ et évaporé. Le produit brut est trituré avec du diéthyléther et le solide formé est filtré pour conduire au composé du titre.
**IR:** ν : >C=O: 1616 cm⁻¹ ; amide: 1508 cm⁻¹; C-O-C: 1230, 1172, 1009 cm⁻¹

### Stade B : {2-[3-(4-{[4-(Benzyloxy)phényl](méthyl)carbamoyl}-1,5-diméthyl-1H-pyrrol-2-yl)-4-{[(3R)-3-méthyl-3,4-dihydroisoquinolin-2(1H)-yl]carbonyl}phényl]éthyl}carbamate de tert-butyle

Le composé de la Préparation I (0,869 g ; 1,67 mmol) et le composé du Stade A (0,558 g ; 1,67 mmol) sont dissous dans du diméthylacétamide (8 mL), puis de l'azote est mis à barboter dans la solution pendant 5 minutes. De l'acétate de potassium (0,33 g ; 3,34 mmol) et du dichlorure de bis(triphénylphosphine)palladium(II) (0,117 g ; 0,167 mmol) sont ajoutés au mélange qui est ensuite chauffé jusqu'à 140 °C et, après 10 minutes d'agitation, de l'eau (80 µL) est ajoutée. L'agitation à 140 °C sous une atmosphère d'azote est poursuivie pendant 16 heures supplémentaires. Le mélange réactionnel est laissé à refroidir jusqu'à température ambiante, puis évaporé. Le résidu est partagé entre du dichlorométhane (100 mL) et de l'eau (20 mL). La phase organique est lavée avec de l'eau (20 mL), séchée sur Na₂SO₄ et évaporée. Le résidu a été purifié par CLHP préparative en utilisant de l'eau-TFA et de l'acétonitrile comme éluants. Le pH des fractions appropriées est ajusté à 12 au moyen d'une solution aqueuse de NaOH, puis l'acétonitrile est évaporé sous pression réduite. Le résidu aqueux est extrait avec du dichlorométhane (2 x 100 mL). La phase organique est séchée sur Na₂SO₄ et évaporée à sec pour conduire au composé du titre.

### Masse haute révolution (ESI+) :

Formule brute: C₄₅H₅₀N₄O₅
[M+H]⁺ calculé: 727.3861
[M+H]⁺ mesuré: 727.3852
**IR:** ν : >C=O: 1705, 1618 cm⁻¹; amide: 1509 cm⁻¹; C-O-C: 1242, 1166, 1012 cm⁻¹

### Stade C : Chlorhydrate de 5-[5-(2-aminoéthyl)-2-{[(3R)-3-méthyl-3,4-dihydroisoquinolin-2(1H)-yl]carbonyl}phényl]-N-[4-(benzyloxy)phényl]-N,1,2-triméthyl-1H-pyrrole-3-carboxamide

Le composé du Stade B (0,35 g ; 0,48 mmol) a été dissous / mis en suspension dans du HCl 4 M dans le dioxane (3 mL). Le mélange hétérogène est agité pendant 30 minutes à température ambiante, puis évaporé à sec pour donner conduire au composé du titre qui est utilisé dans l'étape suivante sans aucune purification.

### Masse haute révolution (ESI+) :

Formule brute: C₄₀H₄₂N₄O₃
[M+H]⁺ calculé: 627.3337
[M+H]⁺ mesuré: 627.3309
**IR:** ν : C-H: 2931 cm⁻¹; >C=O: 1608 cm⁻¹; amide: 1508 cm⁻¹; C-O-C: 1233, 1012 cm⁻¹

### Stade D : 2-(3-{4-[(4-Hydroxyphényl)(méthyl)carbamoyl]-1,5-diméthyl-1H-pyrrol-2-yl}-4-{[(3R)-3-méthyl-3,4-dihydroisoquinolin-2(1H)-yl]carbonyl}phényl)éthyl]carbamate de phényle

Le composé du Stade C (0,10 g ; 0,15 mmol) est mis en solution dans le dichlorométhane. On y ajoute du carbonochloridate de phényle (0,027 g ; 0,17 mmol) et de la diisopropyléthylamine (0,097 g, 0,75 mmol). Le milieu réactionnel est agité à température ambiante pendant 30 minutes. Après dilution dans le dichlorométhane (100 mL), la phase organique phase est lavée avec de l'eau (20 mL), évaporée et concentrée. Le résidu est ensuite dissous dans l'éthanol et un catalyseur Pd/C est ajouté (10 mg). Le milieu réactionnel est hydrogéné à pression atmosphérique à température ambiante. Lorsque la réaction est terminée, le catalyseur est éliminé par filtration, le filtrat est concentré et le produit brut est purifié par chromatographie sur gel de silice en utilisant le dichlorométhane et le méthanol comme éluants, pour conduire au produit du titre.
***Microanalyse élémentaire :*** (%***mesuré*** (***théorique***))
%C=72.36(73.15);%H=6.15(6.14);%N=8.14(8.53)
***Masse haute résolution (ESI*+*) :***
Formule brute : C₄₀H₄₀N₄O₅
[M+H]⁺ calculé : 657.3077
[M+H]⁺ mesuré : 657.3062

### Exemple 46. (exemple de référence) N-(4-Hydroxyphényl)-N,1,2-triméthyl-5-(2-{[(3R)-3-mathyl-3,4-dihydroisoquinolin-2(1H)-yl]carbonyl}-5-{2-[phénoxyacétyl)amino]éthyl}phényl)-1H-pyrrole-3-carboxamide

Le composé du titre est obtenu selon le procédé de l'Exemple 45 en utilisant au Stade D le chlorure de 2-phénoxyacétyle comme agent acylant.

### Microanalyse élémentaire : (%mesuré (théorique))

%C=72.74(73.41);%H=6.38(6.31);%N=7.65(8.35)

### Masse haute résolution (ESI+) :

Formule brute : C₄₁H₄₂N₄O₅
[M+H]⁺ calculé : 671.3235
[M+H]⁺ mesuré : 671.3226

### Exemple 47. Chlorhydrate de N-(4-hydroxyphényl)-1,2-diméthyl-N-(1-méthyl-1H-pyrazol-4-yl)-5-(6-{[(3S)-3-(morpholin-4-ylméthyl)-3,4-dihydroisoquinolin-2(1H)-yl]carbonyl}-1,3-benzodioxol-5-yl)-pyrrole-3-carboxamide

### Microanalyse élémentaire : (%mesuré (théorique))

%C=64.25(64.59);%H=5.4(5.7);%N=11.41(11.59);%Cl-=4.93(4.89)

### Exemple 48. N-(4-Hydroxypbény)-N-(2-méthoxypyrimidin-5-yl)-1,2diméthyl-5-(6-{[(3R)-3-méthyl-3,4-dihydroisoquinolin-2-(1H)-yl]carbonyl}-1,3-benzodioxol-5-yl)-1H-pyrrole-3-carboxamide

### Microanalyse élémentaire : (%mesuré (théorique))

%C=68.07(68.45);%H=5(5.27);%N=10.74(11.09)

### Exemple 49. Dichlorhydrate de N-(4-hydroxyphényl-1,2-diméthyl-N-(1-méthyl-2,3-dihydro-1H-pyrrolo[2,3-b]pyridin-5-yl)-5-(6-{[(3S)-3-(morpholin-4-yl-ylméthyl)-3,-4-dihydroisoquinolin-2(1H)-yl]carbonyl}-1,3-benzodioxol-5-yl)-1H-pyrrole-3-carboxamide

### Stade A : N-(4-{[tert-Butyl(diméthyl)silyl]oxy}phényl)-1,2-diméthyl-N-(1-méthyl-1H-pyrrolo[2,3-b]pyridin-5-yl)-5-(6-{[(3S)-3-(morpholin-4-ylméthyl)-3,4-dihydroisoquinolin-2(1H)-yl]carbonyl}-1,3-beuzodioxol-5-yl)-1H-pyrrole-3-carboxamide

Le composé du titre est obtenu selon le procédé de l'Exemple 1 en utilisant l'acide de la Préparation 11 et la (3*S*)-3-(4-morpholinylméthyl)-1,2,3,4-tétrahydroisoquinoline (voir Préparation 3') au Stade A, ainsi que le composé de la Préparation 11" au Stade C.

### Stade B : Dichlorhydrate de N-(4-hydroxyphéuyl)-1,2-diméthyl-N-(1-méthyl-2,3-dihydro-1H-pyrrolo[2,3-b]pyridin-5-yl)-5-(6-{[(3S)-3-(morpholin-4-ylméthyl)-3,4-dihydroisoquinolin- 2(1H)-yl]carbonyl}-1,3-benzodioxol-5-yl)-1H-pyrrole-3-carboxamide

A une solution de 0,21 g (0,2 mmol) du composé du Stade A dans 3 mL d'acide acétique sont ajoutés 85 mg de cyanoborohydrure de sodium (5 équivalents). Le milieu réactionnel est agité pendant 3 h à température ambiante, puis pendant une nuit à 50°C. On ajoute ensuite 2,6 équivalents de cyanoborohydrure de sodium et le milieu réactionnel est chauffé à 50°C pendant 3 h. L'opération est réitérée une deuxième fois (ajout de 2,6 équivalents de cyanoborohydrure de sodium, puis chauffage à 50°C pendant 3 h). Après coévaporation de l'acide acétique en présence de toluène, le résidu est repris dans 3 mL de méthanol. Le pH de la solution est ajusté à 12 à l'aide d'une solution méthanolique de potasse 1M. Après une nuit d'agitation à température ambiante, le milieu réactionnel est versé sur une solution aqueuse saturée en bicarbonate de sodium et extrait au dichlorométhane. La phase organique résultante est lavée avec de la saumure, séchée sur Na₂SO₄, filtrée et concentrée sous pression réduite. Le produit obtenu est purifié par chromatographie sur gel de silice (dichlorométhane/éthanol/ammoniac 94/6/0,6) puis sur colonne RP18 Lichroprep (eau/acétonitrile/acide trifluoroacétique). Après évaporation de l'acétonitrile, le produit est neutralisé du bicarbonate de sodium. Il est ensuite extrait avec de l'acétate d'éthyle. La phase organique est séchée sur Na₂SO₄, filtrée et concentrée sous pression réduite. Le résidu obtenu est solubilisé dans l'acétonitrile et salifié avec une solution aqueuse de HCl 1M. Après lyophilisation, le produit du titre est obtenu sous la forme d'un solide.
***Masse haute résolution (ESI+) :***
Formule brute : C₄₃H₄₄N₆O₆
[M+H]⁺ calculé : 741.3395
[M+H]⁺ mesuré : 741.3400

### Exemple 50. Chlorhydrate de N-(4-hydroxyphényl)-N-(2-méthoxyprimidin-5-yl)-1,2-diméthyl-5-(6-{[(3S)-3-(morpholin-4-yméthyl)-3,4-dihydroisoquinolin-2-(1H)-y]]carbonyl}-1,3-benzodioxol-5-yl)-1H-pyrrole-3-carboxamide

### Masse haute résolution (ESI+) :

Formule brute : C₄₀H₄₀N₆O₇
[M+H]⁺ calculé : 717.3031
[M+H]⁺ mesuré : 717.3031

### Exemple 51. (exemple de référence) 5-(5-{[(Benzylcarbamoyl)amino]méthyl}-2-{[(3R)-3-méthyl-3,4-dihydroisoquinolin-2(1H)-yl]carbonyl}phényl)-N-(4-hydroxyphényl)-N-1,2-triméthyl-1H-pyrrole-3-carboxamide

Le composé du titre est obtenu selon le procédé de l'Exemple 40 en utilisant au Stade C l'isocyanate de benzyle comme agent acylant.

### Microanalyse élémentaire : (%mesuré (théorique))

%C=73.39(73.26);%H=6.16(6.3);%N=10.08(10.68)

### Masse haute résolution (ESI+) :

Formule brute : C₄₀H₄₁N₅O₄
[M+H]⁺ calculé : 656.3239
[M+H]⁺ mesuré : 656.3226

### Exemple 52. (exemple de référence) 5-(5-{[(Éthylcarbamoyl)amino]méthyl}-2-{[(3R)-3-méthyl-3,4-dihydroisoquinolin-2(1H)-yl]carbonyl}phényl)-N-(4-hydroxyphényl)-N,1,2-triméthyl-1H-pyrrole-3-carboxamide

Le composé du titre est obtenu selon le procédé de l'Exemple 40 en utilisant au Stade C l'isocyanate d'éthyle comme agent acylant.

### Microanalyse élémentaire : (%mesuré (théorique))

%C=71.4(70.8);%H=6.41(6.62);%N=11.24(11.8)

### Masse haute résolution (ESI+) :

Formule brute : C₃₅H₃₉N₅O₄
[M+H]⁺ calculé : 594.3082
[M+H]⁺ mesuré : 594.3069

### Exemple 53. (exemple de référence) N-(4-hydroxyphényl)-N,1,2-triméthyl-5-(2-{[(3R)-3-méthyl-3,4-dihydroisoquinolin-2(1H)-yl]-carbonyl}-5-{[(phénoxyacétyl)amino]méthyl}phenyl)-1H-pyrrole-3-carboxamide

Le composé du titre est obtenu selon le procédé de l'Exemple 40 en utilisant au Stade C le chlorure de 2-phénoxyacétyle comme agent acylant.
***Microanalyse élémentaire : (%mesuré (théorique))***
%C=72.32(73.15);%H=6.21(6.14);%N=7.84(8.53)
***Masse haute résolution* (*ESI*+) *:***
Formule brute : C₄₀H₄₀N₄O₅
[M+H]⁺ calculé : 657.3079
[M+H]⁺ mesuré : 657.3105

### Exemple 54. (exemple de référence) N-(4-Hydroxyphényl)-1,2-diméthyl-5-(2-{[(3R)-3-méthyl-3,4-dihydroisoquinolin-2(1H)-yl]carbonyl}-5-{[(phenoxyacétyl)amino]méthyl}phényl)-N-(1-méthyl-1H-pyrrolo[2,3-b]pyridin-5-yl)-1H-pyrrole-3-carboxamide

### Microanalyse élémentaire : (%mesuré (théorique))

%C=73.87(73.04);%H=5.47(5.74);%N=10.26(10.87)

### Exemple 55. (exemple de référence) 5-(5-[2-({[2-(4-Fluorophényl)éthyl]sulfonyl}amino)éthyl]-2-{[(-3R)-3-méthyl-3,4-dihydroisoquinolin-2(1H)-yl]carbonyl}phényl)-N-(4-hydroxyphényl)-N,1,2-triméthyl-1H-pyrrole-3-carboxamide

Le composé du titre est obtenu selon le procédé de l'Exemple 45 en utilisant au Stade D le chlorure de 2-(4-fluorophényl)éthanesulfonyle comme agent acylant.
***Microanalyse élémentaire : (%mesuré (théorique))***
%C=67.42(68.12);%H=5.76(6);%N=7.27(7.75);S=3.68(4.44)
***Masse haute résolution* (*ESI*+) *:***
Formule brute : C₄₁H₄₃FN₄O₅S
[M+H]⁺ calculé : 723.3018
[M+H]⁺ mesuré : 723.3011

### Exemple 56. (exemple de référence) 5-(5-{2-[(Benzylcarbamoyl)amino]éthyl}-2-{[(3R)-3-méthyl-3,4-dihydroisoquinolin-2(1H)-yl]carbonyl}phényl)-N-(4-hydroxyphényl)-N,1,2-triméthyl-1H-pyrrole-3-carboxamide

Le composé du titre est obtenu selon le procédé de l'Exemple 45 en utilisant au Stade D l'isocyanate de benzyle comme agent acylant.
***Microanalyse élémentaire : (%mesuré (théorique))***
%C=71.14(73.52);%H=6.47(6.47);%N=9.42(10.46)
***Masse haute résolution* (*ESI*+) *:***
Formule brute : C₄₁H₄₃N₅O₄
[M+H]⁺ calculé : 670.3395
[M+H]⁺ mesuré : 670.3390

### Exemple 57. (exemple de référence) N-(4-Hydroxyphényl)-N-1,2,-triméthyl-5-(2-{[(3R)-3-méthyl-3,4-dihydroisoquinolin-2(1H)-yl]carbonyl}-5-2[(phénylacétyl)amino]éthyl}phényl)-1H-pyrrole-3-carboxamide

Le composé du titre est obtenu selon le procédé de l'Exemple 45 en utilisant au Stade D le chlorure de phénylacétyle comme agent acylant.
***Microanalyse élémentaire : (%mesuré (théorique))***
%C=75.46(75.21);%H=6.11(6.46);%N=8.31(8.56)
***Masse haute résolution* (*ESI*+) *:***
Formule brute : C₄₁H₄₂N₄O₄
[M+H]⁺ calculé : 655.3286
[M+H]⁺ mesuré : 655.3285

### Exemple 58. (exemple de référence) N-4-(Hydroxyphényl)-N,1,2-triméthyl-5-(2-{[(3R)-3-méthyl-3,4-dihydroisoquinolin-2(1H)-yl]carbonyl}-5-{2-[(phénylcarbamoyl)amino]éthyl}phényl)-1H-pyrrole-3-carboxamide

Le composé du titre est obtenu selon le procédé de l'Exemple 45 en utilisant au Stade D l'isocyanate de phényle comme agent acylant.
***Microanalyse élémentaire : (%mesuré (théorique))***
%C=71.25(73.26);%H=6.12(6.3);%N=9.61(10.68)
***Masse haute résolution* (*ESI*+) *:***
Formule brute : C₄₀H₄₁N₅O₄
[M+H]⁺ calculé : 656.3239
[M+H]⁺ mesuré : 656.3226

### Exemple 59. (exemple de référence) 5-(4-[({[2-(4-Fluorophényl)éthyl]sulfonyl}amino)méthyl]-2-{[(3R)-3-méthyl-3,4-dihydroisoquinolin-2(1H)-yl]carbonyl}phényl)-N-(-4-hydroxyphényl)-N,1,2-triméthyl-1H-pyrrole-3-carboxamide

Le composé du titre est obtenu selon le procédé de l'Exemple 32 en utilisant au Stade D le chlorure de 2-(4-fluorophényl)éthanesulfonyle comme agent de sulfonylation.

### Microanalyse élémentaire : (%mesuré (théorique))

%H=5.19(5.83);%N=7.28(7.9);S=4.45(4.52);%C=66.17(67.78)

### Masse haute résolution (ESI+) :

Formule brute : C₄₀H₄₁FN₄O₅S
[M+H]⁺ calculé : 709.2866
[M+H]⁺ mesuré : 709.2866

### Exemple 60. Chlorhydrate de 5-(5-chloro-2-{[(3S)-3-{[2-(diméthylamino) éthoxy]méthyl}-3,4-dihydroisoquinolin-2(1H)-yl]carbonyl}phényl)-N-(4-hydroxy phényl)-1,2-diméthyl-N-phényl-1H-pyrrole-3-carboxamide

On utilise le procédé de l'Exemple 29 en remplaçant d'une part le bromhydrate de 4-(2-bromoéthyl)morpholine utilisé au Stade B par le chlorhydrate de 2-chloroéthyldiméthylamine, et en ajoutant d'autre part une quantité catalytique d'iodure de tétrabutylammonium.
***Masse haute résolution* (*ESI*+) *:***
Formule brute : C₄₀H₄₁ClN₄O₄
[M+H]⁺ calculé : 677.2890
[M+H]⁺ mesuré : 677.2887

### Exemple 61. Chlorhydrate de N-(4-hydroxyphényl-1,2-diméthyl-5-(6-{[(3R)-3-méthyl-3,4-dihydroisoquinolin-2(1H)-yl]carbonyl}-1,3-benzodioxol-5-yl)-N-(pyridin-4-yl)-1H-pyrrole-3-carboxamide

### Masse haute résolution (ESI+) :

Formule brute : C₃₆H₃₂N₄O₅
[M+H]⁺ calculé: 601.2445
[M+H]⁺ mesuré : 601.2424

### Exemple 62. 5-(5-Fluoro-4-méthoxy-2-{[(3R)-3-méthyl-3,4-dihydroisoquinolin-2(1H)-yl]carbonyl}phényl)-N-(4-hydroxyphényl)-1,2-diméthyl-N-(1-méthyl-1H-pyrazol-4-yl)-1H-pyrrole-3-Carboxamide

### Microanalyse élémentaire : (%mesuré (théorique))

%C=68.99(69.18);%H=5.89(5.64);%N=11.35(11.52)

### Masse haute résolution (ESI+) :

Formule brute : C₃₅H₃₄FN₅O₄
[M+H]⁺ calculé : 608.2668
[M+H]⁺ mesuré : 608.2640

### Exemple 63. 5-(5-Chloro-2-{[(3R)-3-méthyl-3,4-dihydroisoquinolin-2(1H)-yl]carbonyl}phényl)-N-(4-hydroxyphényl)-1,2-diméthyl-N-(1-méthyl-1H-pyrrolo[2,3-b]pyridin-5-yl)-1H-pyrrole-3-carboxamide

### Microanalyse élémentaire : (%mesuré (théorique))

%C=70.48(70.85);%H=5.67(5.32);%N=11.2(10.87)

### Masse haute résolution (ESI+) :

Formule brute : C₃₈H₃₄ClN₅O₃
[M+H]⁺ calculé : 666.2242
[M+H]⁺ mesuré : 666.2235

### Exemple 64. (exemple de référence) Chlorhydrate de N,1,2-triméthyl-N-(1-méthyl-1H-pyrrolo[2,3-b]pyridin-5-yl)-5-(6-{[(3S)-3-(morpholin-4-ylméthyl)-3,4-dihydroisoquinolin-2(1H)-yl]carbonyl}-1,3-benzodioxol-5-yl)-1H-pyrrole-3-carboxamide

### Masse haute résolution (ESI+) :

Formule brute : C₃₈H₄₀N₆O₅
[M+H]⁺ calculé : 661.3133
[M+H]⁺ mesuré: 661.3125

### Exemple 65. Chlorhydrate de 1,2-diméthyl-N-(1-méthyl-1H-pyrrolo[2,3-b]pyridin-5-yl)-5-(6-{[(3S)-3-(morpholin-4-ylméthyl)-3,4-dihydroisoquinolin-2(1H)-yl]carbonyl}-1,3-benzodioxol-5-yl)-N-phényl-1H-pyrrole-3-carboxamide

### Masse haute résolution (ESI+) :

Formule brute : C₄₃H₄₂N₆O₅
[M+H]⁺ calculé : 723.3289
[M+H]⁺ mesuré : 723.3287

### Exemple 66. (exemple de référence) (4-{4-[(4-hydroxyphényl)(méthyl)carbamoyl]-1,5-diméthyl-1H-pyrrol-2-yl}-3-{[(3R)-3-méthyl-3,4-dihydroisoquinolin-2(1H)-yl]carbonyl}benzyl)carbamate de 4-méthylphényle

Le composé du titre est obtenu selon le procédé de l'Exemple 32 en utilisant au Stade D le 4-méthylphénylcarbonochloridate comme agent acylant.
***Masse haute résolution* (*ESI*+) *:***
Formule brute : C₄₀H₄₀N₄O₅
[M+H]⁺ calculé : 657.3079
[M+H]⁺ mesuré : 657.3076

### Exemple 67. Chlorhydrate de 5-(5-fluoro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(1-méthyl-1H-pyrazol-4-yl)-1H-pyrrole-3carboxamide

### Microanalyse élémentaire : (%mesuré (théorique))

%C=65.69(65.28);%H=5.38(5.77);%N=11.18(12.02);%Cl-=5.61(5.07)

### Exemple 68. 5-(5-Fluoro-4-hydroxy-2-{[(3R)-3-méthyl-3,4-dihydroisoquinolin-2(1H)-yl]carbonyl}phény-N-(4-hydroxyphényl)-1,2-diméthyl-N-(1-méthyl-1H-pyrazole-4-yl)-1H-pyrrole-3-Carboxamide

Une solution de tribromoborane 1N (1,3 équivalent) dans le dichlorométhane est coulée rapidement à température ambiante sur une solution du composé de l'Exemple 62 dans le dichlorométhane. Cette opération est renouvelée 2 fois pour terminer la réaction en 5 h. Le milieu réactionnel est versé sur de l'éthanol anhydre à 5°C. Après 10 min d'agitation, une solution aqueuse saturée de NaHCO₃ est ajoutée. Le mélange est extrait au dichlorométhane, séché sur Na₂SO₄ et concentré à sec. Le produit brut obtenu est purifié par CLHP préparative en utilisant de l'eau, le TFA et l'acétonitrile comme éluants. Le pH des fractions appropriées est ajusté à 12 par une solution aqueuse saturée en NaHCO₃, puis l'acétonitrile est évaporé sous pression réduite. Le résidu aqueux est extrait avec du dichlorométhane. La phase organique est séchée sur Na₂SO₄ et évaporée à sec pour conduire au composé du titre.
***Masse haute résolution* (*ESI*+) *:***
Formule brute : C₃₄H₃₂FN₅O₄
[M+H]⁺ calculé : 594.2511
[M+H]⁺ mesuré : 594.2517

### Exemple 69. Chlorhydrate de N-(4-hydroxyphényl)-1,2-diméthyl-5(6-{[(3S)-3-[(4-méthylpipérazin-1-yl)méthyl]-3,4-dihydroisoquinolin-2(1H)-yl]carbonyl}-1,3-benzodioxol-5-y)-N-(1-méthyl-1H-pyrrolo[2,3-b]pyridin-5-yl)-1H-pyrrole-3-carboxamide

### Masse haute résolution (ESI+) :

Formule brute : C₄₄H₄₅N₇O₅
[M+H]⁺ calculé : 752.3555
[M+H]⁺ mesuré : 752.3552

### Exemple 70. Chlorhydrate de N-(4-hydroxyphényl)-1,2-diméthyl-5-(6-{[(3S)-3-[(4-méthylpipérazin-1-yl)méthyl]-3,4-dihydroisoquinolin-2(1H)-yl]carbonyl}-1,3-benzodioxol-5-yl)-N-(pyridin-4-yl)-1H-pyrrole-3-carboxamide

### Masse haute résolution (ESI+) :

Formule brute : C₄₁H₄₂N₆O₅
[M+H]⁺ calculé : 699.3289
[M+H]⁺ mesuré : 699.3293

### Exemple 71. Chlorhydrate de 5-(5-chloro-2-{[(3S)-3-(morpholin-4-ylméthyl)-3,4-dihydroisoquinolïn-2(1H)-yl]carbonyl}phényl)-N-(4-hydroxyphényl)-1,2-diméthyl-N-(1-méthyl-1H-pyrrolo[2,3-b]pyridin-5-yl)-1H-pyrrole-3-carboxamide

### Masse haute résolution (ESI+) :

Formule brute : C₄₂H₄₁ClN₆O₄
[M+H]⁺ calculé : 723.3289
[M+H]⁺ mesuré : 723.3287

### Exemple 72. 5-(5-Chloro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(1-méthyl-1H-benzimidazol-5-yl)-1H-pyrrole-3-carboxamide

### Microanalyse élémentaire : (%mesuré (théorique))

%C=68.90(69.17);%H=5.32(5.67);%N=11.40(11.52)

### Exemple 73. Dichlorhydrate de N-(4-hydroxyphényl)-1,2-diméthyl-5-(6-{[(3S)-3-(morpholin-4-ylméthyl)-3,4-dihydroisoquinolin-2(1H)-yl]carbonyl}-1,3-benzodioxol-5-yl)-N-(pyridin-4-yl)-1H-pyrrole-3-carboxamide

### Masse haute résolution (ESI+) :

Formule brute : C₄₀H₃₉N₅O₆
[M+H]⁺ calculé : 686.2973
[M+H]⁺ mesuré : 686.2971

### Exemple 74. Chlorhydrate de 5-(5-chloro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(pyridin-4-yl)-1H-pyrrole-3-carboxamide

### Masse haute résolution (ESI+) :

Formule brute : C₃₉H₃₈ClN₅O₄
[M+H]⁺ calculé : 676.2685
[M+H]⁺ mesuré : 676.2684

### Exemple 75. Chlorhydrate de N-(4-hydroxyphényl)-1-méthyl-N-(1-méthyl-1H-pyrazol-4-yl)-5-(6-{[(3S)-(morpholin-4-ylméthyl)-3,4-dihydroisoqilinolin-2(1H)-yl]carbonyl}-1,3-benzodioxol-5-yl)-2-(trifluorométhyl)-1H-pyrrole-3-carboxamide

### Microanalyse élémentaire (% théorique : mesuré)

%C=60.12:59.77%H=4.92:4.76; %N=10.79:10.39 %Cl=4.55:5.17

### Masse haute résolution (ESI+) :

Formule brute : C₃₉ H₃₇ F₃ N₆ O₆
[M+H]⁺ calculé : 743.2799
[M+H]⁺ mesuré : 742.2802

**Exemple 76. 2-(Difluorométhyl)-*N*-(4-hydroxyphényl)-1-méthyl-*N-*(1-méthyl-1*H-*pyrazol-4**-**yl)-5-(6-{[(3*S*)-3-(morpholin-4-ylméthyl)3,4-dihydroisoquinolin-2(1*H*)-yl]carbonyl}-1,3-benzodioxol-5-yl)-1*H*-pyrrole-3-carboxamide**
**Exemple 77. *N*-(4-Hydroxyphényl)-2-(méthoxyméthyl)-1-méthyl-*N*-(1-méthyl-1*H-*pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 78. 2**-(**2,2-Difluoroéthyl)-*N(*-4*-*hydroxyphényl)-1-méthyl-*N*-(1-méthyl-1*H-*pyrazol-4-yl)-5-(6-(6-{[(3*S*)-3-(morpholin-4-ylméthyl)-3,-4-dihydroisoquinolin-2(1*H*)-yl]carbonyl}-1,3-benzodioxol-5-yl)-1*H*-pyrrole-3-carboxamide**
**Exemple 79. *N*-(4-Hydroxyphényl)-1-méthyl-*N-*(1-méthyl-1*H*-pyrazole-4-yl)-5-(6-{[(3*S*)-3-(morpholin-4-ylméthyl)-3,4-dihydroisoquinolin-2(1*H*)-yl]carbonyl}-1,3-benzodioxol-5**-**yl)-2-(2,2,2-trifluoroéthyl)-1*H-*pyrrole-3-carboxamide**
**Exemple 80. *N*-(4-Hydroxyphényl)-2-(2-méthoxyéthyl)-1-méthyl-*N*-(1-méthyl-1*H-*pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 81. Chlorhydrate de *N*-(4-hydroxyphényl)-1-méthyl-5-(6-{[(3*R*)-3-méthyl-3,4-dihydroisoquinolin-2(1*H*)-yl]carbonyl}-1,3-benzodioxol-5-yl)-*N-*(1-méthyl-1*H-*pyrazol-4-yl)-2-[2-(morpholin-4-yl)éthyl]-1*H*-pyrrole-3-carboxamide**
   On procède selon le protocole général de l'Exemple 1 en utilisant au Stade A le composé de la Préparation 22. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.
   ***Masse haute résolution (ESI*+*) :***
   Formule brute : C₄₀ H₄₂ N₆ O₆
   [M+H]⁺ calculé : 703.3239
   [M+H]⁺ mesuré : 703.3236
**Exemple 82. *N*-(4-Hydroxyphényl)-1-méthyl-5-(6-{[(3*R*)-3-méthyl-3,4-dihydroisoquinolin-2(1*H*)-yl]carbonyl}1,3-benzoidoxol-5-yl)-*N*-(1-méthyl-1*H-*pyrazol-4-yl)-2-(morpholin-4-ylméthyl)-1*H*-pyrrole-3-carboxamide**
   On procède selon le protocole général de l'Exemple 1 en utilisant au Stade A le composé de la Préparation 23.
   ***Masse haute résolution** (**ESI***+*) **:***
   Formule brute : C₃₉ H₄₀ N₆ O₆
   [M+H]⁺ calculé : 689.3082
   [M+H]⁺ mesuré : 689.3085
**Exemple 83. *N-(*4-Hydroxyphényl)-1-méthyl-*N*-(1-méthyl-1*H*-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-2-(trifluorométhyl)-1*H*-p**y**rrole-3-carboxamide**
**Exemple 84. 2-(Difluorométhyl)-*N*-(4-hydroxyphényl)-1-méthyl-*N*-(1-méthyl-1*H-*pyrrolo[2,3-*b*]pyridin-5-yl)-5-(6**-**{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 85. *N*-(4-Hydroxyphényl)-2-(méthoxyméthyl)-1-méthyl-*N*-(1-méthyl)-1*H-*pyrrolo[2,3-*b*]pyridin-5-yl)-5-(6{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 86. 2-(2,2-Difluoroéthyl}-*N*-(4-hydroxyphényl)-1-méthyl-*N*-(1-méthyl-1*H-*pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 87. *N*-(4-Hydroxyphényl)-1-méthyl-*N*-(1-méthyl-1*H*-pyrrole[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-2-(2,2,2-trifluoroéthyl)-1*H*-pyrrole-3-carboxamide**
**Exemple 88. Chlorhydrate de *N*-(4-hydroxyphényl-2-(2-méthoxyéthyl)-1-méthyl-*N-*(1-méthyl-1*H*-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
   On procède selon le protocole général de l'Exemple 1 en utilisant les préparations adéquates. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.
   ***Microanalyse élémentaire :* (% *théorique : mesuré)***
   %C=65.97:66.15; %H=5.78:5.46; %N=10.26:10.24; %Cl-=4.33:4.76
   ***Masse haute résolution* (ESI/FIA/HR et MS/MS) *:***
   Formule brute : C₄₅ H₄₆ N₆ O₇
   [M+H]⁺ calculé : 783.3501
   [M+H]⁺ mesuré : 783.3502
**Exemple 89. *N*-(4-Hydroxyphényl)-1-méthyl-*N*-(1-méthyl-1*H*-pyrrolo[2,3-*b*]pyridin-5-yl)-2-[2-(morpholin-4-yl)éthyl]-5-(6-{[(3*R*)-3-méthyl-3,4-dihydroisoquinohn-2(1*H*)-yl]carbonyl}-1,3-benzodioxol-5-yl)-1*H*-pyrrole-3-carboxamide**
**Exemple 90. *N*-(4-Hydroxyphényl)-1-méthyl-*N-*(1-méthyl-1*H*-pyrrolo[2,3-*b*]pyridin-5-yl)-2-(morpholin-4-ylméthyl)-5-(6-{[(3*R*)-3-méthyl-3,4-dihydroisoquinolin-2(1*H*)-yl]carbonyl}-1,3-benzodioxol-5-yl)-1*H*-pyrrole-3-carboxamide**
**Exemple 91. *N*-(4-Hydroxyphényl)-2-méthyl-*N-*(1-méthyl-1*H-*pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H-*pyrrole-carboxamide**
**Exemple 92. Chlorhydrate de 1-éthyl-*N*-(4-hydroxyphényl)-2-méthyl-*N*-(1-méthyl-1*H*-pyrazol-4-yl)**-**5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
   On procède selon le protocole décrit à l'Exemple 1, en utilisant les composés issus des Préparations 20 et 3' au Stade A et le composé issu de la Préparation 1" au Stade C. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.
   ***Microanalyse élémentaire :* (% *théorique : mesuré)***
   %C=64.99:65.61; %H=5.86:5.39; %N=11.37:11.43; %Cl=4.80:4.42
   ***Masse haute résolution* (*ESI*+*) :***
   Formule brute : C₄₀H₄₂N₆O₆
   [M+H]⁺ calculé : 703.3239
   [M+H]⁺ mesuré : 703.3236
**Exemple 93. Chlorhydrate de *N*-(4-hydroxypihényl)-1-(2-méthoxyéthyl)-2-méthyl-*N-*(1-méthyl-1*H*-pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H-*pyrrole-3-carboxamide**
   ***Microanalyse élémentaire :* (% *théorique : mesuré)***
   %C=64.01:64.10; %H=5.90:5.63; %N=10.92:10.88; %Cl-=4.61:4.70.
   ***Masse haute résolution (*ESI**+***) :***
   Formule brute : C₄₁ H₄₄ N₆ O₇
   [M+H]⁺ calculé : 733.3344
   [M+H]⁺ mesuré : 733.3345
**Exemple 94. Chlorhydrate de 1-(2-fluoroéthyl)-*N*-(4-hydroxyphényl)-2-méthyl-*N*-(1-méthyl-1*H*-pyrazol-4-yl)-5-(6-{[(3*S*)-3-(morpliolin-4-ylméthyl)-3,4-dihydroisoquinolin-2(1*H*)-yl]carbonyl}-1,3-benzodioxol-5-yl)-1*H*-pyrrole-3-carboxamide**
   On procède selon le protocole décrits aux Stades A-D de l'Exemple 1, en utilisant les composés issus des Préparations 21 et 3' au Stade A et le composé issu de la Préparation 1" au Stade C. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.
   ***Microanalyse élémentaire :* (% *théorique : mesuré)***
   %C=63.44:63.25; %H=5.59:5.09; %N=11.10:11.02; %Cl=4.68:4.74
   ***Masse haute résolution* (*ESI*+)** *:*
   Formule brute : C₄₀H₄₁FN₆O₆
   [M+H]⁺ calculé : 721.3144
   [M+H]⁺ mesuré : 721.3147
**Exemple 95. Chlorhydrate de 1-(2,2-difluoroéthyl)-*N-*(4-hydroxyphényl)-2-méthyl-*N*-(1-méthyl-1*H*-pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
   Le composé du titre est obtenu selon le procédé de l'Exemple 1 en utilisant l'acide de la Préparation 32 et le composé de la Préparation 3' au Stade A, ainsi que le composé de la Préparation 1" au Stade C. Le composé obtenu est salifié et lyophilisé tel que décrit dans le procédé général pour obtenir le composé du titre.
   ***Microanalyse élémentaire :* (% *théorique : mesuré)***
   %C=61.97:61.89; %H=5.33:5.04; %N=10.84:10.85; %Cl⁻=4.57:4.55
   ***Masse haute résolution*** (***ESI***+/***HR***, ***ESI***-/***LR***) ***:***
   Formule brute : C₄₀ H₄₀ F₂ N₆ O₆
   [M+H]⁺ calculé : 739.3050
   [M+H]⁺ mesuré : 739.3052

### Exemple 96. Chlorhydrate de N-(4-hydroxyphényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1-[2-(morpholin-4-yl)éthyl]-1H-pyrrole-3-carboxamide

### Stade A: N-[4-[tert-butyl(diméthyl)silyl]oxyphényl]-2-méthyl-5-[6[(3R)-3-méthyl-3,4-dihydro-1H-isoquinoline-2-carbonyl]-1,3-benzodioxol-5-yl]-N-(1-méthyl-1H-pyrazol-4-yl)-1-(2-morpholinoéthyl)pyrrole-3-carboxamide

Le composé obtenu au Stade E de l'Exemple 99 est dissous dans 6 mL de tétrahydrofurane. L'iodure de sodium (100 mg, 0,67 mmol) est ajouté, suivi de la morpholine (0,21 mL, 2,53 mmol) au goutte à goutte. Le milieu réactionnel est chauffé en flacon scellé à 90°C pendant 72 h. Après refroidissement, la solution est évaporée à sec et le résidu purifié par chromatographie sur gel de silice en utilisant le dichlorométhane et le méthanol comme éluants. On obtient le produit attendu sous la forme d'une mousse.
**RMN ¹H** (500 MHz, dmso-d6) δ ppm : 7.7-7.45 (4 sl, 1 H), 7.2-6.9 (m, 4 H), 7.1-6.4 (m, 2 H), 7.1-6.9 (4 sl, 1 H), 7-6.8 (4 m, 2 H), 6.75/6.48 (d+sl, 2 H), 6.1 (4 sl, 2 H), 5.25-4.7 (4 sl, 1 H), 5.2-3.8 (m, 2 H), 4.9/4.65/3.8 (3 m, 1 H), 3.75 (4 s, 3 H), 3.5 (sl, 4 H), 3-2 (m, 10 H), 2.41/2.3 (2 sl, 3 H), 1.02/0.95/0.78 (3 dl, 3 H), 0.88 (m, 9 H), 0.1 (m, 6 H)
**IR (ATR)** cm⁻¹: 1626 δ >C=O amides

### Stade B : Chlorhydrate de N-(4-hydroxyphényl)-2-méthyl-5-(6-{[(3R)-3-méthyl-3,4-dihydroisoquinolin-2(1H)-yl]carbonyl}-1,3-benzodioxol-5-yl)-N-(1-méthyl-1H-pyrazol-4-yl)-1-[2-(morpholin-4-yl)éthyl]-1H-pyrrole-3-carboxamide

On procède selon le protocole décrit au Stade D de l'Exemple 1. Le produit obtenu est enfin soumis à une étape de salification en présence de HCl dans l'éther. Après filtration et lyophilisation dans un mélange acétonitrile/eau, on obtient le composé attendu.
***Masse haute résolution (ESI*+*) :***
Formule brute : C₄₀ H₄₂ N₆ O₆
[M+H]⁺ calculé : 703.3239
[M+H]⁺ mesuré : 703.3238

### Exemple 97. Chlorhydrate de N-(4-hydroxyphényl)-2-méthyl-5-(6-{[(3R)-3-méthyl-3,4-dihydroisoquinolin-2(1H)-yl]carbonyl}-1,3-benzodioxol-5-yl)-1-[2-(morpholin-4-yl)éthyl]-N-phényl-1H-pyrrole-3-carboxamide

On procède selon le protocole décrit à l'Exemple 96 en remplaçant le composé de la Préparation 1" par celui de la Préparation 2".
***Microanalyse élémentaire :* (% *théorique : mesuré*)**
%C=68.61:68.12; %H=5.89:5.23; %N=7.62:7.54; %Cl-=4.82:4.66
***Masse haute résolution* (ESI/FIA/HR** et **MS/MS) *:***
Formule brute : C₄₂ H₄₂ N₄ O₆
[M+H]⁺calculé : 699.3177
[M+H]⁺ mesuré : 699.3173

### Exemple 98. Chlorhydrate de 1-[2-(diméthylamino)éthyl]-N-(4-hydroxyphényl)-2-méthyl-5-(6-{[(3R)-3-méthyl-3,4-dihydroisoquinolin-2(1H)-yl]carbonyl}-1,3-benzodioxol-5-yl)-N-phényl-1H-pyrrole-3-carboxamide

On procède selon le protocole décrit à l'Exemple 27 en remplaçant le composé de la Préparation 1" par celui de la Préparation 2". Le produit obtenu est enfin soumis à une étape de salification en présence d'éther chlorhydrique 1M dans l'éther. Après filtration et lyophilisation dans un mélange acétonitrile/eau, on obtient le composé attendu.
***Microanalyse élémentaire :* (% *théorique : mesuré)***
%C=69.30:69.20; %H=5.96:5.48; %N=8.08:8.08; %Cl-=5.11:5.03
***Masse haute résolution* (ESI/FIA/HR** et **MS/MS) *:***
Formule brute : C₄₀ H₄₀ N₄ O₅
[M+H]⁺ calculé : 657.3071
[M+H]⁺ mesuré : 657.3066

### Exemple 99. Chlorhydrate de N-(4-hydroxyphényl)-1-{2-[(2-méthoxyéthyl) (méthyl)amino]éthyl}-2-méthyl-5-(6{[(3R)-3-méthyl-3,4-dihydroisoquinotin-2(1H)-yl]carbonyl}-1,3-benzodioxol-5-yl)-N-(1-méthyl-1H-pyrazol-4-yl)-1H-pyrrole-3-carboxamide

### Stade A : 1-(2-Benzyloxyéthyl)-2-méthyl-5-[6-[(3R)-3-méthyl-3,4-dihydro-1H-isoquinoline-2-carbonyl]-1,3-benzodioxol-5-yl]pyrrole-3-carboxylate d'éthyle

On procède selon le protocole décrit au Stade A de l'Exemple 1 en remplaçant le composé de la Préparation 1 par celui de la Préparation 19.
**RMN ¹H** (500 MHz, dmso-d6) δ ppm : 7.35-6.95 (m, 9 H), 7-6.8 (plusieurs s, 2 H), 6.35-5.85 (plusieurs s, 1 H), 6.15 (plusieurs s, 2 H), 5.15-3.5 (plusieurs m, 4 H), 4.9/4.7/3.95 (plusieurs m, 1 H), 4.4 (m, 2 H), 4.2-3.95 (m, 2 H), 3.55 (m, 2 H), 3.1-2.35 (plusieurs m, 2 H), 2.5-2.2 (plusieurs s, 3 H), 1.25-1.1 (plusieurs m, 3 H), 1.05-0.7 (plusieurs d, 3 H)

### Stade B : Acide 1-(2-benzyloxyéthyl)-2-méthyl-5-[6-[(3R)-3-méthyl-3,4-dihydro-1H-isoquinoline-2-carbonyl]-1,3-benzodioxol-5-yl]pyrrole-3-carboxylique

On procède selon le protocole décrit au Stade B de l'Exemple 1.
**IR (ATR)** cm⁻¹ : 3000-2500 ν -OH, 1675-1625 v -C=O acide carboxylique + amide

### Stade C : 1-(2-Benzyloxyéthyl)-N-[4-[tert-butyl(diméthyl)silyl]oxyphényl]-2-méthyl-5-[6-[(3R)-3-méthyl-3,4-dihydro-1H-isoquinoline-2-carbonyl]-1,3-benzodioxol-5-yl]-N-(1-méthyl-1H-pyrazol-4-yl)pyrrole-3-carboxamide

On procède selon le protocole décrit au Stade C de l'Exemple 1.
**RMN ¹H** (500 MHz, dmso-d6) δ ppm :7.7-7.5 (plusieurs s, 1 H), 7.35-6.5 (plusieurs m, 11 H), 7.1-6.9 (plusieurs s, 1 H), 6.95-6.5 (plusieurs s, 2 H), 6.8/6.5 (m, 2 H), 6.05 (plusieurs s, 2 H), 5.25-4.7 (plusieurs s, 1 H), 5.1-3.6 (plusieurs m, 4 H), 4.85/4.6/3.75 (plusieurs m, 1 H), 4.3 (m, 2 H), 3.7 (2xs, 3 H), 3.4 (m, 2 H), 3.05-2.4 (plusieurs m, 2 H), 2.4-2.25 (plusieurs s, 3 H), 1-0.75 (plusieurs d, 3 H), 0.9 (plusieurs s, 9 H), 0.1 (plusieurs s, 6 H)
**IR (ATR)** cm⁻¹ : 1629 ν >C=O amides

### Stade D : N-[4-[tert-Butyl(diméthyl)silyl]oxyphényl]-1-(2-hydroxyéthyl)-2-méthyl-5-[6-[(3R)-3-méthyl-3,4-dihydro-1H-isoquinoline-2-carbonyl]-1,3-benzodioxol-5-yl]-N-(1-méthyl-1H-pyrazol-4-yl)pyrrole-3-carboxamide

Dans un réacteur d'hydrogénation, le composé du stade précédent est dissous dans 30 mL de méthanol. La solution est dégazée par bullage d'argon et le palladium sur charbon à 10% (550 mg) est ajouté. Cette suspension est agitée sous une pression de 1 bar d'hydrogène pendant 15 heures, puis filtrée sur Whatman®. Le catalyseur est rincé avec du méthanol et le filtrat est évaporé sous vide. Le résidu ainsi obtenu est purifié par chromatographie sur gel de silice en utilisant le dichlorométhane et le méthanol comme éluants. On obtient le produit attendu sous la forme d'une huile.
**RMN ¹H** (500 MHz, pyridine-d5) δ ppm: 7.7-7.4 (4 s, 1 H), 7.3-6.9 (m, 4 H), 7.1-6.8 (4 s, 1 H), 7-6.7 (m, 2 H), 6.9-6.4 (m, 2 H), 6.8-6.4 (4 m, 2 H), 6.1 (m, 2 H), 5.2/5/4.7 (4 s, 1 H), 5.15-3.9 (8 d, 2 H), 4.9-4.8 (m, 1 H), 4.9/4.7/3.85 (3 m, 1 H), 3.9-3.7 (m, 2 H), 3.75 (2 s, 3 H), 3.5-3.2 (m, 2 H), 3-2.4 (m, 2 H), 2.4-2.3 (4 s, 3 H), 1.05/0.95/0.75 (4 d, 3 H), 0.85 (sl, 9 H), 0.15-0 (m, 6 H)
**IR (ATR)** cm⁻¹ :3346 ν -OH alcool primaire, 1621v -C=O amides

### Stade E : 2-[3-[[4-[tert-Butyl(diméthyl)silyl]oxyphényl]-(1-méthyl-1H-pyrazol-4-yl)carbamoyl]-2-méthyl-5-[6-[(3R)-3-méthyl-3,4-dihydro-1H-isoquinoline-2-carbonyl]-1,3-benzodioxol-5-yl]pyrrol-1-yl]éthyl méthanesulfonate

Le composé du stade précédent (2,37g, 3,17 mmol) est dissous dans 30 mL de tétrahydrofurane. Le milieu réactionnel est refroidi à 0°C et sont ajoutés successivement la triéthylamine (1,8 mL, 13,9 mmol) et, goutte à goutte, le chlorure de méthanesulfonyle (0,40 mL, 5,17 mmol). La réaction est agitée pendant 2 heures à 0°C. Le milieu réactionnel est ensuite versé dans une solution aqueuse saturée en hydrogénocarbonate de sodium et extrait 3 fois avec de l'acétate d'éthyle. La phase organique est lavée 3 fois avec de la saumure, séchée sur MgSO₄, filtrée puis évaporée à sec. Le résidu d'évaporation est engagé sans purification dans l'étape suivante.
**RMN ¹H** (500 MHz, dmso-d6) δ ppm : 7.7-7.45 (plusieurs s, 1 H), 7.25-6.4 (m, 8 H), 7.1-6.9 (plusieurs s, 1 H), 6.95-6.65 (plusieurs s, 2 H), 6.1 (plusieurs s, 2 H), 5.2-4.6 (plusieurs s, 1 H), 5.05-3.75 (plusieurs d, 2 H), 4.9/4.6/3.9/3.8 (plusieurs m, 1 H), 4.3-4.05 (m, 2 H), 4.2-3.95 (m, 2 H), 3.75/3.7 (2 s, 3 H), 3.05-2.5 (plusieurs m, 2 H), 3 (plusieurs s, 3 H), 2.45-2.3 (plusieurs s, 3 H), 1.05-0.75 (plusieurs d, 3 H), 0.9 (plusieurs s, 9 H), 0.1 (plusieurs s, 6 H)
**IR (ATR)** cm⁻¹ : 1626 ν -C=O, 1349 ν -SO₂, 1249 δ-CH3, 1172 ν -SO₂

### Stade F : N-(4-(tert-Butyl(diméthyl)silyl]oxyphényl]-1-[2-[2-méthoxyéthyl (méthyl)amino]éthyl]-2-méthyl-5-[6-[(3R)-3-méthyl-3,4-dihydro-1H-isoquinoline-2-carbonyl]-1,3-benzodioxol-5-yl]-N-(1-méthyl,1H-pyrazol-4-yl)pyrrole-3-carboxamide

Le composé du stade précédent (1,33 mg, 1,61 mmol) est dissous dans 6 mL de tétrahydrofurane. L'iodure de sodium (100 mg, 0,67 mmol) est ajouté, suivi de la diméthylamine (0,172 g, 1,932 mmol) au goutte à goutte. Le milieu réactionnel est chauffé en flacon scellé à 60°C pendant 36 h. Après refroidissement, il est évaporé à sec et purifié par chromatographie sur gel de silice en utilisant le dichlorométhane et le méthanol contenant de l'ammoniac comme éluants. On obtient le produit attendu sous la forme d'une mousse.
**RMN 1H (500 MHz, dmso-d6) δ ppm** 7.7-7.45 (4 sl, 1 H), 7.2-6.9 (m, 4 H), 7.1-6.9 (4 sl, 1 H), 7-6.7 (4 m, 2 H), 7/6.45 (2 m, 2 H), 6.8/6.45 (2 m, 2 H), 6.1 (m, 2 H), 5.22/5.05/4.7 (4 sl, 1 H), 5.2-3.8 (m, 2 H), 4.91/4.65/3.9 (3 m, 1 H), 3.75 (2 sl, 3 H), 3.7 (m, 2 H), 3.2 (sl, 3 H), 3-2 (m, 2 H), 2.7-2 (ml, 6 H), 2.7-2 (m, 3 H), 2.45/2.35 (2 sl, 3 H), 1.05/0.95/0.8 (3 dl, 3 H), 0.9 (m, 9 H), 0.1 (m, 6 H)
**IR (ATR)** cm⁻¹ : 1628 (épaulement) δ -C=O amides

### Stade G : Chlorhydrate de N-(4-hydroxyphényl)-1-[2-[2-méthoxyéthyl(méthyl)amino]éthyl]-2-méthyl-5-[6-[(3R)-3-méthyl-3,4-dihydro-1H-isoquinoline-2-carbonyl]-1,3-benzodioxol-5-yl]-N-(1-méthyl-1H-pyrazol-4-yl)pyrrole-3-carboxamide

On procède selon le protocole décrit au Stade D de l'Exemple 1. Le produit obtenu est enfin soumis à une étape de salification en présence d'éther chlorhydrique 1M dans l'éther. Après filtration et lyophilisation dans un mélange acétonitrile/eau, on obtient le composé attendu.
**IR (ATR)** cm⁻¹ : 2000 à 3500 ν -NH⁺/OH, 1615 ν >C=O amides, 1237-1161 δ >C-O-C<, 745 ν >CH-Ar
***Masse haute résolution (ESI*+*) :***
Formule brute : C₄₀ H₄₄ N₆ O₆
[M+H]⁺ calculé : 705.3395
[M+H]⁺ mesuré : 705.3391

### Exemple 100. Chlorhydrate de 5-(5-fluoro-4-méthoxy-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(1-méthyl-1H-pyrazole-4-yl)-1H-pyrrole-3-carboxamide

On procède selon le protocole général de l'Exemple 1 en utilisant les préparations adéquates. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.

### Microanalyse élémentaire : (% théorique : mesuré)

%C=64.23:64.31; %H=5.80:5.43; %N=11.52:11.46; %Cl-=4.86:4.95
***Masse haute résolution* (ESI+) :**
Formule brute : C₃₉ H₄₁ F N₆ O₅
[M+H]⁺ calculé : 693.3195
[M+H]⁺ mesuré : 693.3194

### Exemple 101. Chlorhydrate de 5-(4-fluoro-5-méthoxy-2-{[(3S)-3-(morpholin-4-ylméthyl)-3,4-dihydroisoquinolin-2(1H)-yl]carbonyl]phényl)-N-(4-hydroxyphényl)-1,2-diméthyl-N-(1-3méthyl-1H-pyrazol-4-yl)-1H-pyrrole-3-carboxamide

Le composé du titre est obtenu selon le procédé de l'Exemple 1 en utilisant l'acide de la Préparation 27 et le composé de la Préparation 3' au Stade A, ainsi que le composé de la Préparation 1" au Stade C. Le composé obtenu est salifié et lyophilisé tel que décrit dans le procédé général pour obtenir le composé du titre.
***Microanalyse élémentaire** :* **(*% théorique : mesuré*)**
%C=64.23:63.12; %H=5.80:5.20; %N=11.52:11.38. %Cl-=4.86:5.03
***Masse haute résolution (ESI+*/*HR, ESI-*/*LR) :***
Formule brute : C₃₉ H₄₁ F N₆ O₅
[M+H]⁺ calculé : 693.3195
[M+H]⁺ mesuré : 693.3195

### Exemple 102. Trichlorhydrate de 5-(5-chloro-2-{[(3S)-3-[(4-méthylpipérazin-1-yl)méthyl]-3,4-dihydroisoquinolin-2(1H)-yl]carbonyle-N-(4-hydroxyphényl)-1,2-diméthyl-N-(1-méthyl-1H-pyrrolo[2,3-b]pyridin-5-yl)-1H-pyrrole-3-carboxamide

### Masse haute résolution (ESI+) :

Formule brute : C₄₃ H₄₄ Cl N₇ O₃
[M+H]⁺ calculé : 742.3267
[M+H]⁺ mesuré : 742.3268

**Exemple 103. 5-(5-Chloro-2{[(3*S*)-3-[(4-méthylpipérazin-1-yl)méthyl]3,4-dihydroisoquinolin-2(1*H*)-yl]carbonyl}phényl)-1,2-diméthyl-*N-*(1*-*méthyl-1*H-*pyrrolo[2,3-*b*]pyridin-5yl)-*N*-phényl-1*H*-pyrrole-3-carboxamide**
**Exemple 104. *N*-(4-DHydroxyphényl)-1,2-diméthyl-*N*-(1-méthyl-1*H*-pyrazolo[3,4-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 105. 5-(5-Chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 106. 5-(5-Chloro-2{[(3*S*)-3(morpholin-4-ylméthyl)3,4-dihydroisoquinolin-2(1*H*)-yl]-carbonyl}phényl)-1,2-diméthyl-*N*-(1-méthyl-1*H*-pyrrolo[2,3-*b*]pyridin-5-yl)-*N*-phényl-1*H*-pyrrol-3-carboxamide**
   On procède selon le protocole général de l'Exemple 1 en utilisant les préparations adéquates, étant entendu que le Stade D n'est pas effectué. Le produit attendu est obtenu sous la forme d'une base libre.
   ***Microanalyse élémentaire :* (% *théorique : mesuré)***
   %C=70.72:69.77; %H=5.79:5.96; %N=11.78:11.43
   ***Masse haute résolution* (*ESI+*) *:***
   Formule brute : C₄₂ H₄₁ Cl N₆ O₃
   [M+H]⁺ calculé : 713.3001
   [M+H]⁺ mesuré : 713.2998
**Exemple 107. 5-(5-Chloro-2-{[(3*S*)-(morpholin-4-ylméthyl-3,4-dihydroisoquinolin-2(1*H*)-yl]carbonyl}phényl)-*N*-(4-fluorophényl)-1,2-diméthyl-*N*-(1-méthyl-1*H-*pyrrolo[2,3-*b*]pyridin-5-yl)-1*H*-pyrrole-3-carboxamide**
**Exemple 108. Chlorhydrate de 5-(5-chloro-2-{[(3*S*)-3-(morpholin-4-ylméthyl)-3,4-dihydroisoquinolin-2(1*H*)-yl]carbonyl}phényl-*N*-(5-cyano-1-méthyl-1*H*-pyrrol-3-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et la (3*S*)-3-(4-morpholinylméthyl)-1,2,3,4-tétrahydroisoquinoline (voir Préparation 3') au Stade A, ainsi que le composé de la Préparation 19" au Stade C. Le produit obtenu est enfin soumis à une étape de salification en présence d'éther chlorhydrique dans l'éther. Après filtration et lyophilisation dans un mélange acétonitrile/eau, on obtient le composé attendu.
   ***Microanalyse élémentaire :* (*% théorique : mesuré)***
   %C=64.95:65.09; %H=5.45:5.20; %N=11.36:11.26; %Cl-=4.79:4.62
   ***Masse haute résolution* (ESI/+) *:***
   Formule brute : C₄₀ H₃₉ Cl N₆ O₄
   [M+H]⁺ calculé : 703.2794
   [M+H]⁺ mesuré : 703.2789
**Exemple 109. Chlorhydrate de 5-(5-chloro-2-{[(3*S*)-3-(morpholin-4-ylméthyl)-3,4-dihydroisoquinolin-2-(1*H*)-yl]carbonyl}phényl-*N*-(6-cyanopyridin-2-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 23" au Stade C. Le composé obtenu est salifié et lyophilisé tel que décrit dans le procédé général pour obtenir le composé du titre.
   ***Masse haute résolution (ESI+-*/*FIA*/ *HR, ESI-*/*FIA) :***
   Formule brute : C₄₀ H₃₇ Cl N₆ O₄
   [M+H]⁺ calculé : 701.2638
   [M+H]⁺ mesuré : 701.2639
**Exemple 110. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(4-cyanopyridin-2-yl)-*N*-(4-hydroxyphényl**)-**1**,**2**-**diméthyl**-**1*H***-**pyrrole-3**-**carboxamide**
   ***Microanalyse élémentaire :* (% *théorique : mesuré)***
   %C=65.13:65.72; %H=5.19:4.76; %N=11.39:12.04; %Cl⁻=4.81:4.45
   ***Masse haute résolution (ESI*+-/*FIA*/*HR*, *ESI***-/***FIA***) ***:***
   Formule brute : C₄₀ H₃₇ Cl N₆ O₄
   [M+H]⁺ calculé : 701.2638
   [M+H]⁺ mesuré : 701.2643
**Exemple 111. 5-(5-Chloro-2-{[(3*S*)-3-(morpholin-4-ylméthyl)-3,4-dihydroisoquinolin-2(1*H*)-yl]carbonyl}phényl)-*N*-(4-cyanopyrimidin-2-yl)-*N*-(4-hydroxyphényl)1,2-diméthyl-1*H*-pyrrole-3-carboxamide**
**Exemple 112. 5-(5-Chloro-2-{[(3*S*)-3-(morpholin-4-ylméthyl)-3,4-dihydroisoquinolin-2(1*H*)-yl]carbonyl}phényl)-*N*-[2-(diméthylamino)pyridin-4-yl]-*N*-(4-hydroxyphényl)-1**,**2**-**diméthyl**-**1*H***-**pyrrole**-**3**-**carboxamide**
**Exemple 113. 5-(5-Chloro-2-{[(3*S*)-3-(morpholin-4-ylméthyl)3,4-dihydroisoquinolin-2(1*H*)-yl]carbonyl}phényl)-*N*-(4-hydroxyphényl)-1,2-diméthyl-*N*-(1-méthyl-1*H-*benzimidazol-5-yl)-1*H*-pyrrole-3-carboxamide**
**Exemple 114. 5-(5-Chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(pyrazolo[1,5-*a*]pyrimidin-6-yl)-1*H*-pyrrole-3-carboxamide**
**Exemple 115. Dichlorhydrate de 5-(5-chloro-2-{[(3*S*)-3-[(9a*S*)-hexahydropyrazino[2,1-*c*][1,4]oxazin-8(1*H*)-ylméthy-3,4-dihydroisoquiHnolin-2(1*H*)-yl]carbonyl}phényl)-*N-*(4-hydroxyphényl)-1,2-diméthyl-*N*-(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 1 et le composé de la Préparation 7' au Stade A, ainsi que le composé de la Préparation 1" au Stade C. Le composé obtenu est salifié et lyophilisé tel que décrit dans le procédé général pour obtenir le composé du titre.
   ***Microanalyse élémentaire :* (% *théorique : mesuré)***
   %C=61.01:60.17; %H=5.74:5.09; %N=12.15:12.02; %Cl-=8.78:9.81
   ***Masse haute résolution* (*ESI+-*/*FIA*/*HR, ESI***-/***FIA***) ***:***
   Formule brute : C₄₁ H₄₄ Cl N₇ O₄
   [M+H]⁺ calculé : 734.3216
   [M+H]⁺ mesuré : 734.3220
**Exemple 116. Dichlorhydrate de 5-(5-chloro-2-{[(3*S*)-3-[(9a*R*)-hexahydropyrazino [2,1-*c*][1,4]oxazin-8(1*H*)-ylméthyl]-3,4-dihydroisoquinolin-2(1*H*)-yl]carbonyl}phényl)-*N*-(4-hydroxyphény-1,2-diméthyl-*N*-(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 1 et le composé de la Préparation 9' au Stade A, ainsi que le composé de la Préparation 1" au Stade C. Le composé obtenu est salifié et lyophilisé tel que décrit dans le procédé général pour obtenir le composé du titre.
   ***Microanalyse élémentaire :* (% *théorique : mesuré)***
   %C=61.01:61.90; %H=5.74:5.65; %N=12.15:12.14; %Cl=13.15:11.51
   ***Masse haute résolution* (*ESI*/*FIA*/*HR et MS*/*MS*) *:***
   Formule brute : C₄₁ H₄₄ Cl N₇ O₄
   [M+H]⁺ calculé : 734.3216
   [M+H]⁺ mesuré : 734.3218
**Exemple 117. 5-(5-Chloro-2-{[(3*S*)-3-(fluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide**
**Exemple 118. 5-(5-Choloro-2-{[(3*S*)-3-(difluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide**
**Exemple 119. 5-(5-Chloro-2-{[(3*S*)-3-(trifluorométhyl)-3,4-dihydroisoquinolin-2(1*H*)-yl]carbonyl}phény)-*N*-(4-hydroxyphényl)1,2-diméthyl-*N*-(1-méthyl-1*H*-pyrazol-4-yl)-1*H*-pyrrole-3-carboxamide**
**Exemple 120. 5-(5-Chloro-2-{[(3*S*)-3-[(3-cyanoazétidin-1-yl)méthyl]-3,4-dihydroisoquinolin-2(1*H*)-yl]carbonyl}phényl-*N*-(4-hydroxyphényl)-1,2-diméthyl-*N-*(1-méthyl**-**1*H*-pyrazol-4-yl)-1*H*-pyrrole-3-carboxamide**
**Exemple 121. 5-(5-chloro-2-{[(3*S*)-3-(morpholin-4-ylméthyl)-3,4-dihydroisoquinolin-2(1*H*)-yl]carbonyl}phényl)-*N*-(1-éthyl-1*H*-pyrazol-4-yl)-*N*-(4-hydroxyphényl)-1,2-diméthyl-1H-pyrrole-carboxamide**
**Exemple 122. 5-(5-Chloro-2-{[(3*S*)-3-(morpholin-4-ylméthyl)-3,4-dihydroisoquinolin-2(1*H*)-yl]carbonyl}phényl)-*N*-(1-cyclopropyl-1*H*-pyrazol-4-yl)-*N*-(4-hydroxyphényl)-1**,**2**-**diméthyl**-**1*H***-**pyrrole**-**3**-**carboxamide**
   Le composé du titre est obtenu selon le procédé de l'Exemple 1 en utilisant l'acide de la Préparation 1 et le composé de la Préparation 3' au Stade A, ainsi que le composé de la Préparation 28" au Stade C.
   ***Microanalyse élémentaire :* (% *théorique : mesuré)***
   %C=68.12:67.94; %H=5.86:5.77; %N=11.92:11.65
   ***Masse haute résolution*** (***ESI***+/***HR***, ***ESI***-/***LR***) ***:***
   Formule brute : C₄₀ H₄₁ Cl N₆ O₄
   [M+H]⁺ calculé : 705.2951
   [M+H]⁺ mesuré : 705.2952
**Exemple 123. 5-(5-chloro-2**-**{[(3*S*)-3-(morpholin-4-ylméthyl)-3,4-dihydroisoquinolin-2(1H)-yl]carbonyl}phényl)-*N*-(4-hydroxyphényl)-1,2-diméthyl-*N*-[1-(trifluorométhyl)-1*H*-pyrazol-4-yl]-1*H*-pyrrole-3-carboxamide**
**Exemple 124. 5-(5-chloro**-**2-{[(3*S*)-3-(morpholin-4**-**ylm**é**hyl)**-**3**,**4**-**dihydroisoquinolin-2(1*H*)-yl]carbonyl}phényl)-*N*-(1-(difluorométhyl)-1*H*-pyrazol-4-yl]-*N*-(4-hydroxyphényl)-1,2-diméthyl-1*H*-pyrrole-carboxamide**
**Exemple 125. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-*N*-[1-(2-méthoxyéthyl)-1*H*-pyrazol-4-yl]-1,2-diméthyl-1*H*-pyrrole-3-Carboxamide**
**Exemple 126. 5-(5-Chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-[1-(oxetan-3-yl)-1*H-*pyrazol-4-yl]-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 26" au Stade C.
   ***Masse haute résolution*** (***ESI***+/***HR***, ***ESI***-/***LR***) ***:***
   Formule brute : C₄₀ H₄₁ Cl N₆ O₅
   [M+H]⁺ calculé : 721.2900
   [M+H]⁺ mesuré : 721.2902
**Exemple 127. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*[1-(tétrahydrofuran-3-yl**)-**1*H*-pyrazol-4-yl]-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 22" au Stade C. Le composé obtenu est salifié et lyophilisé tel que décrit dans le procédé général pour obtenir le composé du titre.
   ***Microanalyse élémentaire :* (% *théorique : mesuré)***
   %C=63.81:63.63; %H=5.75:5.74; %N=10.89:10.71; %Cl⁻=4.59:4.52
   ***Masse haute résolution*** (***ESI***+/***HR***, ***ESI***-/***LR***) ***:***
   Formule brute : C₄₁ H₄₃ Cl N₆ O₅
   [M+H]⁺ calculé : 735.3056
   [M+H]⁺ mesuré : 735.3061
**Exemple 128. 5-(5-chloro-2-{[(3*S*)-3-(morpholin-4-ylméthyl)-3,4-dihydroisoquinolin-2(1*H*)**-**yl]carbonyl}phényl)-*N*-[1-(2-hydroxy-2-méthylpropyl)-1*H*-pyrazol-4-yl]-*N*-(4-hydroxyphényl**)-**1**,**2**-**diméthyl-1H-pyrrole**-**3**-**carboxamide**
**Exemple 129. 5-(5-Chloro-2-{[(3*S*)-3-(morpholin-4-ylméthyl)-3,4-dihydroisoquinolin-2(1*H*)-yl]carbonyl}phényl)1,2-diméthyl-*N*-(1-méthyl-2,3-dihydro-1*H*-pyrrolo[2,3-*b*]pyridin-5-yl)-*N*-phényl-1*H*-pyrrole-3-carboxamide**
   Le composé du titre est obtenu selon le procédé décrit au Stade B de l'Exemple 49 en utilisant l'Exemple 106 comme matière première, étant entendu que le produit n'est pas soumis à l'étape de salification.
   ***Microanalyse élémentaire :* (% *théorique : mesuré)***
   %C=70.53:70.51; %H=6.06:5.81; %N=11.75:11.71
   ***Masse haute résolution (*ESI+-/FIA*) :***
   Formule brute : C₄₂ H₄₃ Cl N₆ O₃
   [M+H]⁺ calculé : 715.3158
   [M+H]⁺ mesuré : 715.3159
**Exemple 130. 5-(5-chloro-2-{[(3*S*)-3-(morpholin-4-ylméthyl)-3,4-dihydroisoquinolin-2(1*H*)-yl]carbonyl}phényl)-*N*-(4-fluorophényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 131.** (exemple de référence) **5-(5-(chloro-2-{[(3*S*)-3-(morpholin-4-ylméthyl)-3,4-dihydroisoquinolin-2(1*H*)-yl]carbonyl}phényl)-*N*-(4-hydroxyphényl)-*N*,1,2-triméthyl-1*H*-pyrrole-3-carboxamide**
**Exemple 132. 5-(5-chloro-2-{[(3*S*)-3-(morpholin-4-ylméthyl)-3,4-dihydroisoquinolin-2(1*H*)-yl]carbonyl}phényl)-*N*-(2-hydroxypyrimidin-5-yl)-1,2-diméthyl-*N*-phényl-1*H-*pyrrole-3-carboxamide**
**Exemple 133. 5-(5-chloro-2-{[(3*S*)-3-(morpholin-4-ylméthyl)-3,4-dihydroisoquinolin-2(1*H*)-yl]carbonyl}phényl-*N*-(2-hydroxypyrimidin-5-yl)-1,2-diméthyl-*N*-(1-méthyl-1*H*-pyrrolo[2,3-*b*]pyridin-5-yl)-1*H*-pyrrole-3-carboxamide**
**Exemple 134. 5-(5-chloro-2-{[(3*S*)-3-(morpholin-4-ylméthyl)-3,4-dihydroisoquinolin 2(1*H*)-yl]carbonyl}phényl)-*N*-(2-hydroxypyrimidin-5-yl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 135. 5-(5-chloro-2-{[(3*S*)-3-(morpholin-4-ylméthyl)-3,4-dihydroisoquinolin 2(1*H*)-yl]carbonyl}phényl)-*N*-(2-hydroxypyrimidin-5-yl)-1,2-diméthyl-*N*-(1-méthyl-1*H*-pyrazol-4-yl)-1*H*-pyrrole-3-carboxamide**
**Exemple 136. 5-(5-chloro-2-{[(3*S*)-3-(morpholin-4-ylméthyl)-3,4-dihydroisoquinolin-2(1*H*)-yl]carbonyl}phényl)-*N*-(2-hydroxypyrimidin-5-yl)-1,2-diméthyl-*N*-(1-méthyl-1*H*-pyrazol-4-yl)-1*H*-pyrrole-3-carboxamide**
**Exemple 137. 5-(5-chloro-2-{[(3*S*)-3-(morpholin-4-ylméthyl)-3,4-dihydroisoquinolin-2(1*H*)-yl]carbonyl}phényl)-*N*-(2-hydroxyphényl)-2-diméthyl-*N*-(1-méthyl-1*H-*pyrrolo[2,3-*b*]pyridin-5-yl)-1*H*-pyrrole-3-carboxamide**
**Exemple 138. 5-(5-chloro-2-{[(3*S*)-3-(morpholin-4-ylméthyl)-3,4-dihydroisoquinolin-2(1*H*)-yl]carbonyl}phényl)-*N*-(2-hydroxyphényl)-2-méthyl-*N*-(1-méthyl-2,3-dihydro-1*H*-pyrrolo[2,3-*b*]pyridin-5-yl)-1*H*-pyrrolo-3-carboxamide**
**Exemple 139. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-1*H*-pyrazol-4-yl)-1*H*-pyrrole-3-carboxamide**
**Exemple 140. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H-*pyrrolo[2,3-b]pyridin-5-yl)1*H*-pyrrole-3-carboxamide**
**Exemple** 141. 5-(5-chloro-2-{[(3*S*)-3-(morpholin-4-ylméthyl)-3,4-dihydroisoquinolin **2(1*H*)-yl]carbonyl}phényl)-*N*-(4-hydroxyphényl)-1-méthyl-*N*-(1-méthyl-2,3-dihydro-1*H*-pyrrolo[2,3-*b*]pyridin-5-yl)-1*H*-pyrrole-3-carboxamide**
**Exemple 142. 5-(5-chloro-2-{[(3*S*)-3-(morpholin-4-ylméthyl)-3,4-dihydroisoquinolin-2(1*H*)-yl]carbonyl}phényl)-1-éthyl-*N*-(4-hydroxyphényl)-2-méthyl-*N*-(1-méthyl-1*H-*pyrazol-4-yl)-1*H*-pyrrole-3-carboxamide**
**Exemple 143. 5-(5-chloro-2-{[(3*S*)-3-(morpholin-4-ylméthyl)-3,4-dihydroisoquinolin-2(1*H*)-yl]carbonyl}phényl)-1-éthyl-*N*-(4-hydroxyphényl)-2-méthyl-*N*-(1-méthyl-1*H-*pyrrolo[2,3-*b*]pyridin-5-yl)-1*H*-pyrrole-3-carboxamide**
**Exemple 144. 5-(5-chloro-2-{[(3*S*)-3-(morpholin-4-ylméthyl)-3,4-dihydroisoquinolin-2(1*H*)-yl]carbonyl}phényl)-1-éthyl-*N*-(4-hydroxyphényl)-2-méthyl-*N*-(1-méthyl-2,3-dihydro-1*H*-pyrrolo[2,3-*b*]pyridin-5-yl)-1*H*-pyrrole-3-carboxamide**
**Exemple 145. 5-(5-chloro-2-{[(3*S*)-3-(morpholin-4-ylméthyl)-3,4-dihydroisoquinolin-2(1*H*)-yl]carbonyl}phényl)-1-éthyl-*N*-(4-hydroxyphényl)-1-(2-méthoxyéthyl-2-méthyl-*N*-(1-méthyl-1*H*-pyrazol-4-yl)-1*H-*pyrrole-3-carboxamide**
**Exemple 146. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1-(2-méthoxyéthyl-2-méthyl-*N*-(1-méthyl-1*H*-pyrazol[2,3-*b*]pyridin-5-yl)-1*H-*pyrrole-3-carboxamide**
**Exemple 147. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1-(2-méthoxyéthyl-2-méthyl-*N*-(1-**méthyl-2,3-dihydro-1*H*-pyrrolo[2,3-*b*]pyridin-5-yl)-1*H-*pyrrole-3-carboxamide
**Exemple 148. 5-(5-chloro-2-{[(3*S*)-3-(morpholin-4-ylméthyl)-3,4-dihydroisoquinolin-2(1*H*)-yl]carbonyl}phényl)-1-(2-fluoroéthyl)-*N*-(4-hydroxyphényl)-2-méthyl-*N*-(1-méthyl-1*H*-pyrazol-4-yl)-1*H-*pyrrole-3-carboxamide**
**Exemple 149. 5-(5-chloro-2-{[(3*S*)-3-(morpholin-4-ylméthyl)-3,4-dihydroisoquinolin-2(1*H*)-yl]carbonyl}phényl)-1-(2-fluoroéthyl)-*N*-(4-hydroxyphényl)-2-méthyl-*N*-(1-méthyl-1*H*-pyrrolo[2,3-*b*]pyridin-5-yl)-1*H-*pyrrole-3-carboxamide**
**Exemple 150. 5-(5-chloro-2-{[(3*S*)-3-(morpholin-4-ylméthyl)-3,4-dihydroisoquinolin-2(1*H*)-yl]carbonyl}phényl)-1-(2-fluoroéthyl)-*N*-(4-hydroxyphényl)-2-méthyl-*N*-(1-méthyl-2,3-dihydro-1*H*-pyrrolo[2,3-*b*]pyridin-5-yl)-1*H-*pyrrole-3-carboxamide**
**Exemple 151. 5-(5-chloro-2-{[(3*S*)-3-(morpholin-4-ylméthyl)-3,4-dihydroisoquinolin-2(1*H*)-yl]carbonyl}phényl)-1-(2,2-difluoroéthyl)-*N*-(4-hydroxyphényl)-2-méthyl-*N*-(1-méthyl-1*H*-pyrazol-4-yl)-1*H-*pyrrole-3-carboxamide**
**Exemple 152. 5-(5-chloro-2-{[(3*S*)-3-(morpholin-4-ylméthyl)-3,4-dihydroisoquinolin-2(1*H*)-yl]carbonyl}phényl)-1-(2,2-difluoroéthyl)-*N*-(4-hydroxyphényl)-2-méthyl-*N*-(1-méthyl-1*H*-pyrazol[2,3-*b*]pyridin-5-yl)-1*H-*pyrrole-3-carboxamide**
**Exemple 153. 5-(5-chloro-2-{[(3*S*)-3-(morpholin-4-ylméthyl)-3,4-dihydroisoquinolin-2(1*H*)-yl]carbonyl}phényl)-1-(2,2-difluoroéthyl)-*N*-(4-hydroxyphényl)-2-méthyl-*N*-(1-méthyl-2,3-dihydro-1*H*-pyrrolo[2,3-*b*]pyridin-5-yl)-1*H-*pyrrole-3-carboxamide**
**Exemple 154. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-1*H*-pyrazol-4** yl)-1-(2,2,2-trifluoroéthyl)-1*H-*pyrrole-3-carboxamide
**Exemple 155. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-1*H-*pyrrolol[2,3-*b*]pyridin-5-yl)-1-(2,2,2-trifluoroéthyl)-1*H-*pyrrole-3-carboxamide**
**Exemple 156. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-2,3-dihydro-1*H*-pyrazol[2,3-*b*]pyridin-5-yl)-1-(2,2,2-trifluoroéthyl)-1*H-*pyrrole-3-carboxamide**
**Exemple 157. 5-(5-chloro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-1*H*-pyrazol-4-yl)-1-[2-(morpholin-4-yl)éthyl]-1H-pyrrole-3-carboxamide**
**Exemple 158. 5-(5-chloro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-1*H*-pyrrolo[2,3-*b*]pyridin-5-yl)-1-[2-(morpholin-4-yl)éthyl]-1*H*-pyrrole-3-carboxamide**
**Exemple 159. 5-(5-chloro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-2,3-dihydro-1*H-*pyrrolo[2,3-*b*]pyridin-5-yl)-1-[2-(morpholin-4-yl)éthyl]-1*H*-pyrrole-3-carboxamide**
**Exemple 160. 5-(5-chloro-2-{[(3*R*)-3-méthyl-3,4-dihydroisoquinolin-2(1*H*)-yl]carbonyl}phényl)-*N*-(4-hydroxyphényl)-2-méthyl-1-[2-(morpholin-4-yl)éthyl]-*N-*phényl-1*H*-pyrrole-3-carboxamide**
**Exemple 161. 5-(5-chloro-2-{[(3*R*)-3-méthyl-3,4-dihydroisoquinolin-2(1*H*)-yl]carbonyl}phényl)-1-[2-(diméthylamino)éthyl]-*N*-(4-hydroxyphényl)-2-méthyl-*N*-(1-méthyl-1*H*-pyrazol-4-yl)-1*H*-pyrrole-3-carboxamide**
**Exemple 162. 5-(5-chloro-2-{[(3*R*)-3-méthyl-3,4-dihydroisoquinolin-2(1*H*)-yl]carbonyl}phényl)-1-[2-(diméthylamino)éthyl]-*N*-(4-hydroxyphényl-2-méthyl-*N-*phényl-1*H-*pyrrole-3-carboxamide**
**Exemple 163. 5-(5-chloro-2-{[(3*R*)-3-méthyl-3,4-dihydroisoquinolin-2(1*H*)-yl]carbonyl}phényl)-1-[2-(diméthylamino)éthyl]-*N*-(4-hydroxyphényl-2-méthyl-*N*-(1-méthyl-1*H*-pyrrolo[2,3-*b*]pyridin-5-yl)-1*H-*pyrrole-3-carboxamide**
**Exemple 164. 5-(5-chloro-2-{[(3*R*)-3-méthyl-3,4-dihydroisoquinolin-2(1*H*)-yl]carbonyl}phényl)-1-[2-(diméthylamino)éthyl]-*N*-(4-hydroxyphényl-2-méthyl-*N*-(1-méthyl-2,3-dihydro-1*H*-pyrrolo[2,3-*b*]pyridin-5-yl)-1*H-*pyrrole-3-carboxamide**
**Exemple 165. 5-(5-chloro-2-{[(3*R*)-3-méthyl-3,4-dihydroisoquinolin-2(1*H*)-yl] carbonyl}phényl)-*N*-(4-hydroxyphényl)-1-{2-[(2-méthoxyéthyl)(méthyl)amino]éthyl}-2-méthyl-*N*-(1-méthyl-1*H-*pyrazol-4-yl)-1*H*-pyrrole-3-carboxamide**
**Exemple 166. 5-(5-chloro-2-{[(3*R*)-3-méthyl-3,4-dihydroisoquinolin-2(1*H*)-yl] carbonyl}phényl)-*N*-(4-hydroxyphényl)-1-{2-[(2-méthoxyéthyl)(méthyl)amino]éthyl}-2-méthyl-*N*-(1-méthyl-1*H*-pyrrolo[2,3-*b*]pyridin-5-yl)-1*H-*pyrrole-3-carboxamide**
**Exemple 167. 5-(5-chloro-2-{[(3*R*)-3-méthyl-3,4-dihydroisoquinolin-2(1*H*)-yl] carbonyl}phényl)-*N*-(4-hydroxyphényl)-1-{2-[(2-méthoxyéthyl)(méthyl)amino]éthyl}-2-méthyl-*N-*(1-méthyl-2,3-dihydro-1*H*-pyrrolo[2,3-*b*]pyridin-5-yl)-1*H*-pyrrole-3-carboxamide**
**Exemple 168. 5-(5-chloro-2-{[(3*S*)-3-(morpholin-4-ylméthyl-3,4-dihydroisoquinolin-2(1*H*)-yl]carbonyl}phényl)-*N*-(4-hydroxyphényl)-1-méthyl-*N*-(1-méthyl-1*H*-pyrazol-4-yl)-2-(trifluorométhyl)-1*H-*pyrrole-3-carboxamide**
**Exemple 169. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl-1-méthyl-*N*-(1-méthyl-1*H-*pyrrolo[2,3-*b*]pyridin-5-yl)2-(trifluorométhyl)-1*H*-pyrrole-3-carboxamide**
**Exemple 170. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl-1-méthyl-*N*-(1-méthyl-2,3-dihydro-1*H*-pyrrolo[2,3-*b*]pyridin-5-yl)2-(trifluorométhyl)-1*H*-pyrrole-3-carboxamide**
**Exemple 171. 5-(5-chloro-2-{[(3*S*)-3-(morpholin-4-ylméthyl)-3,4-dihydroisoquinolin-2(1H)-yl]carbonyl}phényl)-2-(difluorométhyl)-*N*-(4-hydroxyphényl-1-méthyl-*N*-(1-méthyl-1*H*-pyrazol-4-yl)-1*H*-pyrrole-3-carboxamide**
**Exemple 172. 5-(5-chloro-2-{[(3*S*)-3-(morpholin-4-ylméthyl)-3,4-dihydroisoquinolin-2(1*H*)-yl]carbonyl}phényl)-2-(difluorométhyl)-*N*-(4-hydroxyphényl-1-méthyl-*N*-(1-méthyl-1*H*-pyrrolo[2,3-*b*]pyridin-5-yl)-1*H*-pyrrole-3-carboxamide**
**Exemple 173. 5-(5-chloro-2-{[(3*S*)-3-(morpholin-4-ylméthyl)-3,4-dihydroisoquinolin-2(1*H*)-yl]carbonyl}phényl)-2-(difluorométhyl)-*N*-(4-hydroxyphényl-1-méthyl-*N*-(1-méthyl-2,3-dihydro-1*H*-pyrrolo[2,3-*b*]pyridin-5-yl)-1*H*-pyrrole-3-carboxamide**
**Exemple 174. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-(méthoxyméthyl)-1-méthyl-*N*-(1-méthyl-1*H*-pyrazol-4-yl)-1*H*-pyrrole-3-carboxamide**
**Exemple 175. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-(méthoxyméthyl)-1-méthyl-*N*-(1-méthyl-1*H*-pyrrolo[2,3-*b*]pyridin-5-yl)-1*H*-pyrrole-3-carboxamide**
**Exemple 176. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-(méthoxyméthyl)-1-méthyl-*N*-(1-méthyl-2,3-dihydro-1*H*-pyrrolo[2,3-*b*]pyridin-5-yl)-1*H*-pyrrole-3-carboxamide**
**Exemple 177. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H*-pyrazol-4-yl)-2-(2,2,2-trifluoroéthyl)-1*H*-pyrrole-3-carboxamide**
**Exemple 178. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H-*pyrrolo[2,3-*b*]pyridin-5-yl)-2-(2,2,2-trifluorométhyl)-1*H*-pyrrole-3-carboxamide**
**Exemple 179. 5-(5-chloro-2-{[(3*S*)-3-(morpholin-4-ylméthyl)-3,4-dihydroisoquinolin 2(1*H*)-yl]carbonyl}phényl)-*N*-(4-hydroxyphényl)-1-méthyl-*N*-(1-méthyl-2,3-dihydro-1*H*-pyrrolo[2,3-*b*]pyridin-5-yl)2-(2,2,2-trifluorométhyl)-1*H*-pyrrole-3-carboxamide**
**Exemple 180. 5-(5-chloro-2-{[(3*S*)-3-(morpholin-4-ylméthyl)-3,4-dihydroisoquinolin-2(1*H*)-yl]carbonyl}phényl)-2-(2,2-difluoroéthyl)-*N*-(4-hydroxyphényl)-1-méthyl-*N*-(1-méthyl-1*H*-pyrazol-4-yl)-1*H*-pyrrole-3-carboxamide**
**Exemple 181. 5-(5-chloro-2-{[(3*S*)-3-(morpholin-4-ylméthyl)-3,4-dihydroisoquinolin-2(1*H*)-yl]carbonyl}phényl)-2-(2,2-difluoroéthyl)-*N*-(4-hydroxyphényl)-1-méthyl-*N*-(1-méthyl-1*H*-pyrrolo[2,3-*b*]pyridin-5-yl)-1*H*-pyrrole-3-carboxamide**
**Exemple 182. 5-(5-chloro-2-{[(3*S*)-3-(morpholin-4-ylméthyl)-3,4-dihydroisoquinolin-2(1*H*)-yl]carbonyl}phényl)-2-(2,2-difluoroéthyl)-*N*-(4-hydroxyphényl)-1-méthyl-*N*-(1-méthyl-2,3-dihydro-1H-pyrrolo[2,3-b]pyridin-5-yl)-1*H*-pyrrole-3-carboxamide**
**Exemple 183. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-(2-méthoxyéthyl)-1-méthyl-*N*-(1-methyl-1*H*-pyrazol-4-yl)-1*H*-pyrrole-3-carboxamide**
**Exemple 184. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-(2-méthoxyméthyl)-1-méthyl-*N*-(1-méthyl-1*H*-pyrrolo[2,3-*b*]pyridin-5-yl)-1*H*-pyrrole-3-carboxamide**
**Exemple 185. 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-2-(2-méthoxyéthyl)-1-méthyl-*N*-(1-méthyl-2,3-dihydro-1*H*-pyrrolo[2,3-*b*]pyridin-5-yl)-1*H*-pyrrole-3-carboxamide**
**Exemple 186. 5-(5-chloro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-1*H*-pyrazol-4-yl-2-(morpholin-4-ylméthyl)-1*H*-pyrrole-3-carboxamide**
**Exemple 187. 5-(5-chloro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-1*H*-pyrrolo[2,3-*b*]pyridin-5-yl)-2-(morpholin-4-ylméthyl)-1*H*-pyrrole-3-carboxamide**
**Exemple 188. 5-(5-chloro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-2,3-dihydro-1*H-*pyrrolo[2,3-b]pyridin-5-yl)-2-(morpholin-4-ylméthyl)-1*H*-pyrrole-3-carboxamide**
**Exemple 189. 5-(5-chloro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-1*H*-pyrazol-4-yl-2-[2-(morpholin-4-yl)éthyl]-1*H*-pyrrole-3-carboxamide**
**Exemple 190. 5-(5-chloro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-1*H*-pyrrolo[2,3-*b*]pyridin-5-yl)-2-[2-(morpholin-4-yl)éthyl]-1*H*-pyrrole-3-carboxamide**
**Exemple 191. 5-(5-chloro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-2,3-dihydro-1*H-*pyrrolo[2,3-*b*]pyridin-5-yl)-2-[2-(morpholin-4-yl)éthyl]-1*H*-pyrrole-3-carboxamide**
**Exemple 192. 5-(5-chloro-2-{[(3*S*)-3-(trifluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H-*pyrrole-3-carboxamide**
**Exemple 193. 5-(5-chloro-2-{[(3*S*)-3-(trifluorométhyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H-*pyrrolo[2,3-*b*]pyridin-5-yl)-1*H*-pyrrole-3-carboxamide**
**Exemple 194. 5-(5-chloro-2-{[(3*S*)-3-(difluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 195. 5-(5-chloro-2-{[(3*S*)-3-(difluorométhyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H-*pyrrolo[2,3-*b*]pyridin-5-yl)-1*H*-pyrrole-3-carboxamide**
**Exemple 196. 5-(5-chloro-2-{[(3*S*)-3-(fluorométhyl-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 197. 5-(5-chloro-2-{[(3*S*)-3-(fluorométhyl-3,4-dihydroisoquinolin-2(1*H*)-yl]carbonyl}phényl)-*N*-(4-hydroxyphényl)-1,2-diméthyl-*N*-(1-méthyl-2,3-dihydro-1*H-*pyrrolo**
**Exemple 198. 5-(5-chloro-2-{[(3*S*)-3-[(9a*S*)-hexahydropyrazino[2,1-*c*][1,4]oxazin-8(1*H*)-ylméthyl]-3,4-dihydroisoquinolin-2(1*H*)-yl]carbonyl}phényl)-N-(4-hydroxyphényl)-1,2-diméthyl-*N*-(1-méthyl-1*H*-pyrrolo[2,3-*b*]pyridin-5-yl)-1*H-*pyrrole-3-carboxamide**
**Exemple 199. 5-(5-chloro-2-{[(3*S*)-3-[(9a*S*)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N*-(1-méthyl-2,3-dihydro-1*H*-pyrrolo[2,3-b]pyridin-5-yl)-1*H*-pyrrole-3-carboxamide**
**Exemple 200. 5-(5-chloro-2-{[(3*S*)-3-[(9aR)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N-*(1méthyl-1*H*-pyrrolo[2,3-*b*]pyridin-5-yl)-1*H-*pyrrole-3-carboxamide**
**Exemple 201. 5-(5-chloro-2-{[(3*S*)-3-[(9a*S*)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N-*(1-méthyl-2,3-dihydro-1*H*-pyrrolo[2,3-*b*]pyridin-5-yl)-1*H*-pyrrole-3-carboxamide**
**Exemple 202. 5-(2-{[(3S)-3-(aminométhyl)-3,4,dihydroisoquinolin-2(1*H*)-yl]carbonyl}-5-chlorophényl)-*N*-(4-hydroxyphényl)-1,2-diméthyl-*N*-(1-méthyl-1*H*-pyrazol-4-yl)-1*H*-pyrrole-3-carboxamide**
**Exemple 203. 5-(2-{[(3*S*)-3-(aminométhyl)-3,4,dihydroisoquinolin-2(1*H*)-yl]carbonyl}-5-chlorophényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 204. 5-(2-{[(3*S*)-3-(aminométhyl)-3,4,dihydroisoquinolin-2(1*H*)-yl]carbonyl}-5-chlorophényl)-*N*-(4-hydroxyphényl)-1,2-diméthyl-*N*-(1-méthyl-2,3-dihydro-1*H-*pyrrolo[2,3-*b*]pyridin-5-yl)-1*H*-pyrrole-3-carboxamide**
**Exemple 205. 5-(5-chloro-2-{[(3*S*)-3-[(3-cyanoazetidin-1-yl)méthyl]-3,4-dihydroisoquinolin-2(1*H*)-yl]carbonyl}phényl)-*N*-(4-hydroxyphényl)-1,2-diméthyl-*N-*(1-méthl-1*H*-pyrrolo[2,3-*b*]pyridin-5-yl)-1*H*-pyrrolo-3-carboxamide**
**Exemple 206. 5-(5-chloro-2-{[(3*S*)-3-[(3-cyanoazetidin-1-yl)méthyl]-3,4-dihydroisoquinolin-2(1*H*)-yl]carbonyl}phényl)-*N*-(4-hydroxyphényl)-1,2-diméthyl-*N-*(1-méthyl-2,3-dihydro-1*H*-pyrrolo[2,3-*b*]pyridin-5-yl)-1*H*-pyrrole-3-carboxamide**
**Exemple 207. 5-(5-Chloro-2-{[(3*S*)-3-(1-oxa-6-azaspiro[3,3]hept-6-ylméthyl)3,4-dihydroisoquinolin-2(1*H*)-yl]carbonyl}phényl)-*N*-(4-hydroxyphényl)-1,2-diméthyl-*N-*(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 1 et le composé de la Préparation 8' au Stade A, ainsi que le composé de la Préparation 1" au Stade C.
   ***Masse haute résolution (ESI+-*/*FIA*/*HR, ESI-*/*FIA):***
   Formule brute : C₃₉ H₃₉ Cl N₆ O₄
   [M+H]⁺calculé : 691.2794
   [M+H]⁺ mesuré : 691.2796
**Exemple 208. 5-(5-chloro-2-{[(3*S*)-3-(1-oxa-6-azaspiro[3,3]hept-6-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrrolo[2,3-*b*]pyridin-5-yl)-1*H*-pyrrole-3-carboxamide**
**Exemple 209. Chlorhydrate de 5-(5-fluoro-4-méthoxy-2-{[(3*S*)-3-(morpholin-4-ylméthyl)-3,4-dihydroisoquinotin-2(1*H*)-yl]carbonyl}phényl)-*N*-1(4-hydroxyphényl)-1,2-diméthyl-*N*-(1-méthyl-1*H*-pyrrolo[2,3-*b*]pyridin-5-yl)-1*H*-pyrrole-3-carboxamide**
   On procède selon le protocole général de l'Exemple 1 en utilisant les préparations adéquates. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.
   ***Microanalyse élémentaire :* (% *théorique : mesuré)***
   %C=66.27:66.84; %H=5.69:5.15; %N=10.78:10.71; %Cl-=4.55:4.46
   ***Masse haute résolution* (ESI+-/FIA) :**
   Formule brute : C₄₃ H₄₃ F N₆ O₅
   [M+H]⁺calculé : 743.3352
   [M+H]⁺ mesuré : 743.3353
**Exemple 210. Chlorhydrate de 5-(5-fluoro-4-méthoxy-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
   ***Stade A : 5-(5-fluoro-4-méthoxy-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-(1-méthyl-1H-pyrrolo[2,3-b]pyridin-5-yl)-1H-pyrrole-3-carboxamide***
   Le composé du titre est obtenu selon le procédé de l'Exemple 1 en utilisant le composé de la Préparation 14 et la (3*S*)-3-(4-morpholinylméthyl)-1,2,3,4-tétrahydroisoquinoline (voir Préparation 3') au Stade A, ainsi que le composé de la Préparation 11" au Stade C.
   ***Stade B : Chlorhydrate de 5-(5-chloro-2-{[(3S)-3-(morpholin-4-ylméthyl)-3,4-dihydroisoquinolin-2(1H)-yl]carbonyl}phényl)-1,2-diméthyl-N-(1-méthyl-2,3-dihydro-1H-pyrrolo[2,3-b]pyridin-5-yl)-N-phényl-1H-pyrrole-3-carboxamide***
   Le composé du titre est obtenu selon le procédé décrit au Stade B de l'Exemple 49 à partir du composé du stade précédent, étant entendu que le produit est finalement soumis à une étape de salification en présence d'éther chlorhydrique 1M dans l'éther. Après filtration et lyophilisation dans un mélange acétonitrile/eau, on obtient le produit attendu.
   ***Microanalyse élémentaire :* (% *théorique : mesuré)***
   %C=60.12:59.77; %H=4.92_{:}4.76; %N=10.79:10.39; %Cl-=4.55:5.17
   ***Masse haute résolution (ESI*/*FIA*/*HR et MS*/*MS) :***
   Formule brute : C₄₀ H₄₀ N₄ O₅
   [M+H]⁺calculé : 657.3071
   [M+H]⁺ mesuré : 657.3066
**Exemple 211. 5-(4-fluoro-5-méthoxy-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrrolo[2,3-*b*]pyridin-5-yl)-1*H*-pyrrole-3-carboxamide**
**Exemple 212. 5-(4-fluoro-5-méthoxy-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*(1-méthyl-2,3-dihydro-1*H*-pyrrolo[2,3-*b*]pyridin-5-yl)-1*H*-pyrrole-3-carboxamide**
**Exemple 213. *N*-(1-éthyl-1*H*-pyrazol-4-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
**Exemple 214. Chlorhydrate de N-(1-cyclopropyl-1*H*-pyrazol-4-yl)-5-(5-fluoro-2-{[(3*S*)-3-(morpholin-4-ylméthyl-3,4-dihydroisoquinolin-2(1*H*)-yl]carbonyl}phényl)-*N*-(4-hydroxyphényl)1,2-diméthyl-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que le composé de la Préparation 28" au Stade C. Le composé obtenu est salifié et lyophilisé tel que décrit dans le procédé général pour obtenir le composé du titre.
   ***Microanalyse élémentaire :* (% *théorique : mesuré)***
   %C=66.24:66.53; %H=5.84:5.39; %N=10.59:10.92 %Cl⁻=4.89:5.68
**Exemple 215. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-[1-(trifluorométhyl)-1*H*-pyrazol-4-yl]-1*H*-pyrrole-3-carboxamide**
**Exemple 216. *N*-[1-(difluorométhyl)-1*H*-pyrazol-4-yl]-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl**)-**1**,**2**-**diméthyl**-**1*H*-pyrrole**-3-**carboxamide**
**Exemple 217. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-*N*-[1,2-diméthoxyéthyl)-1*H*-pyrazol-4-yl]-1,2-diméthyl-1*H*-pyrrrole-3-carboxamide**
**Exemple** 218. **5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-[1-(oxetan-3-yl)-1*H-*pyrazol-4-yl]-1*H*-pyrrole-3-carboxamide**
**Exemple 219. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl)-*N*-(1-tétrahydrofuran-3-yl)-1*H*-pyrazol-4-yl]-1*H*-pyrrole-3-carboxamide**
**Exemple** 220. **5-(5-fluoro-2-{[(3*S*)-3-(morpholin-4-ylméthyl)-3,4-dihydroisoquinolin-2(1*H*)-yl]carbonyl}phényl)-*N*-[1-(2-hydroxy-2-méthylpropyl)-1*H*-pyrazol-4-yl]-*N*-(4-hydroxyphényl)-1,2-diméthyl-1*H*-pyrrole-3-carboxamide**
**Exemple** 221. **5-(5-fluoro-2-{[(3*S*)-3-(morpholin-4-ylméthyl)-3,4-dihydroisoquinolin-2(1*H*)-yl]carbonyl}phényl)-1,2-diméthyl-*N*-(1-méthyl-1*H*-pyrrole[2,3-*b*]pyridin-5-yl)-*N*-phényl-1*H*-pyrrole-3-carboxamide**
**Exemple 222. 5-(5-fluoro-2-{[(3*S*)-3-(morpholin-4-ylméthyl)-3,4-dihydroisoquinolin-2(1*H*)-yl]carbonyl}phényl)-1,2-diméthyl-*N*-(1-méthyl-2,3-dihydro-1*H*-pyrrole[2,3-*b*]pyridin-5-yl)-*N*-phényl-1*H*-pyrrole-3-carboxamide**
**Exemple** 223. **5-(5-fluoro-2-{[(3*S*)-3-(morpholin-4-ylméthyl)-3,4-dihydroisoquinolin-2(1*H*)-yl]carbonyl}phényl)-*N*-(4-fluorophényl)-1,2-diméthyl-*N*-(1-méthyl-1*H-*pyrrolo[2,3-*b*]pyridin-5-yl)-1*H*-pyrrole-3-carboxamide**
**Exemple 224. 5-(5-fluoro-2-{[(3*S*)-3-(morpholin-4-ylméthyl)-3,4-dihydroisoquinolin-2(1*H*)-yl]carbonyl}phényl)-*N*-(4-fluorophényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 225.** (exemple de référence) **5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-*N*,1,2-triméthyl-1*H*-pyrrole-3-carboxamide**
**Exemple 226. 5-(5-fluoro-2-{[(3*S*)-3-(morpholin-4-ylméthyl)-3,4-dihydroisoquinolin-2(1*H*)-yl]carbonyl}phényl)-*N*-(2-hydroxypyridin-5-yl)-1,2-diméthyl-*N*-phényl-1*H-*pyrrole-3-carboxamide**
**Exemple 227. 5-(5-fluoro-2-{[(3*S*)-3-(morpholin-4-ylméthyl)-3,4-dihydroisoquinolin-2(1*H*)-yl]carbonyl}phényl)-*N*-(2-hydroxypyridin-5-yl)-1,2-diméthyl-*N*-(1-méthyl-1*H*-pyrazolo[2,3-*b*]pyridin-5-yl)-1*H*-pyrrole-3-carboxamide**
**Exemple** 228. **5-(5-fluoro-2-{[(3*S*)-3-(morpholin-4-ylméthyl)-3,4-dihydroisoquinolin-2(1*H*)-yl]carbonyl}phényl)-*N*-(2-hydroxypyridin-5-yl)-1,2-diméthyl-*N*-(1-méthyl-2,3-dihydro-1*H*-pyrazolo[2,3-*b*]pyridin-5-yl)-1*H*-pyrrole-3-carboxamide**
**Exemple 229. 5-(5-fluoro-2-{[(3*S*)-3-(morpholin-4-ylméthyl)-3,4-dihydroisoquinolin-2(1*H*)-yl]carbonyl}phényl)-*N*-(2-hydroxypyridin-5-yl)-1,2-diméthyl-*N*-(1-méthyl-1*H*-pyrazol-4-yl)-1*H*-pyrrole-3-carboxamide**
**Exemple 230. Chlorhydrate de *N*-(5-cyano-1-méthyl-1*H*-pyrrol-3-yl)-5-(5-fluoro-2-{[(3*S*)-3-(morpholin-4-ylméthyl)-3,4-dihydroisoquinolin-2(1*H*)-yl]carbonyl}phényl)-*N*-(4(hydroxyphényl)-1,2-diméthyl-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que le composé de la Préparation 19" au Stade C. Le composé obtenu est salifié et lyophilisé tel que décrit dans le procédé général pour obtenir le composé du titre.
   ***Microanalyse élémentaire :* (% *théorique : mesuré)***
   %C=66.43:67.09; %H=5.57:5.21; %N=11.62:11.48 %Cl⁻=4.90:4.75
   ***Masse haute résolution (ESI+) :***
   Formule brute : C₄₀H₃₉FN₆O₄
   [M+H]⁺calculé : 687.3097
   [M+H]⁺ mesuré : 687.3073
**Exemple** 231. **5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-1*H*-pyrazol-4-yl)-1*H*-pyrrole-3-carboxamide**
**Exemple** 232. **5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-1*H-*pyrrolo[2,3-*b*]pyridin-5-yl)-1*H*-pyrrole-3-carboxamide**
**Exemple 233. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-2,3-dihydro-1*H*-pyrrolo[2,3-*b*]pyridin-5-yl)-1*H*-pyrrole-3-carboxamide**
**Exemple 234. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H*-pyrazol-4-yl)-1*H*-pyrrole-3-carboxamide**
**Exemple 235. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H-*pyrrolol[2,3-*b*]pyridin-5-yl)-1*H*-pyrrole-3-carboxamide**
**Exemple 236. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-2,3-dihydro-1*H*-pyrrole[2,3-*b*]pyridin-5-yl)-1*H*-pyrrole-3-carboxamide**
**Exemple 237. 1-éthyl-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-1*H*-pyrazol-4-yl)-1*H*-pyrrole-3-carboxamide**
**Exemple 238. 1-éthyl-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-1*H*-pyrrolo[2,3-*b*]pyridin-5-yl)-1*H*-pyrrole-3-carboxamide**
**Exemple 239. 1-éthyl-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-2,3-dihydro-1*H*-pyrrolo[2,3-*b*]pyridin-5-yl)-1*H*-pyrrole-3-carboxamide**
**Exemple 240. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-(2-méthoxyéthyl)-2-méthyl-*N*-(1-méthyl-1*H*-pyrrol-4-yl)-1*H*-pyrrole-3-carboxamide**
**Exemple 241. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-(2-méthoxyéthyl)-2-méthyl-*N*-(1-méthyl-1*H*-pyrrolo[2,3-*b*]pyridin-5-yl)-1*H*-pyrrole-3-carboxamide**
**Exemple 242. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-(2-méthoxyéthyl)-2-méthyl-*N*-(1-méthyl-2,3-dihydro-1*H*-pyrrolo[2,3-*b*]pyridin-5-yl)-1*H*-pyrrole-3-carboxamide**
**Exemple 243. 1-(2-fluoroéthyl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-1*H*-pyrrol-4-yl)-1*H*-pyrrole-3-carboxamide**
**Exemple 244. 1-(2-fluoroéthyl)-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-1*H*-pyrrolo[2,3-*b*]pyridin-5-yl)-1*H*-pyrrole-3-carboxamide**
**Exemple 245. 1-(2-fluoroéthyl)-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-2,3-dihydro-1*H*-pyrrolo[2,3-*b*]pyridin-5-yl)-1*H*-pyrrole-3-carboxamide**
**Exemple 246. 1-(2,2-difluoroéthyl)-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-1*H*-pyrazol-4-yl)-1*H*-pyrrole-3-carboxamide**
**Exemple 247. 1-(2,2-difluoroéthyl)-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-1*H*-pyrrolo[2,3-*b*]pyridin-5-yl)-1*H*-pyrrole-3-carboxamide**
**Exemple 248. 1-(2,2-difluoroéthyl)-5-(5-fluoro-2-{[(3*S*)-3-(morpholin-4-ylméthyl)-3,4-dihydroisoquinolin-2(1*H*)-yl]carbonyl}phényl-*N*-(4-hydroxyphényl)-2-méthyl-*N*-(1-méthyl-2,3-dihydro-1*H*-pyrrolo[2,3-*b*]pyridin-5-yl)-1*H*-pyrrole-3-carboxamide**
**Exemple 249. 5-(5-fluoro-2-{[(3*S*)-3-(morpholin-4-ylméthyl)-3,4-dihydroisoquinolin-2(1*H*)-yl]carbonyl]phényl)-*N*-(4-hydroxyphényl)2-méthyl-*N*-(1-méthyl-1*H*-pyrazol-4-yl)-1-(2,2,2-trifluoroéthyl)-1*H*-pyrrole-3-carboxamide**
**Exemple 250. 5-(5-fluoro-2-{[(3*S*)-3-(morpholin-4-ylméthyl)-3,4-dihydroisoquinolin-2(1*H*)-yl]carbonyl]phényl)-*N*-(4-hydroxyphényl)-2-méthyl-*N*-(1-méthyl-1*H-*pyrrolo[2,3-*b*]pyridin-5-yl)-1-(2,2,2-trifluoroéthyl)-1*H*-pyrrole-3-carboxamide Exemple 251. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-méthyl-*N*-(1-méthyl-2,3-dihydro-1*H*-pyrrolo[2,3-*b*]pyridin-5-yl)-1-(2,2,2-trifluoroéthyl)-1*H*-pyrrole-3-carboxamide**
**Exemple 252. 5-(5-fluoro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-1*H*-pyrazol-4-yl)-1-[2-(morpholin-4-yl)éthyl]-1*H*-pyrrole-3-carboxamide**
**Exemple 253. 5-(5-fluoro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-1*H*-pyrrolo[2,3-*b*]pyridin-5-yl)-1-[2-(morpholin-4-yl)éthyl]-1*H*-pyrrole-3-carboxamide**
**Exemple 254. 5-(5-fluoro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-2,3-dihydro-1*H-*pyrrolo[2,3,*b*]pyridin-5-yl)-1-[2-(morpholin-4-yl)éthyl]-1*H*-pyrrole-3-carboxamide**
**Exemple 255. 5-(5-fluoro-2-{[(3*R*)-3-méthyl-3,4-dihydroisoquinolin-2(1*H*)-yl]carbonyl}phényl)-*N*-(4-hydroxyphényl)-2-méthyl-1-[2-(morpholin-4-yl)éthyl]-*N-*phényl-1*H*-pyrrole-3-carboxamide**
**Exemple 256. 1-[2-(diméthylamino)éthyl]-5-(5-fluoro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-1*H*-pyrazol-4-yl)-1*H*-pyrrole-3-carboxamide**
**Exemple 257. 1-[2-(diméthylamino)éthyl]-5-(5-fluoro-2-{[(3*R*)-3-méthyl-3,4-dihydroisoquinolin-2(1*H*)-yl]carbonyl}phényl)-*N*-(4-hydroxyphényl)-2-méthyl-*N-*phényl-1H-pyrrole-3-carboxamide**
**Exemple 258. 1-[2-(diméthylamino)éthyl]-5-(5-fluoro-2-{[(3*R*)-3-méthyl-3,4-dihydroisoquinolin-2(1*H*)-yl]carbonyl}phényl)-*N*-(4-hydroxyphényl)2-méthyl-*N*-(1-méthyl-1*H*-pyrrolo|2,3-*b*]pyridin-5-yl)-1*H*-pyrrolo-3-carboxamide**
**Exemple 259. 1-[2-(diméthylamino)éthyl]-5-(5-fluoro-2-{[(3*R*)-3-méthyl-3,4-dihydroisoquinolin-2(1*H*)-yl]carbonyl}phényl)-*N*-(4-hydroxyphényl)2-méthyl-*N*-(1-méthyl-2,3-dihydro-1*H*-pyrrolo[2,3-b]pyridin-5-yl)-1*H*-pyrrole-3-carboxamide**
**Exemple 260. 5-(5-fluoro-2-{[(3*R*)-3-méthyl-3,4-dihydroisoquinolin-2(1*H*)-yl]carbonyl}phényl)-*N*-(4-hydroxyphényl)-1-{2-[(2-méthoxyéthyl)(méthyl)amino]éthyl}-2-méthyl-*N*-(1-méthyl-1*H*-pyrazol-4-yl)-1*H-*pyrrole-3-carboxamide**
**Exemple 261. 5-(5-fluoro-2-{[(3*R*)-3-méthyl-3,4-dihydroisoquinolin-2(1*H*)-yl]carbonyl}phényl)-*N*-(4-hydroxyphényl)-1-{2-[(2-méthoxyéthyl)(méthyl)amino]éthyl}-2-méthyl-*N*-(1-méthyl-1*H*-pyrrolo[2,3-*b*]pyridin-5-yl)-1*H*-pyrrole-3-carboxamide**
**Exemple 262. 5-(5-fluoro-2-{[(3*R*)-3-méthyl-3,4-dihydroisoquinolin-2(1*H*)-yl]carbonyl}phényl)-*N*-(4-hydroxyphényl)-1-{2-[(2-méthoxyéthyl)(méthyl)amino]éthyl}-2-méthyl-*N*-(1-méthyl-2,3-dihydro-1*H-*pyrrolo[2,3-*b*]pyridin-5-yl)-1*H*-pyrrole-3-carboxamide**
**Exemple 263. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H*-pyrazol-4-yl)-2-(trifluorométhyl)-1*H*-pyrrole-3-carboxamide**
**Exemple 264. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H-*pyrrolo[2,3-*b*]pyridin-5-yl)-2-(trifluorométhyl)-1*H*-pyrrole-3-carboxamide**
**Exemple 265. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-2,3-dihydro-1*H*-pyrrolo[2,3-*b*]pyridin-5-yl)-2-(trifluorométhyl)-1*H*-pyrrole-3-carboxamide**
**Exemple 266. 2-(difluorométhyl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H*-pyrazol-4-yl)-1*H*-pyrrole-3-carboxamide**
**Exemple 267. 2-(difluorométhyl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-1*H*-pyrrolo[2,3-*b*]pyridin-5-yl)-1*H*-pyrrole-3-carboxamide**
**Exemple 268. 2-(difluorométhyl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-2,3-dihydro-1*H*-pyrrolo[2,3-*b*]pyridin-5-yl)-1*H*-pyrrole-3-carboxamide**
**Exemple 269. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-(méthoxyéthyl)-1-méthyl-*N*-(1-méthyl-1*H*-pyrazol-4-yl)-1*H*-pyrrole-3-carboxamide**
**Exemple 270. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-(méthoxyéthyl)-1-méthyl-*N*-(1-méthyl-1*H*-pyrrolo[2,3-*b*]pyridin-5-yl)-1*H*-pyrrole-3-carboxamide**
**Exemple 271. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-(méthoxyéthyl)-1-méthyl-*N*-(1-méthyl-2,3-dihydro-1*H*-pyrrolo[2,3-*b*]pyridin-5-yl)-1*H*-pyrrole-3-carboxamide**
**Exemple 272. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl)-*N*-(1-méthyl-1*H*-pyrazol-4-yl)-2-(2,2,2-trifluoroéthyl)-1*H*-pyrrole-3-carboxamide**
**Exemple 273. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl)-*N*-(1-méthyl-1*H-*pyrrolo[2,3-*b*]pyridin-5-yl)-2-(2,2,2-trifluoroéthyl)-1*H*-pyrrole-3-carboxamide**
**Exemple 274. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1-méthyl-*N*-(1-méthyl-2,3-dihydro-1*H*-pyrrolo[2,3-*b*]pyridin-5-yl)-2-(2,2,2-trifluoroéthyl)-1*H*-pyrrole-3-carboxamide**
**Exemple 275. 2-(2,2-difluoroéthyl)-5-(5-fluoro-2-{[(3*S*)-morpholin-4-ylméthyl)-3,4-dihydroisoquinolin-2(1*H*)-yl]carbonyl}phényl)-*N*-(4-hydroxyphényl)-1-méthyl*-N*-(1-méthyl-1*H*-pyrazol-4-yl)-1*H*-pyrrole-3-carboxamide**
**Exemple 276. 2-(2,2-difluoroéthyl)-5-(5-fluoro-2-{[(3*S*)-morpholin-4-ylméthyl)-3,4-dihydroisoquinolin-2(1*H*)-yl]carbonyl}phényl)-*N*-(4-hydroxyphényl)-1-méthyl*-N*-(1-méthyl-1*H*-pyrrolo[2,3-*b*]pyridin-5-yl)-1*H*-pyrrole-3-carboxamide**
**Exemple 277. 2-(2,2-difluoroéthyl)-5-(5-fluoro-2-{[(3*S*)-morpholin-4-ylméthyl)-3,4-dihydroisoquinolin-2(1*H*)-yl]carbonyl}phényl)-*N*-(4-hydroxyphényl)-1-méthyl*-N*-(1-méthyl-2,3-dihydro-1*H*-pyrrolo[2,3-*b*]pyridin-5-yl)-1*H*-pyrrole-3-carboxamide**
**Exemple 278. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-(2-méthoxyéthyl)1-méthyl-*N*-(1-méthyl-1*H*-pyrazol-4-yl)-1*H*-pyrrole-3-carboxamide**
**Exemple 279. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-(2-méthoxyéthyl)-1-méthyl-*N*-(1-méthyl-1*H*-pyrrolo[2,3-*b*]pyridin-5-yl)-1*H*-pyrrole-3-carboxamide**
**Exemple 280. 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-2-(2-méthoxyéthyl)1-méthyl-*N*-(1-méthyl-2,3-dihydro-1*H*-pyrrolo[2,3-*b*]pyridin-5-yl)-1*H*-pyrrole-3-carboxamide**
**Exemple 281. 5-(5-fluoro-2-{[(3R)-3-méthyl-3,4-dihydroisoquinolin-2(1*H*)-yl]carbonyl)phényl)-*N*-(4-hydroxyphényl)-1-méthyl-*N*-(1-méthyl-1*H*-pyrazol-4-yl)-2-(morpholin-4-ylméthyl)-1*H*-pyrrole-3-carboxamide**
**Exemple 282. 5-(5-fluoro-2-{[(3*R*)-3-méthyl-3,4-dihydroisoquinolin-2(1*H*)-yl]carbonyl)phényl)-*N*-(4-hydroxyphényl)-1-méthyl-*N*-(1-méthyl-1*H*-pyrrolo[2,3-*b*]pyridin-5-yl)-2-(morpholin-4-ylméthyl)-1*H*-pyrrole-3-carboxamide**
**Exemple 283. 5-(5-fluoro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-2,3-dihydro-1*H-*pyrrolo-[2,3-*b*]pyridin-5-yl)-2-(morpholin-4-ylméthyl)-1*H*-pyrrole-3-carboxamide**
**Exemple 284. 5-(5-fluoro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-1*H*-pyrazol-4-yl)-2-[2-(morpholin-4-yl)éthyl]-1*H*-pyrrole-3-carboxamide**
**Exemple 285. 5-(5-fluoro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-1*H*-pyrrolo[2,3-*b*]pyridin-5-yl)-2-(morpholin-4-ylméthyl)-1*H*-pyrrole-3-carboxamide**
**Exemple 286. 5-(5-fluoro-2-{[(3*R*)-3-méthyl-3,4-dihydroisoquinolin-2(1*H*)-yl]carbonyl}phényl)-*N*-(4-hydroxyphényl)-1-méthyl-*N*-(1-méthyl-2,3-dihydro-1*H-*pyrrolo[2,3-*b*]pyridin-5-yl)-2-[2-(morpholin-4-yl)éthyl]-1*H*-pyrrole-3-carboxamide**
**Exemple 287. 5-(5-fluoro-2-{[(3*S*)-3-(trifluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide**
**Exemple 288. 5-(5-fluoro-2-{[(3*S*)-3-(trifluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 289. 5-(5-fluoro-2-{[(3*S*)-3-(trifluorométhyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H-*pyrrolo[2,3-*b*]pyridin-5-yl)-1*H*-pyrrole-3-carboxamide**
**Exemple 290. 5-(2-{[(3*S*)-3-difluorométhyl)-3,4-dihydroisoquinolin-2(1*H*)-yl]carbonyl}-5-fluorophényl)-*N*-(4-hydroxyphényl)-1,2-diméthyl-*N*-(1-méthyl-1*H-*pyrazol-4-yl)-1*H*-pyrrole-3-carboxamide**
**Exemple 291. 5-(2-{[(3*S*)-3-difluorométhyl)-3,4-dihydroisoquinolin-2(1*H*)-yl]carbonyl}-5-fluorophényl)-*N*-(4-hydroxyphényl)-1,2-diméthyl-*N*-(1-méthyl-1*H-*pyrrolo[2,3-*b*]pyridin-5-yl)-1*H*-pyrrolo-3-carboxamide**
**Exemple 292. 5-(2-{[(3*S*)-3-difluorométhyl)-3,4-dihydroisoquinolin-2(1*H*)-yl]carbonyl}-5-fluorophényl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrolo-3-carboxamide**
**Exemple 293. 5-(2-{[(3*S*)-3-difluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide**
**Exemple 294. 5-(5-fluoro-2-{[(3*S*)-3-(fluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 295. 5-(5-fluoro-2-{[(3*S*)-3-(fluorométhyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H-*pyrrolo[2,3-*b*]pyridin-5-yl)-1*H*-pyrrole-3-carboxamide**
**Exemple 296. 5-(5-fluoro-2-{[(3*S*)-3-(fluorométhyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H-*pyrrolo[2,3-*b*]pyridin-5-yl)-1*H*-pyrrole-3-carboxamide**
**Exemple 297. 5-(5-fluoro-2-{[(3*S*)-3-[(9a*S*)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N*-(1-méthyl-1*H*-pyrrole[2,3-*b*]pyridin-5-yl)-1*H-*pyrrole-3-carboxamide**
**Exemple 298. 5-(5-fluoro-2-{[(3*S*)-3-[(9a*S*)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 299. 5-(5-fluoro-2-{[(3*S*)-3-[(9a*S*)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N*-(1-méthyl-1*H*-pyrrolo-4-yl)-1*H*-pyrrole-3-carboxamide**
**Exemple 300. 5-(5-fluoro-2-{[(3*S*)-3-[(9a*R*)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H-*pyrrole-3-carboxamide**
**Exemple 301. 5-(5-fluoro-2-{[(3*S*)-3-[(9a*R*)-hexahydropyrazino[2,1-*c*][1,4]oxazin-8(1*H*)-ylméthyl]-3,4-dihydroisoquinolin-2(1*H*)-yl]carbonyl}phényl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 302. 5-(2-{[(3*S*)-3-(aminométhyl)-3,4-dihydroisoquinolin-2(1*H*)-yl]carbonyl}-5-fluorophényl)-*N*-(4-hydroxyphényl)-1,2-diméthyl-*N*-1(1-méthyl-1*H*-pyrazol-4-yl)-1*H-*pyrrole-3-carboxamide**
**Exemple 303. 5-(2-{[(3*S*)-3-(aminométhyl)-3,4-dihydroisoquinolin-2(1*H*)-yl]carbonyl}-5-fluorophényl-*N*-(4-hydroxyphényl)-1,2-diméthyl-*N*-(1-méthyl-1*H*-pyrrole[2,3-*b*]pyridin-5-yl)-1*H*-pyrrole-3-carboxamide**
**Exemple 304. 5-(2-{[(3*S*)-3-(aminométhyl)-3,4-dihydroisoquinolin-2(1*H*)-yl]carbonyl}-5-fluorophényl-*N*-(4-hydroxyphényl)-1,2-diméthyl-*N*-(1-méthyl-2,3-dihydro-1*H-*pyrrolo[2,3-*b*]pyridin-5-yl)-1*H*-pyrrole-3-carboxamide**
**Exemple 305. 5-(2-{[(3*S*)-3-[(3-cyanoazetidin-1-yl)méthyl]-3,4-dihydroisoquinolin-2(1*H*)-yl]carbonyl}-5-fluorophényl)-*N*-(4-hydroxyphényl)1,2-diméthyl-*N*-(1-méthyl-1*H*-pyrazol-4-yl)-1*H*-pyrrole-3-carboxamide**
**Exemple 306. 5-(2-{[(3*S*)-3-[(3-cyanoazetidin-1-yl)méthyl]-3,4-dihydroisoquinolin-2(1*H*)-yl]carbonyl}-5-fluorophényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 307. 5-(2-{[(3*S*)-3-[(3-cyanoazetidin-1-yl)méthyl]-3,4-dihydroisoquinolin-2(1*H*)-yl]carbonyl}-5-fluorophényl)-*N*-(4-hydroxyphényl)-1,2-diméthyl-*N*-(1-méthyl-2,3-dihydro-1*H*-pyrrole[2,3-*b*]pyridin-5-yl)-1*H*-pyrrole-3-carboxamide**
**Exemple 308. 5-(5-fluoro-2-{[(3*S*)-3-(1-oxa-6-azaspiro[3.3]hept-6-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrazol-4-yl)-1*H*-pyrrole-3-carboxamide**
**Exemple 309. 5-(5-fluoro-2-{[(3*S*)-3-(1-oxa-6-azaspiro[3.3]hept-6-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrrolo[2,3-b]pyridin-5-yl)-1*H*-pyrrole-3-carboxamide**
**Exemple 310. *N*-(1-éthyl-1*H*-pyrazol-4-yl)-*N*-(4-hydroxyphényl)-1,2-diméthyl-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 311. N-(1-cyclopropyl-1*H*-pyrazol-4-yl)-*N*-(4-hydroxyphényl)-1,2-diméthyl-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 312. *N*-(4-hydroxyphényl)-1,2-diméthyl-5-(6-{[(3*S*)-3-(morpholin-4-ylméthyl)-3,4-dibydroisoquinolin-2(1*H*)-yl]carbonyl}-1,3-benzodioxol-5-yl)-*N*-[1-(trifluorométhyl)-1*H*-pyrazol-4-yl]-1*H*-pyrrole-3-carboxamide**
**Exemple 313. *N*-[1-(difluorométhyl)-1*H*-pyrazol-4-yl]-*N*-(4-hydroxyphényl)1,2-diméthyl-5-(6-{[(3*S*)-3-(morpholin-4-ylméthyl)-3,4-dihydroisoquinolin-2(1*H*)-yl]carbonyl}1,3-benzodioxol-5-yl)-1*H*-pyrrole-3-carboxamide**
**Exemple 314. *N*-(4-hydroxyphényl)-*N*-[1-(2-méthoxyéthyl)-1*H*-pyrazol-4-yl]-1,2-diméthyl-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 315. *N*-(4-hydroxyphényl)-1,2-diméthyl-5-(6-{[(3*S*)-3-(morpholin-4-ylméthyl)-3,4-dihydroisoquinolin-2(1*H*)-yl]carbonyl}-1,3-benzodioxol-5-yl)-*N*-[1-(oxetan-3-yl)-1*H*-pyrazol-4-yl]-1H-pyrrole-3-carboxamide**
**Exemple 316. *N*-(4-hydroxyphényl)-1,2-diméthyl-5-(6-{[(3*S*)-3-(morpholin-4-ylméthyl)-3,4-dihydroisoquinolin-2(1*H*)-yl]carbonyl}-1,3-benzodioxol-5-yl)-*N*-[1-(tétrahydrofuran-3-yl)-1*H*-pyrazol-4-yl]-1*H*-pyrrole-3-carboxamide**
**Exemple 317. *N*-[1-(2-hydroxy-2-méthylpropyl)-1*H*-pyrazol-4-yl]-*N*-(4-hydroxyphényl)-1,2-diméthyl-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 318. 1,2-diméthyl-*N*-(1-méthyl-1*H*-pyrrolo[2,3-*b*]pyridin-5-yl)-5-(6{[(3*S*)-3-(morpholin-4-ylméthyl)-3,4-dihydroisoquinolin-2(1*H*)-yl]carbonyl}-1,3-benzodioxol-5-yl)-*N*-phényl-1*H*-pyrrole-3-carboxamide**
**Exemple 319. 1,2-diméthyl-*N*-(1-méthyl-2,3-dihydro-1*H*-pyrrolo[2,3-*b*]pyridin-5-yl)-5-(6-{[(3*S*)-3-(moorpholin-4-ylméthyl)-3,4-dihydroisoquinolin-2(1*H*)-yl]carbonyl}-1,3-benzodioxol-5-yl-*N*-phényl-1*H*-pyrrole-3-carboxamide**
**Exemple 320. *N*-(4-fluroophényl)-1,2-diméthyl-*N*-(1-méthyl-1*H*-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 321. *N*-(4-fluroophényl)-1,2-diméthyl-*N*-(1-méthyl-1*H*-pyrrolo[2,3-*b*]pyridin-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 322. (exemple de référence) *N*-(4-hydroxyphényl)-N,1,2-triméthyl-5-(6-{[(3*S*)-3-(morpholin-4-ylméthyl)-3,4-dihydroisoquinolin-2(1*H*)-yl]carbonyl}-1,3-benzodioxol-5-yl)-1*H-*pyrrole-3-carboxamide**
**Exemple 323. *N*,(2-dihydroxypyrimidin-5-yl)-1,2-diméthyl-5-(6-{[(3*S*)-3-morpholin-4-ylméthyl)-3,4-dihydroisoquinolin-2(1*H*)-yl]carbonyl}-1,3-benzodioxol-5-yl)-*N*-phényl-1*H*-pyrrole-3-carboxamide**
**Exemple 324. *N*,(2-dihydroxypyrimidin-5-yl)-1,2-diméthyl-*N*-(1-méthyl-1*H*-pyrrolo[2,3-*b*]pyridin-5-yl)-5-(6-{[(3*S*)-3-(morpholin-4-ylméthyl-3,4-dihydroisoquinolin-2(1*H*)-yl]carbonyl}-1,3-benzodioxol-5-yl)-1*H*-pyrrole-3-carboxamide**
**Exemple 325. *N-*(2-dihydroxypyrimidin-5-yl)-1,2-diméthyl-*N*-(1-méthyl-2,3-dihydro-1*H*-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide,**
**Exemple 326. *N-*(2-dihydroxypyrimidin-5-yl)-1,2-diméthyl-*N*-(1-méthyl-1*H-*pyrazol-4-yl)-5-(6-{[(3*S*)-3-(morpholin-4-ylméthyl)3,4-dihydroisoquinolin-2(1*H*)-yl]carbonyl}-1,3-benzodioxol-5-yl)-1*H*-pyrrole-3-carboxamide**
**Exemple 327. *N*-(5-cyano-1-méthyl-1*H*-pyrrol-3-yl)-*N*-(4-hydroxyphényl)-1,2-diméthyl-5-(6-{[(3*S*)-3-(morpholin-4ylméthyl)-3,4-dihydroisoquinolin-2(1*H*)-yl]carbonyl}-1,3-benzodioxol-5-yl)-1*H*-pyrrole-3-carboxamide**
**Exemple 328. *N*-(4-hydroxyphényl)2-méthyl-*N*-(1-méthyl-1*H*-pyrrole[2,3-b]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-carboxamide**
**Exemple 329. *N*-(4-hydroxyphényl)2-méthyl-*N*-(1-méthyl-1*H*-pyrrolo[2,3-*b*]pyridin-5-yl-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 330. *N*-(4-hydroxyphényl)-1-méthyl-*N*-(1-méthyl-1*H*-pyrrol-4-yl)5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 331. *N*-(4-hydroxyphényl)2-méthyl-*N*-(1-méthyl-1*H*-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 332. *N*-(4-hydroxyphényl)2-méthyl-*N*-(1-méthyl-2,3-dihydro-1*H-*pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 333. 1-éthyl-*N*-(4-hydroxyphényl)-2-méthyl-*N*-(1-méthyl-1*H*-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 334. 1-éthyl-*N*-(4-hydroxyphényl)-2-méthyl-*N*-(1-méthyl-2,3-dihdyro-1*H-*pyrrolo-[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 335. *N*-(4-hydroxyphényl)-1-(2-méthoxyéthyl)-2-méthyl-*N*-(1-méthyl-1*H-*pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 336. *N*-(4-hydroxyphényl)-2-méthyl-*N*-(1-méthyl-1*H*-pyrrolo[2,3-dihydro-1*H*-pyrrolo[2,3-*b*]pyridin-5-yl-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 337. 1-(2-fluoroéthyl)-*N*-(4-hydroxyphényl)-2-méthyl-*N*-(1-méthyl-1*H-*pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 338. 1-(2-fluoroéthyl)-*N*-(4-hydroxyphényl)-2-méthyl-*N*-(1-méthyl-2,3-dihydro-1H-pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1H-pyrrole-3-carboxamide**
**Exemple 339. 1-(2,2-difluoroéthyl)-*N*-(4-hydroxyphényl)-2-méthyl-*N*-(1-méthyl-1*H-*pyrrolo[2,3-*b*]pyridin-5-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple** 340. **1-(2,2-difluoroéthyl)-*N*-(4-hydroxyphényl)-2-méthyl-*N*-(1-méthyl-2,3-dihydro-1*H*-pyrrolo[2,3-*b*]pyridin-5-yl)-5-(6-{[(3*S*)-3-morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 341. *N*-(4-hydroxyphényl)-2-méthyl-*N*-(1-méthyl-1*H*-pyrazol-4-yl)-5-(6-{[(3*S*)-3-morpholin-4-ylméthyl)-3,4-dihydroisoquinolin-2(1*H*)-yl]carbonyl}-1,3-benzodioxol-5-yl)-1-(2,2,2-trifluoroéthyl)-1*H*-pyrrole-3-carboxamide**
**Exemple 342. *N*-(4-hydroxyphényl)-2-méthyl-*N*-(1-méthyl-1*H*-pyrazolo[2,3-b]pyridin-5-yl)-5-(6-{[(3*S*)-3-morpholin-4-ylméthyl)-3,4-dihydroisoquinolin-2(1*H*)-yl]carbonyl}-1,3-benzodioxol-5-yl)-1-(2,2,2-trifluoroéthyl)-1*H*-pyrrole-3-carboxamide**
**Exemple 343. *N*-(4-hydroxyphényl)-2-méthyl-*N*-(1-méthyl-2,3-dihydro-1*H-*pyrrolo[2,3-*b*]pyridin-5-yl)-(6-{[(3*S*)-3-morpholin-4-ylméthyl)-3,4-dihydroisoquinolin-2(1*H*)-yl]carbonyl}-1,3-benzodioxol-5-yl)-1-(2,2,2-trifluoroéthyl)-1*H*-pyrrolo-3-carboxamide**
**Exemple 344. *N*-(4-hydroxyphényl)-2-méthyl-5-(6-{[(3R*)*-3-méthyl-3,4-dihydroisoquinolin-2(1*H*)-yl]carbonyl}-1,3-benzodioxol-5-yl)-N-(1-méthyl-1*H-*pyrrolo[2,3-*b*]pyridin-5-yl)-1-[2-(morpholin-4-yl)éthyl]-1*H*-pyrrole-3-carboxamide**
**Exemple** 345. ***N*-(4-hydroxyphényl)-2-méthyl-5-(6-{[(3R*)*-3-méthyl-3,4-dihydroisoquinolin-2(1*H*)-yl]carbonyl}-1,3-benzodioxol-5-yl)-*N*-(1-méthyl-2,3-dihydro-1*H*-pyrrolo[2,3-*b*]pyridin-5-yl)-1-[2-(morpholin-4-yl)éthyl]-1*H*-pyrrole-3-carboxamide**
**Exemple 346. 1-[2-(diméthylamino)éthyl]-*N*-(4-hydroxyphényl)-2-méthyl-5-(6-{[(3*R*)-3-méthyl-3,4-dihydroisoquinolin-2(1*H*)-yl]carbonyl}-1,3-benzodioxol-5-yl)-*N*-(1-méthyl-1*H*-pyrazol-4-yl)-1*H*-pyrrole-3-carboxamide**
**Exemple 347. 1-[2-(diméthylamino)éthyl]-*N*-(4-hydroxyphényl)-2-méthyl-5-(6-{[(3*R*)-3-méthyl-3,4-dihydroisoquinolin-2(1*H*)-yl]carbonyl}-1,3-benzodioxol-5-yl)-*N*-(1-méthyl-1*H*-pyrazolo[2,3-b]pyridin-5-yl)-1*H*-pyrrole-3-carboxamide**
**Exemple 348. 1-[2-(diméthylamino)éthyl]-*N*-(4-hydroxyphényl)-2-méthyl-5-(6-{[(3*R*)-3-méthyl-3,4-dihydroisoquinolin-2(1*H*)-yl]carbonyl}-1,3-benzodioxol-5-yl)-*N*-(1-méthyl-2,3-dihydro-1*H*-pyrrolo[2,3-*b*]pyridin-5-yl)-1*H*-pyrrole-3-carboxamide**
**Exemple 349. *N*-(4-hydroxyphényl)-1-{2-[(2-méthoxyéthyl)(méthyl)amino]éthyl}-2-méthyl-5-(6-{[(3*R*)-3-méthyl-3,4-dihydroisoquinolin-2(1*H*)-yl]carbonyl}-1,3-benzodioxol-5-yl)-*N*-(1-méthyl-1*H*-pyrrolo[2,3-*b*]pyridin-5-yl)-1*H*-pyrrole-3-carboxamide**
**Exemple 350. *N*-(4-hydroxyphényl)-1-{2-[(2-méthoxyéthyl)(méthyl)amino]éthyl}-2-méthyl-5-(6-{[(3*R*)-3-méthyl-3,4-dihydroisoquinolin-2(1*H*)-yl]carbonyl}-1,3-benzodioxol-5-yl)-*N*-(1-méthyl-2,3-dihydro-1*H*-pyrrolo[2,3-*b*]pyridin-5-yl)-1*H-*pyrrole-3-carboxamide**
**Exemple** 351. ***N*-(4-hydroxyphényl)-1-méthyl-*N*-(1-méthyl-2,3-dihydro-1*H-*pyrrolo[2,3-*b*]pyridin-5-yl)-5-(6-{[(3*S*)-3-morpholin-4-ylméthyl)3,4-dihydroisoquinolin-2(1*H*)-yl]carbonyl}-1,3-benzodioxol-5-yl)-*N*-(trifluorométhyl)-1*H-*pyrrole-3-carboxamide**
**Exemple 352. 2-(difluorométhyl)-*N*-(-4-hydroxyphényl)-1-méthyl-*N*-(1-méthyl-2,3-dihydro-1*H*-pyrrolo[2,3-*b*]pyridin-5-yl)-5-(6-{[(3*S*)-3-morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 353. *N*-(4-hydroxyphényl)-2-(méthoxyméthyl)-1-méthyl-*N*-1(1-méthyl-2,3-dihydro-1*H*-pyrrolo[2,3-*b*]pyridin-5-yl)-5-(6-{[(3*S*)-3-morpholin-4-ylméthyl)-3,4-dihydroisoquinolin-2(1*H*)-yl]carbonyl}-1,3-benzodioxol-5-yl)-1*H*-pyrrole-3-carboxamide**
**Exemple 354. *N*-(4-hydroxyphényl)-1-méthyl-*N*-(1-méthyl-1*H*-pyrazol-4-yl)-5-(6-{[(3*S*)-3-morpholin-4-ylméthyl)-3,4-dihydroisoquinolin-2(1*H*)-yl]carbonyl}-1,3-benzodioxol-5-yl)-2-(2,2,2-trifluoroéthyl)-1*H*-pyrrole-3-carboxamide**
**Exemple 355. *N*-(4-hydroxyphényl)-1-méthyl-*N*-(1-méthyl-1*H*-pyrrolo[2,3-*b*]pyridin-5-yl)-5-(6-{[(3*S*)-3-morpholin-4-ylméthyl)-3,4-dihydroisoquinolin-2(1*H*)-yl]carbonyl}-1,3-benzodioxol-5-yl)-2-(2,2,2-trifluoroéthyl)-1H-pyrrole-3-carboxamide Exemple 356. *N*-(4-hydroxyphényl)-1-méthyl-*N*-(1-méthyl-2,3-dihydro-1*H-*pyrrolo[2,3-*b*]pyridin-5-yl)-5(6-{[(3*S*)-3-morpholin-4-ylméthyl)-3,4-dihydroisoquinolin-2(1*H*)yl]carbonyl}-1,3-benzodioxol-5-yl)2-(2,2,2-trifluoroéthyl)-1*H*-pyrrole-3-carboxamide**
**Exemple 357. 2-(2,2-difluoroéthyl)-*N*-(4-hydroxyphényl)-1-méthyl-*N*-(1-méthyl-2,3-dihydro-1*H*-pyrrolo[2,3-*b*]-pyridin-5-yl)-5-(6-{[(3*S*)-3-(morpholin-4-ylméthyl)-3,4-dihydroisoquinolin-2(1*H)*-yl]carbonyl}-1,3-benzodioxol-5-yl)-1H-pyrrole-3-carboxamide**
**Exemple 358. Chlorhydrate de *N*-(4-hydroxyphényl)-2-(2-méthoxyéthyl)-1-méthyl-*N-*(1-méthyl-2,3-dihydro-1*H*-pyrrolo[2,3-*b*]pyridin-5-yl-5-(6-{[(3*S*)-3-(morpholin-4-ylméthyl)-3,4-dihydroisoquinolin-2(1*H*)-yl]carbonyl}-1,3-benzodioxol-5-yl-1*H-*pyrrole-3-carboxamide**
   Le composé du titre est obtenu selon le procédé décrit au Stade B de l'Exemple 49 en utilisant l'Exemple 88 comme matière première, étant entendu que le produit est finalement solubilisé dans l'acétonitrile et salifié à l'aide d'une solution aqueuse de HCl 0,1 M. Après une étape de lyophilisation, on obtient le produit attendu sous la forme d'un solide.
   ***Microanalyse élémentaire :* (% *théorique : mesuré)***
   %C=65.80:64.71; %H=6.01:5.74; %N=10.23:10.03; %Cl-=4.32:6.47
   ***Masse haute résolution* (ESI/+) :**
   Formule brute : C₄₅H₄₈N₆O₇
   [M+H]⁺calculé : 785.3657
   [M+H]⁺ mesuré : 785.3658
**Exemple 359. *N***-**(4**-**hydroxyphényl)1**-**méthyl-5-(6**-**{[(3*R*)**-**3**-**méthyl**-**3,4-dihydroisoquinolin-2(1*H*)-yl]carbonyl}-1,3-benzodioxol-5-yl)-*N*-(1-méthyl-2,3-dihydro-1*H*-pyrrolo[2,3-*b*]pyridin-5-yl)-2-(morpholin-4-ylméthyl)-1*H*-pyrrole-3-carboxamide**
**Exemple 360. *N-*(4-hydroxyphényl)-1-méthyl-5-(6-{[(3*R*)-3-méthyl-3,4-dihydroisoquinolin**-**2(1*H*)-yl**]**carbonyl}-1,3-benzodioxol-5-yl)-*N*-(1-méthyl-2,3-dihydro-1*H*-pyrrolo[2,3-*b*]pyridin-5-yl)-2-[2-(morpholin-4-yl)éthyl]-1*H*-pyrrole-3-carboxamide,**
**Exemple 361. *N*-(4-hydroxyphényl)-1,2-diméthyl-*N*-(1-méthyl-1*H*-pyrazol-4-yl)-5-(6-{[(3*S*)-3-(trifluorométhyl)-3,4-dihydroisoquinolin-2(1*H*)-yl]carbonyl}-1,3-benzodioxol-5-yl)**-**1*H*-pyrrole-3-carboxamide**
**Exemple 362. *N*-(4-hydroxyphényl)-1,2-diméthyl-*N*-(1-méthyl-1*H*-pyrrolo[2,3-*b*]pyridin-5-yl)-5-(6-{[(3*S*)-3-(trifluorométhyl)-3,4-dihydroisoquinolin-2-(1*H*)-yl]carbonyl}-1,3-benzodioxal-5-yl)-1*H*-pyrrole-3-carboxamide**
**Exemple 363. *N*-(4-hydroxyphényl)-1,2-diméthyl-*N*-(1-méthyl-2,3-dihydro-1*H-*pyrrolo[2,3-*b*]pyridin-5-yl)-5-(6{[(3*S*)-3-(trifluorométhyl)-3,4-dihydroisoquinolin-2(1*H*)-yl]carbonyl}-1,3-benzodioxol-5-yl)-1*H*-pyrroloe-3-carboxamide**
**Exemple 364. 5-(6-{[(3*S*)-3(difluorméthyl)-3,4-dihydroisoquinolin-2(1*H*)-yl]carbonyl}-1,3-benzodioxol-5-yl)-*N***-**(4-hydroxyphényl)-1,2-diméthyl-*N*-(1-méthyl-1*H*-pyrazol-4-yl)-1*H*-pyrrole-3-carboxamide**
**Exemple 365**. **5-(6-{[(3*S*)-3-(difluorométhyl)-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 366. 5-(6-{[(3*S*)-3-(difluorométhyl)-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 367. 5-(6-{[(3*S*)-3-(fluorométhyl)-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-1*H*-pyrazol-4-*yl)-1H-pyrrole-3-carboxamide***
**Exemple 368. 5-(6-{[(3*S*)-3-(fluorométhyl)-3,4-dihydroisoquinolin-2(1*H*)-yl]earbonyl}-1,3-benzodioxol-5-y)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 369. 5-(6-{[(3*S*)-3-(fluorométhyl)-3,4-dihydroisoquinolin-2(1*H*)-yl]earbonyl}-1,3-benzodioxol-5-y)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 370. 5-(6-{[(3*S*)-3-[(9a*S*)-hexahydrophyrazino[2,1-*c*][1,4]oxazin-8(1*H*)-ylméthyl]-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-1*H*-pyrazol-4-yl)-1*H*-pyrrole-3-carboxamide**
**Exemple 371. 5-(6-{[(3*S*)-3-[(9a*S*)-hexahydropyrazino[2,1,*c*][1,4]oxazin-8(1*H*)-ylméthyl]-3,4-dihydroisoquinolin-2(1*H*)-yl]carbonyl}-1,3-benzodioxol-5-yl)-*N*-(4-hydroxyphényl)-1,2-diméthyl-*N-*(1-méthyl-1*H*-pyrrolo[2,3-*b*]pyridin-5-yl)-1*H-*pyrrole-3-carboxamide**
**Exemple 372. 5-(6-{[(3*S*)-3-[(9a*S*)-hexahydropyrazino[2,1-*c*][1,4]oxazin-8(1*H*)-ylméthyl]-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole**-**3-carboxamide**
**Exemple 373. 5-(6{[(3*S*)-3-[(9a*R*)-hexahydropyrazino[2,1-*c*][1,4]oxazin-8(1*H*)-ylméthyl]-3,4**-**dihydroiso**q**uinolin-2(1*H*)-yl]carbonyl}-1,3-benzodioxol-5-yl)-*N*-(4-hydroxyphényl)-1,2-diméthyl-*N*-(1-méthyl-1*H*-pyrazol-4-yl)-1*H*-pyrrole-3-carboxamide**
**Exemple 374. 5-(6{[(3*S*)-3-[(9a*R*)-hexahydropyrazino[2,1-*c*][1,4]oxazin-8(1*H*)-ylméthyl]-3,4**-**dihydroiso**q**uinolin-2(1*H*)-yl]carbonyl}-1,3-benzodioxol-5-yl)-*N*-(4-hydroxyphényl)-1,2-diméthyl-*N*-(1-méthyl-1*H*-pyrrolo-[2,3-*b*]pyridin-5-yl)-1*H-*pyrrole-3-carboxamide**
**Exemple 375. 5-(6-{[(3*S*)-3-[(9a*R*)-hexahydropyrazino[2,1-*c*][1,4]oxazin-8(1*H*)-ylméthyl]-3,4**-**dihydroiso**q**uinolin-2(1*H*)-yl]carbonyl}-1,3-benzodioxol-5-yl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 376. 5-(6-{[(3*S*)-3-(aminométhyl)-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-1*H*-pyrazol-4-yl)-1*H*-pyrrole-3-carboxamide**
**Exemple 377. 5-(6-{[(3*S*)-3-(aminométhyl)-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
**Exemple 378. 5-(6-{[(3*S*)-3-(aminométhyl)-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 379. 5-(6-{[(3*S*)-3-[(3-cyanoazetidin-1-yl)méthyl]-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-1*H*-pyrazol-4-yl)-1*H*-pyrrole-3-carboxamide**
**Exemple 380. 5-(6-{[(3*S*)-3-[(3-cyanoazetidin-1-yl)méthyl]-3,4-dihydroisoquinolin-2(1*H*)-yl]carbonyl}-1,3-benzodioxol-5-yl)-*N*-(4hydroxyphényl)-1,2-diméthyl-*N*-(1-méthyl-1*H*-pyrrolo[2,3-*b*]pyridin-5-yl)-1*H*-pyrrole-3-carboxamide**
**Exemple 381. 5-(6-{[(3*S*)-3-[(3-cyanoazetidin-1-yl)méthyl]-3,4-dihydroisoquinolin-2(1*H*)-yl]carbonyl}-1,3-benzodioxol-5-yl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide**
**Exemple 382. *N*-(4-hydroxyphényl)-1,2-diméthyl-*N*-(méthyl-1*H*-pyrazol-4-yl)-5-(6-{[(3*S*)-3-(1-oxa-6-azaspiro[3.3]hept-6-ylméthyl)-3,4-dihydroisoquinolin-2(1*H*)-yl]carbonyl}-1,3-benzodioxol-5-yl)-1*H*-pyrrole-3-carboxamide**
**Exemple 383. *N*-(4-hydroxyphényl)-1,2-diméthyl-*N*-(1-méthyl-1*H*-pyrrolo[2,3-*b*]pyridin-5-yl)-5-(6-{[(3*S*)-3-(1-oxa-6-azaspiro[2.3]hept-6-ylméthyl)-3,4-dihydroisoquinolin-2(1*H*)-carbonyl}-1,3-benzodioxol-5-yl)-1*H*-pyrrole-3-carboxamide**
**Exemple 384. Chlorhydrate de 5-(5-fluoro-2-{[(3*S*)-3(morpholin-4-ylméthyl)3,4-dihydroisoquinolin-2(1*H*)-yl]carbonyl}phényl)-*N*-(4-hydroxyphényl)-1,2-diméthyl-*N-*(pyridin-4-yl)-1*H***-**pyrrole-3-carboxamide**
   ***Microanalyse élémentaire : (% théorique : mesuré)***
   %C=67.28:67.73; %H=5.65:5.30; %N=10.06:9.41 %Cl-=5.09:5.79
**Exemple 385. Chlorhydrate de 5-(5-fluoro-2-{[(3*S*)-3-morpholin-4-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrrolo[2,3-*b*]pyridin-5-yl)-1*H*-pyrrole-3-carboxamide**
   ***Masse haute résolution (ESI*+*) :***
   Formule brute : C₄₁ H₄₁ Cl N₆ O₄
   [M+H]⁺calculé : 713.3253
   [M+H]⁺ mesuré : 713.3272
**Exemple 386. Chlorhydrate de 5**-**(5-chloro-2-{[(3*S*)-3-morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide**
   ***Stade A : N-[4-[tert-Butyl(dimethyl)silyl]oxyphenyl]-5-[5-chloro-2-[(3S)-3-(morpholinomethyl)-3,4-dihydro-1H-isoquinoline-2-carbonyl]phenyl]-N-(5-cyano-1,2-dimethyl-pyrrol-3-yl)-1,2-dimethyl-pyrrole-3-carboxamide***
   Le composé du titre est obtenu selon le procédé de l'Exemple 1 en utilisant l'acide de la Préparation 1 et le composé de la Préparation 3' au Stade A, ainsi que le composé de la Préparation 18" au Stade C.
   **IR** : v -CN- : 2210 cm⁻¹ ; v -C=O-: 1631 cm⁻¹
   ***Stade B : Chlorhydrate de 5-(5-chloro-2-{[(3S)-3-(morpholin-4-ylméthyl)-3,4-dihydroisoquinolin*-*2(1H)*-*yl]carbonyl}phényl)*-*N*-*(5*-*cyano*-*1*,*2*-*diméthyl*-*1H*-*pyrrol*-*3*-*yl)-N*-(*4*-*hydroxyphényl*)-*1*,*2*-*diméthyl*-*1H*-*pyrrole*-*3*-*carboxamide***
   Le composé du Stade A est déprotégé selon le protocole décrit au Stade D de l'Exemple 1. Le produit ainsi obtenu est enfin soumis à une étape de salification en présence d'éther chlorhydrique dans l'éther. Après filtration et lyophilisation dans un mélange acétonitrile/eau, on obtient le produit attendu.
   **RMN ¹H** (500 MHz, dmso-d6) δ ppm: 11.2 (sl, 1H), 9.39 (sl,1H), 7.83 (d, 1 H), 7.54 (d, 1 H), 7.33 (s, 1 H), 7.14 (m, 2 H), 7 (m, 2 H), 6.8 (d, 2 H), 6.62 (d, 2 H), 6.57 (sl, 1 H), 5.26 (s, 1 H), 5.26 (m, 1 H), 4.64/4.03 (AB, 2 H), 4.01/3.92 (2m, 4 H), 3.75/3.43/3.15/3.02 (4m, 4 H), 3.59 (s, 3 H), 3.3/3.15 (2m, 2 H), 2.97 (s, 3 H), 2.69/2.52 (dd+d, 2 H), 2.06 (s, 3 H), 1.91 (s, 3 H)
   ***Microanalyse élémentaire : (% théorique : mesuré)***
   %C=65.34:65.50; %H=5.62:5.15; %N=11.15:10.84 %Cl-=4.70:4.44
   ***Masse haute résolution (ESI*+*) :***
   Formule brute : C₄₁ H₄₁ Cl N₆ O₄
   [M+H]⁺calculé : 717.2952
   [M+H]⁺ mesuré : 717.2951
**Exemple 387. Chlorhydrate de *N*-(4-hydroxyphényl)-1,2-diméthyl-*N*-(1-méthyl-1*H-*pyrrolo[2,3-*c*]pyridin-5-yl)-5-(6-{[(3*S*)-3-(morpholin-4-ylméthyl)-3,4-dihydroisoquinolin-2-(1*H*)-yl]carbonyl}-1,3-benzodioxol-5-yl)-1*H*-pyrrole-3-carboxamide,**
   ***Microanalyse élémentaire : (% théorique : mesuré)***
   %C=66.62:66.36; %H=5.59:5.62; %N=10.84:10.72 %Cl-=4.57:4.55
   ***Masse haute résolution (ESI*+*) :***
   Formule brute : C₄₃ H₄₂ N₆ O₆
   [M+H]⁺calculé : 739.3239
   [M+H]⁺ mesuré : 739.3241
**Exemple 388. Dichlorhydrate de 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*(1-méthyl-2,3-dihydro-1*H*-pyrrolo[2,3-*b*]pyridin-5-yl)-1*H*-pyrrole-3-carboxamide**
   Le composé du titre est obtenu selon le procédé décrit au Stade B de l'Exemple 49 en utilisant l'Exemple 71 comme matière première, étant entendu que le produit est finalement soumis à une étape de salification en présence d'éther chlorhydrique 1M dans l'éther. Après filtration et lyophilisation dans un mélange acétonitrile/eau, on obtient le composé attendu.
   ***Microanalyse élémentaire : (% théorique : mesuré)***
   %C=62.73:62.96; %H=5.64:4.95; %N=10.45:10.32; %Cl=13.23:12.91
   ***Masse haute résolution (ESI*+*) :***
   Formule brute : C₄₂ H₄₃ Cl N₆ O₄
   [M+H]⁺calculé : 731.3107
   [M+H]⁺ mesuré : 731.3111
**Exemple 389. Chlorhydrate de 5-(5-chloro-2-{[(3*S*)-3-morpholin-4-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrrolo[2,3-*c*]pyridin-5-yl)-1*H*-pyrrole-3-carboxamide**
   On procède selon le protocole général de l'Exemple 1 en utilisant les préparations adéquates. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.
   ***Microanalyse élémentaire : (% théorique : mesuré)***
   %C=65.88:66.28; %H=5.53:5.15; %N=10.98:10.95; %Cl-=4.63:4.47
   ***Masse haute résolution (*ESI/FIA/HR et MS/MS*) :***
   Formule brute : C₄₂ H₄₁ Cl N₅ O₄
   [M+H]⁺calculé : 729.2951
   [M+H]⁺ mesuré : 729.2954
**Exemple 390. 5-(5-Chloro-2-{[(3*S*)-3-(morpholin-4-ylméthyl)-3,4-dihydroisoquinolin-2(1*H*)-yl]carbonyl}phényl)-1,2-diméthyl-*N*-(1-méthyl-1*H*-pyrrolo[2,3-*b*]pyridin-5-yl)-*N*-phényl-1*H*-pyrrole-3-carboxamide**
   On procède selon le protocole général de l'Exemple 1 en utilisant les préparations adéquates, étant entendu que le Stade D n'est pas effectué. Le produit attendu est obtenu sous la forme d'une base libre.
   ***Microanalyse élémentaire : (% théorique : mesuré)***
   %C=70.72:69.77; %H=5.79:5.96; %N=11.78:11.43
   ***Masse haute résolution (ESI+) :***
   Formule brute : C₄₂ H₄₁ Cl N₆ O₃
   [M+H]⁺calculé : 713.3001
   [M+H]⁺ mesuré : 713.2998
**Exemple 391. Chlorhydrate de 5-(5-chloro-2**-**{[(3*S*)-3-morpholin-4-ylméthyl)-3,4-dihydroisoquinolin-2(1*H*)-yl]carbonyl}-*N*-(4-hydroxyphényl)-1,2-diméthyl-*N-*(1-méthyl-1*H*-pyrrolo[3,2-b]pyridin-5-yl)-1*H*-pyrrole-3-carboxamide**
   *Microanalyse **élémentaire (%** théorique : mesuré)*
   %C=65.88:65.69; %H=5.33:4.87; %N=10.98:10.86; %Cl-=4.63:4.51
   ***Masse haute résolution (*ESI+-/FIA*) :***
   Formule brute : C₄₂ H₄₁ Cl N₆ O₄
   [M+H]⁺calculé : 729.2951
   [M+H]⁺ mesuré : 729.2953
**Exemple 392. Chlorhydrate de 5**-**(5-chloro-2-{[(3*R*)-3-[3-(morpholin-4-yl)propyl]-3,4-dihydroisoquinolïn-2(1*H*)-yl]carbonyl}phényl)-*N*-(4-hydroxyphényl)1,2-diméthyl-*N-*phényl-1*H*-pyrrole-3-carboxamide**
   ***Microanalyse élémentaire : (% théorique : mesuré)***
   %C=68.19:68.33; %H=6.00:5.49; %N=10.78:10.71; %Cl-=4.55:4.46 ; %Cl = 9.58 :9.78
   ***Masse haute résolution (*ESI+-/FIA*) :***
   Formule brute : C₄₂ H₄₃ Cl N₄ O₄
   [M+H]⁺calculé : 703.3046
   [M+H]⁺ mesuré : 703.3042
**Exemple 393. Chlorhydrate de 5-(5-chloro-2-{[(3*R*)-3-[3-(morpholin-4-yl)propyl]-3,4-dihydroisoquinolin-2(1*H*)-yl]carbonyl}phényl)-*N*-(4-hydroxyphényl)-1,2-diméthyl-*N-*(1-méthyl-1*H*-pyrrolo[2,3-*b*]pyridin-5-yl)-1*H*-pyrrole-3-carboxaroide**
   ***Masse haute résolution (*ESI+-/FIA*) :***
   Formule brute : C₄₄ H₄₅ Cl N₆ O₄
   [M+H]⁺calculé : 757.3264
   [M+H]⁺ mesuré : 757.3263
**Exemple 394. 5-(5-Chloro-2-{[(3*R*)-3-[3-(morpholin-4-yl)propyl]-3,4-dihydroisoquinolin-2(1*H*)-yl]carbonyl}phényl)-*N*-(4-hydroxyphényl)-1,2-diméthyl-*N-*(1-méthyl-2,3-dihydro-1*H*-pyrrolo[2,3-*b*]pyridin-5-yl)-1*H*-pyrrole-3-carboxamide**
   Le composé du titre est obtenu selon le procédé décrit au Stade B de l'Exemple 49 en utilisant l'Exemple 393 comme matière première, étant entendu que le produit n'est pas soumis à l'étape de salification.
   ***Microanalyse élémentaire : (% théorique : mesuré)***
   %C=69.60:69.56; %H=6.21:6.24; %N=11.07:11.08
   ***Masse haute résolution** (***ESI**+-/**FIA***) :*
   Formule brute : C₄₄ H₄₇ Cl N₆ O₄
   [M+H]⁺calculé : 759.3420
   [M+H]⁺ mesuré : 759.3422
**Exemple 395. Chlohydrate de 5-(5-chloro-2-{[(3*S*)-3-(morpholin-4-ylméthyl)-3,4-dihydroisoquinolin-2(1*H*)-yl]carbonyl}phényl)-*N*-(3-cyano-4-méthoxyphényl)-*N*-(4-hydroxyphény)**-**1**,**2**-**diméthyl-1*H*-pyrrole-3-carboxamide**
   On procède selon le protocole général de l'Exemple 1 en utilisant les préparations adéquates. Le produit obtenu est enfin solubilisé dans l'acétonitrile et salifié à l'aide d'une solution aqueuse de HCl 0,1 M. Après une étape de lyophilisation, on obtient le composé attendu sous la forme d'un solide.
   ***Masse haute résolution (*ESI/FIA/HR et MS/MS*) :***
   Formule brute : C₄₂ H₄₀ Cl N₅ O₅
   [M+H]⁺calculé : 730.2791
   [M+H]⁺ mesuré : 730.2790
**Exemple 396. Clorhydrate de *N*-(3-fluoro-4-hydroxyphényl)-1,2-diméthyl-*N*-(1-méthyl-1*H*-pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide**
   Le composé du titre est obtenu selon le procédé de l'Exemple 1 en utilisant l'acide de la Préparation 11 et la (3*S*)-3-(4-morpholinylméthyl)-1,2,3,4-tétrahydroisoquinoline (voir Préparation 3') au Stade A, ainsi que le composé de la Préparation 20" au Stade C. Le produit obtenu est enfin soumis à une étape de salification en présence d'éther chlorhydrique 1M dans l'éther. Après filtration et lyophilisation dans un mélange acétonitrile/eau, on obtient le composé attendu.
   ***Microanalyse élémentaire : (% théorique : mesuré)***
   %C=65.79:65.43; %H=5.39:5.19; %N=11.31:11.21 ; %Cl-=4.77 :4.34
   ***Masse haute résolution (*ESI/FIA/HR et MS/MS*) :***
   Formule brute : C₃₉ H₃₉ F N₆ O₆
   [M+H]⁺calculé : 707.2988
   [M+H]⁺ mesuré : 707.2988
**Exemple 397. 5-(5-Chloro-2-{[(3*S*)-3-(morpholin-4-ylméthyl)-3,4-dihydroisoquinolin-2(1*H*)-yl]carbonyl}phényl)-*N*-(4-hydroxyphényl)-*N*-(2-méthoxypyrimidin-5-yl)-1,2-diméthyl-1*H*-pyrrole-3-carboxamide**
   ***Masse haute résolution (*ESI+) :**
   Formule brute : C₃₉ H₃₉ Cl N₆ O₅
   [M+H]⁺calculé : 707.2743
   [M+H]⁺ mesuré : 707.2746
**Exemple 398. Chlorhydrate de 5-(5-chloro-2-{[(3*S*)-3-(morpholin-4-ylméthy)-3,4-dihydroisoquinolin-2(1*H*)-yl]carbonyl}phényl)-*N*-(3-cyanophényl)-*N*-(4-hydroxyphényl)-1,2-diméthyl-1*H*-pyrrole-3-carboxamide**
   **Microanalyse élémentaire : *(% théorique : mesuré)***
   %C=66.85:66.75; %H=5.34:5.42; %N=9.51:9.73; %Cl=9.62:9.67 ; %Cl-=4.81:4.71
   ***Masse haute résolution (ESI+-*/*FIA*/*HR, ESI-*/*FIA) :***
   Formule brute : C₄₁ H₃₈ Cl N₅ O₄
   [M+H]⁺calculé : 700.2685
   [M+H]⁺ mesuré : 700.2686
**Exemple 399. Chlorhydrate de 5-(5-chloro-2-{[(3*S*)-3-(morpholin-4-ylméthyl)-3,4-dihydroisoquinolin**-**2(1*H*)-yl]carbonyl}phényl)-*N*-(3-fluoro-4-hydroxyphényl)1,2-diméthyl-*N*-(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 1 et la (3*S*)-3-(4-morpholinylméthyl)-1,2,3,4-tétrahydroisoquinoline (voir Préparation 3') au Stade A, ainsi que le composé de la Préparation 20" au Stade C. Le produit obtenu est enfin soumis à une étape de salification en présence d'éther chlorhydrique 1M dans l'éther. Après filtration et lyophilisation dans un mélange acétonitrile/eau, on obtient le composé attendu.
   ***Microanalyse élémentaire : (% théorique : mesuré)***
   %C=62.21:61.32; %H=5.36:5.18; %N=11.46_{:}11.14; %Cl= 9.66:10.16; %Cl⁻=4.83:5.23
   ***Masse haute résolution (ESI*+-/*FIA*/*HR, ESI-lFIA) :***
   Formule brute : C₃₈ H₃₈ Cl F N₅ O₄
   [M+H]⁺ calculé : 697.2700
   [M+H]⁺ mesuré : 697.2704
**Exemple 400. Chlorhydrate de 2-[{[5-(5-chloro-2-{[(3*S*)-3-(morpholin-4-ylméthyl)-3,4-dihydroisoquinolin-2(1*H*)-yl]carbonyl}phényl)-1,2-diméthyl-1*H*-pyrrol-3-yl]carbonyl}(4-hydroxyphényl)amino]pyridine-4-carboxylate de méthyle**
   Le composé du titre est un produit secondaire qui se forme au cours de la synthèse de l'Exemple 110 (au dernier stade avant l'étape de salification), du fait de l'hydrolyse de la fonction nitrile en ester méthylique. Le composé est séparé de celui de l'Exemple 110 par chromatographie sur gel de silice dans un mélange de méthanol et de dichlorométhane.
   ***Microanalyse élémentaire : (% théorique : mesuré)***
   %C=63.90:64.43; %H=5.36:5.01; %N=9.09:9.34; %Cl⁻=4.60:4.46
   ***Masse haute résolution (ESI*+/*HR et ESI-*/*LR) :***
   Formule brute : C₄₁ H₄₀ Cl N₅ O₆
   [M+H]⁺calculé : 734.2740
   [M+H]⁺ mesuré : 734.2743
**Exemple 401. Chlorhydrate de 5-(5-chloro-2-{[(3*S*)-3-(morpholin-4-ylméthyl)-3,4-dihydroisoquinolïn-2(1*H*)-yl]carbonyl}phényl)-*N*-(3-cyano-4-fluorophényl)-*N*-(4-hydroxyphényl)-1**,**2**-**diméthyl-1*H*-pyrrole-3-carboxamide**
   ***Microanalyse élémentaire : (% théorique mesuré)***
   %C=65.25:64.23; %H=5.07:4.71; %N=9.28:9.36; %Cl= 9.40:9.59; %Cl⁻=4.70:4.50
   ***Masse haute résolution (ESI*+-*I*/*FIA*/ *HR, ESI-lFIA) :***
   Formule brute : C₄₁ H₃₇ Cl F N₅ O₄
   [M+H]⁺ calculé : 718.2591
   [M+H]⁺ mesuré : 718.2593
**Exemple 402. Chlorhydrate de 5-(5-fluoro-2-{[(3S)-3-(morpholin-4-ylméthyl)-3,4-dihydroisoquinolin**-**2(1*H***)-**yl]carbonyl}phényl)-*N*-(4-hydroxyphényl)1,2-diméthyl-*N-*{1-[(3*S* ou *R*)-tétrahydrofuran-3-yl]-1*H*-pyrazol-4-yl}-1*H*-pyrrole-3-carboxamide**
   ***Microanalyse élémentaire : (% théorique mesuré)***
   %C=65.20:65.92; %H=5.87:5.78; %N=11.13:10.36 %Cl⁻=4.69 :4.79)
   ***Masse haute résolution (ESI*+*) :***
   Formule brute : C₄₁H₄₃FN₆O₅
   [M+H]⁺calculé : 719.3359
   [M+H]⁺ mesuré : 719.3362
   **et**
**Exemple 403. Chlorhydrate de 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*{1-[(3*R* ou *S*)-tétrahydrofuran-3-yl]-1*H*-pyrazol-4-yl}-1*H*-pyrrole-3-carboxamide**
   ***Microanalyse élémentaire : (% théorique : mesuré)***
   %C=65.20:66.04; %H=5.87:5.87; %N=11.13:10.62 ; %Cl⁻=4.69:4.76
   ***Masse haute résolution (ESI*/+*) :***
   Formule brute : C₄₁H₄₃FN₆O₅
   [M+H]⁺ calculé : 719.3359
   [M+H]⁺ mesuré : 719.3350

   Les composés des Exemples 402 et 403 sont obtenus selon le procédé de l'Exemple 1 en utilisant l'acide de la Préparation 8 et le composé de la Préparation 3' au Stade A, ainsi que le composé de la Préparation 22" au Stade C. Les diastéréoisomères obtenus sont séparés par chromatographie chirale puis salifiés et lyophilisés tel que décrit dans le procédé général pour donner les composés du titre.
**Exemple 404. Chlorhydrate de 5-(5-chloro-2-{[(3*S*)-(morpholin-4-ylméthyl)3-4-dihydroisoquinolin-2(1*H*)-yl]-carbonyl}phényl)-*N*-(2-cyanopyrimidin-4-yl)-*N*-(4-hydroxyphényl**)-**1**,**2**-**diméthyl-1*H*-pyrrole**-**3**-**carboxamide**
**Exemple 405. Dichlorhydrate de 5-(5-chloro-2-{[(3*S*)-3-(morpholin-4-ylméthyl)-3,4-dihydroisoquinolin-2(1*H*)-yl]carbonyl}phényl)-1,2-diméthyl-*N*-(1-méthyl-1*H-*pyrrolo[2,3-*b*]pyridin-5-yl)-*N*-(pyridazin-4-yl)-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'aminé adéquate au Stade C, étant entendu que le Stade D n'est pas effectué. Le composé obtenu est salifié et lyophilisé tel que décrit dans le procédé général pour obtenir le composé du titre.
   ***Masse haute résolution (ESI*+-/*FIA*/*HR, ESI-*/*FIA) :***
   Formule brute : C₄₀ H₃₉ Cl N₈ O₃
   [M+H]⁺calculé : 715.2906
   [M+H]⁺ mesuré : 715.2909
**Exemple 406. Chlorhydrate de *N*-(5-cyano-1,2-diméthyl-1*H*-pyrrol-3-yl)-*N*-(4-hydroxyphényl)-1,2-diméthyl-5-(2-{[(3*S*)-3-(morpholin-4-ylméthyl)-3,4-dihydroisoquinolin-2(1*H*)**-**yl]-carbonyl}phényl)-1*H*-pyrrole-3-carboxamide**
   Le composé du titre est obtenu selon le procédé de l'Exemple 1 en utilisant l'acide de la Préparation 25 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=68.46:68.27; %H=6.03:5.12; %N=11.68:11.75; %Cl⁻=4.93:4.73
   ***Masse haute résolution (ESI*/*FIAlHR et MS*/*MS*, *ESI-*/*FIA) :***
   Formule brute : C₄₁ H₄₂ N₆ O₄
   [M+H]⁺ calculé : 683.3340
   [M+H]⁺ mesuré : 683.3334
**Exemple 407. Chlorhydrate de *N-*(3-fluoro-4-hydroxyphényl)-5-(5-méthoxy-2-{[(3*S*)-3-(morpholin-4-ylméthyl)-3,4-dihydroisoquinolin-2(1*H*)-yl]carbonyl}phényl)-1,2-diméthyl-*N*-(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 12 et le composé de la Préparation 3' au Stade A, ainsi que le composé de la Préparation 20" au Stade C. Le composé obtenu est salifié et lyophilisé tel que décrit dans le procédé général pour obtenir le composé du titre.
   ***Microanalyse élémentaire : (% théorique : mesuré)***
   %C=64.23:63.94; %H=5.80:5.00; %N=11.52:11.56; %Cl⁻=4.86:4.99
   ***Masse haute résolution (ESI*+-/*FIA*/*HR et MS*/*MS*, *ESI-*/*FIA) :***
   Formule brute : C₃₉ H₄₁ F N₆ O₅
   [M+H]⁺ calculé : 693.3195
   [M+H]⁺ mesuré : 693.3191
**Exemple 408. Chlorhydrate de *N-*(5-cyano-1,2-diméthyl-1*H*-pyrrol-3-yl)-*N*-(4-hydroxyphényl)-5-(5-méthoxy-2-{[(3*S*)-3-(morpholin-4-ylméthyl)-3,4-dihydroisoquinolin-2(1*H*)-yl]carbonyl}phényl)1,2-diméthyl-1*H*-pyrrole-3-carboxamide**
   Le composé du titre est obtenu selon le procédé de l'Exemple 1 en utilisant l'acide de la Préparation 12 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=67.32:67.30; %H=6.05:5.28; %N=11.22:11.15; %Cl⁻=4.73:4.59
   ***Masse haute résolution (ESI*+-/*FIA*/*HR et ESI-*/*FIA) :***
   Formule brute : C₄₂ H₄₄ N₆ O₅
   [M+H]⁺ calculé : 713.3446
   [M+H]⁺ mesuré : 713.3443
**Exemple 409. Chlorhydrate de *N-*(4-hydroxyphényl)**-**1,2-diméthyl-*N*-(1-méthyl-1*H-*pyrrolo[2,3-*b*]pyridin-5-yl)-5-(2-{[(3*S*)-3-(morpholin-4-ylméthyl)-3,4-dihydroisoqninolin-2(1*H*)-yl]carbonyl}phényl)-1*H*-pyrrole-3-carboxamide**
   Le composé du titre est obtenu selon le procédé de l'Exemple 1 en utilisant l'acide de la Préparation 25 et le composé de la Préparation 3' au Stade A, ainsi que le composé de la Préparation 11" au Stade C. Le composé obtenu est salifié et lyophilisé tel que décrit dans le procédé général pour obtenir le composé du titre.
   ***Microanalyse élémentaire : (% théorique : mesuré)***
   %C=68.98:68.95; %H=5.93:4.76; %N=11.49:11.43
   ***Masse haute résolution (ESI*/*FIA*/*HR et MS*/*MS) :***
   Formule brute : C₄₂ H₄₂ N₆ O₄
   [M+H]⁺calculé : 695.3340
   [M+H]⁺ mesuré : 695.3341
**Exemple 410. Chlorhydrate de *N-*(4-hydroxyphényl)-1,2-diméthyl-*N*-(1-méthyl-2,3-dihydro-1*H*-pyrrolo[2,3-*b*]pyridin-5-yl)-5-(2-{[(3*S*)-3-(morpholin-4-ylméthyl)-3,4-dihydroisoquinolin-2(1*H*)-yl]carbonyl}phényl)-1*H*-pyrrole-3-carboxamide**
   Le composé du titre est obtenu selon le procédé décrit au Stade B de l'Exemple 49 en utilisant l'Exemple 409 comme matière première. Le composé obtenu est salifié et lyophilisé tel que décrit dans le procédé général pour obtenir le composé du titre.
   ***Masse haute résolution (ESI*/*FIA*/*HR et ESI-*/*FIA) :***
   Formule brute : C₄₂ H₄₄ N₆ O₄
   [M+H]⁺calculé : 697.3497
   [M+H]⁺ mesuré : 697.3497
**Exemple 411. Chlorhydrate de 5-(5-chloro-2-{[(3*R*)-3-méthyl-3,4-dihydroisoquinolin-2(1*H*)-yl]carbonyl}phényl)-*N*-(4-hydroxyphényl)-2-méthyl-*N*-(1-méthyl-1*H*-pyrazol-4-yl)-1-[2-(morpholin-4-yl)éthyl]-1*H*-pyrrole-3-carboxamide**
   Le composé du titre est obtenu selon le procédé de l'Exemple 96 en utilisant l'acide de la Préparation 30, le composé de la Préparation 1', ainsi que le composé de la Préparation 1". Le composé obtenu est salifié et lyophilisé tel que décrit dans le procédé général pour obtenir le composé du titre.
   ***Masse haute résolution (ESIlFIAlHR et MS*/*MS) :***
   Formule brute : C₃₉ H₄₁ Cl N₆ O₄
   [M+H]⁺calculé : 693.2951
   [M+H]⁺ mesuré : 693.2947
**Exemple 412. Chlorhydrate de 5-(5-chloro-2-{[(3*R*)-3-[3-(morpholin-4-yl)propyl]-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 6' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=66.06:65.61; %H=5.93:5.22; %N=10.75:10.69; %Cl⁻=4.53:4.68
   ***Masse haute résolution (ESI*/*FIAlHR et MS*/*MS, ESI-*/*FIA) :***
   Formule brute : C₄₃ H₄₅ Cl N₆ O₄
   [M+H]⁺calculé : 745.3264
   [M+H]⁺ mesuré : 745.3260
**Exemple 413. Chlorhydrate de *N*-(5-cyano-1,2-diméthyl-1*H*-pyrrol-3-yl)-*N*-(4-hydroxyphényl)-1,2-dyméthyl-5-(7-{[(3*S*)-3-(morpholin-4-ylméthyl)-3,4-dihydroisoquinolin-2(1*H*)-yl]carbonyl}-2,3-dihydro-1,4-benzodioxin-6-yl)-1*H-*pyrrole-3-carboxamide**
   Le composé du titre est obtenu selon le procédé de l'Exemple 1 en utilisant l'acide de la Préparation 13 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=66.44:66.40; %H=5.83:4.84; %N=10.81:10.79; %Cl⁻=4.56:4.22
   ***Masse haute résolution (ESIlFIAlHR et MS*/*MS, ESI-*/*FIA) :***
   Formule brute : C₄₃ H₄₄ N₆ O₆
   [M+H]⁺calculé : 741.3395
   [M+H]⁺ mesuré : 741.3397
**Exemple 414. Chlorhydrate de 5**-**(5**-**chloro**-**2-{(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*(5-méthyl-5*H*-pyrrolo[3,2-*d*]pyrimidin-2-yl)-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'amine adéquate au Stade C. Le composé obtenu est salifié et lyophilisé tel que décrit dans le procédé général pour obtenir le composé du titre.
   ***Microanalyse élémentaire : (% théorique : mesuré)***
   %C=64.23:64.38; %H=5.39:5.25; %N=12.79:12.62; %Cl⁻=4.62:4.39
   ***Masse haute résolution (ESIlFIAlHR et MS*/*MS, ESI-lFIA) :***
   Formule brute : C₄₁ H₄₀ Cl N₇ O₄
   [M+H]⁺calculé : 730.2903
   [M+H]⁺ mesuré : 730.2904
**Exemple 415. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1-méthyl-1*H*-pyrrole-3-carboxamide**
   Le composé du titre est obtenu selon le procédé de l'Exemple 1 en utilisant l'acide de la Préparation 3 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=64.95:65.14; %H=5.45:5.34; %N=11.36:11.36; %Cl⁻=4.79:4.67
   ***Masse haute résolution (ESI*+-*lFIAlHR, ESI-lFIA) :***
   Formule brute : C₄₀ H₃₉ Cl N₆ O₄
   [M+H]⁺calculé : 703.2794
   [M+H]⁺ mesuré : 703.2795
**Exemple 416. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*[1-(trideutériométhyl)-1*H*-pyrazol-4-yl]-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 25" au Stade C. Le composé obtenu est salifié et lyophilisé tel que décrit dans le procédé général pour obtenir le composé du titre.
   ***Microanalyse élémentaire : (% théorique : mesuré)***
   %C=63.51:63.41; %H=5.63:5.42; %N=11.69:11.61; %Cl⁻=4.93:4.85
   ***Masse haute résolution (ESI*+-*lFIAlHR,ESI-lFIA) :***
   Formule brute : C₃₈ H₃₆ Cl D₃ N₆ O₄
   [M+H]⁺calculé : 682.2982
   [M+H]⁺ mesuré : 682.2986
**Exemple 417. Chlorhydrate de *N*-(5-cyano-1,2-diméthyl-1*H*-pyrrol-3-yl)-*N*-(4-hydroxyphényl)-5-(4-méthoxy-2-{[(3*S*)-3-(morpholin-4-ylméthyl)-3,4-dihydroisoquinolin-2(1*H*)-yl]carbonyl}phényl)-1,2-diméthyl-1*H*-pyrrole-3-carboxamide**
   Le composé du titre est obtenu selon le procédé de l'Exemple 1 en utilisant l'acide de la Préparation 31 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=67.32:67.56; %H=6.05:5.84; %N=11.22:11.21; %Cl⁻=4.73:4.71
   ***Masse haute résolution (ESIlFIA*/*HR et MS*/*MS, ESI-lFIA) :***
   Formule brute : C₄₂ H₄₄ N₆ O₅
   [M+H]⁺calculé : 713.3446
   [M+H]⁺ mesuré : 713.3446
**Exemple 418. Chlorhydrate de *N*-(4-hydroxyphényl)-5-(4-méthoxy-2-{[(3*S*)-3-(morpholin-4-ylméthyl)-3,4-dihydroisoquinolin-2(1*H*)-yl]carbonyl}phényl)-1,2-diméthyl-*N*-(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 31 et le composé de la Préparation 3' au Stade A, ainsi que le composé de la Préparation 1" au Stade C. Le composé obtenu est salifié et lyophilisé tel que décrit dans le procédé général pour obtenir le composé du titre.
   ***Microanalyse élémentaire : (% théorique : mesuré)***
   %C=65.86:65.51; %H=6.09:6.09; %N=11.82:11.73; %Cl⁻=4.98:5.14
   ***Masse haute résolution (ESI*/*FIA*/*HR, ESI-lFIA) :***
   Formule brute : C₃₉ H₄₂ N₆ O₅
   [M+H]⁺calculé : 675.3289
   [M+H]⁺ mesuré : 675.3286
**Exemple 419. Chlorhydrate de *N-*(3-cyanophényl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1**,**2**-**diméthyl**-**1*H***-**pyrrole**-**3**-**carboxamide**
   Le composé du titre est obtenu selon le procédé de l'Exemple 1 en utilisant l'acide de la Préparation 8 et le composé de la Préparation 3' au Stade A, ainsi que le composé de la Préparation 41" au Stade C. Le composé obtenu est salifié et lyophilisé tel que décrit dans le procédé général pour obtenir le composé du titre.
   ***Masse haute résolution (ESI*+*) :***
   Formule brute : C₄₁H₃₈FN₅O₄
   [M+H]⁺calculé : 684.2988
   [M+H]⁺ mesuré : 684.2975
**Exemple 420. Chlorhydrate de 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*[1-(trideutériométhyl)-1*H*-pyrazol-4-yl]-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que le composé de la Préparation 25" au Stade C. Le composé obtenu est salifié et lyophilisé tel que décrit dans le procédé général pour obtenir le composé du titre.
   ***Masse haute résolution (ESI*/+*) :***
   Formule brute : C₃₈D₃H₃₆FN₆O₄
   [M+H]⁺calculé : 666.3285
   [M+H]⁺ mesuré : 666.3265
**Exemple 421. Chlorhydrate de *N*-(5-cyano-1,2-diméthyl-1*H*-pyrrol-3-yl)-5-(5-fluoro-2-{[(3*S*)**-**3**-**(morpholin**-**4**-**ylméthyl)-3**,**4**-**dihydroisoquinolin**-**2(1*H*)-yl**]**carbonyl**}**phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Masse haute résolution (ESI*/*FIA*/*HR et MS*/*MS*) *:***
   Formule brute : C₄₁ H₄₁ F N₆ 04
   [M+H]⁺ calculé : 701.3246
   [M+H]⁺ mesuré : 701.3282
**Exemple 422. Chlorhydrate de *N*-(4-hydroxyphényl)-1,2-diméthyl-*N*-(1-méthyl-1*H-*pyrazol-4-yl)-5-(2-{[(3*S*)-3-(morpholin-4-ylméthyl)-3,4-dihydroisoquinolin-2(1*H*)-yl**]**carbonyl}phényl)-1*H*-pyrrole**-**3**-**carboxamide**
   Le composé du titre est obtenu selon le procédé de l'Exemple 1 en utilisant l'acide de la Préparation 25 et le composé de la Préparation 3' au Stade A, ainsi que le composé de la Préparation 1" au Stade C. Le composé obtenu est salifié et lyophilisé tel que décrit dans le procédé général pour obtenir le composé du titre.
   ***Microanalyse élémentaire : (% théorique : mesuré)***
   %C=67.00:67.47; %H=6.07:5.54; %N=12.34:12.46; %Cl⁻=5.20:4.58
   ***Masse haute résolution (ESI*+-/*FIA*/*HR, ESI-*/*FIA) :***
   Formule brute : C₃₈ H₄₀ N₆ O₄
   [M+H]⁺calculé : 645.3184
   [M+H]⁺ mesuré : 645.3182
**Exemple 423. 5-(5-Fluoro-2-{[(3*S*)-3-(morpholin-4-ylméthyl)3,4-dihydroisoquinolin-2(1*H*)-yl]carbonyl}phényl)-*N*-(4-hydroxyphényl)1,2-diméthyl-*N*-[1-(oxétan-3-yl)-1*H-*pyrazol-4-yl]-1*H*-pyrrole-3-carboxamode**
   Le composé du titre est obtenu selon le procédé de l'Exemple 1 en utilisant l'acide de la Préparation 8 et le composé de la Préparation 3' au Stade A, ainsi que le composé de la Préparation 26" au Stade C.
   ***Microanalyse élémentaire : (% théorique : mesuré)***
   %C=68.17:67.82; %H=5.86:5.97; %N=11.92:11.48
   ***Masse haute résolution (ESI*/*FIAlHR et MS*/*MS*) *:***
   Formule brute : C₄₀H₄₁FN₆O₅
   [M+H]⁺calculé : 705.3202
   [M+H]⁺ mesuré : 705.3207
**Exemple 424. Chlorhydrate de *N*-(4-hydroxyphényl)-*N*-(2-méthoxypyrimidin-5-yl)-1,2**-**diméthyl**-**5-(2-{[(3*S*)**-**3-(marpholin-4-ylméthyl)-3**,**4**-**dihydroisoquinolin**-**2(1*H*)-yl]carbonyl}phényl)-1*H*-pyrrole-3-carboxamide**
   Le composé du titre est obtenu selon le procédé de l'Exemple 1 en utilisant l'acide de la Préparation 25 et le composé de la Préparation 3' au Stade A, ainsi que le composé de la Préparation 12" au Stade C. Le composé obtenu est salifié et lyophilisé tel que décrit dans le procédé général pour obtenir le composé du titre.
   ***Microanalyse élémentaire : (% théorique : mesuré)***
   %C=66.05:65.63; %H=5.83:5.45; %N=11.85:11.93; %Cl⁻=5.00:4.91
   ***Masse haute résolution (ESI*+-/*FIA*/*HR, ESI***-/***FIA***) ***:***
   Formule brute : C₃₉ H₄₀ N₆ O₅
   [M+H]⁺calculé : 673.3133
   [M+H]⁺ mesuré : 673.3129
**Exemple 425. Chlorhydrate de *N*-(3-cyano-5-méthoxyphényl)-5-(5-fluoro-2-{[(35)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrale-3-carboxamide**
   Le composé du titre est obtenu selon le procédé de l'Exemple 1 en utilisant l'acide de la Préparation 8 et le composé de la Préparation 3' au Stade A, ainsi que l'amine adéquate au Stade C. Le composé obtenu est salifié et lyophilisé tel que décrit dans le procédé général pour obtenir le composé du titre.
   ***Microanalyse élémentaire : (% théorique : mesuré)***
   %C=67.25:66.55; %H=5.51:5.28; %N=9.33:8.55 %Cl⁻=4.73:4.67
**Exemple 426**. **5-(5-Fluoro-2-{[(3*S*)-3-(morpholin-4-ylméthyl)-3,4-dihydroisoquinolin-2(1*H*)-yl]carbonyl}phényl)-*N*-(4-hydroxyphényl)-*N*-(2-méthoxypyrimidin-5-yl)-1,2-diméthyl**-**1*H***-**pyrrole**-**3**-**carboxamide**
   Le composé du titre est obtenu selon le procédé de l'Exemple 1 en utilisant l'acide de la Préparation 8 et le composé de la Préparation 3' au Stade A, ainsi que le composé de la Préparation 12" au Stade C.
   ***Masse haute résolution (ESI*/*FIA*/*HR et MS***/***MS***) ***:***
   Formule brute : C₃₉H₃₉FN₆O₅
   [M+H]⁺calculé : 691.3038
   [M+H]⁺ mesuré: 691.3060
**Exemple 427. Chlorhydrate de *N*-(3-cyano-4-méthoxyphényl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que l'amine adéquate au Stade C. Le composé obtenu est salifié et lyophilisé tel que décrit dans le procédé général pour obtenir le composé du titre.
   ***Microanalyse élémentaire : (% théorique : mesuré)***
   %C=67.24:66.41; %H=5.51:5.35; %N=9.33:8.97 %Cl⁻=4.73:4.81
**Exemple 428. Chlorhydrate de *N*-(5-cyano-1,2-diméthyl-1*H*-pyrrol-3-yl)-5-(4-fluoro-2-{[(3S)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 26 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=66.79:66.97; %H=5.74:5.36; %N=11.40:11.45; %Cl⁻=4.81:4.53
   ***Masse haute résolution (ESI*+-/*FIA*/*HR, ESI-*/*FIA) :***
   Formule brute : C₄₁ H₄₁ F N₆ O₄
   [M+H]⁺calculé : 701.3246
   [M+H]⁺ mesuré : 701.3245
**Exemple 429. Chlorhydrate de *N*-(5-cyano-1,2-diméthyl-1*H*-pyrrol-3-yl)-5-(4-fluoro-5-méthoxy-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 27 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=65.75:65.43; %H=5.78:5.57; %N=10.95:10.81; %Cl⁻=4.62:4.54
   ***Masse haute résolution (ESI*/*FIA*/*HR et MS*/*MS*, *ESI-*/*FIA) :***
   Formule brute : C₄₂ H₄₃ F N₆ O₅
   [M+H]⁺calculé : 731.3352
   [M+H]⁺ mesuré : 731.3351
**Exemple 430. 5-(5-Chloro-2-{[3-(trifluorométhyl)-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide**
   Le composé du titre est obtenu selon le procédé de l'Exemple 1 en utilisant l'acide de la Préparation 1 et la 3-(trifluorométhyl)-1,2,3,4-tétrahydroisoquinoline racémique au Stade A, ainsi que le composé de la Préparation 11" au Stade C.
   ***Masse haute résolution (ESI*+-/*FIA*/*HR et MS*/*MS, ESI-*/*FIA) :***
   Formule brute : C₃₈ H₃₁ Cl F₃ N₅ O₃
   [M+H]⁺calculé : 698.2140
   [M+H]⁺ mesuré : 698.2144
**Exemple 431. Chlorhydrate de 5-(5-chloro-2-{[(3*S*)-3-(morpholin-4-ylméthy)-3,4-dihydroisoquinolin-2(1*H*)-yl]carbonyl}phényl)-*N*-(1,3-diméthyl-1*H*-pyrazol-4-yl)-*N-*(4-hydroxyphényl)-1,2-diméthyt-1*H*-pyrrole-3-carboxamide**
   ***Microanalyse élémentaire : (% théorique : mesuré)***
   %C=64.19:64.37; %H=5.80:5.18; %N=11.52:11.55; %Cl⁻=4.86:4.68
   ***Masse haute résolution (ESI*+-/*FIA*/*HR, ESI***-/***FIA***) ***:***
   Formule brute : C₃₉ H₄₁ Cl N₆ O₄
   [M+H]⁺calculé : 693.2951
   [M+H]⁺ mesuré : 693.2952
   **et**
**Exemple 432. Chlorhydrate de 5-5-chloro-2-{[(3*S*)-3-(morpholin-4-ylméthyl)-3,4-dihydroisoquinolin-2(1*H*)-yl]carbonyl}phényl)-*N*-(1,5-diméthyl-1*H-*pyrazol-4-yl)-*N-*(4-hydroxyphényl)-1,2-diméthyl-1*H*-pyrrole-3-carboxamide**
   ***Microanalyse élémentaire : (% théorique : mesuré)***
   %C=64.19:64.43; %H=5.80:5.22; %N=11.52:11.60; %Cl⁻=4.86:4.66
   ***Masse haute résolution (ESI*+-/*FIA*/*HR, ESI***-/***FIA***) ***:***
   Formule brute : C₃₉ H₄₁ Cl N₆ O₄
   [M+H]⁺calculé : 693.2951
   [M+H]⁺ mesuré : 693.2953

   Les composés des Exemples 431 et 432 sont obtenus selon le procédé de l'Exemple 1 en utilisant l'acide de la Préparation 1 et le composé de la Préparation 3' au Stade A, ainsi que le mélange de la Préparation 27" au Stade C. A l'issue du Stade D, les isomères sont séparés par HPLC préparative en utilisant l'acétonitrile et l'eau-TFA comme éluants. Après évaporation du solvant et neutralisation au bicarbonate de sodium, les produits sont soumis à une étape de salification en présence d'éther chlorhydrique 1M dans l'éther. Après filtration et lyophilisation dans un mélange acétonitrile/eau, on obtient les produits du titre.
**Exemple 433. Chlorhydrate de *N*-(5-cyano-1-méthyl-1*H*-pyrrol-3-yl)-*N*-(4-hydroxyphényl)-1,2-diméthyl-5-(2-{[(3*S*)-3-(morpholin-4-ylméthyl)-3,4-dihydroisoquinolin-2(1*H*)-yl]carbonyl}phenyl)-1*H*-pyrrole-3-carboxamide**
   Le composé du titre est obtenu selon le procédé de l'Exemple 1 en utilisant l'acide de la Préparation 25 et le composé de la Préparation 3' au Stade A, ainsi que le composé de la Préparation 19" au Stade C. Le composé obtenu est salifié et lyophilisé tel que décrit dans le procédé général pour obtenir le composé du titre.
   ***Microanalyse élémentaire : (% théorique : mesuré)***
   %C=68.12:68.29; %H=5.86:5.40; %N=11.92:12.05; %Cl⁻=5.03:4.92
   ***Masse haute résolution (ESI*/*FIA*/*HR et MS*/*MS*) *:***
   Formule brute : C₄₀ H₄₀ N₅ O₄
   [M+H]⁺calculé : 669.3184
   [M+H]⁺ mesuré : 669.3184
**Exemple 434. Chlorhydrate de 5-(5-fluoro-2-{[(3*S*)-3-(morpholin-4-ylméthyl)-3,4-dihydroisoquinolin-2(1H)-yl]-carbonyl}phényl)-*N*-(4-hydroxyphényl)-1,2-diméthyl-*N-*(1-méthyl-2,3-dihydro-1*H*-pyrrolo[2,3-*b*]pyridin-5-yl)-1*H*-pyrrole-3-carboxamide**
   Le composé du titre est obtenu selon le procédé décrit au Stade B de l'Exemple 49 en utilisant l'Exemple 385 comme matière première. Le composé obtenu est salifié et lyophilisé tel que décrit dans le procédé général pour obtenir le composé du titre.
   ***Microanalyse élémentaire : (% théorique : mesuré)***
   %C=67.15 :68.03; %H=5.90:5.50; %N=11.19:10.59 %Cl⁻=4.72:5.55
**Exemple 435. Chlorhydrate de *N*-(4-cyanopyridin-2-yl)-5-(5-fluoro-4-méthoxy-2-{[(3*S*)-3-(morpholin-4-ylmethyl)-3,4-dihydroisoquinolin-2(1*H*)-yl]carbonyl}phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 14 et le composé de la Préparation 3' au Stade A, ainsi que l'aminé adéquate au Stade C. Le composé obtenu est salifié et lyophilisé tel que décrit dans le procédé général pour obtenir le composé du titre.
   ***Masse haute résolution (ESI*+-/*FIA*/*HR, ESI*-/*FIA) :***
   Formule brute : C₄₁ H₃₉ F N₆ O₅
   [M+H]⁺calculé : 715.3039
   [M+H]⁺ mesuré : 715.3040
**Exemple 436. Chlorhydrate de 5-(5-chloro-2-{[(3*S*)-3-(morpholin-4-ylméthyl)-3,4-dihydroisoquinolin-2(1H)-yl]carbonyl}phényl)-*N*-(5-cyanothiophén-2-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'aminé adéquate au Stade C. Le composé obtenu est salifié et lyophilisé tel que décrit dans le procédé général pour obtenir le composé du titre.
   ***Microanalyse élémentaire : (% théorique : mesuré)***
   %C=63.07:63.09; %H=5.02:4.78; %N=9.43:9.35; %S=4.32:4.09; %Cl⁻=4.77:4.59
   ***Masse haute résolution (ESI*/*FIA*/*HR et MS*/*MS*, *ESI*+-/*FIA) :***
   Formule brute : C₃₉ H₃₆ Cl N₅ O₄ S
   [M+H]⁺calculé : 706.2249:
   [M+H]⁺ mesuré : 706.2250
**Exemple 437. Chorhydrate de *N-*(3-cyano-4-fluorophényl)-5-(5-fluoro-2{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que l'amine adéquate au Stade C. Le composé obtenu est salifié et lyophilisé tel que décrit dans le procédé général pour obtenir le composé du titre.
   ***Masse haute résolution (ES*+*) :***
   Formule brute : C₄₁H₃₇F₂N₅O₄
   [M+H]⁺ calculé : 702.2894
   [M+H]⁺ mesuré : 702.2886
**Exemple 438. Chlorhydrate de 5-(5-chloro-2-{[(3*S*)-3-(morpholin-4-ylméthyl)3,4-dihydroisoquinolin-2(1*H*)-yl]carbonyl}phényl)-*N*-(4-hydroxyphényl)-1,2-diméthyl-*N-*(pyrazin-2-yl)-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'aminé adéquate au Stade C. Le composé obtenu est salifié et lyophilisé tel que décrit dans le procédé général pour obtenir le composé du titre.
   ***Microanalyse élémentaire : (% théorique : mesuré)***
   %C=63.95:63.96; %H=5.37:5.17; %N=11.78:11.61; %Cl⁻=4.97:4.57
   ***Masse haute résolution (ESI+*/*HR, ESI-*/*LR) :***
   Formule brute : C₃₈ H₃₇ Cl N₆ O₄
   [M+H]⁺calculé : 677.2638
   [M+H]⁺ mesuré : 677.2639
**Exemple 439. Chlorhydrate de 5-(5-fluoro-4-méthoxy-2-{[(3*S*)-3-(morpholin-4-ylméthyl)-3**,**4**-**dihydroisoquinolin**-**2(1*H*)**-**yl**]**carbonyl}phényl)-*N*-4**-**hydroxyphényl)-*N-*(2-méthoxypyrimidin-5-yl)-1,2-diméthyl-1*H*-pyrrole-3-carboxamide**
   Le composé du titre est obtenu selon le procédé de l'Exemple 1 en utilisant l'acide de la Préparation 14 et le composé de la Préparation 3' au Stade A, ainsi que le composé de la Préparation 12" au Stade C. Le composé obtenu est salifié et lyophilisé tel que décrit dans le procédé général pour obtenir le composé du titre.
   ***Masse haute résolution (ESI*/*FIA*/*HR et MS*/*MS, ESI-*/*FIA*) *:***
   Formule brute : C₄₀ H₄₁ F N₆ O₆
   [M+H]⁺ calculé : 721.3144
   [M+H]⁺ mesuré : 721.3144
**Exemple 440. Chlorhydrate de *N*-(5-cyano-1,2-diméthyl-1*H*-pyrrol-3-yl)-5-(5-fluoro-4-méthoxy-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 14 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=65.75:65.98; %H=5.78:5.50; %N=10.95:10.87; %Cl⁻=4.62:4.42
   ***Masse haute résolution (ESI*+/*HR, ESI-*/*LR) :***
   Formule brute : C₄₂ H₄₃ F N₆ O₅
   [M+H]⁺ calculé : 731.3352
   [M+H]⁺ mesuré : 731.3353
**Exemple 441. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-1,2-diméthyl-*N*-phényl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 29" au Stade C, étant entendu que le Stade D n'est pas effectué. Le composé obtenu est salifié et lyophilisé tel que décrit dans le procédé général pour obtenir le composé du titre.
   ***Microanalyse élémentaire : (% théorique : mesuré)***
   %C=66.75:66.44; %H=5.74:5.59; %N=11.39:11.45; %Cl⁻=4.81:4.43
   ***Masse haute résolution (ESI+*/*HR, ESI-*/*LR) :***
   Formule brute : C₄₁ H₄₁ Cl N₆ O₃
   [M+H]⁺ calculé : 701.3001
   [M+H]⁺ mesuré : 701.2998
**Exemple 442. Chlorhydrate de 5-(5-chloro-2-{[(3*S*)-3-(morpholin-4-ylméthyl)-3,4-dihydroisoquinolin-2(1*H*)-yl]carbonyl}phényl-*N*-(5-cyanothiophén-3-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 42" au Stade C. Le composé obtenu est salifié et lyophilisé tel que décrit dans le procédé général pour obtenir le composé du titre.
   ***Microanalyse élémentaire : (% théorique : mesuré)***
   %C=63.07:63.14; %H=5.02:4.87; %N=9.43:9.41; %S=4.32:4.24; %Cl⁻=4.77:4.57
   ***Masse haute résolution (ESI+*/*HR, ESI-*/*LR) :***
   Formule brute : C₃₉ H₃₆ Cl N₅ O₄ S
   [M+H]⁺ calculé : 706.2249
   [M+H]⁺ mesuré : 706.2252
**Exemple 443. 5-(5-Fluoro-4-hydroxy-2-{[(3*S*)-3-(morpholin-4-ylméthyl)-3,4-dihydroisoquinolin-2(1*H*)-yl]carbonyl}phényl)-1,2-diméthyl-*N*-(1-méthyl-1*H-*pyrrolo[2,3-*b*]pyridin-5-yl)-*N*-phényl-1*H*-pyrrole-3-carboxamide**
   ***Stade A : 5-(5-Fluoro-4-méthoxy-2-{[(3S)-3-(morpholin-4-ylméthyl)-3,4-dihydroisoquinolin-2(1H)-yl]carbonyl}phényl)-1,2-diméthyl-N-(1-méthyl-1H-pyrrolo[2,3-b]pyridin-5-yl)-N-phényl-1H-pyrrole-3-carboxamide***
   Le composé du titre est obtenu selon le procédé de l'Exemple 1 (Stades A à C) en utilisant l'acide de la Préparation 28 et le composé issu de la Préparation 3' au Stade A, ainsi que le composé de la Préparation 30" au Stade C.***Stade B :* 5-(5-Fluoro-4-hydroxy-2-{[(3*S*)-3-(morpholin-4-ylméthyl)-3,4-dihydroisoquinolin-2(1*H*)-yl]carbonyl}phényl)-1,2-diméthyl-*N*-(1-méthyl-1*H-*pyrrolo[2,3-*b*]pyridin-5-yl)-*N*-phényl-1*H*-pyrrole-3-carboxamide**
   A une solution du composé du Stade A (1 g ; 1,37 mmol) dans du dichlorométhane anhydre (10 mL) est ajouté au goutte à goutte à 0°C une solution molaire de tribromure de bore dans le dichlorométhane (1,8 mL ; 1,8 mmol). Après 15 heures d'agitation à température ambiante, le milieu réactionnel est coulé au goutte à goutte sur une solution d'éthanol (15 mL) à -10°C. Après une heure d'agitation, une solution aqueuse saturée en bicarbonate de sodium est ajouté, et le milieu réactionnel est extrait au dichlorométhane. Après séchage au MgSO₄, le résidu est purifié sur colonne de gel de silice en utilisant un mélange de dichlorométhane et de méthanol comme éluant pour conduire au produit attendu.
   ***Masse haute résolution (ESI*+/*HR, ESI-*/*LR) :***
   Formule brute : C₄₂ H₄₁ F N₆ O₄
   [M+H]⁺ calculé : 713.3246
   [M+H]⁺ mesuré : 713.3244
**Exemple 444. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-*N*-(5-méthoxypyrazin-2-yl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'amine adéquate au Stade C. Le composé obtenu est salifié et lyophilisé tel que décrit dans le procédé général pour obtenir le composé du titre.
   ***Microanalyse élémentaire : (% théorique : mesuré)***
   %C=62.99:62.72; %H=5.42:5.24; %N=11.30:11.19; %Cl⁻=4.77:4.67
   ***Masse haute résolution (ESI*+/*HR, ESI-*/*LR) :***
   Formule brute : C₃₉ H₃₉ Cl N₆ O₅
   [M+H]⁺ calculé : 707.2743
   [M+H]⁺ mesuré : 707.2747
**Exemple 445. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*[1-(trideutériométhyl)-2,3-dihydro-1*H*-pyrrolo[2,3-*b*]pyridin-5-yl]-1*H*-pyrrole-3-carboxamide**
   ***Stade A : 5-(5-Chloro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-[1-(trideutériométhyl)-1H-pyrrolo[2,3-b]pyridin-5-yl]-1H-pyrrole-3-carboxamide***
   L'intermédiaire est obtenu selon le procédé de l'Exemple 1 en utilisant l'acide de la Préparation 1 et le composé de la Préparation 3' au Stade A, ainsi que le composé de la Préparation 31" au Stade C.***Stade B : Chlorhydrate de 5-(5-chloro-2-{[(3S)-3-(morpholin-4-ylméthyl)-3,4-dihydroisoquinolin-2(1H)-yl]carbonyl}phényl)-N-(4-hydroxyphényl)-1,2-diméthyl-N-[1-(trideutériométhyl)-2,3-dihydro-1H-pyrrolo[2,3-b]pyridin-5-yl]-1H-pyrrole-3-carboxamide***
   On procède selon le protocole décrit au Stade B de l'Exemple 49, étant entendu que le produit est ensuite soumis à une étape de salification en présence d'éther chlorhydrique 1M dans l'éther. Après filtration et lyophilisation dans un mélange acétonitrile/eau, on obtient le produit du titre.
   ***Microanalyse élémentaire : (% théorique : mesuré)***
   %C=65.45:65.33; %H=5.78:5.59; %N=10.90:10.82; %Cl⁻=4.60:4.28
   ***Masse haute résolution (ESI*+/*HR, ESI-*/*LR) :***
   Formule brute : C₄₂ H₄₀ Cl D₃ N₆ O₄
   [M+H]⁺ calculé : 734.3295
   [M+H]⁺ mesuré : 734.3300
**Exemple 446. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(4-fluoropyrazin-2-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'amine adéquate au Stade C. Le composé obtenu est salifié et lyophilisé tel que décrit dans le procédé général pour obtenir le composé du titre.
   ***Microanalyse élémentaire : (% théorique : mesuré)***
   %C=62.28:62.16; %H=5.10:4.97; %N=11.04:11.35; %Cl⁻=4.85:4.48
   ***Masse haute résolution (ESI+*/*HR, ESI-*/*LR) :***
   Formule brute : C₃₈ H₃₆ Cl F N₆ O₄
   [M+H]⁺ calculé : 695.2543
   [M+H]⁺ mesuré : 695.2545
**Exemple 447. Chlorhydrate de 5-(4-fluoro-2-{[(3*S*)-3-(morpholin-4-ylméthyl)-3,4-dihydroisoquinolin-2(1*H*)-yl]carbonyl}phényl)-*N*-(4-hydroxyphényl-1,2-diméthyl-*N-*(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 26 et le composé de la Préparation 3' au Stade A, ainsi que le composé de la Préparation 1" au Stade C. Le composé obtenu est salifié et lyophilisé tel que décrit dans le procédé général pour obtenir le composé du titre.
   ***Microanalyse élémentaire : (% théorique : mesuré)***
   %C=65.28:65.27; %H=5.77:5.51; %N=12.02:1.90; %Cl⁻=5.07:4.74
   ***Masse haute résolution (ESI+*/*HR, ESI-*/*LR):***
   Formule brute : C₃₈ H₃₉ F N₆ O₄
   [M+H]⁺ calculé : 663.3090
   [M+H]⁺ mesuré : 663.3084
**Exemple 448. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-[5-cyano-1-(trideutériométhyl)-1*H-*pyrrol-3-yl]-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 32" au Stade C. Le composé obtenu est salifié et lyophilisé tel que décrit dans le procédé général pour obtenir le composé du titre.
   ***Microanalyse élémentaire : (% théorique : mesuré)***
   %C=64.69:64.23; %H=5.45:5.47; %N=11.32:11.16; %Cl⁻=4.77:4.56
   ***Masse haute résolution (ESI+*/*HR, ESI-*/*LR) :***
   Formule brute : C₄₀ H₃₆ Cl D₃ N₆ O₄
   [M+H]⁺calculé : 706.2982
   [M+H]⁺ mesuré : 706.2985
**Exemple 449. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-[5-cyano-2-méthyl-1-(trideutériométhyl)-1*H*-pyrrol-3-yl]-N-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 33" au Stade C. Le composé obtenu est salifié et lyophilisé tel que décrit dans le procédé général pour obtenir le composé du titre.
   ***Microanalyse élémentaire : (% théorique : mesuré)***
   %C=65.07:64.55; %H=5.62:5.51; %N=11.11:10.98; %Cl⁻=4.68:4.58
   ***Masse haute résolution (ESI+*/*HR, ESI-*/*LR) :***
   Formule brute : C₄₁ H₃₈ Cl D₃ N₆ O₄
   [M+H]⁺ calculé : 720.3139
   [M+H]⁺ mesuré : 720.3143
**Exemple 450. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(3-cyano-1-méthyl-1*H*-pyrazol-5-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 34" au Stade C. Le composé obtenu est salifié et lyophilisé tel que décrit dans le procédé général pour obtenir le composé du titre.
   ***Microanalyse élémentaire : (% théorique : mesuré)***
   %C=63.24:62.62; %H=5.31:5.09; %N=13.24:13.04; %Cl⁻=4.79:4.37
   ***Masse haute résolution (ESI+*/*HR, ESI-*/*LR) :***
   Formule brute : C₃₉ H₃₈ Cl N₇ O₄
   [M+H]⁺ calculé : 704.2747
   [M+H]⁺ mesuré : 704.2747
**Exemple 451. Chlorhydrate de 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrazolo[3,4-b]pyridin-5-yl)-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que le composé de la Préparation 17" au Stade C. Le composé obtenu est salifié et lyophilisé tel que décrit dans le procédé général pour obtenir le composé du titre.
   ***Microanalyse élémentaire : (% théorique : mesuré)***
   %C=65.64:66.28; %H=5.51:5.45; %N=13.07:12.17 %Cl⁻=4.73:5.51
**Exemple 452. Chlorhydrate de *N*-(1,3-diméthyl-1*H*-pyrazol-4-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   ***Microanalyse élémentaire : (% théorique : mesuré)***
   %C=65.68:64.77; %H=5.94:5.55; %N=11.78:10.69 %Cl⁻=4.97:6.48 **et**
**Exemple 453. Chlorhydrate de *N*-(1,5-diméthyl-1*H*-pyrazol-4-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   ***Microanalyse élémentaire : (% théorique : mesuré)***
   %C=65.68:65.43; %H=5.94:5.62; %N=11.78:10.95 %Cl⁻=4.97:5.60
   Les composés des Exemples 452 et 453 sont obtenus selon le procédé de l'Exemple 1 en utilisant l'acide de la Préparation 8 et le composé de la Préparation 3' au Stade A, ainsi que le mélange de la Préparation 27" au Stade C. A l'issue du Stade D, les isomères sont séparés par HPLC préparative en utilisant l'acétonitrile et l'eau-TFA comme éluants. Après évaporation du solvant et neutralisation au bicarbonate de sodium, les produits sont soumis à une étape de salification en présence d'éther chlorhydrique 1M dans l'éther. Après filtration et lyophilisation dans un mélange acétonitrile/eau, on obtient les produits du titre.
**Exemple 454. 5-(5-Chloro-2{[3-(trifluorométhyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide issu de la Préparation 1 et la 3-(trifluorométhyl)-1,2,3,4-tétrahydroisoquinoline racémique au Stade A, ainsi que le composé de la Préparation 18" au Stade C.
   ***Microanalyse élémentaire : (% théorique : mesuré)***
   %C=64.77:64.81; %H=4.55:4.52; %N=10.21:10.33
   ***Masse haute résolution (ESI+*/*HR) :***
   Formule brute : C₃₇ H₃₁ Cl F₃ N₅ O₃
   [M+H]⁺ calculé : 686.2140
   [M+H]⁺ mesuré : 686.2145
**Exemple 455. Chlorhydrate de 5-(5-chloro-2-{[(3*R*)-3-méthyl-3,4-dihydroisoquinolin-2(1*H*)-yl]carbonyl}phényl)-*N*-{5-cyano-2-méthyl-1-[2-(morpholin-4-yl)éthyl]-1*H-*pyrrol-3-yl}-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 1' au Stade A, ainsi que le composé de la Préparation 35" au Stade C. Le composé obtenu est salifié et lyophilisé tel que décrit dans le procédé général pour obtenir le composé du titre.
   ***Microanalyse élémentaire : (% théorique : mesuré)***
   %C=65.71:66.07; %H=5.78:5.82; %N=10.95:10.66; %Cl⁻=4.62:4.45
   ***Masse haute résolution (ESI+*/*HR, ESI-*/*LR) :***
   Formule brute : C₄₂ H₄₃ Cl N₆ O₄
   [M+H]⁺ calculé : 731.3107
   [M+H]⁺ mesuré : 731.3109
**Exemple 456. Chlorhydrate de 5-(5-chloro-2-{[(3*R*)-3-méthyl-3,4-dihydroisoquinolin-2(1*H*)-yl]carbonyl}phényl)-*N*-(4-hydroxyphényl)-1,2-diméthyl-*N*-{2-[2-(morpholin-4-yl)éthoxy]pyrimidin-5-yl}-1*H*-pyrrole-3-carboxamide**
   Le composé du titre est obtenu selon le procédé de l'Exemple 1 en utilisant l'acide de la Préparation 1 et le composé de la Préparation 1' au Stade A, ainsi que le composé de la Préparation 36" au Stade C. Le composé obtenu est salifié et lyophilisé tel que décrit dans le procédé général pour obtenir le composé du titre.
   ***Microanalyse élémentaire : (% théorique : mesuré)***
   %C=63.41:63.51; %H=5.59:5.26; %N=11.09:11.10; %Cl⁻=4.68:4.46
   ***Masse haute résolution (ESI+*/*HR, ESI-*/*LR):***
   Formule brute : C₄₀ H₄₁ Cl N₆ O₅
   [M+H]⁺ calculé : 721.2900
   [M+H]⁺ mesuré : 721.2907
**Exemple 457. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(6-cyano-5-méthoxypyridin-2-yl)-*N-*(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'amine adéquate au Stade C. Le composé obtenu est salifié et lyophilisé tel que décrit dans le procédé général pour obtenir le composé du titre.
   ***Microanalyse élémentaire : (% théorique : mesuré)***
   %C=64.15:63.95; %H=5.25:4.79; %N=10.95:10.97; %Cl⁻=4.62:4.22
   ***Masse haute résolution (ESI+*/*HR) :***
   Formule brute : C₄₁ H₃₉ Cl N₆ O₅
   [M+H]⁺ calculé : 731.2743
   [M+H]⁺ mesuré : 731.2746
**Exemple 458. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(5-cyano-1,4-diméthyl-1*H*-pyrrol-3-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 37" au Stade C. Le composé obtenu est salifié et lyophilisé tel que décrit dans le procédé général pour obtenir le composé du titre.
   ***Microanalyse élémentaire : (% théorique : mesuré)***
   %C=65.34:65.68; %H=5.62:5.31; %N=11.15:11.15; %Cl⁻=4.70:4.33
   ***Masse haute résolution (ESI+*/*HR, ESI-*/*LR) :***
   Formule brute : C₄₁ H₄₁ Cl N₆ O₄
   [M+H]⁺ calculé : 717.2951
   [M+H]⁺ mesuré : 717.2954
**Exemple 459. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(5-cyano-1-éthyl-2-méthyl-1*H-*pyrrol-3-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 38" au Stade C. Le composé obtenu est salifié et lyophilisé tel que décrit dans le procédé général pour obtenir le composé du titre.
   ***Microanalyse élémentaire : (% théorique : mesuré)***
   %C=65.71;65.29; %H=5.78:5.51; %N=10.95:10.95; %Cl⁻=4.62:4.39
   ***Masse haute résolution (ESI+*/*HR, ESI-*/*LR) :***
   Formule brute : C₄₂ H₄₃ Cl N₆ O₄
   [M+H]⁺ calculé : 731.3107
   [M+H]⁺ mesuré : 731.3109
**Exemple 460. Chlorhydrate de 5-(5-chloro-2-{[(3*R*)-3-[3-(morpholin-4-yl)propyl]-3,4-dihydroisoquinolin-2(1*H*)-yl]carbonyl}phényl)-*N*-(4-hydroxyphényl)-1,2-diméthyl-*N-*[1-(tétrahydrofuran-3-yl)-1*H*-pyrazol-4-yl]-1*H*-pyrrole-3-carboxamide**
   Le composé du titre est obtenu selon le procédé de l'Exemple 1 en utilisant l'acide de la Préparation 1 et le composé de la Préparation 6' au Stade A, ainsi que le composé de la Préparation 22" au Stade C. Le composé obtenu est salifié et lyophilisé tel que décrit dans le procédé général pour obtenir le composé du titre.
   ***Microanalyse élémentaire : (% théorique : mesuré)***
   %C=64.58:64.24; %H=6.05:5.88; %N=10.51:10.53; %Cl⁻=4.43:4.39
   ***Masse haute résolution (ESI+*/*HR, ESI-*/*LR) :***
   Formule brute : C₄₃ H₄₇ Cl N₆ O₅
   [M+H]⁺ calculé : 763.3369
   [M+H]⁺ mesuré : 763.3371
**Exemple 461. Chlorhydrate de 5-(5-chloro-2-{[(3*R*)-3-[3-(morpholin-4-yl)propyl]-3,4-dihydroisoquinolin-2(1*H*)-yl]carbonyl}phényl)-*N*-(5-cyano-1,2-diméthyl-1*H*-pyrrol-3-yl)-1,2-diméthyl-*N*-phényl-1*H*-pyrrole-3-carboxamide**
   Le composé du titre est obtenu selon le procédé de l'Exemple 1 en utilisant l'acide de la Préparation 1 et le composé de la Préparation 6' au Stade A, ainsi que le composé de la Préparation 29" au Stade C, étant entendu que le Stade D n'est pas effectué. Le composé obtenu est salifié et lyophilisé tel que décrit dans le procédé général pour obtenir le composé du titre.
   ***Microanalyse élémentaire : (% théorique : mesuré)***
   %C=67.44:66.68; %H=6.05:5.80; %N=10.97:10.95; %Cl⁻=4.63:4.57
   ***Masse haute résolution (ESI+*/*HR) :***
   Formule brute : C₄₃ H₄₅ Cl N₆ O₃
   [M+H]⁺ calculé : 729.3314
   [M+H]⁺ mesuré : 729.3316
**Exemple 462. 5-(5-Chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(5-méthyl-1,2-oxazol-3-yl)-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que l'amine adéquate au Stade C.
   ***Microanalyse élémentaire : (% théorique : mesuré)***
   %C=67.10:66.64; %H=5.63:5.40; %N=10.30:10.24
   ***Masse haute résolution (ESI+*/*HR, ESI-*/*LR) :***
   Formule brute : C₃₈ H₃₈ Cl N₅ O₅
   [M+H]⁺ calculé : 680.2634
   [M+H]⁺ mesuré : 680.2637
**Exemple 463. *N*-(2-Ethoxypyrimidin-5-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 8 et le composé de la Préparation 3' au Stade A, ainsi que le composé de la Préparation 39" au Stade C.
   ***Microanalyse élémentaire : (% théorique : mesuré)***
   %C=68.17:67.52; %H=5.86:5.60; %N=11.92:11.43
**Exemple 464. Dichlorhydrate de 5-(5-chloro-2-{[(3*S*)-3-(morpholin-4-ylméthyl)-3,4-dihydroisoquinolin-2(1*H*)-yl]carbonyl}phényl)-*N*-(5-hydroxypyridin-2-yl)-1,2-diméthyl-*N*-(1-méthyl-1*H*-pyrrolo[2,3-*b*]pyridin-5-yl)-1*H*-pyrrole-3-carboxamide**
   Le composé du titre est obtenu selon le procédé de l'Exemple 1 en utilisant l'acide de la Préparation 1 et le composé de la Préparation 3' au Stade A, ainsi que le composé de la Préparation 40" au Stade C. Le composé obtenu est salifié et lyophilisé tel que décrit dans le procédé général pour obtenir le composé du titre.
   ***Masse haute résolution (ESI+*/*HR, ESI-*/*LR) :***
   Formule brute : C₄₁ H₄₀ Cl N₇ O₄
   [M+H]⁺ calculé : 730.2903
   [M+H]⁺ mesuré : 730.2907
**Exemple 465. Chlorhydrate de 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide**
   Le composé du titre est obtenu selon le procédé de l'Exemple 1 en utilisant l'acide de la Préparation 29 et le composé de la Préparation 3' au Stade A, ainsi que le composé de la Préparation 18" au Stade C. Le composé obtenu est salifié et lyophilisé tel que décrit dans le procédé général pour obtenir le composé du titre.
   ***Microanalyse élémentaire : (% théorique : mesuré)***
   %C=65.34:64.81; %H=5.62:5.27; %N=11.15:10.95 %Cl⁻=4.70:5.09
   ***Masse haute résolution (ESI+) :***
   Formule brute : C₄₁ H₄₁ Cl N₆ O₄
   [M+H]⁺ calculé : 717.2951
   [M+H]⁺ mesuré : 717.2952
**Exemple 466. Chlorhydrate de 5-(4-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N-*(1-méthyl-1*H*-pyrazol-4-yl)-1*H*-pyrrole-3-carboxamide**
   Le composé du titre est obtenu selon le procédé de l'Exemple 1 en utilisant l'acide de la Préparation 29 et le composé de la Préparation 3' au Stade A, ainsi que le composé de la Préparation 1" au Stade C. Le composé obtenu est salifié et lyophilisé tel que décrit dans le procédé général pour obtenir le composé du titre.
   ***Microanalyse élémentaire : (% théorique : mesuré)***
   %C=66.77:62.82; %H=5.63:5.29; %N=11.74:11.75; %Cl⁻=5.01:5.23
   ***Masse haute résolution (ESI+*/*HR, ESI-*/*LR) :***
   Formule brute : C₃₈ H₃₉ Cl N₆ O₄
   [M+H]⁺ calculé : 679.2794
   [M+H]⁺ mesuré : 679.2796
**Exemple 467. 5-(5-chloro-2-{[3,4-dihydroisoquinolin-2(1*H*)-yl]carbonyl}phényl)-*N*-(4-hydroxyphényl)-1,2-diméthyl-*N*-phényl-1*H*-pyrrole-3-carboxamide**
   Le composé du titre est obtenu selon le procédé de l'Exemple 1 en utilisant l'acide de la Préparation 1 et la 1,2,3,4-tétrahydroisoquinoline au Stade A, ainsi que le composé de la Préparation 2" au Stade C.
   ***Microanalyse élémentaire : (% théorique : mesuré)***
   %C=72.97:72.93; %H=5.25:5.08; %N=7.29:7.34
   ***Masse haute résolution (ESI*/*FIA*/*HR et MSlMS) :***
   Formule brute : C₃₅ H₃₀ Cl N₃ O₃
   [M+H]⁺ calculé : 576.2048
   [M+H]⁺ mesuré : 576.2067

### ETUDE PHARMACOLOGIQUE

### EXEMPLE A : Inhibition de Bcl-2 par la technique de polarisation de fluorescence

Les essais de polarisation de fluorescence ont été réalisés en microplaques (384 puits). La protéine Bcl-2 étiquetée (histag-Bcl-2 tel que Bcl-2 correspond au numéro d'ordre primaire UniProtKB^{®}: P10415), à la concentration finale de 2,50.10⁻⁸M, est mélangée avec un peptide fluorescent (Fluorescein-REIGAQLRRMADDLNAQY), à la concentration finale de 1,00. 10⁻⁸M dans une solution tampon (Hepes 10 mM, NaCl 150 mM, Tween20 0,05%, pH 7,4), en présence ou en absence de concentrations croissantes de composés à tester. Après incubation de 2 heures, la polarisation de fluorescence est mesurée.
Les résultats sont exprimés en IC₅₀ (concentration en composé qui inhibe à 50 % la polarisation de fluorescence) et sont présentés dans le tableau 1 ci-dessous.

Les résultats montrent que les composés de l'invention inhibent l'interaction entre la protéine Bcl-2 et le peptide fluorescent décrit précédemment.

### EXEMPLE B : Cytotoxicité in vitro.

Les études de cytotoxicité ont été réalisées sur la lignée tumorale de leucémie RS4 ;11. Les cellules sont réparties dans des microplaques et exposées aux composés à tester pendant 48 heures. La viabilité cellulaire est ensuite quantifiée par un essai colorimétrique, le Microculture Tétrazolium Assay (Cancer Res., 1987, 47, 939-942).
Les résultats sont exprimés en IC₅₀ (concentration en composé qui inhibe à 50 % la viabilité cellulaire) et sont présentés dans le tableau 1 ci-dessous.

Les résultats montrent que les composés de l'invention sont cytotoxiques.

**Tableau 1 : IC₅₀ d'inhibition de Bcl-2 (test de polarisation de fluorescence) et de cytotoxicité pour les cellules RS4 ;11**

| | IC₅₀ (nM) Bcl-2 FP | IC₅₀ (nM) MTT RS4;11 | | IC₅₀ (nM) Bcl-2 FP | IC₅₀ (nM) MTT RS4;11 |
|---|---|---|---|---|---|
| **Exemple 1** | 5,0 | 33,6 | **Exemple 29** | 6,6 | 47,5 |
| **Exemple 2** | ND | 1660 | **Exemple 30** | 4,7 | 771 |
| **Exemple 3** | 24,6 | 94,9 | **Exemple 31** | 7,2 | 89,3 |
| **Exemple 4** | ND | 231 | **Exemple 32** | 13,3 | 240 |
| **Exemple 5** | 21,2 | 44,8 | **Exemple 33** | 10,9 | 57,8 |
| **Exemple 6** | 25,2 | 69 | **Exemple 34** | 8,2 | 47 |
| **Exemple 7** | 25,6 | 90,6 | **Exemple 35** | 50,0 | 560 |
| **Exemple 8** | ND | 255 | **Exemple 36** | 71,4 | >600 |
| **Exemple 9** | 22,4 | 87,5 | **Exemple 37** | 60,4 | >600 |
| **Exemple 10** | 16,2 | 205 | **Exemple 38** | 7,5 | 134 |
| **Exemple 11** | 14,2 | 202 | **Exemple 39** | 7,2 | 19,7 |
| **Exemple 12** | 5,5 | 39,6 | **Exemple 40** | 64,4 | 431 |
| **Exemple 13** | 4,4 | 19,8 | **Exemple 41** | 10,0 | 22,6 |
| **Exemple 14** | 3,7 | 8,23 | **Exemple 42** | 5,2 | 4,36 |
| **Exemple 15** | 11,1 | 69,4 | **Exemple 43** | 5,1 | 28,9 |
| **Exemple 16** | 12,6 | 22,7 | **Exemple 44** | 3,0 | 5,41 |
| **Exemple 17** | 8,0 | 75,2 | **Exemple 45** | 38,9 | 403 |
| **Exemple 18** | 3,9 | 27,6 | **Exemple 46** | 76,6 | >600 |
| **Exemple 19** | 6,0 | 65,5 | **Exemple 47** | 5,9 | 44,5 |
| **Exemple 20** | 4,9 | 164 | **Exemple 48** | 4,4 | 14,9 |
| **Exemple 21** | 4,7 | 79,9 | **Exemple 49** | 4,0 | 14,1 |
| **Exemple 22** | 6,6 | 45,7 | **Exemple 50** | 5,9 | 33,1 |
| **Exemple 23** | 3,6 | 25,4 | **Exemple 51** | 26,7 | 354 |
| **Exemple 24** | 6,2 | 79,1 | **Exemple 52** | 28,9 | 433 |
| **Exemple 25** | 4,0 | 33,3 | **Exemple 53** | 43,5 | 293 |
| **Exemple 26** | 4,1 | 541 | **Exemple 54** | 18,0 | 30,5 |
| **Exemple 27** | 5,2 | 93,4 | **Exemple 55** | 209,6 | >600 |
| **Exemple 28** | 7,5 | 95,3 | **Exemple 56** | 75,0 | >600 |
| **Exemple 57** | 80,0 | >600 | **Exemple 102** | 2 | 6,51 |
| **Exemple 58** | 133,3 | >600 | **Exemple 108** | 3,9 | 15,4 |
| **Exemple 59** | 134,0 | >600 | **Exemple 109** | 91,5 | 930 |
| **Exemple 60** | ND | 18,3 | **Exemple 110** | 8,5 | 39,5 |
| **Exemple 61** | 4,5 | 37,8 | **Exemple 115** | 3,6 | 18,3 |
| **Exemple 62** | 14,3 | 127 | **Exemple 116** | 3,4 | 47,8 |
| **Exemple 63** | 5,1 | 11,2 | **Exemple 122** | 3,4 | 82,4 |
| **Exemple 65** | 21,9 | 113 | **Exemple 126** | 4,2 | 49,2 |
| **Exemple 66** | 16,9 | 241 | **Exemple 127** | 5,3 | 111 |
| **Exemple 67** | 12,5 | 98,6 | **Exemple 129** | 18,1 | 275 |
| **Exemple 68** | 2,1 | 20,7 | **Exemple 207** | 4,6 | 32,2 |
| **Exemple 69** | 5,3 | 3,68 | **Exemple 209** | 7,4 | 25,4 |
| **Exemple 70** | 8,5 | 63,5 | **Exemple 210** | 8,8 | 47,2 |
| **Exemple 71** | 6,1 | 12,9 | **Exemple 214** | 6,3 | 236 |
| **Exemple 72** | 7,7 | 43,6 | **Exemple 230** | 5,1 | 18,3 |
| **Exemple 73** | 8 | 42,6 | **Exemple 358** | 14,8 | 165 |
| **Exemple 74** | 4 | 45,4 | **Exemple 384** | 9,7 | 216 |
| **Exemple 75** | 52,9 | 367 | **Exemple 385** | 5,7 | 28,7 |
| **Exemple 81** | ND | 249 | **Exemple 386** | 2,6 | 9,23 |
| **Exemple 82** | 19,5 | 427 | **Exemple 387** | 20,6 | 243 |
| **Exemple 88** | 15,9 | 69,6 | **Exemple 388** | 3,6 | 16,5 |
| **Exemple 92** | 8,4 | 37,8 | **Exemple 389** | 14,7 | 208 |
| **Exemple 93** | 19,7 | 368 | **Exemple 390** | 21,2 | 173 |
| **Exemple 94** | 8,6 | 104 | **Exemple 391** | 30,3 | 255 |
| **Exemple 95** | 7 | 174 | **Exemple 392** | 3,8 | 11,4 |
| **Exemple 96** | 13,5 | 161 | **Exemple 393** | 2,1 | 1,95 |
| **Exemple 97** | 19 | 98 | **Exemple 394** | 2,5 | 2,76 |
| **Exemple 98** | 7,6 | 68,3 | **Exemple 395** | 3,5 | 7,57 |
| **Exemple 99** | 6,4 | 108 | **Exemple 396** | 15,7 | 116 |
| **Exemple 100** | 22,9 | 193 | **Exemple 397** | 4,5 | 37,1 |
| **Exemple 101** | 21,1 | 743 | **Exemple 398** | 3,9 | 13,4 |
| **Exemple 399** | 9,9 | 155 | **Exemple 432** | 3,4 | 76,6 |
| **Exemple 400** | 49 | 469 | **Exemple 433** | 2,6 | 48,2 |
| **Exemple 401** | 8 | 24,3 | **Exemple 434** | 3,5 | 45,7 |
| **Exemple 402** | 26,1 | 241 | **Exemple 435** | 14,9 | 441 |
| **Exemple 403** | 24,2 | 289 | **Exemple 436** | 49,1 | 338 |
| **Exemple 404** | 24,4 | 85,5 | **Exemple 437** | 4,4 | 70,5 |
| **Exemple 406** | 4,2 | 10,6 | **Exemple 438** | 11,3 | 379 |
| **Exemple 407** | 20,6 | 150 | **Exemple 439** | 6,1 | 329 |
| **Exemple 408** | 5,4 | 4,46 | **Exemple 440** | 2,6 | 13,6 |
| **Exemple 409** | 12,2 | 41,9 | **Exemple 441** | 3,5 | 132 |
| **Exemple 410** | 9,9 | 61,6 | **Exemple 442** | 3 | 42,8 |
| **Exemple 411** | 5,5 | 32,8 | **Exemple 443** | ND | 57,9 |
| **Exemple 412** | 3,1 | 0,398 | **Exemple 444** | 5,5 | 181 |
| **Exemple 413** | 3,1 | 9,43 | **Exemple 445** | 2 | 19 |
| **Exemple 415** | 3,8 | 29,9 | **Exemple 446** | 7,8 | 249 |
| **Exemple 416** | 2,9 | 42,5 | **Exemple 447** | 20 | >600 |
| **Exemple 417** | 3 | 11 | **Exemple 448** | 2,4 | 12,1 |
| **Exemple 418** | 10,1 | 159 | **Exemple 449** | 1,7 | 11,9 |
| **Exemple 419** | 2,8 | 15,8 | **Exemple 450** | 2,7 | 53,2 |
| **Exemple 420** | 6,1 | 51,8 | **Exemple 451** | 2,9 | 102 |
| **Exemple 421** | 2,5 | 3,73 | **Exemple 452** | 8,3 | 112 |
| **Exemple 422** | 9,2 | 101 | **Exemple 453** | 6,9 | 99,9 |
| **Exemple 423** | 11,7 | 344 | **Exemple 454** | 6,5 | 158 |
| **Exemple 424** | 13,6 | 102 | **Exemple 455** | 1,9 | 11,8 |
| **Exemple 425** | 4,2 | 62 | **Exemple 456** | 2,7 | 80,6 |
| **Exemple 426** | 5,2 | 139 | **Exemple 457** | ND | 80,4 |
| **Exemple 427** | 2,2 | 23,3 | **Exemple 458** | 10,5 | 197 |
| **Exemple 428** | 3,5 | 59,2 | **Exemple 459** | 2,5 | 5,93 |
| **Exemple 429** | 3,1 | 109 | **Exemple 460** | 2,8 | 22,9 |
| **Exemple 430** | 19,9 | 328 | **Exemple 461** | 3,1 | 18,6 |
| **Exemple 431** | 3 | 89 | **Exemple 462** | 13,3 | 368 |
| **Exemple 463** | ND | 40,8 | **Exemple 466** | ND | 493 |
| **Exemple 464** | 36,6 | 723 | **Exemple 467** | 19,2 | 188 |
| **Exemple 465** | ND | 50,6 | | | |

| | | | | | |
|---|---|---|---|---|---|
| ND : non déterminé | | | | | |

### EXEMPLE C : Induction de l'activité caspase in vivo.

La capacité des composés de l'invention à activer la caspase 3 est évaluée dans un modèle de xénogreffe de cellules leucémiques RS4 ;11.
1.10⁷ cellules RS4 ;11 sont greffées par voie sous-cutanée dans des souris immunodéprimées (souche SCID). 25 à 30 jours après la greffe, les animaux sont traités par voie orale par les différents composés. Seize heures après le traitement, les masses tumorales sont récupérées, lysées et l'activité caspase 3 est mesurée dans les lysats tumoraux.
Cette mesure enzymatique est réalisée en dosant l'apparition d'un produit de clivage fluorigénique (activité DEVDase, Promega). Elle est exprimée sous la forme d'un facteur d'activation correspondant au rapport entre les deux activités caspases : celle pour les souris traitées divisée par celle pour les souris contrôles.

Les résultats montrent que les composés de l'invention sont capables d'induire l'apoptose dans les cellules tumorales RS4 ;11 *in vivo.*

### EXEMPLE D : Quantification de la forme clivée de la caspase 3 in vivo.

La capacité des composés de l'invention à activer la caspase 3 est évaluée dans un modèle de xénogreffe de cellules leucémiques RS4 ;11.
1.10⁷ cellules RS4 ;11 sont greffées par voie sous-cutanée dans des souris immunodéprimées (souche SCID). 25 à 30 jours après la greffe, les animaux sont traités par voie orale par les différents composés. Après le traitement, les masses tumorales sont récupérées, lysées et la forme clivée (activée) de la caspase 3 est quantifiée dans les lysats tumoraux.
Cette quantification est réalisée en utilisant l'essai « Meso Scale Discovery (MSD) ELISA platform » qui dose spécifiquement la forme clivée de la caspase 3. Elle est exprimée sous la forme d'un facteur d'activation correspondant au rapport entre la quantité de caspase 3 clivée chez les souris traitées divisée par la quantité de caspase 3 clivée chez les souris contrôles.

Les résultats montrent que les composés de l'invention sont capables d'induire l'apoptose dans les cellules tumorales RS4 ; 11 *in vivo.*

**Tableau 2 : Facteurs d'activation des caspases (caspase 3 clivée essai MSD dans les tumeurs des souris traitées versus souris contrôles) in vivo, après traitement par voie orale (doses précisées entre parenthèses)**

| Composé testé | Temps au bout duquel est prélevé la tumeur (T) | Facteur d'activation ± SEM (versus contrôle) |
|---|---|---|
| **Exemple 25** | 2 h | 45,7 ± 2,0 (25 mg/kg) |
| **Exemple 28** | 2 h | 72,3 ± 5,4 (12,5 mg/kg) |
| **Exemple 47** | 2 h | 12,3 ± 2,4 (25 mg/kg) |
| **Exemple 61** | 2 h | 76,0 ± 5,2 (12,5 mg/kg) |
| **Exemple 67** | 2 h | 29,8 ± 4,0 (25 mg/kg) |
| **Exemple 71** | 2 h | 46,8 ± 16,1 (25 mg/kg) |
| **Exemple 74** | 2 h | 24,5 ± 7,4 (12,5 mg/kg) |
| **Exemple 108** | 2 h | 22,6 ± 2,4 (12,5 mg/kg) |
| **Exemple 230** | 2 h | 42,2 ± 9,3 (25 mg/kg) |
| **Exemple 386** | 2 h | 52,0 ± 8,6 (12,5 mg/kg) |
| **Exemple 388** | 2 h | 85,7 ± 3,7 (25 mg/kg) |
| **Exemple 421** | 2 h | 38,7 ± 10,7 (12,5 mg/kg) |
| **Exemple 449** | 2 h | 50,5 ± 3,4 (12,5 mg/kg) |

### EXEMPLE E : Activité anti-tumorale in vivo.

L'activité anti-tumorale des composés de l'invention est évaluée dans un modèle de xénogreffe de cellules leucémiques RS4 ;11.
1.10⁷ cellules RS4 ;11 sont greffées par voie sous-cutanée dans des souris immunodéprimées (souche SCID). 25 à 30 jours après la greffe, lorsque la masse tumorale a atteint environ 150 mm³, les souris sont traitées par voie orale par les différents composés dans 2 schémas différents (traitement quotidien pendant cinq jours par semaine durant deux semaines, ou deux traitements par semaine pendant deux semaines). La masse tumorale est mesurée 2 fois par semaine depuis le début du traitement.

Les résultats obtenus montrent donc que les composés de l'invention sont capables d'induire une régression tumorale significative pouvant être complète durant la période de traitement.

### EXEMPLE F : Composition pharmaceutique : Comprimés

| | |
|---|---|
| 1000 comprimés dosés à 5 mg d'un composé choisi parmi les exemples 1 à 467 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 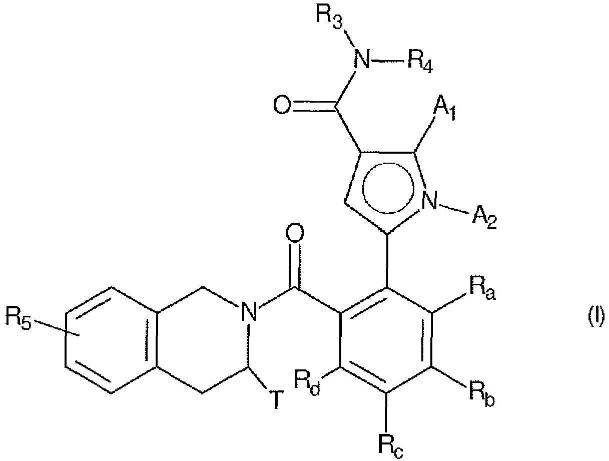 dans laquelle :
◆ A₁ représente un atome d'hydrogène ou d'halogène, un polyhalogénoalkyle (C₁-C₆) linéaire ou ramifié, un groupement alkyle (C₁-C₆) linéaire ou ramifié ou un groupement cycloalkyle,
◆ A₂ représente un groupement alkyle (C₁-C₆) linéaire ou ramifié éventuellement substitué par un groupement choisi parmi halogène, hydroxy, alkoxy (C₁-C₆) linéaire ou ramifié, NR'R" et morpholine, ou bien A₂ représente un polyhalogénoalkyle (C₁-C₆) linéaire ou ramifié ou un groupement cyclopropyle. étant entendu que R' et R" représentent, indépendamment l'un de l'autre, un atome d'hydrogène ou un groupement alkyle (C₁-C₆) linéaire ou ramifié,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ R₁ et R₂ représentent indépendamment l'un de l'autre un atome d'hydrogène ou un groupement alkyle (C₁-C₆) linéaire ou ramifié,
ou bien R₁ et R₂ forment avec l'atome d'azote qui les porte un hétérocycloalkyle,
◆ R₃ représente un groupement aryle ou hétéroaryle, étant entendu qu'un ou plusieurs atomes de carbone des groupements précédents, ou de leurs éventuels substituants, peut(vent) être deutéré(s),
◆ R₄ représente un groupement phényle, 4-hydroxyphényle, 3-fluoro-4-hydroxyphényle, 2-hydroxypyrimidine ou 3-hydroxypyridine, étant entendu qu'un ou plusieurs atomes de carbone des groupements précédents, ou de leurs éventuels substituants, peut(vent) être deutéré(s),
◆ R₅ représente un atome d'hydrogène ou d'halogène, un groupement alkyle (C₁-C₆) linéaire ou ramifié, ou un groupement alkoxy (C₂-C₆) linéaire ou ramifié,
◆ R₆ représente un atome d'hydrogène ou un groupement alkyle (C₁-C₆) linéaire ou ramifié,
◆ Rₐ et R_{d} représentent chacun un atome d'hydrogène et (R_{b},R_{c}) forment ensemble avec les atomes de carbone qui les portent un groupe 1,3-dioxolane, un groupe 1,4-dioxane, ou bien Rₐ, R_{c} et R_{d} représentent chacun un atome d'hydrogène et R_{b} représente un atome d'hydrogène ou d'halogène ou un groupement méthoxy, ou bien Rₐ et R_{d} représentent chacun un atome d'hydrogène, R_{b} représente un atome d'hydrogène ou d'halogène et R_{c} un groupement hydroxy ou méthoxy, ou bien : Rₐ et R_{d} représentent chacun un atome d'hydrogène, R_{b} représente un groupement hydroxy ou méthoxy et R_{c} un atome d'halogène,
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre ou azote (en incluant les azotes quaternaires),
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi alkyle (C₁-C₆) linéaire ou ramifié, spiro (C₃-C₆), alkoxy (C₁-C₆) linéaire ou ramifié, (C₁-C₆)alkyl-S-, hydroxy, oxo (ou N-oxyde le cas échéant), nitro, cyano, -COOR', -OCOR', NR'R", polyhalogénoalkyle (C₁-C₆) linéaire ou ramifié, trifluorométhoxy, (C₁-C₆)alkylsulfonyl, halogène, aryle, hétéroaryle, aryloxy, arylthio, cycloalkyle, hétérocycloalkyle éventuellement substitué par un ou plusieurs atomes d'halogène ou groupements alkyles,
ses énantiomères et diastéréoisomères, ainsi que ses sels d'addition à un acide ou à une base pharmaceutiquement acceptable.

2. Composé de formule (1) selon la revendication 1 dans lequel A₁ représente un atome d'hydrogène ou un groupement méthyle.

3. Composé de formule (I) selon la revendication 1 ou 2 dans lequel A₁ et A₂ représentent tous les deux un groupement méthyle.

4. Composé de formule (I) selon l'une des revendications 1 à 3 dans lequel T représente un méthyle, un groupement aminométhyle, (morpholin-4-yl)méthyle, (4-méthylpipérazin-1-yl)méthyle, 2-(morpholin-4-yl)éthyle, [2-(morpholin-4-yl)éthoxy]méthyle, hydroxyméthyle, [2-(diméthylamino)éthoxy]méthyle, hexahydropyrazino[2,1*-c*][1,4] oxazin-8(1*H*)-ylméthyle, 1-oxa-6-azaspiro[3.3]hept-6-ylméthyle, 3-(morpholin-4-yl)propyle, ou trifluorométhyle.

5. Composé de formule (I) : 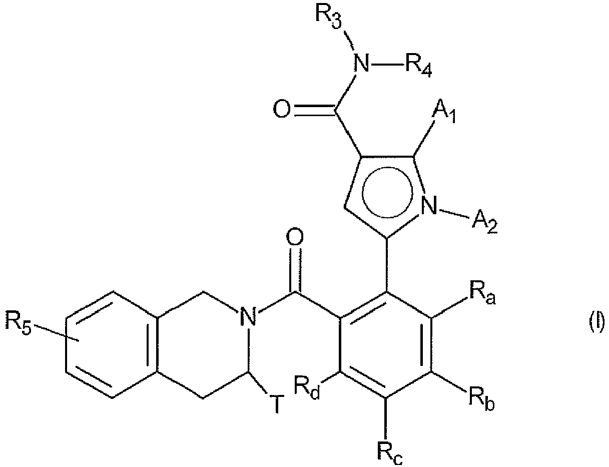 selon la revendication 1 dans lequel :
- A₁, A₂, Rₐ, R_{b}, R_{c}, R_{d}, T, R₄ et R₅ sont tels que définis dans les revendications 1 à 4,
- R₃ représente un groupement choisi parmi phényle, 1*H*-pyrazole, 1*H*-indole, 1*H-*indazole, pyridine, pyrimidine, 1*H*-pyrrolo[2,3-*b*]pyridine, 2,3-dihydro-1*H-*pyrrolo[2,3-*b*]pyridine, 1*H*-benzimidazole, 1*H*-pyrrole, 1*H*-pyrrolo[2,3-*c*]pyridine, 1*H*-pyrrolo[3,2-*b*]pyridine, 5*H*-pyrrolo[3,2-*d*]pyrimidine, thiophène, pyrazine, 1*H-*pyrazolo[3,4-*b*]pyridine, 1,2-oxazole, pyrazolo[1,5-*a*]pyrimidine, ces groupements comportant éventuellement un ou plusieurs substituants choisis parmi halogène, alkyle (C₁-C₆) linéaire ou ramifié, alkoxy (C₁-C₆) linéaire ou ramifié, cyano, cyclopropyle, oxétane, tétrahydrofurane, -CO-O-CH₃, trideutériométhyle, 2-(morpholin-4-yl)éthyle, ou 2-(morpholin-4-yl)éthoxy,
ses énantiomères et diastéréoisomères, ainsi que ses sels d'addition à un acide ou à une base pharmaceutiquement acceptable.

6. Composé de formule (I) selon la revendication 1 choisi parmi le groupe suivant :
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide,
- 5-(5-chloro-2-{[(3*R*)-3-méthyl-3,4-dihydroisoquinolin-2(1*H*)-yl]carbonyl}phényl)-*N*-(4-hydroxyphényl)-1,2-diméthyl-*N*-(pyridin-4-yl)-1*H*-pyrrole-3-carboxamide,
- *N*-(4-hydroxyphényl)-1,2-diméthyl-*N*-(1-méthyl-1*H*-pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide,
- *N*-(4-hydroxyphényl)-1,2-diméthyl-5-(6-{[(3*R*)-3-méthyl-3,4-dihydroisoquinolin-2(1*H*)-yl]carbonyl}-1,3-benzodioxol-5-yl)-*N*-(pyridin-4-yl)-1*H*-pyrrole-3-carboxamide,
- 5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H-*pyrrolo[2,3-*b*]pyridin-5-yl)-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(pyridin-4-yl)-1*H-*pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(5-cyano-1-méthyl-1*H*-pyrrol-3-yl)-*N*-(4-hydroxyphényl)-1,2-diméthyl-1*H*-pyrrole-3-carboxamide,
- *N*-(5-cyano-1-méthyl-1*H*-pyrrol-3-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-2,3-dihydro-1*H*-pyrrolo[2,3*-b*]pyridin-5-yl)-1*H*-pyrrole-3-carboxamide,
- *N*-(5-cyano-1,2-diméthyl-1*H*-pyrrol-3-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide,
- 5-(5-chloro-2-{[(3*S*)-3-(morpholin-4-ylméthyl)-3,4-dihydroisoquinolin-2(1*H*)-yl]carbonyl}phényl)-*N*-[5-cyano-2-méthyl-1-(trideutériométhyl)-1*H*-pyrrol-3-yl]-*N*-(4-hydroxyphényl)-1,2-diméthyl-1*H*-pyrrole-3-carboxamide,
leurs énantiomères et diastéréoisomères, ainsi que leurs sels d'addition à un acide ou à une base pharmaceutiquement acceptable.

7. Composé de formule (I) selon la revendication 6 qui est le 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide.

8. Composé de formule (I) selon la revendication 6 qui est le 5-(5-chloro-2-{[(3*R*)-3-méthyl-3,4-dihydroisoquinolin-2(1*H*)-yl]carbonyl}phényl)-*N*-(4-hydroxyphényl)-1,2-diméthyl-*N*-(pyridin-4-yl)-1*H*-pyrrole-3-carboxamide.

9. Composé de formule (I) selon la revendication 6 qui est le *N*-(4-hydroxyphényl)-1,2-diméthyl-*N*-(1-méthyl-1*H*-pyrazol-4-yl)-5-(6-{[(3*S*)-3-(morpholin-4-ylméthyl)-3,4-dihydroisoquinolin-2(1*H*)-yl]carbonyl}-1,3-benzodioxol-5-yl)-1*H*-pyrrole-3-carboxamide.

10. Composé de formule (I) selon la revendication 6 qui est le *N*-(4-hydroxyphényl)-1,2-diméthyl-5-(6-{[(3*R*)-3-méthyl-3,4-dihydroisoquinolin-2(1*H*)-yl]carbonyl}-1,3-benzodioxol-5-yl)-*N*-(pyridin-4-yl)-1*H*-pyrrole-3-carboxamide.

11. Composé de formule (I) selon la revendication 6 qui est le 5-(5-fluoro-2-{[(3S)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H*-pyrazol-4-yl)-1*H*-pyrrole-3-carboxamide.

12. Composé de formule (I) selon la revendication 6 qui est le 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(1-méthyl-1*H*-pyrrolo[2,3-*b*]pyridin-5-yl)-1*H*-pyrrole-3-carboxamide.

13. Composé de formule (I) selon la revendication 6 qui est le 5-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-*N*-(pyridin-4-yl)-1*H*-pyrrole-3-carboxamide.

14. Composé de formule (I) selon la revendication 6 qui est le 5-(5-chloro-2-{[(3*S*)-3-(morpholin-4-ylméthyl)-3,4-dihydroisoquinolin-2(1*H*)-yl]carbonyl}phényl)-*N*-(5-cyano-1-méthyl-1*H-*pyrrol-3-yl)-*N*-(4-hydroxyphényl)-1,2-diméthyl-1*H*-pyrrole-3-carboxamide.

15. Composé de formule (I) selon la revendication 6 qui est le *N*-(5-cyano-1-méthyl-1*H-*pyrrol-3-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide.

16. Composé de formule (I) selon la revendication 6 qui est le 5-(5-chloro-2-{[(3*S*)-3-(morpholin-4-ylméthyl)-3,4-dihydroisoquinolin-2(1*H*)-yl]carbonyl}phényl)-*N*-(5-cyano-1,2-diméthyl-1*H*-pyrrol-3-yl)-*N*-(4-hydroxyphényl)-1,2-diméthyl-1*H*-pyrrole-3-carboxamide.

17. Composé de formule (I) selon la revendication 6 qui est le 5-(5-chloro-2-{[(3*S*)-3-(morpholin-4-ylméthyl)-3,4-dihydroisoquinolin-2(1*H*)-yl]carbonyl)phényl)-*N*-(4-hydroxyphényl)-1,2-diméthyl-*N*-(1-méthyl-2,3-dihydro-1*H*-pyrrolo[2,3-*b*]pyridin-5-yl)-1*H*-pyrrole-3-carboxamide.

18. Composé de formule (I) selon la revendication 6 qui est le *N*-(5-cyano-1,2-diméthyl-1*H*-pyrrol-3-yl)-5-(5-fluoro-2-{[(3*S*)-3-(morpholin-4-ylméthyl)-3,4-dihydroisoquinolin-2(1*H*)-yl]carbonyl}phényl)-*N*-(4-hydroxyphényl)-1,2-diméthyl-1*H*-pyrrole-3-carboxamide.

19. Composé de formule (I) selon la revendication 6 qui est le 5-(5-chloro-2-{[(3*S*)-3-(morpholin-4-ylméthyl)-3,4-dihydroisoquinolin-2(1*H*)-yl]carbonyl}phényl)-*N*-[5-cyano-2-méthyl-1-(trideutériométhyl)-1*H*-pyrrol-3-yl]-*N*-(4-hydroxyphényl)-1,2-diméthyl-1*H-*pyrrole-3-carboxamide.

20. Procédé de préparation des composés de formule (I) selon la revendication 1 **caractérisé en ce que** l'on utilise comme produit de départ le composé de formule (II) : 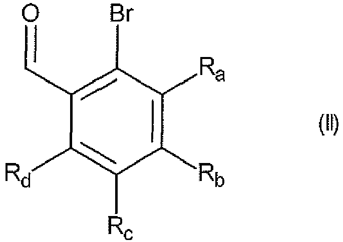 dans laquelle Rₐ, R_{b}, R_{c} et R_{d} sont tels que définis dans la formule (I),
composé de formule (II) qui est soumis à une réaction de Heck, en milieu aqueux ou organique, en présence d'un catalyseur au palladium, d'une base, d'une phosphine et du composé de formule (III) : 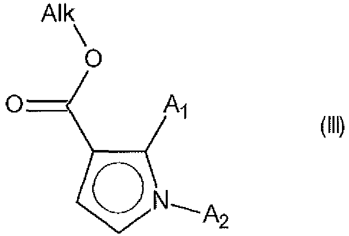 dans laquelle les groupements A₁ et A₂ sont tels que définis dans la formule (I) et Alk représente un alkyle (C₁-C₆) linéaire ou ramifié,
pour obtenir le composé de formule (IV) : 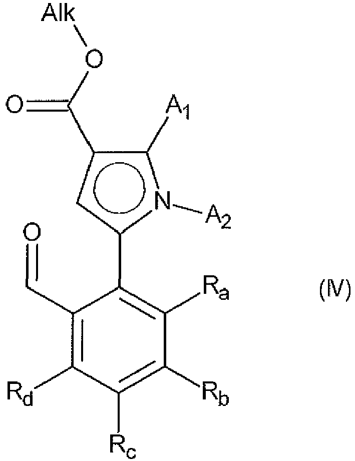 dans laquelle A₁, A₂, Rₐ, R_{b}, R_{c} et R_{d} sont tels que définis dans la formule (I) et Alk est tel que défini précédemment,
composé de formule (IV) dont la fonction aldéhyde est oxydée en acide carboxylique pour former le composé de formule (V) : 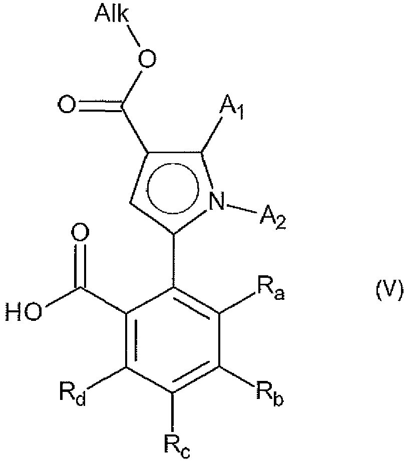 dans laquelle A₁, A₂, Rₐ, R_{b}, R_{c} et R_{d} sont tels que définis dans la formule (I) et Alk est tel que défini précédemment,
composé de formule (V) qui subit ensuite un couplage peptidique avec un composé de formule (VI) : 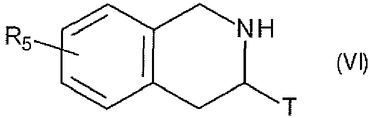 dans laquelle T et R₅ sont tels que définis dans la formule (I),
pour conduire au composé de formule (VII) : 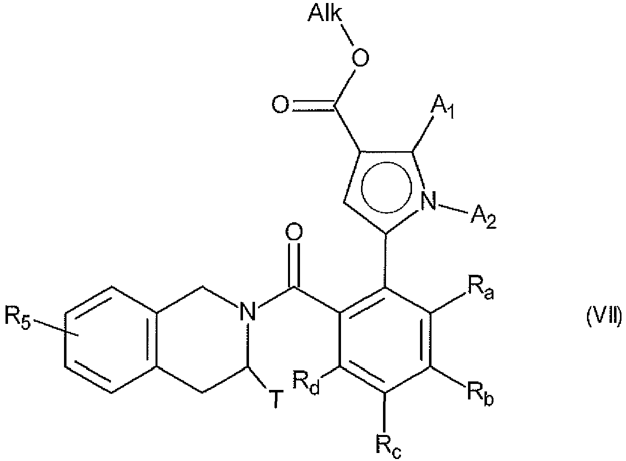 dans laquelle A₁, A₂, Rₐ, R_{b}, R_{c}, R_{d}, T et R₅ sont tels que définis dans la formule (I) et Alk est tel que défini précédemment,
composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

21. Procédé de préparation selon la revendication 20 d'un composé de formule (I) dans lequel l'un des groupements R₃ ou R₄ est substitué par une fonction hydroxy **caractérisé en ce que** l'amine NHR₃R₄ est préalablement soumise à une réaction de protection de la fonction hydroxy avant tout couplage avec l'acide carboxylique du composé de formule (VII), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

22. Composition pharmaceutique contenant un composé de formule (I) selon l'une quelconque des revendications 1 à 19 ou un de ses sels d'addition avec un acide ou une base pharmaceutiquement acceptable en combinaison avec un ou plusieurs excipients pharmaceutiquement acceptables.

23. Composition pharmaceutique selon la revendication 22 pour son utilisation en tant qu'agent pro-apoptotique.

24. Composition pharmaceutique selon la revendication 22 pour son utilisation dans le traitement des cancers, des maladies auto-immunes et du système immunitaire.

25. Composition pharmaceutique selon la revendication 22 pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

26. Utilisation d'une composition pharmaceutique selon la revendication 22 pour la fabrication d'un médicament utile en tant qu'agent pro-apoptotique.

27. Utilisation d'une composition pharmaceutique selon la revendication 22 pour la fabrication d'un médicament destiné au traitement des cancers, des maladies immunitaires et auto-immunes.

28. Utilisation d'une composition pharmaceutique selon la revendication 22 pour la fabrication d'un médicament destiné au traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

29. Composé de formule (1) selon l'une des revendications 1 à 19, ou un de ses sels d'addition avec un acide ou une base pharmaceutiquement acceptable, pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

30. Utilisation d'un composé de formule (I) selon l'une des revendications 1 à 19, ou un de ses sels d'addition avec un acide ou une base pharmaceutiquement acceptable, pour la fabrication d'un médicament destiné au traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes non hodgkiniens, des mélanomes, des hémopathies malignes, des myélomes, du cancer de l'ovaire, du cancer du poumon non à petites cellules, du cancer de la prostate et du cancer du poumon à petites cellules.

31. Association d'un composé de formule (I) selon l'une quelconque des revendications 1 à 19 avec un agent anticancéreux choisi parmi les agents génotoxiques, les poisons mitotiques, les anti-métabolites, les inhibiteurs du protéasome, les inhibiteurs de kinases ou les anticorps.

32. Composition pharmaceutique contenant une association selon la revendication 31 en combinaison avec un ou plusieurs excipients pharmaceutiquement acceptables.

33. Association selon la revendication 31 pour son utilisation dans le traitement des cancers.

34. Utilisation d'une association selon la revendication 31 pour la fabrication d'un médicament utile dans le traitement des cancers.

35. Composé de formule (I) selon l'une quelconque des revendications 1 à 19 pour son utilisation en association avec une radiothérapie dans le traitement des cancers.

## Patentansprüche

1. Verbindung der Formel (I): 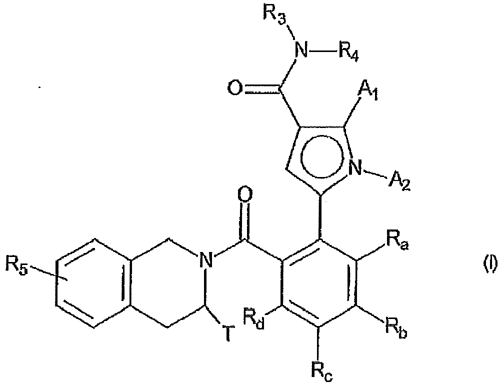 in der:
◆ A₁ ein Wasserstoff- oder Halogenatom, eine geradkettige oder verzweigte (C₁-C₆)-Polyhalogenalkylgruppe, eine geradkettige oder verzweigte (C₁-C₆)-Alkylgruppe oder eine Cycloalkylgruppe bedeutet,
◆ A₂ eine geradkettige oder verzweigte, gegebenenfalls durch eine Gruppe ausgewählt aus Halogen, Hydroxy, geradkettigem oder verzweigtem (C₁-C₆)-Alkoxy, NR'R" und Morpholin substituierte (C₁-C₆)-Alkylgruppe bedeutet oder A₂ eine geradkettige oder verzweigte (C₁-C₆)-Polyhalogenalkylgruppe oder eine Cyclopropylgruppe bedeutet,
wobei es sich versteht, dass R' und R" unabhängig voneinander ein Wasserstoffatom oder eine geradkettige oder verzweigte (C₁-C₆)-Alkylgruppe darstellen,
◆ T ein Wasserstoffatom, eine geradkettige oder verzweigte, gegebenenfalls durch ein bis drei Halogenatome substituierte (C₁-C₆)-Alkylgruppe, eine (C₁-C₄)-Alkyl-NR₁R₂- oder eine (C₁-C₄)-Alkyl-OR₆-gruppe bedeutet,
◆ R₁ und R₂ unabhängig voneinander ein Wasserstoffatom oder eine geradkettige oder verzweigte (C₁-C₆)-Alkylgruppe bedeuten,
oder R₁ und R₂ gemeinsam mit dem sie tragenden Stickstoffatom eine Heterocycloalkylgruppe bilden,
◆ R₃ eine Aryl- oder Heteroarylgruppe bedeutet, wobei es sich versteht, dass eines oder mehrere Kohlenstoffatome der vorhergehenden Gruppen oder ihrer eventuellen Substituenten deuteriert sein können,
◆ R₄ eine Phenyl-, 4-Hydroxyphenyl-, 3-Fluor-4-hydroxyphenyl-, 2-Hydroxypyrimidin- oder 3-Hydroxypyridin-gruppe bedeutet, wobei es sich versteht, dass eines oder mehrere Kohlenstoffatome der vorhergehenden Gruppen oder ihrer eventuellen Substituenten deuteriert sein können,
◆ R₅ ein Wasserstoff- oder Halogenatom, eine geradkettige oder verzweigte (C₁-C₆)-Alkylgruppe oder eine geradkettige oder verzweigte (C₁-C₆)-Alkoxygruppe bedeutet,
◆ R₆ Wasserstoffatom oder eine geradkettige oder verzweigte (C₁-C₆)-Alkylgruppe bedeutet,
◆ Rₐ und R_{d} jeweils ein Wasserstoffatom bedeuten und (R_{b},R_{c}) gemeinsam mit den sie tragenden Kohlenstoffatomen eine 1,3-Dioxolangruppe oder eine 1,4-Dioxangruppe bilden, oder Rₐ, R_{c} und R_{d} jeweils ein Wasserstoffatom bedeuten und R_{b} ein Wasserstoff- oder Halogenatom oder eine Methoxygruppe bedeutet, oder Rₐ und R_{d} jeweils ein Wasserstoffatom bedeuten, R_{b} ein Wasserstoff- oder Halogenatom bedeutet und R_{c} eine Hydroxy- oder Methoxygruppe, oder : Rₐ und R_{d} jeweils ein Wasserstoffatom bedeuten, R_{b} eine Hydroxy- oder Methoxygruppe und R_{c} ein Halogenatom bedeuten,
wobei es sich versteht, dass:
- man unter "Aryl" eine Phenyl-, Naphthyl-, Biphenyl- oder Indenylgruppe versteht,
- man unter "Heteroaryl" jede mono- oder bicyclische Gruppe versteht, die aus 5 bis 10 Kettengliedern gebildet ist, mindestens einen aromatischen Teil aufweist und 1 bis 4 Heteroatome ausgewählt aus Sauerstoff, Schwefel oder Stickstoff (einschließlich quaternären Stickstoff) enthält,
- man unter "Cycloalkyl) jede nichtaromatische, mono- oder bicyclische carbocyclische Gruppe mit 3 bis 10 Kettengliedern versteht,
- man unter "Heterocycloalkyl" jede nichtaromatische, mono- oder bicyclische, kondensierte oder spirogebundene Gruppe versteht, die aus 3 bis 10 Kettengliedern gebildet ist und 1 bis 3 Heteroatome ausgewählt aus Sauerstoff, Schwefel, SO, SO₂ oder Stickstoff enthält,
wobei die in dieser Weise definierten Aryl-, Heteroaryl-, Cycloalkyl- und Heterocycloalkylgruppen und die Alkyl-, Alkenyl-, Alkinyl- und Alkoxygruppen durch 1 bis 3 Gruppen ausgewählt aus geradkettigem oder verzweigtem (C₁-C₆)-Alkyl, (C₃-C₆)-spiro, geradkettigem oder verzweigtem (C₁-C₆)-Alkoxy, (C₁-C₆)-Alkyl-S-, Hydroxy, Oxo (oder gegebenenfalls *N*-Oxid), Nitro, Cyano, -COOR', -OCOR', NR'R", geradkettigem oder verzweigtem (C₁-C₆)-Polyhalogenalkyl, Trifluormethoxy, (C₁-C₆)-Alkylsulfonyl, Halogen, Aryl, Heteroaryl, Aryloxy, Arylthio, Cycloalkyl und Heterocycloalkyl, gegebenenfalls substituiert durch ein oder mehrere Halogenatome oder Alkylgruppen, substituiert sein können,
ihre Enantiomeren und Diastereoisomeren sowie ihre Additionssalze mit einer pharmazeutisch annehmbaren Säure oder Base.

2. Verbindung der Formel (I) nach Anspruch 1, worin A₁ ein Wasserstoffatom oder eine Methylgruppe bedeutet.

3. Verbindung der Formel (I) nach Anspruch 1 oder 2, worin A₁ und A₂ jeweils eine Methylgruppe bedeuten.

4. Verbindung der Formel (I) nach einem der Ansprüche 1 bis 3, worin T Methyl, eine Aminomethyl-, (Morpholin-4-yl)-methyl-, (4-Methylpiperazin-1-yl)-methyl-, 2-(Morpholin-4-yl)-ethyl-, [2-(Morpholin-4-yl)-ethoxy]-methyl-, Hydroxymethyl-, [2-(Dimethylamino)-ethoxy]-methyl-, Hexahydropyrazino[2,1-*c*][1,4]oxazin-8(1*H*)-ylmethyl-, 1-Oxa-6-azaspiro[3,3]hept-6-ylmethyl-, 3-(Morpholin-4-yl)-propyl- oder Trifluormethylgruppe bedeutet.

5. Verbindung der Formel (I): 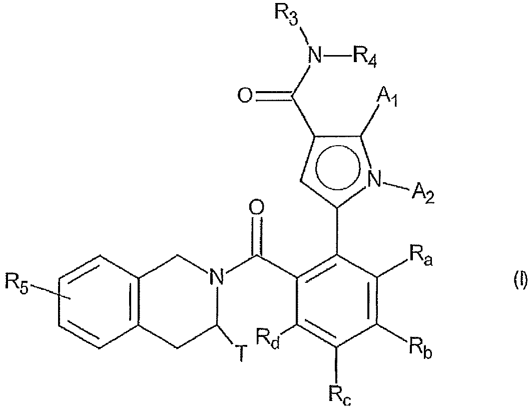 nach Anspruch 1, worin:
- A₁, A₂, Rₐ, R_{b}, R_{c}, R_{d}, T, R₄ und R₅ die in den Ansprüchen 1 bis 4 angegebenen Bedeutungen besitzen,
- R₃ eine Gruppe bedeutet ausgewählt aus Phenyl, 1*H*-Pyrazol, 1*H*-Indol, 1*H-*Indazol, Pyridin, Pyrimidin, 1*H*-Pyrrolo[2,3-*b*]pyridin, 2,3-Dihydro-1*H-*pyrrolo[2,3-b]pyridin, 1*H*-Benzimidazol, 1*H*-Pyrrol, 1*H*-pyrrolo[2,3-*c*]pyridin, 1*H*-Pyrrolo[3,2-*b*]pyridin, 5*H*-Pyrrolo[3,2-*d*]pyrimidin, Thiophen, Pyrazin, 1*H-*Pyrazolo[3,4-b]pyridin, 1,2-Oxazol, Pyrazolo[1,5-*a*]pyrimidin, wobei diese Gruppen gegebenenfalls einen oder mehrere Substituenten aufweisen ausgewählt aus Halogen, geradkettigem oder verzweigtem (C₁-C₆)-Alkyl, geradkettige oder verzweigtem (C₁-C₆)-Alkoxy, Cyano, Cyclopropyl, Oxetan, Tetrahydrofuran,-CO-O-CH₃, Trideuteriomethyl, 2-(Morpholin-4-yl)-ethyl oder 2-(Morpholin-4-yl)-ethoxy,
ihre Enantiomere und Diastereoisomere sowie ihre Additionssalze mit einer pharmazeutisch annehmbaren Säure oder Base.

6. Verbindung der Formel (I) nach Anspruch 1, ausgewählt aus der folgenden Gruppe:
- 5-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(1-methyl-1*H*-pyrazol-4-yl)-1*H-*pyrrol-3-carboxamid,
- 5-(5-Chlor-2-{[(*3R*)-3*-*methyl-3,4-dihydroisochinolin-2(1*H*)-yl]-carbonyl}-phenyl)-*N*-(4-hydroxyphenyl)-1,2-dimethyl-*N*-(pyridin-4-yl)-1*H-*pyrrol-3-carboxamid,
- *N*-(4-Hydroxyphenyl)-1,2-dimethyl-*N*-(1-methyl-1*H*-pyrazol-4-yl)-5-(6-{[(3*S*)-3-(morpholin-4-ylmethyl)-3,4-dihydroisochinolin-2(1*H*)-yl]-carbonyl}-1,3-benzodioxol-5-yl)-1*H*-pyrrol-3-carboxamid,
- *N-(*4-Hydroxyphenyl)-1,2-dimethyl-5-(6-{[(3*R*)-3-methyl-3,4-dihydroisochinolin-2(1*H*)-yl]-carbonyl}-1,3-benzodioxol-5-yl)-*N*-(pyridin-4-yl)-1*H*-pyrrol-3-carboxamid,
- 5-(5-Fluor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(1-methyl-1*H-*pyrazol-4-yl)-1*H*-pyrrol-3-carboxamid,
- 5-(5-Chlor-2-{[(3*S)-*3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(1-methyl-1*H-*pyrrolo[2,3-*b*]pyridin-5-yl)-1*H*-pyrrol-3-carboxamid,
- 5-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(pyridin-4-yl)-1*H-*pyrrol-3-carboxamid,
- 5-(5-Chlor-2-{[(3*S*)-3-(morpholin-4-ylmethyl)-3,4-dihydroisochinolin-2(1*H)-*yl]-carbonyl}-phenyl)-*N*-(5-cyano-1-methyl-1*H-*pyrrol-3-yl)-*N*-(4-hydroxyphenyl)-1,2-dimethyl-1*H*-pyrrol-3-carboxamid,
- *N*-(5-Cyano-1-methyl-1*H*-pyrrol-3-yl)-5-(5-fluor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-1*H*-pyrrol-3-carboxamid,
- 5-(5-Chlor-2-{[(3*S*)-3-(morpholin-4-ylmethyl)-3,4-dihydroisochinolin-2(1*H*)-yl]-carbonyl}-phenyl)-*N*-(5-cyano-1,2-dimethyl-1*H*-pyrrol-3-yl)-*N*-(4-hydroxyphenyl)-1,2-dimethyl-1*H-*pyrrol-3-carboxamid,
- 5-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(1-methyl-2,3-dihydro-1*H*-pyrrolo[2,3-*b*]pyridin-5-yl)-1*H*-pyrrol-3-carboxamid,
- *N*-(5-Cyano-1,2-dimethyl-1*H*-pyrrol-3-yl)-5-(5-fluor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-1*H-*pyrrol-3-carboxamid,
- 5-(5-Chlor-2-{[(3*S*)-3-(morpholin-4-ylmethyl)-3,4-dihydroisochinolin-2(1*H*)-yl]-carbonyl}-pheny)-*N*-[5-cyano-2-methyl-1-(trideuteriomethyl)-1*H*-pyrrol-3-yl]-*N-*(4-hydroxyphenyl)-1,2-dimethyl-1*H*-pyrrol-3-carboxamid,
deren Enantiomere und Diastereoisomere sowie deren Additionssalze mit einer pharmazeutisch annehmbaren Säure oder Base.

7. Verbindung der Formel I) nach Anspruch 6, nämlich 5-(5-Chlor-2-{[(3*S*)-3-(morpholin-4-ylmethyl)-3,4-dihydroisochinolin-2(1*H*)-yl]-carbonyl}-phenyl)-*N*-(4-hydroxymethyl)-1,2-dimethyl-*N*-(1-methyl-1*H-*pyrazol-4-yl)-1*H-*pyrrol-3-carboxamid.

8. Verbindung der Formel (I) nach Anspruch 6, nämlich 5-(5-Chlor-2-{[(3*R*)-3-methyl-3,4-dihydroisochinolin-2-(1*H*)-yl]-carbonyl}-phenyl)-*N*-(4-hydroxyphenyl)-1,2-dimethyl-*N-*(pyridin-4-yl)-1*H-*pyrrol-3-carboxamid.

9. Verbindung der Formel (I) nach Anspruch 6, nämlich *N*-(4-Hydroxyphenyl)-1,2-dimethyl-*N*-(1-methyl-1*H*-pyrazol-4-yl)-5-(6-{[(3*S*)-3-(morpholin-4-ylmethyl)-3,4-dihydroisochinolin-2(1*H*)-yl]-carbonyl}-1,3-benzodioxol-5-yl)-1*H-*pyrrol-3-carboxamid.

10. Verbindung der Formel (I) nach Anspruch 6, nämlich *N*-(4-Hydroxyphenyl)-1,2-dimethyl-5-(6-{[(3*R*)-3-methyl-3,4-dihyroisochinolin-2(1*H*)-yl]-carbonyl}-1,3-benzodioxol-5-yl)-*N*-(pyridin-4-yl)-1*H-*pyrrol-3-carboxamid.

11. Verbindung der Formel (I) nach Anspruch 6, nämlich 5-(5-Fluor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(1-methyl-1*H-*pyrazol-4-yl)-1*H*-pyrrol-3-carboxamid.

12. Verbindung der Formel (I) nach Anspruch 6, nämlich 5-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(1-methyl-1*H*-pyrrolo[2,3-*b*]pyridin-5-yl)-1*H*-pyrrol-3-carboxamid.

13. Verbindung der Formel (I) nach Anspruch 6, nämlich 5-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(pyridin-4-yl)-1*H-*pyrrol-3-carboxamid.

14. Verbindung der Formel (I) nach Anspruch 6, nämlich 5-(5-Chlor-2-{[(3*S*)-3-(morpholin-4-ylmethyl)-3,4-dihydroisochinolin-2(1*H*)-yl]-carbonyl}-phenyl)-*N*-(5-cyano-1-methyl-1*H-*pyrrol-3-yl)-*N*-(4-hydroxyphenyl)-1,2-dimethyl-1*H*-pyrrol-3-carboxamid.

15. Verbindung der Formel (I) nach Anspruch 6, nämlich *N*-(5-Cyano-1-methyl-1*H-*pyrrol-3-yl)-5-(5-fluor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-1*H*-pyrrol-3-carboxamid.

16. Verbindung der Formel (I) nach Anspruch 6, nämlich 5-(5-Chlor-2-{[(3*S*)-3-(morpholin-4-ylmethyl)-3,4-dihydroisochinolin-2(1*H*)-yl]-carbonyl}-phenyl)-*N*-(5-cyano-1,2-dimethyl-1*H*-pyrrol-3-yl)-*N*-(4-hydroxyphenyl)-1,2-dimethyl-1*H*-pyrrol-3-carboxamid.

17. Verbindung der Formel (I) nach Anspruch 6, nämlich 5-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-*N*-(1-methyl-2,3-dihydro-1*H*-pyrrolo[2,3-*b*]pyridin-5-yl)-1*H*-pyrrol-3-carboxamid.

18. Verbindung der Formel (I) nach Anspruch 6, nämlich *N*-(5-Cyano-1,2-dimethyl-1*H-*pyrrol-3-yl)-5-(5-fluor-2-{[(3*S*)-3-(morpholin-4-ylmethyl)-3,4-dihydroisochinolin-2(1*H*)-yl]-carbonyl}-phenyl)-*N*-(4-hydroxyphenyl)-1,2-dimethyl-1*H*-pyrrol-3-carboxamid.

19. Verbindung der Formel (I) nach Anspruch 6, nämlich 5-(5-Chlor-2-{[(3*S*)-3-(morpholin-4-ylmethyl)-3,4-dihydroisochinolin-2(1*H*)-yl]-carbonyl}-phenyl)-*N*-[5-cyano-2-methyl-1-(trideuteriomethyl)-1*H*-pyrrol-3-yl]-*N*-(4-hydroxyphenyl)-1,2-dimethyl-1*H-*pyrrol-3-carboxamid.

20. Verfahren zur Herstellung der Verbindungen der Formel (I) nach Anspruch 1, **dadurch gekennzeichnet, dass** man als Ausgangsprodukt die Verbindung der Formel (II) verwendet: 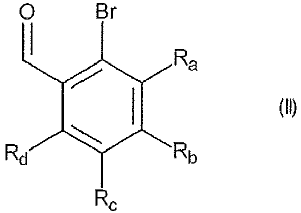 in der Rₐ, R_{b}, Rₑ und R_{d} die bezüglich der Formel (I) angegebenen Bedeutungen besitzen,
welche Verbindung der Formel (II) einer Heck-Reaktion in wässrigem oder organischem Medium in Gegenwart eines Palladiumkatalysators, einer Base, eines Phosphins und der Verbindung der Formel (III): 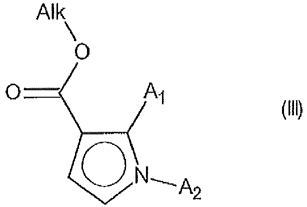 in der die Gruppen A₁ und A₂ die bezüglich der Formel (I) angegebenen Bedeutungen besitzen und Alk eine geradkettige oder verzweigte (C₁-C₆)-Alkylgruppe bedeutet,
unterworfen wird zur Bildung der Verbindung der Formel (IV): 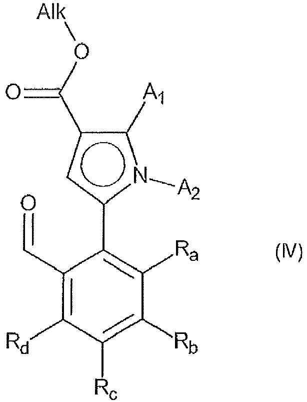 in der A₁, A₂, Rₐ, R_{b}, Rₑ und R_{d} die bezüglich der Formel (I) angegebenen Bedeutungen besitzen und Alk die oben angegebene Bedeutung aufweist,
die Aldehydfunktion der Verbindung der Formel (IV) zu der Carbonsäure oxidiert wird zur Bildung der Verbindung der Formel (V): 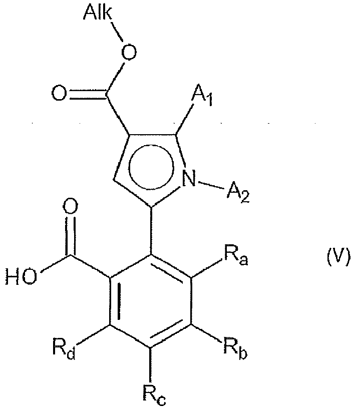 in der A₁, A₂, Rₐ, R_{b}, Rₑ und R_{d} die bezüglich der Formel (I) angegebenen Bedeutungen besitzen und Alk die oben angegebene Bedeutung aufweist,
welche Verbindung der Formel (V) anschließend einer Peptidkupplung mit einer Verbindung der Formel (VI): 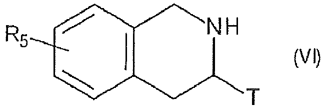 in der T und R₅ die bezüglich der Formel (I) angegebenen Bedeutungen besitzen,
unterworfen wird zur Bildung der Formel (VII): 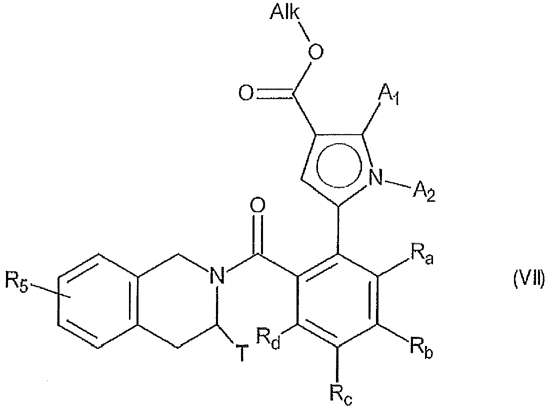 in der A₁, A₂, Rₐ, R_{b}, R_{c}, R_{d}, T und R₅ die bezüglich der Formel (I) angegebenen Bedeutungen besitzen und Alk die oben angegebene Bedeutung aufweist,
die Esterfunktion der Verbindung der Formel (VII) hydrolysiert wird zur Bildung der entsprechenden Carbonsäure oder des entsprechenden Carboxylats, welche(s) in ein Säurederivat wie das entsprechende Acylchlorid oder Anhydrid umgewandelt wird, vor der Kupplung mit einem Amin NHR₃R₄, worin R₃ und R₄ die bezüglich der Formel (I) angegebenen Bedeutungen besitzen, zur Bildung der Verbindung der Formel (I), welche Verbindung der Formel (I) mit Hilfe einer klassischen Trennmethode gereinigt werden kann, welche man gewünschtenfalls in ihre Additionssalze mit einer pharmazeutisch annehmbaren Säure oder Base umwandelt und die man gegebenenfalls mit Hilfe einer klassischen Trennmethode in ihre Isomeren auftrennt,
wobei es sich versteht, dass zu jedem geeigneten Zeitpunkt im Verlaufe des oben beschriebenen Verfahrens bestimmte Gruppen (Hydroxy, Amino...) der Reaktionsteilnehmer oder Synthesezwischenprodukte zum Zwecke der Synthese geschützt und dann wieder von ihren Schutzgruppen befreit werden können.

21. Verfahren nach Anspruch 20 zur Herstellung einer Verbindung der Formel (I), bei der eine der Gruppen R₃ oder R₄ durch eine Hydroxyfunktion substituiert ist, **dadurch gekennzeichnet, dass** das Amin NHR₃R₄ zuvor einer Reaktion zum Schutz der Hydroxygruppe unterworfen wird vor der Kupplung mit der Carbonsäure der Verbindung der Formel (VII) oder einem entsprechenden Säurederivat, worauf die gebildete geschützte Verbindung der Formel (I) einer Reaktion zur Abspaltung der Schutzgruppe unterzogen wird und gegebenenfalls in eines ihrer Additionssalze mit einer pharmazeutisch annehmbaren Säure oder Base umgewandelt wird.

22. Pharmazeutische Zubereitung enthaltend eine Verbindung der Formel (I) nach einem der Ansprüche 1 bis 19 oder eines ihrer Additionssalze mit einer pharmazeutisch annehmbaren Säure oder Base in Kombination mit einem oder mehreren pharmazeutisch annehmbaren Hilfsstoffen.

23. Pharmazeutische Zubereitung nach Anspruch 22 zur Verwendung als pro-apoptotisches Mittel.

24. Pharmazeutische Zubereitung nach Anspruch 22 zur Verwendung bei der Behandlung von Krebserkrankungen, Autoimmunkrankheiten und Erkrankungen des Immunsystems.

25. Pharmazeutische Zubereitung nach Anspruch 22 zur Verwendung bei der Behandlung von Blasenkrebs, Gehirnkrebs, Brustkrebs, Uteruskrebs, chronisch-lymphatischen Leukämien, Kolorektalkrebs, Speiseröhrenkrebs, Leberkrebs, lymphoblastischen Leukämien, Nicht-Hodgkin-Lymphomen, Melanomen, bösartigen Hämopathien, Myelomen, Ovarialkrebs, nicht-kleinzelligem Lungenkrebs, Prostatakrebs und kleinzelligem Lungenkrebs.

26. Verwendung einer pharmazeutischen Zubereitung nach Anspruch 22 zur Herstellung eines Arzneimittels, das als pro-apoptotisches Mittel nützlich ist.

27. Verwendung einer pharmazeutischen Zubereitung nach Anspruch 22 zur Herstellung eines Arzneimittels, das zur Behandlung von Krebs-, Immun- und Autoimmunerkrankungen bestimmt ist.

28. Verwendung einer pharmazeutischen Zubereitung nach Anspruch 22 zur Herstellung eines Arzneimittels zur Behandlung von Blasenkrebs, Gehirnkrebs, Brustkrebs, Uteruskrebs, chronisch-lymphatischen Leukämien, Kolorektalkrebs, Speiseröhrenkrebs, Leberkrebs, lymphoblastischen Leukämien, Nicht-Hodgkin-Lymphomen, Melanomen, bösartigen Hämopathien, Myelomen, Ovarialkrebs, nicht-kleinzelligem Lungenkrebs, Prostatakrebs und kleinzelligem Lungenkrebs.

29. Verbindung der Formel (I) nach einem der Ansprüche 1 bis 19 oder eines ihrer Additionssalze mit einer pharmazeutisch annehmbaren Säure oder Base zur Verwendung bei der Behandlung von Blasenkrebs, Gehirnkrebs, Brustkrebs, Uteruskrebs, chronisch-lymphatischen Leukämien, Kolorektalkrebs, Speiseröhrenkrebs, Leberkrebs, lymphoblastischen Leukämien, Nicht-Hodgkin-Lymphomen, Melanomen, bösartigen Hämopathien, Myelomen, Ovarialkrebs, nicht-kleinzelligem Lungenkrebs, Prostatakrebs und kleinzelligem Lungenkrebs.

30. Verwendung einer Verbindung der Formel (I) nach einem der Ansprüche 1 bis 19 oder eines ihrer Additionssalze mit einer pharmazeutisch annehmbaren Säure oder Base zur Herstellung eines Arzneimittels zur Behandlung von Blasenkrebs, Gehirnkrebs, Brustkrebs, Uteruskrebs, chronisch-lymphatischen Leukämien, Kolorektalkrebs, Speiseröhrenkrebs, Leberkrebs, lymphoblastischen Leukämien, Nicht-Hodgkin-Lymphomen, Melanomen, bösartigen Hämopathien, Myelomen, Ovarialkrebs, nicht-kleinzelligem Lungenkrebs, Prostatakrebs und kleinzelligem Lungenkrebs.

31. Kombination einer Verbindung der Formel (I) nach einem der Ansprüche 1 bis 19 mit einem Antikrebsmittel ausgewählt aus genotoxischen Mitteln, mitotischen Giften, Anti-Metalboliten, Proteasominhibitoren, Kinaseinhibitoren oder Antikörpern.

32. Pharmazeutsche Zubereitung enthaltend eine Kombination nach Anspruch 31 in Kombination mit einem oder mehreren pharmazeutisch annehmbaren Hilfsstoffen.

33. Kombination nach Anspruch 31 zur Verwendung bei der Behandlung von Krebserkrankungen.

34. Verwendung einer Kombination nach Anspruch 31 zur Herstellung eines Arzneimittels zur Behandlung von Krebserkrankungen.

35. Verbindung der Formel (I) nach einem der Ansprüche 1 bis 19 zur Verwendung in Kombination mit einer Strahlentherapie bei der Behandlung von Krebserkrankungen.

## Claims

1. Compound of formula (I): 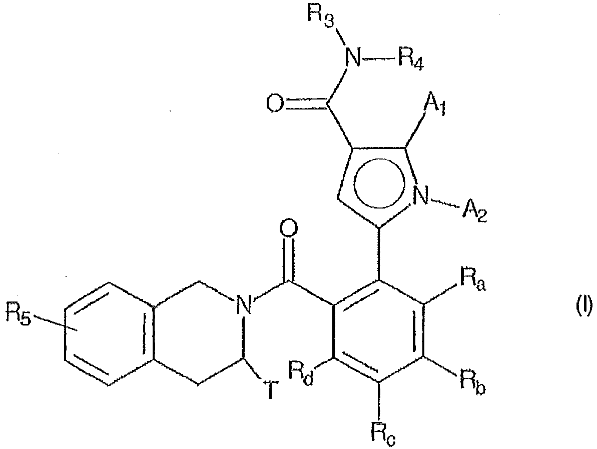 wherein:
◆ A₁ represents a hydrogen or halogen atom, a linear or branched (C₁-C₆)polyhaloalkyl group, a linear or branched (C₁-C₆)alkyl group or a cycloalkyl group,
◆ A₂ represents a linear or branched (C₁-C₆)alkyl group optionally substituted by a group selected from halogen, hydroxy, linear or branched (C₁-C₆)alkoxy, NR'R" and morpholine, or A₂ represents a linear or branched (C₁-C₆)polyhaloalkyl group or a cyclopropyl group,
it being understood that R' and R", each independently of the other, represent a hydrogen atom or a linear or branched (C₁-C₆)alkyl group,
◆ T represents a hydrogen atom, a linear or branched (C₁-C₆)alkyl group optionally substituted by from one to three halogen atoms, a group (C₁-C₄)alkyl-NR₁R₂, or a group (C₁-C₄)alkyl-OR₆,
◆ R₁ and R₂, each independently of the other, represent a hydrogen atom or a linear or branched (C₁-C₆)alkyl group,
or R₁ and R₂ form with the nitrogen atom carrying them a heterocycloalkyl,
◆ R₃ represents an aryl or heteroaryl group, it being understood that one or more carbon atoms of the preceding groups, or of their possible substituents, may be deuterated,
◆ R₄ represents a phenyl group, a 4-hydroxyphenyl group, a 3-fluoro-4-hydroxyphenyl group, a 2-hydroxypyrimidine group or a 3-hydroxypyridine group, it being understood that one or more carbon atoms of the preceding groups, or of their possible substituents, may be deuterated,
◆ R₅ represents a hydrogen or halogen atom, a linear or branched (C₁-C₆)alkyl group, or a linear or branched (C₁-C₆)alkoxy group,
◆ R₆ represents a hydrogen atom or a linear or branched (C₁-C₆)alkyl group,
◆ Rₐ and R_{d} each represent a hydrogen atom and (R_{b},R_{c}) form together with the carbon atoms carrying them a 1,3-dioxolane group or a 1,4-dioxane group, or Rₐ, R_{c} and R_{d} each represent a hydrogen atom and R_{b} represents a hydrogen or halogen atom or a methoxy group,
or Rₐ and R_{d} each represent a hydrogen atom, R_{b} represents a hydrogen or halogen atom and R_{c} represents a hydroxy or methoxy group, or: Rₐ and R_{d} each represent a hydrogen atom, R_{b} represents a hydroxy or methoxy group and R_{c} represents a halogen atom,
it being understood that:
- "aryl" means a phenyl, naphthyl, biphenyl or indenyl group,
- "heteroaryl" means any mono- or bi-cyclic group composed of from 5 to 10 ring members, having at least one aromatic moiety and containing from 1 to 4 hetero atoms selected from oxygen, sulphur and nitrogen (including quaternary nitrogens),
- "cycloalkyl" means any mono- or bi-cyclic, non-aromatic, carbocyclic group containing from 3 to 10 ring members,
- "heterocycloalkyl" means any mono- or bi-cyclic, non-aromatic, condensed or spiro group composed of from 3 to 10 ring members and containing from 1 to 3 hetero atoms selected from oxygen, sulphur, SO, SO₂ and nitrogen,
it being possible for the aryl, heteroaryl, cycloalkyl and heterocycloalkyl groups so defined and the groups alkyl, alkenyl, alkynyl and alkoxy to be substituted by from 1 to 3 groups selected from linear or branched (C₁-C₆)alkyl, (C₃-C₆)spiro, linear or branched (C₁-C₆)alkoxy, (C₁-C₆)alkyl-S-, hydroxy, oxo (or *N*-oxide where appropriate), nitro, cyano, -COOR', -OCOR', NR'R", linear or branched (C₁-C₆)polyhaloalkyl, trifluoromethoxy, (C₁-C₆)alkylsulphonyl, halogen, aryl, heteroaryl, aryloxy, arylthio, cycloalkyl, heterocycloalkyl optionally substituted by one or more halogen atoms or alkyl groups,
its enantiomers and diastereoisomers, and addition salts thereof with a pharmaceutically acceptable acid or base.

2. Compound of formula (I) according to claim 1, wherein A₁ represents a hydrogen atom or a methyl group.

3. Compound of formula (I) according to claim 1 or 2, wherein A₁ and A₂ both represent a methyl group.

4. Compound of formula (I) according to one of claims 1 to 3, wherein T represents a methyl, aminomethyl, (morpholin-4-yl)methyl, (4-methylpiperazin-1-yl)methyl, 2-(morpholin-4-yl)ethyl, [2-(morpholin-4-yl)ethoxy]methyl, hydroxymethyl, [2-(dimethylamino)ethoxy]methyl, hexahydropyrazino[2,1-*c*][1,4]oxazin-8(1*H*)-ylmethyl, 1-oxa-6-azaspiro[3.3]hept-6-ylmethyl, 3-(morpholin-4-yl)propyl or trifluoromethyl group.

5. Compound of formula (I): 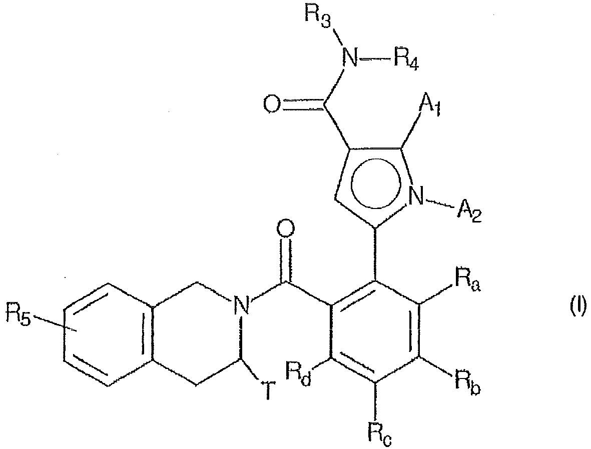 according to claim 1, wherein:
- A₁, A₂, Rₐ, R_{b}, Rₑ, R_{d}, T, R₄ and R₅ are as defined in claims 1 to 4,
- R₃ represents a group selected from phenyl, 1*H-*pyrazole, 1*H-*indole, 1*H-*indazole, pyridine, pyrimidine, 1*H-*pyrrolo[2,3-*b*]pyridine, 2,3-dihydro-1*H-*pyrrolo[2,3-*b*]pyridine, 1*H-*benzimidazole, 1*H-*pyrrole, 1*H-*pyrrolo[2,3-*c*]pyridine, 1*H-*pyrrolo[3,2-b]pyridine, 5*H*-pyrrolo[3,2-*d*]pyrimidine, thiophene, pyrazine, 1*H-*pyrazolo[3,4-b]pyridine, 1,2-oxazole, and pyrazolo[1,5-*a*]pyrimidine, those groups optionally having one or more substituents selected from halogen, linear or branched (C₁-C₆)alkyl, linear or branched (C₁-C₆)alkoxy, cyano, cyclopropyl, oxetane, tetrahydrofuran, -CO-O-CH₃, trideuteriomethyl, 2-(morpholin-4-yl)ethyl and 2-(morpholin-4-yl)ethoxy,
its enantiomers and diastereoisomers, and addition salts thereof with a pharmaceutically acceptable acid or base.

6. Compound of formula (I) according to claim 1, selected from the following group:
- 5-(5-chloro-2-{[(3*S*)-3-(morpholin-4-ylmethyl)-3,4-dihydroisoquinolin-2(1*H*)-yl]-carbonyl}phenyl)-*N*-(4-hydroxyphenyl)-1,2-dimethyl-*N*-(1-methyl-1*H*-pyrazol-4-yl)-1*H*-pyrrole-3-carboxamide,
- 5-(5-chloro-2-{[(3*R*)-3-methyl-3,4-dihydroisoquinolin-2(1*H*)-yl]carbonyl}phenyl)-*N*-(4-hydroxyphenyl)-1,2-dimethyl-*N*-(pyridin-4-yl)-1*H*-pyrrole-3-carboxamide,
- *N*-(4-hydroxyphenyl)-1,2-dimethyl-*N*-(1-methyl-1*H*-pyrazol-4-yl)-5-(6-{[(3*S*)-3-(morpholin-4-ylmethyl)-3,4-dihydroisoquinolin-2(1*H*)-yl]carbonyl}-1,3-benzodioxol-5-yl)-1*H-*pyrrole-3-carboxamide,
- *N*-(4-hydroxyphenyl)-1,2-dimethyl-5-(6-{[(3*R*)-3-methyl-3,4-dihydroisoquinolin-2(1*H*)-yl]carbonyl}-1,3-benzodioxol-5-yl)-*N*-(pyridin-4-yl)-1*H*-pyrrole-3-carboxamide,
- 5-(5-fluoro-2-{[(3*S*)-3-(morpholin-4-ylmethyl)-3,4-dihydroisoquinolin-2(1*H*)-yl]-carbonyl}phenyl)-*N*-(4-hydroxyphenyl)-1,2-dimethyl-*N*-(1-methyl-1*H*-pyrazol-4-yl)-1*H-*pyrrole-3-carboxamide,
- 5-(5-chloro-2-{[(3*S*)-3-(morpholin-4-ylmethyl)-3,4-dihydroisoquinolin-2(1*H*)-yl]-carbonyl}phenyl)-*N*-(4-hydroxyphenyl)-1,2-dimethyl-*N-*(1-methyl-1*H-*pyrrolo[2,3-*b*]pyridin-5-yl)-1*H-*pyrrole-3-carboxamide,
- 5-(5-chloro-2-{[(3*S*)-3-(morpholin-4-ylmethyl)-3,4-dihydroisoquinolin-2(1*H)-*yl]-carbonyl}phenyl)-*N-(*4-hydroxyphenyl)-1,2-dimethyl-*N*-(pyridin-4-yl)-1*H-*pyrrole-3-carboxamide,
- 5-(5-chloro-2-{[(3*S*)-3-(morpholin-4-ylmethyl)-3,4-dihydroisoquinolin-2(1*H*)-yl]-carbonyl}phenyl)-*N-*(5-cyano-1-methyl-1*H-*pyrrol-3-yl)-*N-*(4-hydroxyphenyl)-1,2-dimethyl-1*H-*pyrrole-3-carboxamide,
- *N*-(5-cyano-1-methyl-1*H*-pyrrol-3-yl)-5-(5-fluoro-2-{[(3*S*)-3-(morpholin-4-ylmethyl)-3,4-dihydroisoquinolin-2(1*H*)-yl]carbonyl}phenyl)-*N*-(4-hydroxyphenyl)-1,2-dimethyl-1*H-*pyrrole-3-carboxamide,
- 5-(5-chloro-2-{[(3*S*)-3-(morpholin-4-ylmethyl)-3,4-dihydroisoquinolin-2(1*H*)-yl]-carbonyl}phenyl)-*N*-(5-cyano-1,2-dimethyl-1*H-*pyrrol-3-yl)-*N-*(4-hydroxyphenyl)-1,2-dimethyl-1*H-*pyrrole-3-carboxamide,
- 5-(5-chloro-2-{[(3*S*)-3-(morpholin-4-ylmethyl)-3,4-dihydroisoquinolin-2(1*H*)-yl]-carbonyl}phenyl)-*N-*(4-hydroxyphenyl)-1,2-dimethyl-*N-*(1-methyl-2,3-dihydro-1*H-*pyrrolo[2,3-*b*]pyridin-5-yl)-1*H-*pyrrole-3-carboxamide,
- *N*-(5-cyano-1,2-dimethyl-1*H*-pyrrol-3-yl)-5-(5-fluoro-2-{[(3*S*)-3-(morpholin-4-yl-methyl)-3,4-dihydroisoquinolin-2(1*H*)-yl]carbonyl}phenyl)-*N*-(4-hydroxyphenyl)-1,2-dimethyl-1*H-*pyrrole-3-carboxamide,
- 5-(5-chloro-2-{[(3*S*)-3-(molpholin-4-ylmethyl)-3,4-dihydroisoquinolin-2(1*H*)-yl]-carbonyl}phenyl)-*N-*[5-cyano-2-methyl-1-(trideuteriomethyl)-1*H*-pyrrol-3-yl]-*N-*(4-hydroxyphenyl)-1,2-dimethyl-1*H-*pyrrole-3-carboxamide,
their enantiomers and diastereoisomers, and addition salts thereof with a pharmaceutically acceptable acid or base.

7. Compound of formula (I) according to claim 6 which is 5-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-1,2-dimethyl-*N*-(1-methyl-1*H-*pyrazol-4-yl)-1*H-*pyrrole-3-carboxamide.

8. Compound of formula (I) according to claim 6 which is 5-(5-chloro-2-{[(3*R*)-3-methyl-3,4-dihydroisoquinolin-2(1*H*)-yl]carbonyl}phenyl)-*N*-(4-hydroxyphenyl)-1,2-dimethyl-*N-*(pyridin-4-yl)-1*H-*pyrrole-3-carboxamide.

9. Compound of formula (I) according to claim 6 which is *N*-(4-hydroxyphenyl)-1,2-dimethyl-*N-*(1-methyl-1*H-*pyrazol-4-yl)-5-(6-[(3*S*)-3-(morpholin-4-ylmethyl)-3,4-dihydroisoquinolin-2(1*H*)-yl]carbonyl}-1,3-benzodioxol-5-yl)-1*H*-pyrrole-3-carboxamide.

10. Compound of formula (I) according to claim 6 which is *N*-(4-hydroxyphenyl)-1,2-dimethyl-5-(6-{[(3*R*)-3-methyl-3,4-dihydroisoquinolin-2(1*H*)-yl]carbonyl}-1,3-benzodioxol-5-yl)-*N-*(pyridin-4-yl)-1*H-*pyrrole-3-carboxamide.

11. Compound of formula (I) according to claim 6 which is 5-(5-fluoro-2-{[(3*S*)-3-(morpholin-4-ylmethyl)-3,4-dihydroisoquinolin-2(1*H*)-yl]carbonyl}phenyl)-*N*-(4-hydroxyphenyl)-1,2-dimethyl-*N*-(1-methyl-1*H*-pyrazol-4-yl)-1*H*-pyrrole-3-carboxamide.

12. Compound of formula (I) according to claim 6 which is 5-(5-chloro-2-{[(3*S*)-3-(morpholin-4-ylmethyl)-3,4-dihydroisoquinolin-2(1*H)-*yl]carbonyl}phenyl)-*N*-(4-hydroxyphenyl)-1,2-dimethyl-*N*-(1-methyl-1*H*-pynolo[2,3-*b*]pyridin-5-yl)-1*H*-pynole-3-carboxamide.

13. Compound of formula (I) according to claim 6 which is 5-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-1,2-dimethyl-*N-*(pyridin-4-yl)-1*H-*pyrrole-3-carboxamide.

14. Compound of formula (I) according to claim 6 which is 5-(5-chloro-2-{[(3*S*)-3-(morpholin-4-ylmethyl)-3,4-dihydroisoquinolin-2(1*H*)-yl]carbonyl}phenyl)-*N*-(5-cyano-1-methyl-1*H*-pyrrol-3-yl)-*N*-(4-hydroxyphenyl)-1,2-dimethyl-1*H*-pyrrole-3-carboxamide.

15. Compound of formula (I) according to claim 6 which is *N*-(5-cyano-1-methyl-1*H-*pyrrol-3-yl)-5-(5-fluoro-2-{[(3*S*)-3-(morpholin-4-ylmethyl)-3,4-dihydroisoquinolin-2(1*H*)-yl]carbonyl}phenyl)-*N-*(4-hydroxyphenyl)-1,2-dimethyl-1*H-*pyrrole-3-carboxamide.

16. Compound of formula (I) according to claim 6 which is 5-(5-chloro-2-{[(3*S*)-3-(morpholin-4-ylmethyl)-3,4-dihydroisoquinolin-2(1*H*)-yl]carbonyl}phenyl)-*N*-(5-cyano-1,2-dimethyl-1*H-*pynol-3-yl)-*N-*(4-hydroxyphenyl)-1,2-dimethyl-1*H-*pyrrole-3-carboxamide.

17. Compound of formula (I) according to claim 6 which is 5-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-1,2-dimethyl-*N-*(1-methyl-2,3-dihydro-1*H-*pyrrolo[2,3-*b*]pyridin-5-yl)-1*H*-pyrrole-3-carboxamide.

18. Compound of formula (I) according to claim 6 which is *N*-(5-cyano-1,2-dimethyl-1*H*-pyrrol*-*3-yl)-5-(5-fluoro-2-{[(3*S*)-3-(morpholin-4-ylmethyl)-3,4-dihydroisoquinolin-2(1*H*)-yl]carbonyl}phenyl)-*N*-(4-hydroxyphenyl)-1,2-dimethyl-1*H*-pyrrole-3-carboxamide.

19. Compound of formula (I) according to claim 6 which is 5-(5-chloro-2-{[(3*S*)-3-(morpholin-4-ylmethyl)-3,4-dihydroisoquinolin-2(1*H*)-yl]carbonyl}phenyl)-*N*-[5-cyano-2-methyl-1-(trideuteriomethyl)-1*H-*pyrrol-3-yl]-*N-*(4-hydroxyphenyl)-1,2-dimethyl-1*H-*pyrrole-3-carboxamide.

20. Process for the preparation of compounds of formula (I) according to claim 1, **characterised in that** there is used as starting material the compound of formula (II): 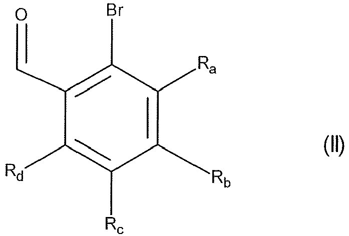 wherein Rₐ, R_{b}, R_{c} and R_{d} are as defined for formula (I),
which compound of formula (II) is subjected to a Heck reaction, in an aqueous or organic medium, in the presence of a palladium catalyst, of a base, of a phosphine and of the compound of formula (III): 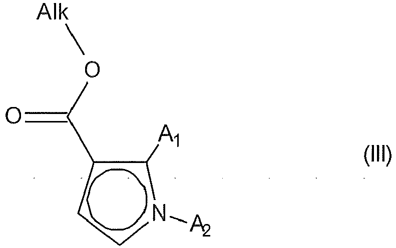 wherein the groups A₁ and A₂ are as defined for formula (I) and Alk represents a linear or branched (C₁-C₆)alkyl,
to obtain the compound of formula (IV): 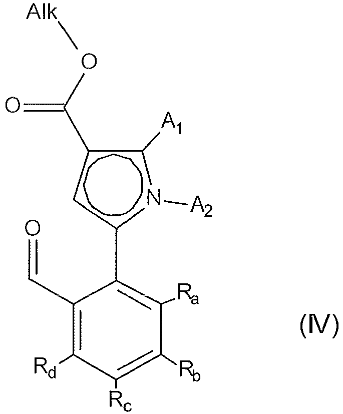 wherein A₁, A₂, Rₐ, R_{b}, R_{c} and R_{d} are as defined for formula (I) and Alk is as defined hereinbefore,
the aldehyde function of which compound of formula (IV) is oxidised to a carboxylic acid to form the compound of formula (V): 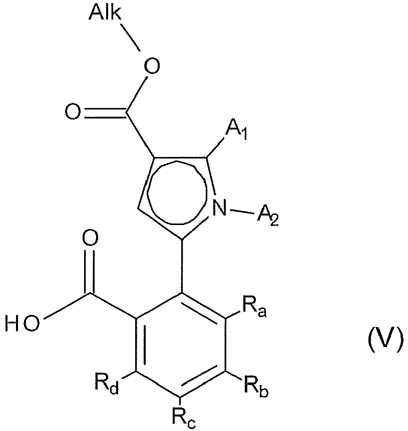 wherein A₁, A₂, Rₐ, R_{b}, R_{c} and R_{d} are as defined for formula (I) and Alk is as defined hereinbefore,
which compound of formula (V) is then subjected to peptide coupling with a compound of formula (VI): 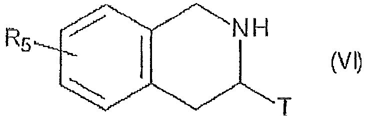 wherein T and R₅ are as defined for formula (I),
to yield the compound of formula (VII): 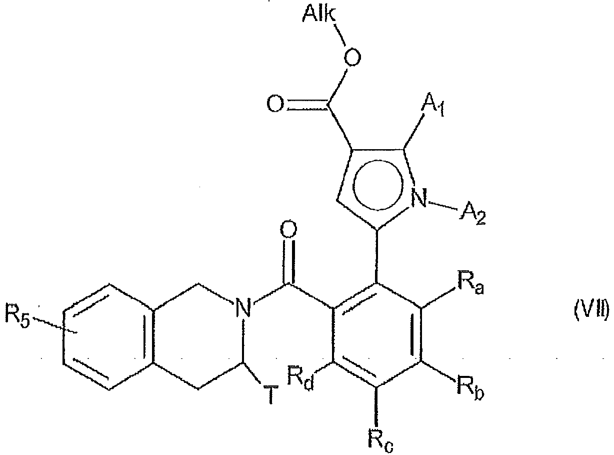 wherein A₁, A₂, Rₐ, R_{b}, R_{c}, R_{d}, T and R₅ are as defined for formula (I) and Alk is as defined hereinbefore,
the ester function of which compound of formula (VII) is hydrolysed to yield the corresponding carboxylic acid or carboxylate, which may be converted into an acid derivative such as the corresponding acyl chloride or anhydride before being coupled with an amine NHR₃R₄ wherein R₃ and R₄ have the same meanings as for formula (I), to yield the compound of formula (I),
which compound of formula (I) may be purified according to a conventional separation technique, which is converted, if desired, into its addition salts with a pharmaceutically acceptable acid or base and which is optionally separated into its isomers according to a conventional separation technique,
it being understood that, at any time considered appropriate in the course of the above-described process, certain groups (hydroxy, amino...) of the reagents or intermediates of synthesis may be protected and then deprotected according to the requirements of synthesis.

21. Process according to claim 20 for the preparation of a compound of formula (I) wherein one of the groups R₃ or R₄ is substituted by a hydroxy function, **characterised in that** the amine NHR₃R₄ is subjected beforehand to a reaction protecting the hydroxy function prior to any coupling with the carboxylic acid formed from the compound of formula (VII), or with a corresponding acid derivative thereof, the resulting protected compound of formula (I) subsequently undergoes a deprotection reaction and is then optionally converted into one of its addition salts with a pharmaceutically acceptable acid or base.

22. Pharmaceutical composition comprising a compound of formula (I) according to any one of claims 1 to 19 or an addition salt thereof with a pharmaceutically acceptable acid or base in combination with one or more pharmaceutically acceptable excipients.

23. Pharmaceutical composition according to claim 22 for use as a pro-apoptotic agent.

24. Pharmaceutical composition according to claim 22 for use in the treatment of cancers, auto-immune diseases and diseases of the immune system.

25. Pharmaceutical composition according to claim 22 for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

26. Use of a pharmaceutical composition according to claim 22 in the manufacture of a medicament for use as a pro-apoptotic agent.

27. Use of a pharmaceutical composition according to claim 22 in the manufacture of a medicament intended for the treatment of cancers, diseases of the immune system and auto-immune diseases.

28. Use of a pharmaceutical composition according to claim 22 in the manufacture of a medicament intended for the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

29. Compound of formula (I) according to one of claims 1 to 19, or an addition salt thereof with a pharmaceutically acceptable acid or base, for use in the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

30. Use of a compound of formula (I) according to one of claims 1 to 19, or an addition salt thereof with a pharmaceutically acceptable acid or base, in the manufacture of a medicament intended for the treatment of cancers of the bladder, brain, breast and uterus, chronic lymphoid leukaemias, colorectal cancer, cancers of the oesophagus and liver, lymphoblastic leukaemias, non-Hodgkin lymphomas, melanomas, malignant haemopathies, myelomas, ovarian cancer, non-small-cell lung cancer, prostate cancer and small-cell lung cancer.

31. Association of a compound of formula (I) according to any one of claims 1 to 19 with an anti-cancer agent selected from genotoxic agents, mitotic poisons, antimetabolites, proteasome inhibitors, kinase inhibitors and antibodies.

32. Pharmaceutical composition comprising an association according to claim 31 in combination with one or more pharmaceutically acceptable excipients.

33. Association according to claim 31 for use in the treatment of cancers.

34. Use of an association according to claim 31 in the manufacture of a medicament for use in the treatment of cancers.

35. Compound of formula (I) according to any one of claims 1 to 19 for use in association with radiotherapy in the treatment of cancers.
